# EUROPEAN PATENT APPLICATION

(11) **EP 4 296 273 A1**
(43) Date of publication of application: **27.12.2023**
(21) Application number: 22776127.7
(22) Date of filing: 24.03.2022
(51) Int. Cl.: C07H 19/16, C07H 1/00, A61K 31/7076, A61P 25/00, A61P 29/00

(54) **ADENOSINE DERIVATIVE HAVING ANTAGONISTIC ACTION ON A2A AND A3 ADENOSINE RECEPTORS AND METHOD FOR PREPARING SAME**

(30) Priority: 26.03.2021 KR 20210039963
(71) Applicant: Future Medicine Co., Ltd., Seongnam-si, Gyeonggi-do 13449 (KR); HK inno.N Corporation, Seoul 04551 (KR)
(72) Inventor: LEE, Hyuk Woo, Seongnam-si Gyeonggi-do 13449 (KR); SON, Seo Hyun, Seongnam-si Gyeonggi-do 13449 (KR); KANG, Ji Yoon, Seongnam-si Gyeonggi-do 13449 (KR); JANG, Hyu Jeong, Seongnam-si Gyeonggi-do 13449 (KR); AHN, Sang Yeop, Seongnam-si Gyeonggi-do 13449 (KR); KIM, Ji Won, Seoul 05622 (KR); BYUN, Joon Seok, Suwon-si Gyeonggi-do 16698 (KR); JI, Seung Hee, Yongin-si Gyeonggi-do 16862 (KR); KIM, Seo Hyun, Gwacheon-si Gyeonggi-do 13832 (KR); CHOI, Jong Ryoul, Guri-si Gyeonggi-do 11929 (KR)
(74) Representative: Roos, Peter
(86) International application number: PCT/KR2022/004147
(87) International publication number: WO 2022/203427

(57) **Abstract**

The present invention relates to: an adenosine derivative capable of acting as an antagonist on A_{2A} and A₃ adenosine receptors through the introduction of substituents into the 2nd and 8th carbons and 6th and/or 9th amine(s); a pharmaceutical composition including the same; and a method for preparing the same. The adenosine derivative regulates the activity of an A_{2A} or A₃ adenosine receptor, and thus can effectively prevent or treat brain diseases, cardiovascular diseases, sleep disorders, inflammation, immune system diseases and the like in which the receptors are involved.

## Description

### [Technical Field]

The present invention relates to an adenosine derivative that acts on A_{2A} and A₃ adenosine receptors and a method for preparing the same, and more specifically, to a truncated adenosine derivative in which substituents are introduced into the 2nd and 8th carbons and 6th and/or 9th amine(s) as an antagonist of A_{2A} and A₃ adenosine derivatives, and a method for preparing the same.

### [Background Art]

Adenosine derivatives are known to be involved in various diseases such as brain diseases, cardiovascular diseases, sleep disorders, inflammation, and immune system diseases. Therefore, research has been conducted to develop therapeutic agents for the above diseases by employing such adenosine receptors as important targets for the development of new drugs.

Adenosine receptors consist of small units of A1, A2A, A2B, and A3, and it is possible to derive a drug capable of effectively preventing or treating various diseases by understanding the functions and action points of each small unit and developing adenosine derivatives that act selectively.

The present inventors found that a truncated adenosine derivative in which substituents are introduced into the 2nd and 8th carbons and 6th and/or 9th amine(s) can selectively inhibit A_{2A} and A₃ adenosine receptors, thereby completing the present invention.

### [Disclosure]

### [Technical Problem]

Accordingly, a problem to be solved by the present invention is to provide a truncated adenosine derivative in which substituents are introduced into the 2nd and 8th carbons and 6th and/or 9th amine(s) and that can act as an antagonist of A_{2A} and A₃ adenosine receptors.

Another problem to be solved by the present invention is to provide a method for preparing or synthesizing the adenosine derivative.

Still another problem to be solved by the present invention is to provide a pharmaceutical composition for preventing or treating various diseases such as brain diseases, cardiovascular diseases, sleep disorders, inflammation, and immune system diseases, including the adenosine derivative.

The problems of the present invention are not limited to the aforementioned technical problems, and other technical problems, which have not been mentioned, may be clearly understood by a person with ordinary skill in the art from the following description.

### [Technical Solution]

The adenosine derivative according to an exemplary embodiment of the present invention for solving the problems is represented by the following Chemical Formula 1 or 2.

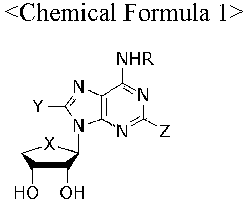

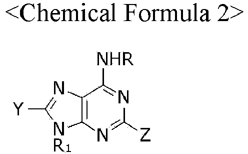

In Chemical Formulae 1 and 2,
X is oxygen (O) or sulfur (S),
Y and Z are each independently a substituted or unsubstituted C₂ to C₁₀ heteroaryl; a substituted or unsubstituted C₂ to C₁₀ heterocycloalkyl; a substituted or unsubstituted C₆ to C₂₀ aryl or alkylaryl; or a substituted or unsubstituted C₂ to C₁₀ alkynyl group,
R is hydrogen (H); a substituted or unsubstituted C₁ to C₁₀ alkyl; or a substituted or unsubstituted C₆ to C₂₀ aryl or alkylaryl, and
R₁ is hydrogen (H); a substituted or unsubstituted C₁ to C₁₀ alkyl; a substituted or unsubstituted C₆ to C₂₀ heteroaryl; or a substituted or unsubstituted heterocycloalkyl.

When Y or Z is substituted, Y or Z may be substituted with one or more selected from the group consisting of an alkyl, an aryl, carboxylic acid, an alkoxy, an alkylamide, a heteroaryl, benzonitrile, benzamide, benzoic acid, a halogen and a carboxylate.

The heteroaryl may be furanyl, thiophenyl, pyrrolyl, pyranyl, pyrazolyl, pyridinyl, triazolyl, imidazolyl, thiazolyl, pyrimidinyl, pyrazinyl, pyridazinyl, indolyl, quinolinyl or purinyl.

The heterocycloalkyl may be tetrahydrofuranyl, thiolanyl, pyrrolidinyl, tetrahydropyranyl, piperidinyl, dioxanyl, morpholino or tetrahydropyrimidinyl.

The aryl may be phenyl, naphthalenyl, anthracenyl or phenanthrenyl, and the alkylaryl may be benzyl.

The alkynyl may be ethynyl, propynyl, butynyl, pentynyl, hexynyl, heptynyl, octynyl, nonynyl or decynyl.

The alkyl may be methyl, ethyl, propyl, butyl or pentyl.

Specifically, Y and Z may be each independently a C₂ to C₁₀ alkynyl unsubstituted or substituted with an alkyl, an aryl or carboxylic acid; a C₆ to C₁₀ alkylaryl; or a C₂ to C₁₀ heteroaryl or heterocycloalkyl unsubstituted or substituted with an alkyl, an alkoxy, an alkylamide, an aryl, a heteroaryl, benzonitrile, benzamide, benzoic acid, a halogen or a carboxylate.

R may be hydrogen; methyl; or benzyl unsubstituted or substituted with a halogen.

R₁ may be hydrogen; a C₁ to C₁₀ alkyl unsubstituted or substituted with an alkyl, a halogen, a hydroxy, an alkoxy or an amino; or a C₆ to C₂₀ heteroaryl or heterocycloalkyl unsubstituted or substituted with an alkyl.

More specifically, Y and Z may be each independently ethynyl unsubstituted or substituted with C₄H₉, C₅H₁₁, C₆H₁₃, C₇H₁₅, C₈H₁₇, phenyl, phenol or butyric acid; a benzyl; or furanyl, thiophenyl, pyrazinyl, pyrrolyl, pyridinyl, pyrimidinyl, piperidinyl, piperazinyl, imidazolyl, thiazolyl, benzo[d]thiazolyl or morpholino unsubstituted or substituted with a methyl, ethyl, methoxy, ethylamide, propylamide, phenyl, benzonitrile, benzamide, benzoic acid, halogen or acetate.

R may be hydrogen; methyl; or halogenated benzyl.

R₁ may be hydrogen; ethyl, propyl, or butyl unsubstituted or substituted with an alkyl, a halogen, a hydroxy, a methoxy, an ethoxy or an amino; or a piperidinyl unsubstituted or substituted with methyl, ethyl or propyl.

Even more specifically, the adenosine derivative according to an exemplary embodiment of the present invention may be represented by any one of the following Chemical Formulae 1-1 to 1-7 or Chemical Formulae 2-1 to 2-7.

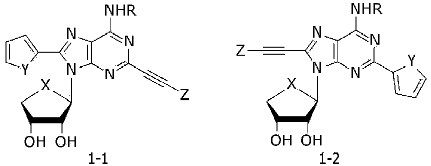

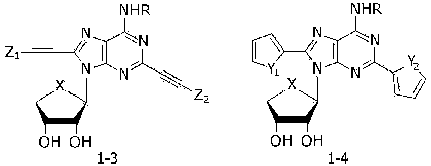

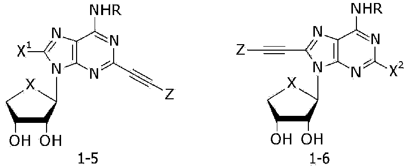

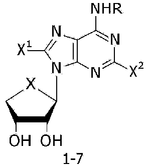

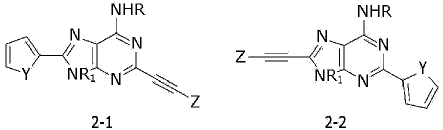

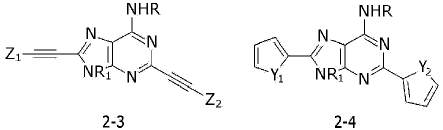

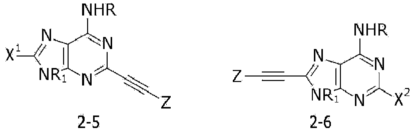

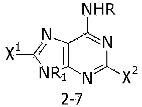

In Chemical Formulae 1-1 to 1-7 or Chemical Formulae 2-1 to 2-7,
X is oxygen or sulfur,
Y, Y₁ and Y₂ are each independently oxygen, sulfur or nitrogen,
Z, Z₁ and Z₂ are each independently C₄H₉, C₅H₁₁, C₆H₁₃, C₇H₁₅, C₈H₁₇, propylamino, butyric acid or phenyl,
R is hydrogen, methyl or iodobenzyl,
X¹ and X² are each independently benzyl, furanyl, methylfuranyl, dimethylfuranyl, thiophenyl, pyridinyl, pyrrolyl, piperidinyl, ethylpiperidinyl, piperidinyl propanoate, piperidinylpropanamide, piperidinyl acetate, thiazolylpiperidinyl, piperazinyl, (methoxyethoxy)phenylpiperazinyl, piperazinylbenzonitrile, piperazinylfluorobenzonitrile, piperazinylbenzamide, piperazinylbenzoic acid, piperazinylfluorobenzoic acid, morpholino, ethylmorpholino, imidazolyl, methylimidazolyl, thiazolyl, benzo[d]thiazolyl or hexynyl, and
R₁ is hydrogen, ethyl, propyl, butyl, methylpropyl, methylbutyl, chloroethyl, methoxyethyl, ethoxyethyl, EtOH, propanediol, methylaminoethyl, piperidinyl or ethylpiperidinyl.

For example, the adenosine derivative of the present invention may be represented by the following Chemical Formulae A-1 to A-5 or Chemical Formulae B-1 to B-2.

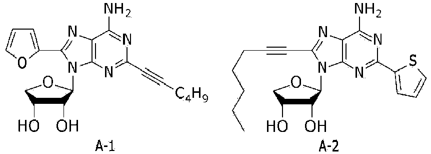

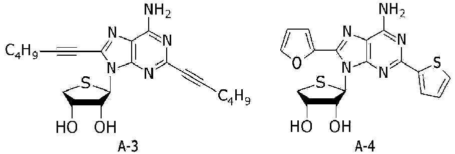

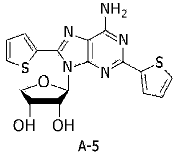

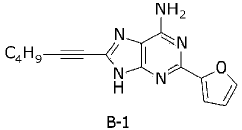

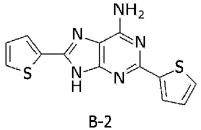

The method for preparing the adenosine derivative according to an exemplary embodiment of the present invention for solving another problem includes sequentially obtaining compounds represented by the following Chemical Formula S 1-1 to S 1-5. 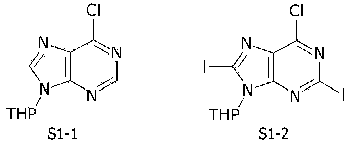 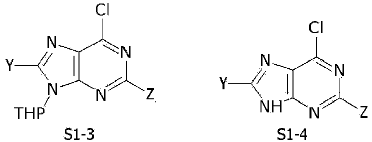 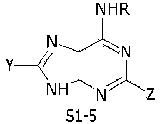
In Chemical Formulae S1-1 to S1-5,
Y and Z are each independently a substituted or unsubstituted C₂ to C₁₀ heteroaryl; a substituted or unsubstituted C₂ to C₁₀ heterocycloalkyl; a substituted or unsubstituted C₆ to C₂₀ aryl or alkylaryl; or a substituted or unsubstituted C₂ to C₁₀ alkynyl group, and
R is hydrogen (H); a substituted or unsubstituted C₁ to C₁₀ alkyl; or a substituted or unsubstituted C₆ to C₂₀ aryl or alkylaryl.
Specific examples of Y, Z and R are as described above.

The method for preparing the adenosine derivative may specifically include:
(a) obtaining a compound represented by Chemical Formula S1-1;
(b) obtaining a compound represented by Chemical Formula S1-2 by adding TMP, n-BuLi and I₂;
(c-1) adding one or more of Pd(dba)₂, CuI and DIPA, and an alkyne;
(c-2) obtaining a compound represented by Chemical Formula S1-3 by adding Pd(PPh₃)₂Cl₂ and a heterocyclic compound;
(d) obtaining a compound represented by Chemical Formula S 1-4 by adding PPTS; and
(e) obtaining a compound represented by Chemical Formula S 1-5 by adding *tert*-butanol. Steps (c-1) and (c-2) may be performed simultaneously or in reverse order.

The method for preparing an adenosine derivative of the present invention as described above may have a final yield of 50% or more, specifically about 52% or more.

The method for preparing an adenosine derivative may further include sequentially obtaining compounds represented by the following Chemical Formulae S2-1 to S2-2 instead of Chemical Formula S1-5 in Chemical Formula S1-4.

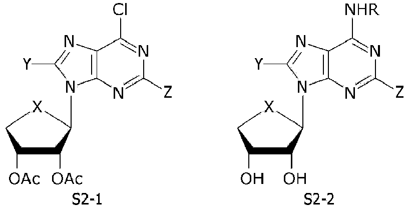

In Chemical Formula S2-1 or S2-2,
X is oxygen (O) or sulfur (S), and specific examples of Y, Z and R are as described above.

The adenosine derivative according to still another exemplary embodiment of the present invention for solving the problem is represented by the following Chemical Formula 3.

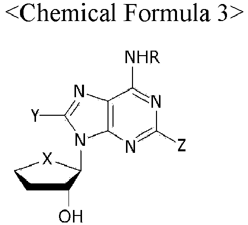

In Chemical Formula 3,
X is oxygen (O) or sulfur (S),
Y and Z are each independently a substituted or unsubstituted C₂ to C₁₀ heteroaryl; a substituted or unsubstituted C₂ to C₁₀ heterocycloalkyl; a substituted or unsubstituted C₆ to C₂₀ aryl or alkylaryl; or a substituted or unsubstituted C₂ to C₁₀ alkynyl group,
R is hydrogen (H); a substituted or unsubstituted C₁ to C₁₀ alkyl; or a substituted or unsubstituted C₆ to C₂₀ aryl or alkylaryl, and
when Y or Z is substituted, Y or Z may be substituted with one or more selected from the group consisting of an alkyl, an aryl, carboxylic acid, an alkoxy, an alkylamide, a heteroaryl, a nitrile, an amide, a halogen, a cycloalkyl and a carboxylate.

The heteroaryl may be furanyl, thiophenyl, pyrrolyl, pyranyl, pyrazolyl, pyridinyl, triazolyl, imidazolyl, thiazolyl, pyrimidinyl, pyrazinyl, pyridazinyl, indolyl, quinolinyl or purinyl.

The heterocycloalkyl may be tetrahydrofuranyl, thiolanyl, pyrrolidinyl, tetrahydropyranyl, piperidinyl, dioxanyl, morpholino or tetrahydropyrimidinyl.

The aryl may be phenyl, naphthalenyl, anthracenyl or phenanthrenyl, and the alkylaryl may be benzyl.

The alkynyl may be ethynyl, propynyl, butynyl, pentynyl, hexynyl, heptynyl, octynyl, nonynyl or decynyl.

The alkyl may be methyl, ethyl, propyl, butyl or pentyl.

Specifically, Y and Z may be each independently a C₂ to C₁₀ alkynyl unsubstituted or substituted with an alkyl, an aryl, a cycloalkyl or carboxylic acid; a C₆ to C₁₀ alkylaryl; or a C₂ to C₁₀ heteroaryl or heterocycloalkyl unsubstituted or substituted with an alkyl, an alkoxy, an alkylamide, an aryl, a heteroaryl, benzonitrile, benzamide, benzoic acid, a halogen or a carboxylate.

R may be hydrogen; methyl; or benzyl unsubstituted or substituted with a halogen.

More specifically, Y and Z may be each independently ethynyl unsubstituted or substituted with methyl, ethyl, propyl, butyl, pentyl, hexyl, heptyl, octyl, phenyl, tolyl, a cycloalkyl, an alkylamide, phenol or butyric acid; a benzyl; or furanyl, thiophenyl, pyrazinyl, pyrrolyl, pyridinyl, pyrimidinyl, piperidinyl, piperazinyl, imidazolyl, thiazolyl, benzo[d]thiazolyl or morpholino unsubstituted or substituted with a methyl, ethyl, methoxy, ethylamide, propylamide, phenyl, benzonitrile, benzamide, benzoic acid, halogen or acetate.

R may be hydrogen; methyl; or halogenated benzyl.

Even more specifically, the adenosine derivative accoording to another exemplary embodiment of the present invention may be represented by the following Chemical Formula 3-1.

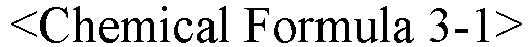

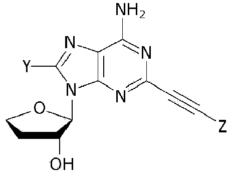

In Chemical Formula 3-1,
Y is benzyl, furanyl, methylfuranyl, dimethylfuranyl, thiophenyl, thiazolyl, pyridinyl, methoxypyridinyl, pyrrolyl, methylpyrrolyl, pyrimidinyl, piperidinyl, ethylpiperidinyl, piperidinyl propanoate, piperidinylpropanamide, piperidinyl acetate, thiazolylpiperidinyl, piperazinyl, (methoxyethoxy)phenylpiperazinyl, piperazinylbenzonitrile, piperazinylfluorobenzonitrile, piperazinylbenzamide, piperazinylbenzoic acid, piperazinylfluorobenzoic acid, morpholino, ethylmorpholino, imidazolyl, methylimidazolyl, thiazolyl or benzo[d]thiazolyl, and
Z is methyl, ethyl, propyl, butyl, pentyl, hexyl, heptyl, octyl, phenyl, tolyl, cyclopropyl, propylamide, butyric acid or phenyl.

For example, Y may be furanyl, methylfuranyl, thiophenyl, methylpyrrolyl, methoxypyridinyl, pyrimidinyl or thiazolyl, and Z may be methyl, ethyl, propyl, pentyl, hexyl, heptyl, octyl, phenyl, tolyl or cyclopropyl.

The method for preparing the adenosine derivative according to still another exemplary embodiment of the present invention for solving another problem includes the following Steps (a) to (h) according to the following Reaction Scheme 1. 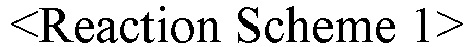 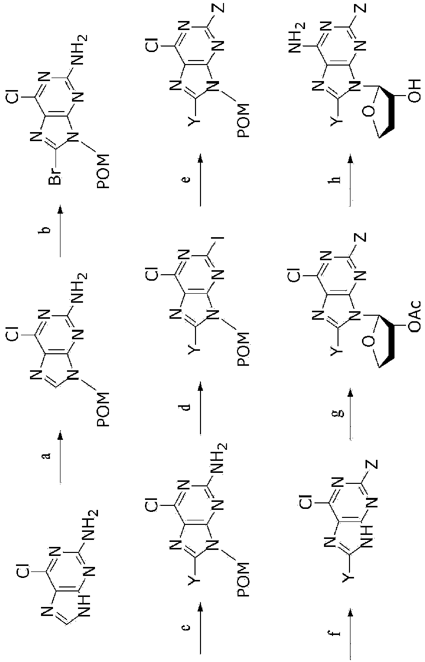
(a) forming a pivaloyloxymethyl (POM) group by being allowed to react with chloromethyl pivalate;
(b) performing bromination by being allowed to react with N-bromosuccinimide (NBS);
(c) substituting a -Br group with a -Y group by being allowed to react with stannyl-Y;
(d) substituting a -NH₂ group with a -I group by being allowed to react with one or more of CuI, I₂ and CH₂I₂;
(e) substituting the -I group with a -Z group by being allowed to react with Z;
(f) removing the POM group by being allowed to react with NaOH;
(g) being allowed to react with tetrahydrofuran-2,3-diyl diacetate; and
(h) being allowed to react with NH₃

Y and Z are as described above, and specifically, Y may be a substituted or unsubstituted C₂ to C₁₀ heteroaryl; or a substituted or unsubstituted C₂ to C₁₀ heterocycloalkyl, and Z may be a substituted or unsubstituted C₂ to C₁₀ alkyne. More specific exemplary embodiments are as described above.

The stannyl may be stannyl unsubstituted or substituted with an alkyl.

The method for preparing the adenosine derivative according to yet another exemplary embodiment of the present invention for solving the problems includes the following Steps (i) to (m) according to the following Reaction Scheme 2. 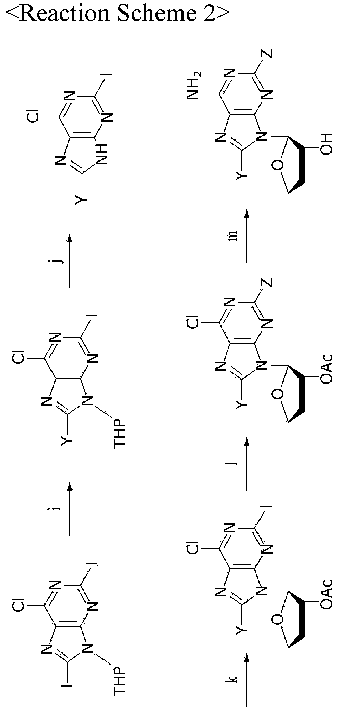
(i) substituting an -I group with a -Y group by being allowed to react with stannyl-Y;
(j) removing a tetrahydropyran (THP) group by being allowed to react with pyridinium p-toluenesulfonate (PPTS);
(k) being allowed to react with tetrahydrofuran-2,3-diyl diacetate;
(1) substituting an -I group with a -Z group by being allowed to react with Z; and
(m) being allowed to react with NH₃
Y and Z are as described above, and specifically, Y may be a substituted or unsubstituted C₂ to C₁₀ heteroaryl; or a substituted or unsubstituted C₂ to C₁₀ heterocycloalkyl, and Z may be a substituted or unsubstituted C₂ to C₁₀ alkyne. More specific exemplary embodiments are as described above.

The stannyl may be stannyl unsubstituted or substituted with an alkyl.

Meanwhile, tetrahydrofuran-2,3-diyl diacetate may be prepared by a method including steps according to the following Reaction Scheme 3. 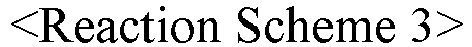 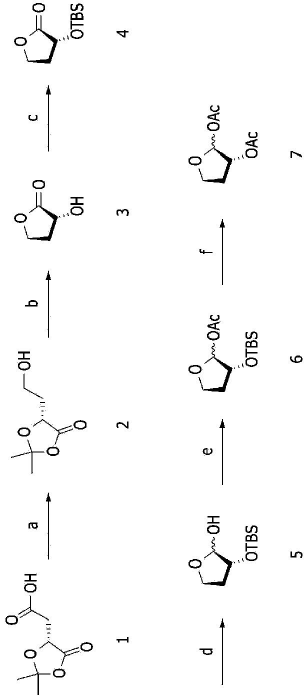
(a) being allowed to react with one or more of a borane-THF complex and THF;
(b) being allowed to react with p-toluenesulfonic acid (PTSA);
(c) being allowed to react with tert-butyldimethylsilyl chloride (TBSCl);
(d) being allowed to react with diisobutylaluminum hydride (DIBAL-H);
(e) being allowed to react with acetic anhydride; and
(f) being allowed to react with tetrabutylammonium fluoride (TBAF) and acetic anhydride

The adenosine derivative of the present invention may be provided in the form of a pharmaceutically acceptable salt. As a salt, acid addition salts formed by various organic or inorganic acids, which are pharmaceutically acceptable, are useful. However, the salt is not limited thereto, and the adenosine derivative of the present invention may also be provided in the form of all salts, hydrates and solvates which may be prepared by typical methods.

The adenosine derivative of the present invention as described above may be included in a pharmaceutical composition capable of preventing or treating A_{2A} or A₃ adenosine receptor-related diseases. A_{2A} or A₃ adenosine receptor-related diseases may include brain diseases, cardiovascular diseases, sleep disorders, inflammation and immune system diseases and the like in which A_{2A} or A₃ adenosine receptors are involved. However, the A_{2A} or A₃ adenosine receptor-related diseases are not limited thereto.

The pharmaceutical composition may be administered systemically or locally, and may be formulated using an excipient (or a diluent) such as a filler, an extender, a binder, a wetting agent, a disintegrating agent, and a surfactant, which may be generally used for administration.

A preferred dose of the pharmaceutical composition varies depending on various factors such as the condition and body weight of a patient, the degree of a disease, the form of drug, the administration route, and the duration, but may be appropriately selected by the person skilled in the art. Further, the administration route may be changed depending on the condition of a patient and the severity thereof.

Specific details of other embodiments are included in the detailed description.

### [Advantageous Effects]

Adenosine derivatives according to exemplary embodiments of the present invention can be utilized to prevent or treat brain diseases, cardiovascular diseases, sleep disorders, inflammation and immune system diseases, and the like in which A_{2A} or A₃ adenosine receptors are involved.

The effects according to the embodiments of the present invention are not limited to the contents exemplified above, and more various effects are included in the present specification.

### [Modes of the Invention]

The benefits and features of the present invention, and the methods of achieving the benefits and features will become apparent with reference to embodiments to be described below in detail. However, the present invention is not limited to the embodiments to be disclosed below and may be implemented in various other forms, and the embodiments are only provided for rendering the disclosure of the present invention complete and for fully representing the scope of the invention to a person with ordinary skill in the technical field to which the present invention pertains, and the present invention will be defined only by the scope of the claims.

The terms used in the present specification are used merely to describe embodiments, and are not intended to limit the present invention. In the present specification, the 'and/or' includes each and all combinations of one or more of the items mentioned. Further, the singular form also includes the plural forms unless specifically stated in a phrase. The terms 'comprises' and/or 'comprising' used in the specification do not exclude the presence or addition of one or more other constituent elements in addition to the referenced constituent elements. The numerical range indicated by using '-' or 'to' indicates a numerical range including values described before and after it as a lower limit and an upper limit, respectively, unless otherwise stated. 'About' or 'approximately' means a value or numerical range within 20% of the value or numerical range described thereafter.

Further, in describing the constituent elements of the examples of the present invention, terms such as first, second, A, B, (a), and (b) may be used. These terms are merely for distinguishing one constituent element from another, and the nature, turn, or order of the corresponding constituent element is not limited by the term.

Unless otherwise defined, all the terms (including technical and scientific terms) used in the present specification will be able to be used as a meaning which may be commonly understood to a person with ordinary skill in the art to which the present invention pertains. In addition, the terms defined in generally used dictionaries are not to be interpreted ideally or excessively unless clearly and specifically defined.

Moreover, in describing the examples of the present invention, when it is determined that the specific description of relevant known configurations or functions obstructs the understanding for the examples of the present invention, the detailed description thereof will be omitted.

As used herein, 'prevention' refers to suppressing the onset of a symptom or disease in an individual who does not yet have the symptom or disease, but may suffer from the symptom or disease.

As used herein, 'treatment' refers to (a) suppression of the development (exacerbation) of a symptom or disease, (b) reduction or amelioration of a symptom or disease, or (c) elimination of a symptom or disease, from an individual.

As used herein, 'individual' refers to an animal, particularly a mammal, including a human having a symptom or disease that can be 'prevented' or 'treated' by administering the composition of the present invention.

As used herein, 'substituted Cₙ to Cₙ₊ₘ' compound includes a case where the number of all carbons of a compound including a substituted moiety is n to n+m, as well as a case where the number of carbons of the compound except for the substituted moiety is n to n+m.

Hereinafter, embodiments of the present invention will be described in detail with reference to Preparation Examples and Experimental Examples, but it is obvious that the effects of the present invention are not limited by the following Experimental Examples.

### Preparation Examples: Preparation of adenosine derivative of present invention (1)

### Preparation Example 1-1: Synthesis of E2,3-tri-O-acetyl-D-erythrofuranose (3)

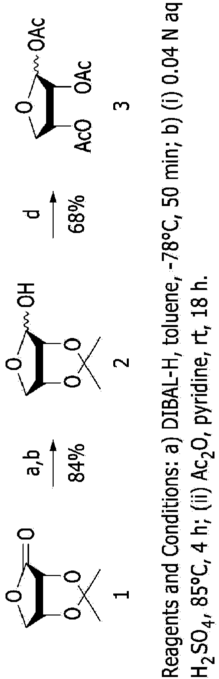

### Preparation Example 1-2: Synthesis of 2,3-O-isopropylidene-D-erythrose (2)

Starting Material (1) (13.2 g, 83 mmol) is suspended in anhydrous toluene (300 mL) under nitrogen, DIBAL-H (100 mL, 1 M in toluene, 100 mmol) is slowly added thereto at -78°C, and then the resultant is stirred for 50 min. After it is confirmed that the reaction is completed, MeOH (10 mL) is slowly added to terminate the reaction. The reaction mixture is filtered through celite and EtOAC (500 mL), and the filtrate is concentrated under reduced pressure. The resulting residue is purified by column chromatography under the condition of hexane/EtOAC = 3/1 to obtain a target compound (2) (5.75 g, 43%).

### Preparation Example 1-3: Synthesis of L2,3-tri-O-acetyl-D-erythrofuranose (3)

Starting Material (2) (5.5 g, 34 mmol) is dissolved in 0.04 *N* H₂SO₄ (30 mL), and the resultant is stirred at 85°C for 4 hours, and then cooled to room temperature. The reaction mixture is neutralized with a saturated aqueous NaHCOs solution and then concentrated under reduced pressure at 45°C. The resulting residue is suspended in pyridine (30 mL) and acetic anhydride (7 mL), and the resultant is stirred at room temperature for 18 hours, and then concentrated under reduced pressure. EtOAC (200 mL) is added to the resulting residue, the resultant is filtered through celite, and then the filtrate is concentrated under reduced pressure. The resulting residue is purified by column chromatography under the condition of hexane/EtOAC = 4/1 to obtain a target compound (3) (2.06 g, 25%) in the form of a white solid: ¹H NMR (600 MHz, CDCl₃) *δ* 6.19 (d, *J* = 1.8 Hz, 1H), 5.48 (d, *J* = 5.4 Hz, 1H), 5.33 (dd, *J* = 1.6, 9.6 Hz, 1H), 4.32 (dd, *J* = 6.0, 9.6 Hz, 1H), 4.00 (dd, *J* = 4.8, 10.2 Hz, 1H), 2.12 (s, 3 H), 2.09 (s, 6 H); Rf = 0.45 (hexane/EtOAc = 4:1).

### Preparation Example 2: Synthesis of 1,2,3-tri-O-acetyl-thio-D-erythrofuranose (5)

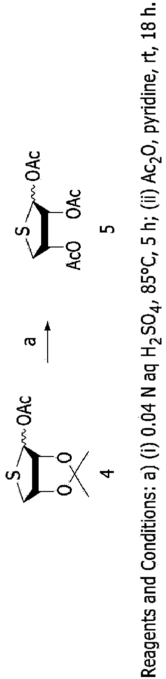

Starting Material (4)(3.5 g, 16 mmol) is dissolved in 0.04 *N* H₂SO₄ (20 mL), and the resultant is stirred at 85°C for 5 hours, and then cooled to room temperature. The reaction mixture is neutralized with a saturated aqueous NaHCO₃ solution and then concentrated under reduced pressure at 45°C. The resulting residue is suspended in pyridine (15 mL) and acetic anhydride (5 mL), and the resultant is stirred at room temperature for 18 hours, and then concentrated under reduced pressure. EtOAC (200 mL) is added to the resulting residue, the resultant is filtered through celite, and then the filtrate is concentrated under reduced pressure. The resulting residue is purified by column chromatography under the condition of hexane/EtOAC = 4/1 to obtain a target compound (5) (1.6 g, 38%) in the form of a white solid: ¹H NMR (600 MHz, CDCl₃) *δ* 5.84 (d, *J* = 1.8 Hz, 1H), 5.49 (s, 1 H), 5.48 (s, 1 H), 3.25 (m, 1 H), 2.98 (m, 1H), 2.13 (s, 3 H), 2.10 (s, 3 H), 2.08 (s, 3 H); β form Rf = 0.39, α form Rf = 0.29, (hexane/EtOAc = 1:1).

### Preparation Example 3: Synthesis of 11a to 11t

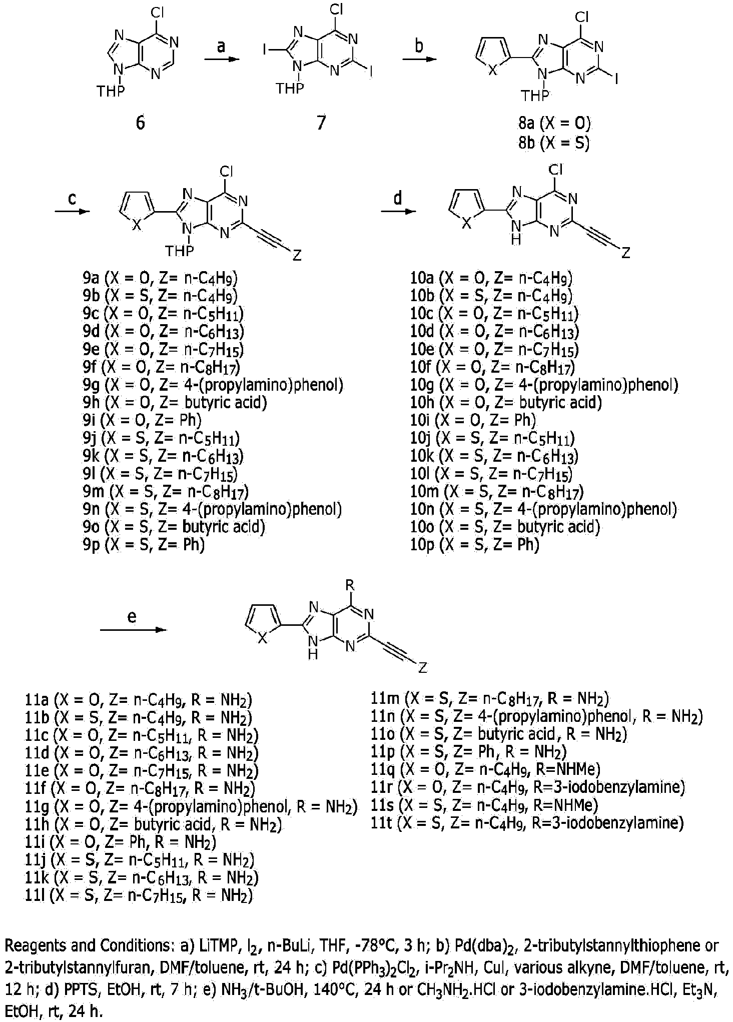

### 6-Chloro-2,8-diiodo-9-(tetrahydro-2H-pyran-2-yl)-9H-purine (7)

2,2,6,6-tetramethylpiperidine (TMP, 59.2 g, 419 mmol) is dissolved in anhydrous THF (170 mL) under nitrogen, n-BuLi (285 mL, 2.5 M in hexane, 457 mmol) is slowly added thereto at -78°C, and the resulting mixture is stirred at 0°C for 2 hours. The temperature of the reaction mixture is lowered to -78°C, a starting material 6-chloro-9-(tetrahydro-2H-pyran-2-yl)-9H-purine (20 g, 84 mmol) dissolved in anhydrous THF (110 mL) is slowly added thereto, and the resulting mixture is stirred for 3 hours. Iodine (102 g, 402 mmol) dissolved in THF (200 mL) is slowly added to the reaction mixture at -78°C, the resulting mixture is stirred for 3 hours, and then the temperature is gradually increased to 0°C over 5 hours. After it is confirmed that the reaction is completed, a 10% sodium thiosulfate solution (550 mL) and an aqueous saturated NH₄Cl solution (250 mL) are added thereto to terminate the reaction. The reaction mixture is extracted with EtOAC (500 mL), dried over anhydrous MgSO₄ and concentrated under reduced pressure. The resulting residue is purified by flash column chromatography under the condition of DCM/MeOH = 100/1 to obtain a target compound (7) (26.9 g, 66%) in the form of an ivory solid: ¹H NMR (600 MHz, CDCl₃) *δ* 5.66 (dd, *J* = 2.4, 10.8 Hz, 1H), 4.20 (m, 1 H), 3.74 (td, *J* = 2.4, 12 Hz, 1H), 3.04 (m, 1 H), 2.17 (m, 1 H), 1.62-1.91 (m, 4 H).

### 6-chloro-8-(furan-2-yl)-2-iodo-9-(tetrahydro-2H-pyran-2-yl)-9H-purine (8a)

After the starting material (7) (35 g, 71 mmol) is dissolved in DMF/toluene (250 mL) under nitrogen, Pd(dba)₂ (0.01 mmol) and 2-tributylstannylfuran (74.55 mmol) are added thereto, and the resulting mixture is stirred at room temperature for 24 hours. After it is confirmed that the reaction is completed, the reaction mixture is extracted with EtOAc (350 mL), dried over anhydrous MgSO₄ and concentrated under reduced pressure. The resulting residue is purified by flash column chromatography under the condition of DCM/MeOH = 100/1 to obtain a target compound (8a) (16.8 g, 55%) in the form of an ivory solid: ¹H NMR (600 MHz, CDCl₃) *δ* 7.91 (d, 1H), 7.13 (d, 1H), 6.70 (t, *J* = 7.2 Hz, 1H), 5.80 (t, *J* = 7.7 Hz, 1H), 3.67-3.57 (m, 2H), 2.17-1.92 (m, 2H), 1.74-1.64 (m, 2H), 1.58-1.55 (m, 2H).

### 6-chloro-2-iodo-9-(tetrahydro-2H-pyran-2-yl)-8-(thiophen-2-yl)-9H-purine (8b)

A target compound (8b) is obtained using 2-tributylstannylthiofuran by the same synthetic method as for Compound (8a): ¹H NMR (600 MHz, CDCl₃) *δ* 7.87 (d, 1H), 7.85 (d, 1H), 7.24 (t, 1H, *J* = 7.4 Hz), 5.75 (t, 1H, *J* = 7.2 Hz), 3.55-3.48 (m, 2H), 2.07-1.84 (m, 2H), 1.65-1.54 (m, 2H), 1.48-1.45 (m, 2H).

### 6-Chloro-8-(furan-2-yl)-2-(hex-1-yn-1-yl)-9-(tetrahydro-2H-pyran-2-yl)-9H-purine (9a)

After Starting Material (8a) (3.7 g, 8.7 mmol) is dissolved in DMF/toluene (100 mL) under nitrogen, Pd(dba)₂ (250 mg), CuI (166 mg), DIPA (1.9 mL), and 1-hexyne (1.2 mL) are added thereto, and the resulting mixture is stirred at room temperature for 16 hours. After it is confirmed that the reaction is completed, the reaction mixture is extracted with EtOAc/H₂O, dried over anhydrous MgSO₄ and filtered through celite, and then the filtrate is concentrated under reduced pressure. The resulting residue is purified by column chromatography under the condition of hexane/EtOAc = 5/1 to obtain a target compound (9a) (3.1 g, 92%) as a brown solid: ¹H NMR (600 MHz, CDCl₃) *δ* 7.72 (t, 1 H), 7.47 (d, 1 H), 6.65 (dd, 1 H), 6.07 (dd, *J* = 2.4, 11.4 Hz, 1H), 4.25 (m, 1 H), 3.80 (dt, *J* = 9.6, 12 Hz, 1H), 2.87 (m, 1 H), 2.50 (t, *J* = 7.2 Hz, 1H), 2.15 (m, 1 H), 1.47-1.91 (m, 8 H), 0.96 (t, *J* = 7.2 Hz, 3H).

### 6-Chloro-2-(hex-1-yn-1-yl)-9-(tetrahydro-2H-pyran-2-yl)-8-(thiophen-2-yl)-9H-purine (9b)

A target compound (9b) is obtained by the same synthetic method as for Compound (9a): ¹H NMR (600 MHz, CDCl₃) *δ* 8.01 (d, *J* = 3.5 Hz, 1 H), 7.44 (d, *J* = 4.9 Hz, 1 H), 7.10 (t, *J* = 3.8 Hz, 1 H), 5.77 (dd, *J* = 2.2, 11.1 Hz, 1 H), 4.18 (d, *J* = 11.4 Hz, 1 H), 3.68-3.76 (m, 1 H), 3.02-3.14 (m, 1 H), 2.54 (t, *J* = 7.0 Hz, 2 H), 2.14 (d, *J* = 11.0 Hz, 1 H), 1.90 (d, *J* = 12.1 Hz, 1 H), 1.62-1.87 (m, 5 H), 1.45-1.57 (m, 2 H), 0.95 (t, *J* = 7.2 Hz, 3 H).

### 6-chloro-8-(furan-2-yl)-2-(hept-1-yn-1-yl)-9-(tetrahydro-2H-pyran-2-yl)-9H-purine (9c)

A target compound (9c) is obtained by the same synthetic method as for Compound (9a): ¹H NMR (600 MHz, CDCl₃) *δ* 7.91 (d, 1H), 7.13 (d, 1H), 6.70 (t, *J* = 7.2 Hz, 1H), 5.78 (t, *J* = 7.7 Hz, 1H), 3.67-3.57 (m, 2H), 2.46 (t, *J* = 7.4 Hz, 2H), 2.20-1.96 (m, 2H), 1.88-1.75 (m, 2H), 1.65-1.53 (m, 2H), 1.44 (m, 2H), 1.33 (m, 2H), 1.27 (m, 2H), 1.01 (t, *J* = 7.7 Hz, 3H).

### 6-chloro-8-(furan-2-yl)-2-(oct-1-yn-1-yl)-9-(tetrahydro-2H-pyran-2-yl)-9H-purine (9d)

A target compound (9d) is obtained by the same synthetic method as for Compound (9a): ¹H NMR (600 MHz, CDCl₃) *δ* 7.87 (d, 1H), 7.15 (d, 1H), 6.64 (t, *J* = 7.4 Hz, 1H), 5.88 (t, *J* = 7.7 Hz, 1H), 3.70-3.64 (m, 2H), 2.35 (t, *J* = 7.4 Hz, 2H), 2.30-2.24 (m, 2H), 1.98-1.85 (m, 2H), 1.75-1.63 (m, 2H), 1.44 (m, 2H), 1.35 (m, 2H), 1.23-1.14 (m, 4H), 0.98 (t, *J* = 7.7 Hz, 3H).

### 6-chloro-8-(furan-2-yl)-2-(non-1-yn-1-yl)-9-(tetrahydro-2H-pyran-2-yl)-9H-purine (9e)

A target compound (9e) is obtained by the same synthetic method as for Compound (9a): ¹H NMR (600 MHz, CDCl₃) *δ* 8.01 (d, 1H), 7.33 (d, 1H), 6.75 (t, *J* = 7.2 Hz, 1H), 6.01 (t, *J* = 7.7 Hz, 1H), 3.94-3.85 (m, 2H), 2.45 (t, *J* = 7.4 Hz, 2H), 2.39-2.31 (m, 2H), 2.05-1.96 (m, 2H), 1.85-1.71 (m, 2H), 1.54 (m, 2H), 1.42 (m, 2H), 1.34-1.14 (m, 6H), 1.00 (t, *J* = 7.4 Hz, 3H).

### 6-chloro-2-(dec-1-yn-1-yl)-8-(furan-2-yl)-9-(tetrahydro-2H-pyran-2-yl)-9H-purine (9f)

A target compound (9f) is obtained by the same synthetic method as for Compound (9a): ¹H NMR (600 MHz, CDCl₃) *δ* 7.92 (d, 1H), 7.41 (d, 1H), 6.68 (t, *J* = 7.7 Hz, 1H), 5.94 (t, *J* = 7.7 Hz, 1H), 3.88-3.77 (m, 2H), 2.36 (t, *J* = 7.2 Hz, 2H), 2.28-2.20 (m, 2H), 1.98-1.87 (m, 2H), 1.79-1.66 (m, 2H), 1.46 (m, 4H), 1.37-1.33 (m, 4H), 1.28-1.19 (m, 4H), 1.05 (t, *J* = 7.7 Hz, 3H).

### 4-((5-(6-chloro-8-(furan-2-yl)-9-(tetrahydro-2H-pyran-2-yl)-9H-purin-2-yl)pent-4-yn-1-yl)amino)phenol (9g)

A target compound (9g) is obtained by the same synthetic method as for Compound (9a): ¹H NMR (600 MHz, CDCl₃) *δ* 9.44 (s, 1H), 7.91 (d, 1H), 7.13 (d, 1H), 6.73 (t, *J* = 7.2 Hz, 1H), 6.69 (d, 2H), 6.64 (d, 2H), 6.57 (d, 2H), 6.55 (d, 2H), 6.20 (s, 1H), 5.80 (t, *J* = 7.7 Hz, 1H), 3.35 (d, 2H), 3.67-3.57 (m, 2H), 2.46 (d, 1H), 2.17-1.92 (m, 2H), 1.75-1.64 (m, 2H), 1.69 (d, 2H).

### 6-(6-chloro-8-(furan-2-yl)-9-(tetrahydro-2H-pyran-2-yl)-9H-purin-2-yl)hex-5-ynoic acid (9h)

A target compound (9h) is obtained by the same synthetic method as for Compound (9a): ¹H NMR (600 MHz, CDCl₃) *δ* 11.8 (s, 1H), 7.87 (d, 1H), 7.20 (d, 1H), 6.85 (t, *J* = 7.2 Hz, 1H), 5.77 (t, *J* = 7.4 Hz, 1H), 3.51-3.46 (m, 2H), 2.51 (d, 2H), 2.35 (d, 2H), 2.15-1.89 (m, 2H),1.75-1.64 (m, 2H), 1.70 (d, 2H).

### 6-chloro-8-(furan-2-yl)-2-(phenylethynyl)-9-(tetrahydro-2H-pyran-2-yl)-9H-purine (9i)

A target compound (9i) is obtained by the same synthetic method as for Compound (9a): ¹H NMR (600 MHz, CDCl₃) *δ* 7.85 (d, 1H), 7.60 (d, 1H), 7.58 (d, 1H) 7.55 (d, 1H), 7.53 (d, 1H), 7.48 (d, 1H), 7.25 (d, 1H), 6.83 (t, *J* = 7.4 Hz, 1H), 5.88 (t, *J* = 7.7 Hz, 1H), 3.40-3.35 (m, 2H), 2.20-2.11 (m, 2H), 1.80-1.74 (m, 2H), 1.48-1.38 (m, 2H).

### 6-chloro-2-(hept-1-yn-1-yl)-9-(tetrahydro-2H-pyran-2-yl)-8-(thiophen-2-yl)-9H-purine (9j)

A target compound (9j) is obtained using 2-tributylstannylthiofuran by the same synthetic method as for Compound (9a): ¹H NMR (600 MHz, CDCl₃) *δ* 7.88 (d, 1H), 7.11 (d, 1H), 6.65 (t, *J* = 7.4 Hz, 1H), 5.77 (t, *J* = 7.7 Hz, 1H), 3.65-3.51 (m, 2H), 2.51 (t, *J* = 7.4 Hz, 2H), 2.22-1.94 (m, 2H), 1.84-1.71 (m, 2H), 1.63-1.52 (m, 2H), 1.38 (m, 2H), 1.31 (m, 2H), 1.21 (m, 2H), 0.97 (t, *J* = 7.7 Hz, 3H).

### 6-chloro-2-(oct-1-yn-1-yl)-9-(tetrahydro-2H-pyran-2-yl)-8-(thiophen-2-yl)-9H-purine (9k)

A target compound (9k) is obtained by the same synthetic method as for Compound (9a): ¹H NMR (600 MHz, CDCl₃) *δ* 7.85 (d, 1H), 7.18 (d, 1H), 6.58 (t, *J* = 7.2 Hz, 1H), 5.85 (t, *J* = 7.7 Hz, 1H), 3.72-3.61 (m, 2H), 2.30 (t, *J* = 7.4 Hz, 2H), 2.24-2.2.11 (m, 2H), 1.98-1.84 (m, 2H), 1.73-1.64 (m, 2H), 1.44 (m, 2H), 1.25 (m, 2H), 1.21-1.14 (m, 4H), 0.99 (t, *J* = 7.4 Hz, 3H).

### 6-chloro-2-(non-1-yn-1-yl)-9-(tetrahydro-2H-pyran-2-yl)-8-(thiophen-2-yl)-9H-purine (9I)

A target compound (91) is obtained by the same synthetic method as for Compound (9a): ¹H NMR (600 MHz, CDCl₃) *δ* 7.98 (d, 1H), 7.43 (d, 1H), 6.85 (t, *J* = 7.4 Hz, 1H), 6.05 (t, *J* = 7.2 Hz, 1H), 3.88-3.81 (m, 2H), 2.43 (t, *J* = 7.4 Hz, 2H), 2.37-2.31 (m, 2H), 2.11-1.94 (m, 2H), 1.79-1.71 (m, 2H), 1.51 (m, 2H), 1.46 (m, 2H), 1.31-1.25 (m, 6H), 0.98 (t, *J* = 7.4 Hz, 3H).

### 6-chloro-2-(dec-1-yn-1-yl)-9-(tetrahydro-2H-pyran-2-yl)-8-(thiophen-2-yl)-9H-purine (9m)

A target compound (9m) is obtained by the same synthetic method as for Compound (9a): ¹H NMR (600 MHz, CDCl₃) *δ* 8.00 (d, 1H), 7.46 (d, 1H), 6.70 (t, *J* = 7.2 Hz, 1H), 6.00 (t, *J* = 7.4 Hz, 1H), 3.88-3.79 (m, 2H), 2.41 (t, *J* = 7.2 Hz, 2H), 2.30-2.24 (m, 2H), 1.97-1.89 (m, 2H), 1.76-1.62 (m, 2H), 1.51 (m, 4H), 1.40-1.32 (m, 4H), 1.25-1.14 (m, 4H), 1.03 (t, *J* = 7.2 Hz, 3H).

### 4-((5-(6-chloro-9-(tetrahydro-2H-pyran-2-yl)-8-(thiophen-2-yl)-9H-purin-2-yl)pent-4-yn-1-yl)amino)phenol (9n)

A target compound (9n) is obtained by the same synthetic method as for Compound (9a): ¹H NMR (600 MHz, CDCl₃) *δ* 9.35 (s, 1H), 8.01 (d, 1H), 7.20 (d, 1H), 6.63 (t, *J* = 7.2 Hz, 1H), 6.70 (d, 2H), 6.61 (d, 2H), 6.54 (d, 2H), 6.50 (d, 2H), 6.18 (s, 1H), 5.75 (t, *J* = 7.4 Hz, 1H), 3.33 (d, 2H), 3.64-3.55 (m, 2H), 2.41 (d, 1H), 2.20-1.99 (m, 2H), 1.87-1.71 (m, 2H), 1.61 (d, 2H).

### 6-(6-chloro-9-(tetrahydro-2H-pyran-2-yl)-8-(thiophen-2-yl)-9H-purin-2-yl)hex-5-ynoic acid (9o)

A target compound (9o) is obtained by the same synthetic method as for Compound (9a): ¹H NMR (600 MHz, CDCl₃) *δ* 12.1 (s, 1H), 7.81 (d, 1H), 7.15 (d, 1H), 6.88 (t, *J* = 7.7 Hz, 1H), 5.68 (t, *J* = 7.2 Hz, 1H), 3.58-3.49 (m, 2H), 2.51 (d, 2H), 2.40 (d, 2H), 2.12-1.97 (m, 2H),1.76-1.61 (m, 2H), 1.57 (d, 2H).

### 6-chloro-2-(phenylethynyl)-9-(tetrahydro-2H-pyran-2-yl)-8-(thiophen-2-yl)-9H-purine (9p)

A target compound (9p) is obtained by the same synthetic method as for Compound (9j): ¹H NMR (600 MHz, CDCl₃) *δ* 7.88 (d, 1H), 7.58 (d, 1H), 7.55 (d, 1H) 7.51 (d, 1H), 7.49 (d, 1H), 7.47 (d, 1H), 7.21 (d, 1H), 6.75 (t, *J* = 7.7 Hz, 1H), 5.75 (t, *J* = 7.7 Hz, 1H), 3.41-3.36 (m, 2H), 2.31.2-18 (m, 2H), 1.75-1.64 (m, 2H), 1.45-1.31 (m, 2H).

### 6-Chloro-8-(furan-2-yl)-2-(hex-1-yn-1-yl)-9H-purine (10a)

After Starting Material (9a) (2.6 g, 8.7 mmol) is dissolved in THF (20 mL) and EtOH (20 mL), pyridiump-toluenesulfonate (0.1 g, 0.39 mmol) dissolved in water (2 mL) is slowly added thereto at room temperature, and the resultant is stirred at room temperature for 7 hours. After it is confirmed that the reaction is completed, the reaction mixture is concentrated under reduced pressure, MeOH (20 mL) is added thereto, and the resultant is again stirred. The resulting solid is filtered to obtain a yellow target compound (10a) (2.1 g, 80%): ¹H NMR (600 MHz, CDCl₃) *δ* 7.65(d, *J* = 1.2 Hz, 1H), 7.47 (d, *J* = 3.6 Hz, 1H), 6.66 (dd, *J* = 1.2, 3.6 Hz, 1H), 2.44 (t, 2 H), 1.45-1.62(m, 4 H), 0.92 (t, 3 H).

### 6-Chloro-2-(hex-1-yn-1-yl)-8-(thiophen-2-yl)-9H-purine (10b)

A target compound (10b) is obtained by the same synthetic method as for Compound (10a): ¹H NMR (600 MHz, DMSO-*d*₆) *δ* 14.24 (bs, 1 H), 7.89 (d, *J* = 3.5 Hz, 1 H), 7.74 (d, *J* = 4.8 Hz, 1 H), 7.18 (dd, *J* = 3.6, 4.8 Hz, 1 H), 2.57 (t, *J* = 7.0 Hz, 2 H), 1.55-1.59 (m, 2 H), 1.43-1.47 (m, 2 H), 0.92 (t, *J* = 7.2 Hz, 3 H).

### 6-chloro-8-(furan-2-yl)-2-(hept-1-yn-1-yl)-9H-purine (10c)

A target compound (10c) is obtained by the same synthetic method as for Compound (10a): ¹H NMR (600 MHz, CDCl₃) *δ* 7.71 (d, 1H), 7.03 (d, 1H), 6.75 (t, *J* = 7.4 Hz, 1H), 2.41 (t, *J* = 7.7 Hz, 2H), 1.35 (m, 2H), 1.29 (m, 2H), 1.00 (t, *J* = 7.7 Hz, 3H).

### 6-chloro-8-(furan-2-yl)-2-(oct-1-yn-1-yl)-9H-purine (10d)

A target compound (10d) is obtained by the same synthetic methods as for Compound (10a): ¹H NMR (600 MHz, CDCl₃) *δ* 7.84 (d, 1H), 7.20 (d, 1H), 6.64 (t, *J* = 7.4 Hz, 1H), 2.38 (t, *J* = 7.2 Hz, 2H), 1.44 (m, 2H), 1.35 (m, 2H), 1.28-1.17 (m, 4H), 0.99 (t, *J* = 7.4 Hz, 3H).

### 6-chloro-8-(furan-2-yl)-2-(non-1-yn-1-yl)-9H-purine (10e)

A target compound (10e) is obtained by the same synthetic method as for Compound (10a): ¹H NMR (600 MHz, CDCl₃) *δ* 7.91 (d, 1H), 7.35 (d, 1H), 6.71 (t, *J* = 7.4 Hz, 1H), 2.43 (t, *J* = 7.7 Hz, 2H), 1.58 (m, 2H), 1.42 (m, 2H), 1.31-1.15 (m, 6H), 0.97 (t, *J* = 7.4 Hz, 3H).

### 6-chloro-2-(dec-1-yn-1-yl)-8-(furan-2-yl)-9H-purine (10f)

A target compound (10f) is obtained by the same synthetic method as for Compound (10a): ¹H NMR (600 MHz, CDCl₃) *δ* 7.98 (d, 1H), 7.45 (d, 1H), 6.71 (t, *J* = 7.4 Hz, 1H), 1.80-1.67 (m, 2H), 1.51 (m, 4H), 1.37-1.31 (m, 4H), 1.29-1.19 (m, 4H), 1.01 (t, *J* = 7.2 Hz, 3H).

### 4-((5-(6-chloro-8-(furan-2-yl)-9H-purin-2-yl)pent-4-yn-1-yl)amino)phenol (10g)

A target compound (10g) is obtained by the same synthetic method as for Compound (10a): ¹H NMR (600 MHz, CDCl₃) *δ* 9.35 (s, 1H), 7.81 (d, 1H), 7.11 (d, 1H), 6.73 (t, *J* = 7.7 Hz, 1H), 6.64 (d, 2H), 6.58 (d, 2H), 6.56 (d, 2H), 6.53 (d, 2H), 6.20 (s, 1H), 2.46 (d, 1H), 1.77-1.61 (m, 2H), 1.51 (d, 2H).

### 6-(6-chloro-8-(furan-2-yl)-9H-purin-2-yl)hex-5-ynoic acid (10h)

A target compound (10h) is obtained by the same synthetic method as for Compound (10a): ¹H NMR (600 MHz, CDCl₃) *δ* 12.0 (s, 1H), 7.91 (d, 1H), 7.130 (d, 1H), 6.79 (t, *J* = 7.7 Hz, 1H), 2.35 (d, 2H), 1.70 (d, 2H).

### 6-chloro-8-(furan-2-yl)-2-(phenylethynyl)-9H-purine (10i)

A target compound (10i) is obtained by the same synthetic method as for Compound (10a): ¹H NMR (600 MHz, CDCl₃) *δ* 7.90 (d, 1H), 7.75 (d, 1H), 7.51 (d, 1H) 7.48 (d, 1H), 7.47 (d, 1H), 7.45 (d, 1H), 7.35 (d, 1H), 6.83 (t, *J* = 7.7 Hz, 1H).

### 6-Chloro-2-(hept-1-yn-1-yl)-8-(thiophen-2-yl)-9H-purine (10j)

A target compound (10j) is obtained by the same synthetic method as for Compound (10a). ¹H NMR (600 MHz, CD₃OD) *δ* 7.97 (d, *J* = 3.2 Hz, 1H), 7.80 (dd, *J* = 5.0, 0.9 Hz, 1H), 7.27 (dd, *J* = 5.0, 3.7 Hz, 1H), 2.49 (t, *J* = 7.1 Hz, 2H), 1.67 (q, *J* = 7.3 Hz, 2H), 1.52-1.38 (m, 4H), 0.96 (t, *J* = 7.1 Hz, 3H);

### 6-Chloro-2-(oct-1-yn-1-yl)-8-(thiophen-2-yl)-9H-purine (10k)

A target compound (10k) is obtained by the same synthetic method as for Compound (10a). ¹H NMR (600 MHz, CD₃OD) *δ* 7.95 (dd, *J* = 3.7, 0.9 Hz, 1H), 7.79 (dd, *J* = 5.1, 0.9 Hz, 1H), 7.26 (dd, *J* = 5.1, 4.1 Hz, 1H), 2.49 (t, *J* = 7.1 Hz, 2H), 1.70-1.62 (m, 2H), 1.55-1.48 (m, 2H), 1.40-1.33 (m, 4H), 0.95-0.92 (m, 3H);

### 6-Chloro-2-(non-1-yn-1-yl)-8-(thiophen-2-yl)-9H-purine (101)

A target compound (101) is obtained by the same synthetic method as for Compound (10a). ¹H NMR(600 MHz, CD₃OD) *δ* 7.94 (dd, *J* = 3.7, 0.9 Hz, 1H), 7.79 (dd, *J* = 5.0, 1.4 Hz, 1H), 7.26-7.23 (m, 1H), 2.48 (t, *J* = 7.1 Hz, 2H), 1.65 (q, *J* = 7.3 Hz, 2H), 1.52-1.46 (m, 2H), 1.36 (q, *J* = 7.3 Hz, 6H), 0.91 (t, *J* = 6.9 Hz, 3H);

### 6-Chloro-2-(dec-1-yn-1-yl)-8-(thiophen-2-yl)-9H-purine (10m)

A target compound (10m) is obtained by the same synthetic method as for Compound (10a). ¹H NMR (600 MHz, CD₃OD) *δ* 7.95 (d, *J* = 3.7 Hz, 1H), 7.79 (d, *J* = 5.0 Hz, 1H), 7.25 (t, *J* = 4.3 Hz, 1H), 2.48 (t, *J* = 7.1 Hz, 2H), 1.69-1.62 (m, 2H), 1.50 (t, *J* = 7.1 Hz, 2H), 1.33 (d, *J* = 10.5 Hz, 8H), 0.90 (t, *J* = 6.6 Hz, 3H);

### 4-((5-(6-Chloro-8-(thiophen-2-yl)-9H-purin-2-yl)pent-4-yn-1-yl)amino)phenol (10n)

A target compound (10n) is obtained by the same synthetic method as for Compound (10a). ¹H NMR (600 MHz, CD₃OD) *δ* 7.78 (d, *J* = 3.2 Hz, 1H), 7.65 (d, *J* = 5.0 Hz, 1H), 7.18-7.12 (m, 3H), 6.85 (d, *J* = 8.7 Hz, 2H), 3.68 (t, *J* = 7.1 Hz, 2H), 3.38 (t, *J* = 7.8 Hz, 2H), 2.10 (t, *J* = 7.1 Hz, 2H);

### 6-(6-Chloro-8-(thiophen-2-yl)-9H-purin-2-yl)hex-5-ynoic acid (10o)

A target compound (10o) is obtained by the same synthetic method as for Compound (10a). ¹H NMR (600 MHz, CD₃OD) *δ* 7.97 (s, 1H), 7.76 (d, *J* = 5.0 Hz, 1H), 7.25 (t, *J* = 4.3 Hz, 1H), 2.57 (t, *J* = 7.1 Hz, 2H), 2.51 (t, *J* = 7.3 Hz, 2H), 1.96 (q, *J* = 7.3 Hz, 2H);

### 6-Cloro-2-(phenylethynyl)-8-(thiophen-2-yl)-9H-purine (10p)

A target compound (10p) is obtained by the same synthetic method as for Compound (10a). 1H-NMR (600 MHz, CD₃OD) *δ* 7.99 (d, *J* = 3.7 Hz, 1H), 7.81 (d, *J* = 4.9 Hz, 1H), 7.66-7.64 (m, 2H), 7.45 (t, *J* = 6.7 Hz, 3H), 7.27 (t, *J* = 4.3 Hz, 1H);

### 8-(Furan-2-yl)-2-(hex-1-yn-1-yl)-9H-purin-6-amine (11a)

Starting Material (10a) (1.1 g, 2.19 mmol) is dissolved in *t*-BuOH (15 mL), the resulting solution is put into a steel bomb, a saturated ammonia *t*-BuOH solution (30 mL) is added, and then the resultant is stirred at 100°C for 10 hours. After the temperature of the reaction mixture is lowered to -10°C, and then MeOH (50 mL) is added thereto. The resulting solid is filtered to obtain a target compound (11a) (750 mg, 86%): ¹H NMR (DMSO-*d*₆): *δ* 7.86 (d, *J* = 4.8Hz, 1H), 7.58 (d, *J* = 3.6Hz, 1H), 7.47 (bs, 1 H), 7.29 (d, *J* = 4.8 Hz, 1H), 5.95 (d, *J* = 7.2 Hz, 1H), 5.53 (d, *J* = 6.0 Hz, 1H), 5.25 (bs, 2 H), 4.41 (dd, *J* = 3.6, 9.6 Hz, 1H), 4.30 (s, 1 H), 3.82 (d, *J* = 9.6Hz, 1H), 2.43 (t, 2 H), 1.53 (m, 2 H), 1.43 (m, 2 H), 0.92 (t, *J* = 7.2 Hz, 2H).

### 2-(Hex-1-yn-1-yl)-8-(thiophen-2-yl)-9H-purin-6-amine (11b)

A target compound (11b) is obtained by the same synthetic method as for Compound (11a): ¹H NMR (600 MHz, CD₃OD) *δ* 7.85 (dd, *J* = 1.2, 3.6 Hz, 1 H), 7.47 (dd, *J* = 1.2, 4.8 Hz, 1 H), 7.09 (dd, *J* = 3.6, 5.2 Hz, 1 H), 2.53 (t, *J* = 6.8 Hz, 2 H), 1.61-1.69 (m, 2 H), 1.49-1.58 (m, 2 H), 0.98 (t, *J* = 7.6 Hz, 3 H).

### 8-(furan-2-yl)-2-(hept-1-yn-1-yl)-9H-purin-6-amine (11c)

A target compound (11c) is obtained by the same synthetic method as for Compound (11a): ¹H NMR (600 MHz, CDCl₃) *δ* 7.75 (d, 1H), 7.03 (d, 1H), 6.98 (s, 2H), 6.72 (t, *J* = 7.2 Hz, 1H), 2.42 (t, *J* = 7.4 Hz, 2H), 1.37 (m, 2H), 1.29 (m, 2H), 1.00 (t, *J* = 7.2 Hz, 3H).

### 8-(furan-2-yl)-2-(oct-1-yn-1-yl)-9H-purin-6-amine (11d)

A target compound (11d) is obtained by the same synthetic method as for Compound (11a): ¹H NMR (600 MHz, CDCl₃) *δ* 7.87 (d, 1H), 7.20 (d, 1H), 7.00 (s, 2H), 6.61 (t, *J* = 7.7 Hz, 1H), 2.39 (t, *J* = 7.4 Hz, 2H), 1.48 (m, 2H), 1.31 (m, 2H), 1.28-1.17 (m, 4H), 0.97 (t, *J* = 7.7 Hz, 3H).

### 8-(furan-2-yl)-2-(non-1-yn-1-yl)-9H-purin-6-amine (11e)

A target compound (11e) is obtained by the same synthetic method as for Compound (11a): ¹H NMR (600 MHz, CDCl₃) *δ* 7.91 (d, 1H), 7.38 (d, 1H), 6.89 (s, 2H), 6.72 (t, *J* = 7.7 Hz, 1H), 2.45 (t, *J* = 7.7 Hz, 2H), 1.58 (m, 2H), 1.42 (m, 2H), 1.31-1.15 (m, 6H), 0.99 (t, *J* = 7.2 Hz, 3H).

### 2-(dec-1-yn-1-yl)-8-(furan-2-yl)-9H-purin-6-amine (11f)

A target compound (11f) is obtained by the same synthetic method as for Compound (11a): ¹H NMR (600 MHz, CDCl₃) *δ* 7.98 (d, 1H), 7.45 (d, 1H), 6.88 (s, 2H), 6.71 (t, *J* = 7.4 Hz, 1H), 1.80-1.69 (m, 2H), 1.51 (m, 4H), 1.42-1.31 (m, 4H), 1.30-1.19 (m, 4H), 1.00 (t, *J* = 7.4 Hz, 3H).

### 4-((5-(6-amino-8-(furan-2-yl)-9H-purin-2-yl)pent-4-yn-1-yl)amino)phenol (11g)

A target compound (11g) is obtained by the same synthetic method as for Compound (11a): ¹H NMR (600 MHz, CDCl₃) *δ* 9.31 (s, 1H), 7.82 (d, 1H), 7.11 (d, 1H), 6.85 (s, 2H), 6.75 (t, *J* = 7.4 Hz, 1H), 6.64 (d, 2H), 6.58 (d, 2H), 6.55 (d, 2H), 6.53 (d, 2H), 6.20 (s, 1H), 2.46 (d, 1H), 1.76-1.61 (m, 2H), 1.51 (d, 2H).

### 6-(6-amino-8-(furan-2-yl)-9H-purin-2-yl)hex-5-ynoic acid (11h)

A target compound (11h) is obtained by the same synthetic method as for Compound (11a): ¹H NMR (600 MHz, CDCl₃) *δ* 11.8 (s, 1H), 7.91 (d, 1H), 7.130 (d, 1H), 6.91 (s, 2H), 6.79 (t, *J* = 7.4 Hz, 1H), 2.35 (d, 2H), 1.75 (d, 2H).

### 8-(furan-2-yl)-2-(phenylethynyl)-9H-purin-6-amine (11i)

A target compound (11i) is obtained by the same synthetic method as for Compound (11a): ¹H NMR (600 MHz, CDCl₃) *δ* 8.01 (d, 1H), 7.77 (d, 1H), 7.52 (d, 1H) 7.48 (d, 1H), 7.46 (d, 1H), 7.45 (d, 1H), 7.35 (d, 1H), 6.97 (s, 2H), 6.85 (t, *J* = 7.4 Hz, 1H).

### 2-(Hept-1-yn-1-yl)-8-(thiophen-2-yl)-9H-purin-6-amine (11j)

A target compound (11j) is obtained by the same synthetic method as for Compound (11a): ¹H NMR (600 MHz, DMSO-*d*₆) *δ* 7.82 (d, *J* = 3.7 Hz, 1H), 7.73 (d, *J* = 4.6 Hz, 1H), 7.32 (s, 2H), 7.22 (dd, *J* = 5.0, 3.7 Hz, 1H), 2.40 (t, *J* = 6.9 Hz, 2H), 1.53 (q, *J* = 7.3 Hz, 2H), 1.43-1.29 (m, 4H), 0.89 (t, *J* = 7.1 Hz, 3H).

### 2-(oct-1-yn-1-yl)-8-(thiophen-2-yl)-9H-purin-6-amine (Ilk)

A target compound (11k) is obtained by the same synthetic method as for Compound (11a): ¹H NMR (600 MHz, DMSO-*d*₆) *δ* 7.81 (d, *J* = 3.2 Hz, 1H), 7.72 (d, *J* = 5.0 Hz, 1H), 7.26 (s, 2H), 7.21 (dd, *J* = 4.8, 3.9 Hz, 1H), 2.39 (t, *J* = 6.9 Hz, 2H), 1.51 (q, *J* = 7.3 Hz, 2H), 1.39 (t, *J* = 7.5 Hz, 2H), 1.28-1.25 (m, 4H), 0.86 (t, *J* = 6.9 Hz, 3H).

### 2-(Non-1-yn-1-yl)-8-(thiophen-2-yl)-9H-purin-6-amine (111)

A target compound (11l) is obtained by the same synthetic method as for Compound (11a): ¹H NMR (600 MHz, CD₃OD) *δ* 7.77 (dd, *J* = 3.9, 1.1 Hz, 1H), 7.65 (dd, *J* = 5.1, 0.9 Hz, 1H), 7.21 (dd, *J* = 5.1, 3.7 Hz, 1H), 2.45 (t, *J* = 7.1 Hz, 2H), 1.63 (q, *J* = 7.4 Hz, 2H), 1.52-1.46 (m, 2H), 1.38-1.31 (m, 6H), 0.91 (t, *J* = 6.9 Hz, 3H).

### 2-(Dec-1-yn-1-yl)-8-(thiophen-2-yl)-9H-purin-6-amine (11m)

A target compound (11m) is obtained by the same synthetic method as for Compound (11a): ¹H NMR (600 MHz, CD₃OD) *δ* 7.77 (dd, *J* = 3.9, 1.1 Hz, 1H), 7.66 (dd, *J* = 5.0, 0.9 Hz, 1H), 7.21 (dd, *J* = 5.0, 3.7 Hz, 1H), 2.45 (t, *J* = 7.1 Hz, 2H), 1.64 (t, *J* = 7.5 Hz, 2H), 1.50 (t, *J* = 7.3 Hz, 2H), 1.35-1.32 (m, 8H), 0.90 (t, *J* = 6.9 Hz, 3H).

### 4-((5-(6-Amino-8-(thiophen-2-yl)-9H-purin-2-yl)pent-4-yn-1-yl)amino)phenol (11n)

A target compound (11n) is obtained by the same synthetic method as for Compound (11a): ¹H NMR (600 MHz, CD₃OD) *δ* 7.69 (dd, *J* = 3.7, 0.9 Hz, 1H), 7.60 (dd, *J* = 4.9, 1.0 Hz, 1H), 7.19-7.16 (m, 3H), 6.90 (dd, *J* = 6.7, 2.1 Hz, 2H), 3.78 (t, *J* = 7.1 Hz, 2H), 3.49 (q, *J* = 7.4 Hz, 2H), 2.14-2.21 (m, 2H).

### 6-(6-Amino-8-(thiophen-2-yl)-9H-purin-2-yl)hex-5-ynoic acid (11o)

A target compound (11o) is obtained by the same synthetic method as for Compound (11a): ¹H NMR (600 MHz, DMSO-*d*₆) *δ* 7.77 (d, *J* = 3.7 Hz, 1H), 7.69 (d, *J* = 4.9 Hz, 1H), 7.20 (t, *J* = 4.3 Hz, 1H), 2.38 (td, *J* = 15.0, 7.7 Hz, 4H), 1.74 (t, *J* = 7.3 Hz, 2H).

### 2-(Penylethynyl)-8-(thiophen-2-yl)-9H-purin-6-amine (11p)

A target compound (11p) is obtained by the same synthetic method as for Compound (11a): ¹H NMR (600 MHz, DMSO-*d*₆) *δ* 7.84 (d, *J* = 3.1 Hz, 1H), 7.75 (d, *J* = 4.3 Hz, 1H), 7.61-7.58 (m, 2H), 7.46 (q, *J* = 2.2 Hz, 3H), 7.41 (s, 2H, D₂O exchangeable), 7.24 (t, *J* = 4.3 Hz, 1H).

### 8-(furan-2-yl)-2-(hex-1-yn-1-yl)-N-methyl-9H-purin-6-amine (11q)

After anhydrous EtOH (0.3 M) is added to Starting Material (10a) under nitrogen and the resulting mixture is stirred, triethylamine (4 eq) and 3-iodobenzylamine (3 eq) are added thereto and the resulting mixture is stirred at room temperature for 24 hours. The reaction mixture is separated by flash column chromatography to obtain a target compound (11q): ¹H NMR (600 MHz, DMSO-*d*₆) *δ* 7.86 (d, *J* = 4.8 Hz, 1H), 7.78-7.76 (m, 2H), 7.58 (d, *J* = 3.6Hz, 1H), 7.47 (bs, 1H), 7.30 (d, *J* = 5.2 Hz, 1H), 7.29 (d, *J* = 4.8 Hz, 1H), 7.26 (s, 1H), 5.95 (d, *J* = 7.2 Hz, 1H), 5.53 (d, *J* = 6.0 Hz, 1H), 5.25 (bs, 1H), 4.41 (dd, *J* = 3.6, 9.6 Hz, 1H), 4.35 (s, 2H), 4.30 (s, 1H), 3.82 (d, *J* = 9.6Hz, 1H), 2.83 (s, 3H) 2.43 (t, 2 H), 1.53 (m, 2H), 1.43 (m, 2H), 0.92 (t, *J* = 7.2 Hz, 2H).

### 8-(furan-2-yl)-2-(hex-1-yn-1-yl)-N-(3-iodobenzyl)-9H-purin-6-amine (11r)

A target compound (11r) is obtained using methylamine by the same synthetic method as for Compound (11q): ¹H NMR (600 MHz, DMSO-*d*₆) δ7.86 (d, *J* = 4.8Hz, 1H), 7.59 (d, *J* = 3.6Hz, 1H), 7.47 (bs, 1H), 7.29 (d, *J* = 4.8 Hz, 1H), 5.85 (d, *J* = 7.2 Hz, 1H), 5.53 (d, *J* = 6.0 Hz, 1H), 5.25 (bs, 1H), 4.41 (dd, *J* = 3.6, 9.6 Hz, 1H), 4.30 (s, 1H), 3.82 (d, *J* = 9.6Hz, 1H), 2.83 (s, 3H) 2.43 (t, 2 H), 1.53 (m, 2H), 1.43 (m, 2H), 0.92 (t, *J* = 7.2 Hz, 2H).

### 2-(hex-1-yn-1-yl)-N-methyl-8-(thiophen-2-yl)-9H-purin-6-amine (11s)

A target compound (11s) is obtained using Starting Material (10b) by the same synthetic method as for Compound (11q): ¹H NMR (600 MHz, DMSO-*d*₆) δ7.88 (d, *J* = 4.8 Hz, 1H), 7.77-7.76 (m, 2H), 7.58 (d, *J* = 3.6Hz, 1H), 7.47 (bs, 1H), 7.31 (d, *J* = 5.2 Hz, 1H), 7.29 (d, *J* = 4.8 Hz, 1H), 7.25 (s, 1H), 5.95 (d, *J* = 7.2 Hz, 1H), 5.53 (d, *J* = 6.0 Hz, 1H), 5.25 (bs, 1H), 4.43 (dd, *J* = 3.6, 9.6 Hz, 1H), 4.35 (s, 2H), 4.30 (s, 1H), 3.82 (d, *J* = 9.6Hz, 1H), 2.83 (s, 3H) 2.43 (t, 2 H), 1.53 (m, 2H), 1.43 (m, 2H), 0.92 (t, *J* = 7.2 Hz, 2H).

### 2-(hex-1-yn-1-yl)-N-(3-iodobenzyl)-8-(thiophen-2-yl)-9H-purin-6-amine (11t)

A target compound (11t) is obtained by the same synthetic method as for Compound (11q): ¹H NMR (600 MHz, DMSO-*d*₆) δ7.87 (dd, *J* = 1.2, 3.6 Hz, 1 H), 7.51 (dd, *J* = 1.2, 4.8 Hz, 1 H), 7.11 (dd, *J* = 3.6, 5.2 Hz, 1 H), 5.35 (bs, 1H), 2.83 (s, 3H), 2.53 (t, *J* = 6.8 Hz, 2 H), 1.61-1.69 (m, 2 H), 1.49-1.58 (m, 2 H), 0.98 (t, *J* = 7.6 Hz, 3 H).

### Preparation Example 4: Synthesis of 13a to 13p

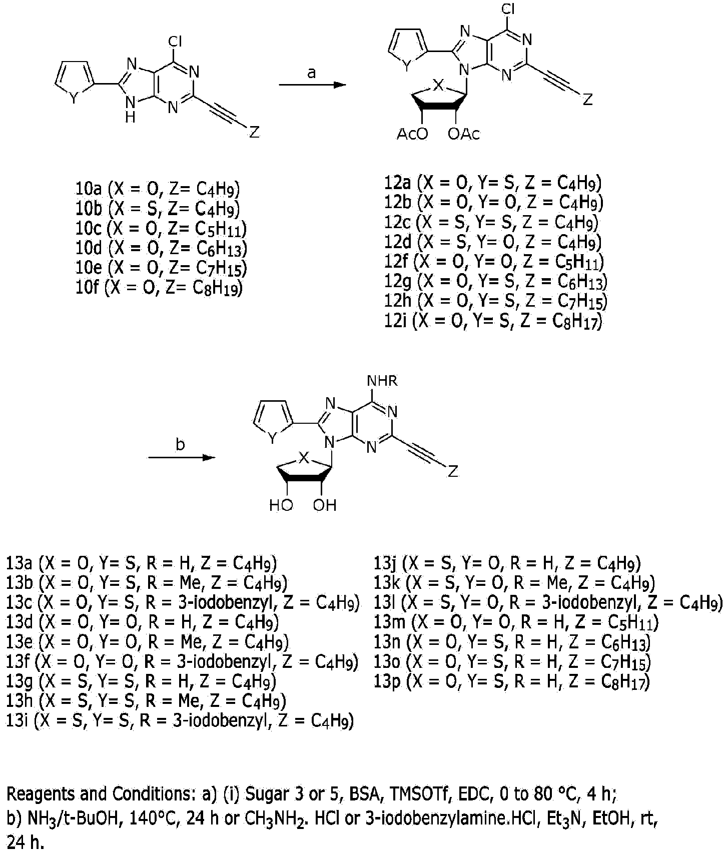

### (2R,3R,4S)-2-(6-Chloro-8-(furan-2-yl)-2-(hex-1-yn-1-yl)-9H-purin-9-yl)tetrahydrothiophene-3,4-diyl diacetate (12a)

Starting Material (11a) (500 mg, 1.66 mmol) is suspended in an anhydrous EDC (5 mL) solvent under nitrogen, N,O-bis(trimethylsilyl)acetamide (BSA) (582 mg, 2.85 mmol) is slowly added thereto at room temperature, and then the resulting mixture is stirred at 40°C for 1 hour. After the reaction mixture is cooled to room temperature, a solution of Sugar 3 (506 mg, 1.86 mmol) dissolved in anhydrous EDC (3 mL) is added thereto, TMSOTf (236 mg, 1.1 mmol) is slowly added thereto at 0°C, and the resulting mixture is stirred at 80°C for 3 hours. After it is confirmed that the reaction is completed, an aqueous saturated NaHCOs solution (30 mL) is added thereto at 0°C, and extraction is performed with DCM (3 x 50 mL). The resulting organic layer is dried over anhydrous MgSO₄ and then concentrated under reduced pressure. The resulting residue is purified by flash column chromatography under the condition of hexane/EtOAc = 4/1 to obtain a target compound (12a) (710 mg, 85%) in the form of a colorless syrup: ¹H NMR (600 MHz, CDCl₃) *δ* 7.74 (t, 1 H), 7.41 (d, 1 H), 6.76 (d, 1 H), 6.70 (d, 1 H), 6.60 (m, 1 H), 5.99 (d, 1 H), 4.01 (dd, *J* = 3.6, 12 Hz, 1H), 3.15 (dd, *J* = 1.8, 12 Hz, 1H), 2.50 (t, *J* = 7.2 Hz, 2H), 2.21 (s, 3 H), 1.97 (s, 3 H), 1.70-1.50 (m, 4 H), 0.97 (t, *J* = 7.2 Hz, 3H).

### (2R,3R,4R)-2-(6-Chloro-8-(furan-2-yl)-2-(hex-1-yn-1-yl)-9H-purin-9-yl)tetrahydrofuran-3,4-diyl diacetate (12b)

A target compound (12b) is obtained by the same synthetic method as for Compound (12a): ¹H NMR (600 MHz, CDCl₃) *δ* 7.84 (t, 1 H), 7.50 (d, 1 H), 6.80 (d, 1 H), 6.72 (d, 1 H), 6.70 (m, 1 H), 5.99 (d, 1 H), 4.08 (dd, *J* = 3.6, 12 Hz, 1H), 3.20 (dd, *J* = 1.8, 12 Hz, 1H), 2.51 (t, *J* = 7.4 Hz, 2H), 2.21 (s, 3 H), 1.97 (s, 3 H), 1.71-1.51 (m, 4 H), 1.01 (t, *J* = 7.7 Hz, 3H).

### (2R,3R,4S)-2-(6-Chloro-2-(hex-1-yn-1-yl)-8-(thiophen-2-yl)-9H-purin-9-yl)tetrahydrothiophene-3,4-diyl diacetate (12c)

A target compound (12c) is obtained by the same synthetic method as for Compound (12a): ¹H NMR (600 MHz, CDCl₃) δ 7.88 (t, 1 H), 7.53 (d, 1 H), 6.75 (d, 1 H), 6.74 (d, 1 H), 6.72 (m, 1 H), 6.00 (d, 1 H), 4.01 (dd, J = 3.8, 12 Hz, 1H), 3.25 (dd, J = 1.6, 12 Hz, 1H), 2.50 (t, J = 7.4 Hz, 2H), 2.23 (s, 3 H), 1.97 (s, 3 H), 1.71-1.58 (m, 4 H), 0.97 (t, J = 7.2 Hz, 3H).

### (2R,3R,4R)-2-(6-Chloro-2-(hex-1-yn-1-yl)-8-(thiophen-2-yl)-9H-purin-9-yl)tetrahydrofuran-3,4-diyl diacetate (12d)

A target compound (12d) is obtained by the same synthetic method as for Compound (12a): ¹H NMR (600 MHz, CDCl₃) δ 7.91 (t, 1 H), 7.61 (d, 1 H), 6.81 (d, 1 H), 6.77 (d, 1 H), 6.74 (m, 1 H), 5.98 (d, 1 H), 4.02 (dd, J = 3.6, 12 Hz, 1H), 3.25 (dd, J = 1.6, 12 Hz, 1H), 2.52 (t, J = 7.7 Hz, 2H), 2.25 (s, 3 H), 1.99 (s, 3 H), 1.75-1.61 (m, 4 H), 0.99 (t, J = 7.4 Hz, 3H).

### (2R,3R,4R)-2-(6-chloro-8-(furan-2-yl)-2-(hept-1-yn-1-yl)-9H-purin-9-yl)tetrahydrofuran-3,4-diyl diacetate (12e)

A target compound (12e) is obtained by the same synthetic method as for Compound (12a): ¹H NMR (600 MHz, CDCl₃) δ 7.84 (t, 1 H), 7.50 (d, 1 H), 6.80 (d, 1 H), 6.72 (d, 1 H), 6.70 (m, 1 H), 5.99 (d, 1 H), 4.08 (dd, J = 3.6, 12 Hz, 1H), 3.20 (dd, J = 1.8, 12 Hz, 1H), 2.51 (t, J = 7.4 Hz, 2H), 2.21 (s, 3 H), 1.97 (s, 3 H), 1.71-1.51 (m, 4 H), 1.29-1.28 (m 2H), 1.01 (t, J = 7.7 Hz, 3H).

### (2R,3R,4R)-2-(6-chloro-2-(oct-1-yn-1-yl)-8-(thiophen-2-yl)-9H-purin-9-yl)tetrahydrofuran-3,4-diyl diacetate (12f)

A target compound (12f) is obtained by the same synthetic method as for Compound (12a): ¹H NMR (600 MHz, CDCl₃) δ 7.91 (t, 1 H), 7.61 (d, 1 H), 6.81 (d, 1 H), 6.77 (d, 1 H), 6.74 (m, 1 H), 5.98 (d, 1 H), 4.02 (dd, J = 3.6, 12 Hz, 1H), 3.25 (dd, J = 1.6, 12 Hz, 1H), 2.52 (t, J = 7.7 Hz, 2H), 2.25 (s, 3H), 1.99 (s, 3 H), 1.81-1.79 (m, 4H), 1.75-1.61 (m, 4H), 0.99 (t, J = 7.4 Hz, 3H).

### (2R,3R,4R)-2-(6-chloro-2-(non-1-yn-1-yl)-8-(thiophen-2-yl)-9H-purin-9-yl)tetrahydrofuran-3,4-diyl diacetate (12g)

A target compound (12g) is obtained by the same synthetic method as for Compound (12a): ¹H NMR (800 MHz, CD₃OD) d 7.71 (dd, J = 0.9, 5.1 Hz, 1 H), 7.65 (dd, J = 0.9, 3.6 Hz, 1 H), 7.23 (dd, J = 3.6, 5.0 Hz, 1 H), 6.11 (d, J = 6.4 Hz, 1 H), 5.50 (dd, J = 4.8, 6.3 Hz, 1 H), 4.63 (dd, J = 3.5, 9.6 Hz, 1 H), 4.46 (t, J = 4.6 Hz, 1 H), 3.97 (dd, J = 1.2, 6.0 Hz, 1 H), 3.08 (bs, 3 H), 2.45 (t, J = 7.2 Hz, 2 H), 1.78-1.76 (m, 4H), 1.61-1.65 (m, 4 H), 1.50-1.55 (m, 2 H), 0.98 (t, J = 7.3 Hz, 3 H).

### (2R,3R,4R)-2-(6-chloro-2-(dec-1-yn-1-yl)-8-(thiophen-2-yl)-9H-purin-9-yl)tetrahydrofuran-3,4-diyl diacetate (12h)

A target compound (12h) is obtained by the same synthetic method as for Compound (12a): ¹H NMR (600 MHz, CDCl₃) δ 7.88 (t, 1 H), 7.53 (d, 1 H), 6.75 (d, 1 H), 6.74 (d, 1 H), 6.72 (m, 1 H), 6.00 (d, 1 H), 4.01 (dd, J = 3.8, 12 Hz, 1H), 3.25 (dd, J = 1.6, 12 Hz, 1H), 2.50 (t, J = 7.4 Hz, 2H), 2.23 (s, 3 H), 1.97 (s, 3 H), 1.82-1.80 (m, 4H), 1.71-1.68 (m, 4 H), 1.62-1.60 (m, 4H), 0.97 (t, J = 7.2 Hz, 3H).

### (2R,3R,4S)-2-(6-Amino-8-(furan-2-yl)-2-(hex-1-yn-1-yl)-9H-purin-9-yl)tetrahydrothiophene-3,4-diol (13a)

Starting Material (12a) (1.1 g, 2.187 mmol) is dissolved in t-BuOH (30 mL), the resulting solution is put into a steel bomb, a saturated ammonia t-BuOH solution (30 mL) is quickly added, and then the resultant is stirred at 100°C for 10 hours. After the temperature of the reaction mixture is lowered to -10°C, MeOH (50 mL) is added, and the resulting solid is filtered to obtain a target compound (13a) (760 mg, 87%): ¹H NMR (600 MHz, MeOD) δ 7.81 (d, J = 1.6 Hz, 1 H), 7.19 (d, J = 3.6 Hz, 1 H), 6.68 (dd, J = 1.6, 3.6 Hz, 1 H), 6.39 (d, J = 7.6 Hz, 1 H), 5.64 (dd, J = 3.6, 8.0 Hz, 1 H), 4.54 (d, J = 2.0 Hz, 1 H), 3.70 (dd, J = 3.2, 11.6 Hz, 1 H), 2.90 (dd, J = 1.6, 11.2 Hz, 1 H), 2.43 (t, J = 7.6 Hz, 2 H), 1.57-1.64 (m, 2 H), 1.46-1.55 (m, 2 H), 0.96 (t, J = 7.2 Hz, 3 H).

### (2S,3R,4R)-2-(2-(hex-1-yn-1-yl)-6-(methylamino)-8-(thiophen-2-yl)-9H-purin-9-yl)tetrahydrofuran-3,4-diol (13b)

Starting Material (12a) (1.1 g, 2.187 mmol) is dissolved in EtOH (30 mL), triethylamine and methylamine hydrochloride are added thereto, and then the resultant is stirred at room temperature for 24 hours. MeOH (50 mL) is put into the reaction mixture and the resulting solid is filtered to obtain a target compound (13b) (79%): ¹H NMR (800 MHz, CD₃OD) d 7.71 (dd, J = 0.9, 5.1 Hz, 1H), 7.65 (dd, J = 0.9, 3.6 Hz, 1 H), 7.23 (dd, J = 3.6, 5.0 Hz, 1H), 6.11 (d, J = 6.4 Hz, 1 H), 5.50 (dd, J = 4.8, 6.3 Hz, 1 H), 4.63 (dd, J = 3.5, 9.6 Hz, 1 H), 4.46 (t, J = 4.6 Hz, 1 H), 3.97 (dd, J = 1.2, 6.0 Hz, 1 H), 3.08 (bs, 3 H), 2.45 (t, J = 7.2 Hz, 2 H), 1.61-1.65 (m, 2 H), 1.50-1.55 (m, 2 H), 0.98 (t, J = 7.3 Hz, 3 H).

### (2S,3R,4R)-2-(2-(hex-1-yn-1-yl)-6-((3-iodobenzyl)amino)-8-(thiophen-2-yl)-9H-purin-9-yl)tetrahydrofuran-3,4-diol (13c)

A target compound (13c) is obtained using 3-iodobenzylamine hydrochloride by the same synthetic method as for Compound (13b): ¹H NMR (800 MHz, DMSO-d₆) d 8.55 (bs, 1 H), 7.88 (dd, J = 0.7, 5.1 Hz, 1 H), 7.74 (s, 1 H), 7.58-7.60 (m, 2 H), 7.35 (d, J = 6.4 Hz, 1 H), 7.29 (dd, J = 3.7, 4.8 Hz, 1 H), 7.11 (t, J = 7.7 Hz, 1 H), 5.97 (d, J = 6.8 Hz, 1 H), 5.51 (d, J = 6.5 Hz, 1 H), 5.22-5.24 (m, 2 H), 4.41 (dd, J = 3.3, 9.3 Hz, 1 H), 3.83 (d, J = 8.9 Hz, 1 H), 2.42 (t, J = 7.1 Hz, 2 H), 1.52-1.56 (m, 2 H), 1.42-1.46 (m, 2 H), 0.92 (t, J = 7.3 Hz, 3 H).

### (2R,3R,4R)-2-(6-Amino-8-(furan-2-yl)-2-(hex-1-yn-1-yl)-9H-purin-9-yl)tetrahydrofuran-3,4-diol (13d)

A target compound (13d) is obtained by the same synthetic method as for Compound (13a): ¹H NMR (800 MHz, CD₃OD) d 7.82 (dd, J = 0.4, 1.6 Hz, 1 H), 7.19 (dd, J = 0.4, 3.4 Hz, 1 H), 6.70 (dd, J = 1.7, 3.5 Hz, 1 H), 6.31 (d, J = 6.3 Hz, 1 H), 5.44 (dd, J = 4.8, 6.2 Hz, 1 H), 4.63 (dd, J = 3.5, 9.6 Hz, 1 H), 4.47-4.48 (m, 1 H), 3.98 (dd, J = 1.3, 9.6 Hz, 1 H), 2.44 (t, J = 7.1 Hz, 2 H), 1.60-1.63 (m, 2 H), 1.49-1.54 (m, 2 H), 0.97 (t, J = 7.3 Hz, 3 H).

### (2S,3R,4R)-2-(8-(furan-2-yl)-2-(hex-1-yn-1-yl)-6-(methylamino)-9H-purin-9-yl)tetrahydrofuran-3,4-diol (13e)

A target compound (13e) is obtained by the same synthetic method as for Compound (13b): ¹H NMR (800 MHz, CD₃OD) d 7.71 (d, J = 5.1 Hz, 1 H), 7.65 (d, J = 3.6 Hz, 1 H), 7.23 (dd, J = 3.6, 5.1 Hz, 1 H), 6.11 (d, J = 6.4 Hz, 1 H), 5.50 (dd, J = 4.8, 6.3 Hz, 1 H), 4.63 (dd, J = 3.5, 9.6 Hz, 1 H), 4.45-4.47 (m, 1 H), 3.97 (dd, J = 1.3, 9.6 Hz, 1 H), 3.08 (bs, 3 H), 2.45 (t, J = 7.1 Hz, 2 H), 1.61-1.65 (m, 2 H), 1.50-1.55 (m, 2 H), 0.98 (t, J = 7.3 Hz, 3 H).

### (2S,3R,4R)-2-(8-(furan-2-yl)-2-(hex-1-yn-1-yl)-6-((3-iodobenzyl)amino)-9H-purin-9-yl)tetrahydrofuran-3,4-diol (13f)

A target compound (13f) is obtained by the same synthetic method as for Compound (13b): ¹H NMR (600 MHz, CDCl₃+CD₃OD) δ 7.65 (s, 1 H), 7.51 (d, J = 3.4 Hz, 1 H), 7.47 (d, J = 7.8 Hz, 1 H), 7.43 (d, J = 4.9 Hz, 1 H), 7.26 (bs, 1 H), 7.05 (t, J = 3.7 Hz, 1 H), 6.94 (t, J = 7.8 Hz, 1 H), 5.97 (d, J = 5.4 Hz, 1 H), 5.31 (bs, 1 H), 4.67 (bs, 2 H), 4.51 (dd, J = 3.8, 9.6 Hz, 1 H), 4.43-4.49 (m, 1 H), 3.91 (d, J = 9.5 Hz, 1 H), 2.32 (t, J = 7.1 Hz, 2 H), 1.47-1.58 (m, 2 H), 1.33-1.44 (m, 2 H), 0.83 (t, J = 7.3 Hz, 3 H).

### (2R,3R,4S)-2-(6-Amino-2-(hex-1-yn-1-yl)-8-(thiophen-2-yl)-9H-purin-9-yl)tetrahydrothiophene-3,4-diol (13g)

A target compound (13g) is obtained by the same synthetic method as for Compound (13b): ¹H NMR (600 MHz, DMSO-d₆) δ 7.84 (d, J = 4.0 Hz, 1 H, 7.60 (d, J = 3.2 Hz, 1 H), 7.42 (bs, 1 H, D₂O exchangeable), 7.27 (dd, J = 4.0, 5.2 Hz, 1 H), 6.01 (d, J = 7.6 Hz, 1 H), 5.39-5.46 (m, 2 H, D₂O exchangeable), 5.31 (d, J = 3.6 Hz, 1 H), 4.33 (d, J = 1.6 Hz, 1 H), 3.44 (dd, J = 3.2, 11.2 Hz, 1H), 2.77 (d, J = 10.4 Hz, 1 H), 2.40 (t, J = 7.2 Hz, 1 H), 1.47-1.54 (m, 2 H), 1.35-1.44 (m, 2H), 0.88 (t, 7.2 Hz, 3 H).

### (2R,3R,4S)-2-(2-(hex-1-yn-1-yl)-6-(methylamino)-8-(thiophen-2-yl)-9H-purin-9-yl)tetrahydrothiophene-3,4-diol (13h)

A target compound (13h) is obtained by the same synthetic method as for Compound (13b): ¹H NMR (600 MHz, DMSO-d₆) δ 7.86 (bs, 1 H, D2O), 7.83 (dd, J = 3.2, 5.2 Hz, 1 H), 7.60 (dd, J = 0.8, 3.6 Hz, 1 H), 7.27 (dd, J = 3.6, 4.8 Hz, 1 H), 6.02 (d, J = 8.4 Hz, 1 H), 5.48 (d, J = 6.4 Hz, 1 H,D2O exchangeable), 5.39 - 5.43 (m, D2O exchangeable), 5.34 (d, J = 3.2 Hz, 1 H), 4.33 (d, J = 1.6 Hz, 1 H), 3.44 (dd, J = 4.0, 11.6 Hz, 1 H), 2.89 (bs, 3 H), 2.77 (dd. J = 1.2, 11.2 Hz, 1 H), 2.41 (t, J = 7.2 Hz, 1 H), 1.49 - 1.56 (m, 2 H), 1.36 - 1.45 (m, 2 H), 0.89 (t, 7.2 Hz, 3 H).

### (2R,3R,4S)-2-(2-(hex-1-yn-1-yl)-6-((3-iodobenzyl)amino)-8-(thiophen-2-yl)-9H-purin-9-yl)tetrahydrothiophene-3,4-diol (13i)

A target compound (13i) is obtained by the same synthetic method as for Compound (13b): ¹H NMR (600 MHz, DMSO-d₆) δ 8.49 (bs, 1 H, D₂O exchangeable), 7.85 (dd, J = 2.4, 6.4 Hz, 1 H), 7.17 (bs, 1 H), 7.61 (dd, J = 0.8, 3.6 Hz, 1 H), 7.55 (d, J = 8.0 Hz, 1 H), 7.31-7.34 (m, 1 H), 7.28 (dd, J = 4.0, 4.8 Hz, 1 H), 7.08 (t, J = 7.6 Hz, 1 H), 6.03 (d, J = 7.6 Hz, 1 H), 5.46 (d, J = 6.8 Hz, 1 H, D₂O exchangeable), 5.39-5.41 (m, 1 H, D₂O exchangeable), 5.31 (d, J = 3.6 Hz, 1 H), 4.59 (bs, 2 H), 4.33 (s, 1 H), 3.44 (dd, J = 4.0, 11.2 Hz, 1 H), 2.77 (d, J = 10.0 Hz, 1 H), 2.40 (t, J = 7.6 Hz, 2 H), 1.48-1.55 (m, 2 H), 1.38-1.45 (m, 2 H), 0.89 (t, J = 7.2 Hz, 3 H).

### (2R,3R,4R)-2-(6-Amino-2-(hex-1-yn-1-yl)-8-(thiophen-2-yl)-9H-purin-9-yl)tetrahydrofuran-3,4-diol (13j)

A target compound (13j) is obtained by the same synthetic method as for Compound (13a): ¹H NMR (600 MHz, MeOD) δ 7.81 (d, J = 1.6 Hz, 1 H), 7.19 (d, J = 3.6 Hz, 1 H), 6.68 (dd, J = 1.6, 3.6 Hz, 1 H), 6.39 (d, J = 7.6 Hz, 1 H), 5.64 (dd, J = 3.6, 8.0 Hz, 1 H), 4.54 (d, J = 2.0 Hz, 1H), 3.70 (dd, J = 3.2, 11.6 Hz, 1 H), 2.90 (dd, J = 1.6, 11.2 Hz, 1 H), 2.43 (t, J = 7.6 Hz, 2 H), 1.57-1.64 (m, 2 H), 1.46-1.55 (m, 2 H), 0.96 (t, J = 7.2 Hz, 3 H).

### (2R,3R,4S)-2-(8-(furan-2-yl)-2-(hex-1-yn-1-yl)-6-(methylamino)-9H-purin-9-yl)tetrahydrothiophene-3,4-diol (13k)

A target compound (13k) is obtained by the same synthetic method as for Compound (13b): ¹H NMR (600 MHz, MeOD) δ 7.79 (s, 1 H), 7.16 (d, J = 4.0 Hz, 1 H), 6.67 (dd, J = 2.0, 3.6 Hz, 1 H), 6.37 (d, 8.0 Hz, 1 H), 5.63 (dd, J = 3.6, 7.6 Hz, 1 H), 4.53 (d, J = 2.0 Hz, 1 H), 3.70 (dd, J = 3.6, 11.6 Hz, 1 H), 3.08 (bs, 3 H), 2.89 (dd, J = 1.2, 11.2 Hz, 1 H), 2.45 (t, J = 6.8 Hz, 2 H), 1.59-1.65 (m, 2 H), 1.47-1.56 (m, 2 H), 0.97 (t, J = 7.2 Hz, 3 H).

### (2R,3R,4S)-2-(8-(furan-2-yl)-2-(hex-1-yn-1-yl)-6-((3-iodobenzyl)amino)-9H-purin-9-yl)tetrahydrothiophene-3,4-diol (131)

A target compound (13l) is obtained by the same synthetic method as for Compound (13b): ¹H NMR (600 MHz, DMSO-d₆) δ 8.56 (bs, 1 H, D₂O exchangeable), 7.99 (dd, J = 1.2, 2.0 Hz, 1H), 7.72 (s, 1 H), 7.55 (d, 7.6 Hz, 1 H), 7.32 (d, J = 8.4 Hz, 1 H), 7.10 (dd, J = 0.8, 3.2 Hz, 1 H), 7.08 (t, J = 8.0 Hz, 1 H), 6.75 (dd, J = 2.0, 3.6 Hz, 1 H), 6.16 (d, J = 8.0 Hz, 1 H), 5.39 (d, J = 6.4 Hz, 1 H, D₂O exchangeable), 5.33-5.38 (m, 1 H, D₂O exchangeable), 5.29 (d, J = 4.0 Hz, 1 H), 4.59 (bs, 2 H), 4.35 (s, 1 H), 3.44 (dd, J = 3.2, 10.8 Hz, 1 H), 2.77 (dd, J = 1.2, 10.8 Hz, 1 H), 2.40 (t, J = 7.2 Hz, 2 H), 1.48-1.55 (m, 2 H), 1.36-1.45 (m, 2 H), 0.89 (t, J = 7.6 Hz, 3 H).

### (2R,3R,4R)-2-(6-amino-8-(furan-2-yl)-2-(hept-1-yn-1-yl)-9H-purin-9-yl)tetrahydrofuran-3,4-diyl diacetate (13m)

A target compound (13m) is obtained by the same synthetic method as for Compound (13a): ¹H NMR (600 MHz, MeOD) δ 7.81 (d, J = 1.6 Hz, 1 H), 7.19 (d, J = 3.6 Hz, 1 H), 6.68 (dd, J = 1.6, 3.6 Hz, 1 H), 6.39 (d, J = 7.6 Hz, 1 H), 5.64 (dd, J = 3.6, 8.0 Hz, 1 H), 4.54 (d, J = 2.0 Hz, 1H), 3.70 (dd, J = 3.2, 11.6 Hz, 1 H), 2.90 (dd, J = 1.6, 11.2 Hz, 1 H), 2.43 (t, J = 7.6 Hz, 2 H), 1.70-1.68 (m, 2H), 1.57-1.64 (m, 2 H), 1.46-1.55 (m, 2 H), 0.96 (t, J = 7.2 Hz, 3 H).

### (2R,3R,4R)-2-(6-amino-2-(oct-1-yn-1-yl)-8-(thiophen-2-yl)-9H-purin-9-yl)tetrahydrofuran-3,4-diyl diacetate (13n)

A target compound (13n) is obtained by the same synthetic method as for Compound (13a): ¹H NMR (800 MHz, CD₃OD) d 7.82 (dd, J = 0.4, 1.6 Hz, 1 H), 7.19 (dd, J = 0.4, 3.4 Hz, 1 H), 6.70 (dd, J = 1.7, 3.5 Hz, 1 H), 6.31 (d, J = 6.3 Hz, 1 H), 5.44 (dd, J = 4.8, 6.2 Hz, 1 H), 4.63 (dd, J = 3.5, 9.6 Hz, 1 H), 4.47-4.48 (m, 1 H), 3.98 (dd, J = 1.3, 9.6 Hz, 1 H), 2.44 (t, J = 7.1 Hz, 2 H), 1.75-1.78 (m, 2H), 1.60-1.63 (m, 2 H), 1.51-1.54 (m, 2H), 1.49-1.54 (m, 2 H), 0.97 (t, J = 7.3 Hz, 3 H).

### (2R,3R,4R)-2-(6-amino-2-(non-1-yn-1-yl)-8-(thiophen-2-yl)-9H-purin-9-yl)tetrahydrofuran-3,4-diyl diacetate (13o)

A target compound (13o) is obtained by the same synthetic method as for Compound (13a): ¹H NMR (600 MHz, DMSO-d₆) δ 7.84 (d, J = 4.0 Hz, 1 H, 7.60 (d, J = 3.2 Hz, 1 H), 7.42 (bs, 1 H, D₂O exchangeable), 7.27 (dd, J = 4.0, 5.2 Hz, 1 H), 6.01 (d, J = 7.6 Hz, 1 H), 5.39-5.46 (m, 2 H, D₂O exchangeable), 5.31 (d, J = 3.6 Hz, 1 H), 4.33 (d, J = 1.6 Hz, 1 H), 3.44 (dd, J = 3.2, 11.2 Hz, 1 H), 2.77 (d, J = 10.4 Hz, 1 H), 2.40 (t, J = 7.2 Hz, 1 H), 1.77-1.79 (m, 2H), 1.62-1.65 (m, 2H), 1.47-1.54 (m, 4 H), 1.35-1.44 (m, 2 H), 0.88 (t, 7.2 Hz, 3 H).

### (2R,3R,4R)-2-(6-amino-2-(dec-1-yn-1-yl)-8-(thiophen-2-yl)-9H-purin-9-yl)tetrahydrofuran-3,4-diyl diacetate (13p)

A target compound (13p) is obtained by the same synthetic method as for Compound (13a): ¹H NMR (600 MHz, MeOD) *δ* 7.81 (d, *J* = 1.6 Hz, 1 H), 7.19 (d, *J* = 3.6 Hz, 1 H), 6.68 (dd, *J* = 1.6, 3.6 Hz, 1 H), 6.39 (d, *J* = 7.6 Hz, 1 H), 5.64 (dd, *J* = 3.6, 8.0 Hz, 1 H), 4.54 (d, *J* = 2.0 Hz, 1H), 3.70 (dd, *J* = 3.2, 11.6 Hz, 1 H), 2.90 (dd, *J* = 1.6, 11.2 Hz, 1 H), 2.43 (t, *J* = 7.6 Hz, 2 H), 1.74-1.77 (m, 2H), 1.67-1.70 (m, 2H), 1.57-1.64 (m, 4 H), 1.46-1.55 (m, 4 H), 0.96 (t, *J* = 7.2 Hz, 3 H).

### Preparation Example 5: Synthesis of 14a to 14r

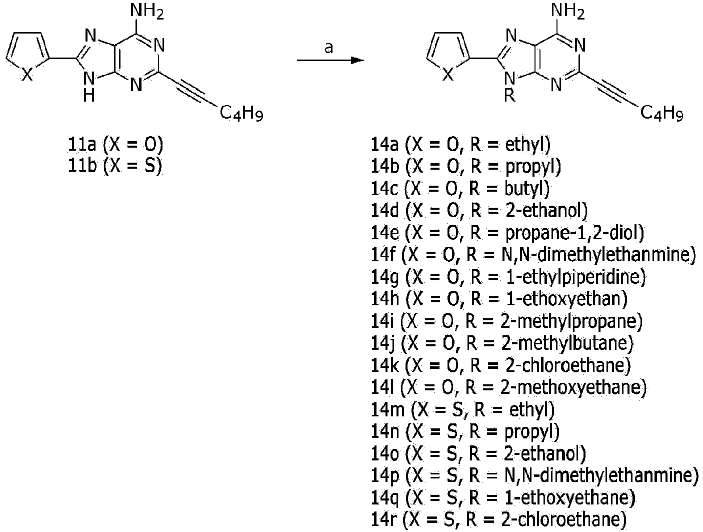

### Reagents and Conditions: a) R-Cl or R-Br, Cs₂CO₃ or K₂CO₃, DMF, 70°C, overnight

### 9-ethyl-8-(furan-2-yl)-2-(hex-1-yn-1-yl)-9H-purin-6-amine (14a)

After Starting Material (11a) (1 g, 3.55 mmol) is dissolved in DMF (10 mL) under nitrogen, K₂CO₃ and ethyl chloride (2.0 eq) are added thereto, and the resulting mixture is stirred at 70°C for 15 hours. After it is confirmed that the reaction is completed, the reaction mixture is extracted with EtOAc (50 mL), and then dried over anhydrous MgSO₄ and concentrated under reduced pressure. The resulting residue is purified by column chromatography under the condition of DCM/MeOH = 50/1 to obtain a target compound (14a) (640 mg, 2.07 mmol, 58%): ¹H NMR (600 MHz, CDCl₃) δ 7.76 (dd, J = 3.6, 1.1 Hz, 1H), 7.42 (dd, J = 5.1, 1.2 Hz, 1H), 7.15 (dd, J = 5.1, 3.7 Hz, 1H), 5.60 (brs, 2 H), 4.52 (q, J = 7.2 Hz, 2H), 2.42 (t, J = 7.1 Hz, 2H), 1.61 (t, J = 7.3 Hz, 3H), 1.55 (m, 2 H), 1.45 (m, 2 H), 0.92 (t, J = 7.2 Hz, 3H).

### 8-(furan-2-yl)-2-(hex-1-yn-1-yl)-9-propyl-9H-purin-6-amine (14b)

A target compound (14b) is obtained by the same synthetic method as for Compound (14a): ¹H NMR (600 MHz, CDCl₃) δ 7.75 (dd, J = 3.6, 1.1 Hz, 1H), 7.41 (dd, J = 5.1, 1.2 Hz, 1H), 7.13 (dd, J = 5.1, 3.7 Hz, 1H), 5.58 (brs, 2 H), 4.34 (m, 2 H), 2.42 (t, J = 7.1 Hz, 2H), 2.12 (m, 2 H), 0.95 (t, J = 7.2 Hz, 3H), 1.54 (m, 2 H), 1.44 (m, 2 H), 0.93 (t, J = 7.2 Hz, 3H).

### 9-butyl-8-(furan-2-yl)-2-(hex-1-yn-1-yl)-9H-purin-6-amine (14c)

A target compound (14c) is obtained by the same synthetic method as for Compound (14a): ¹H NMR (600 MHz, CDCl₃) δ 7.76 (dd, J = 3.6, 1.1 Hz, 1H), 7.43 (dd, J = 5.1, 1.2 Hz, 1H), 7.14 (dd, J = 5.1, 3.7 Hz, 1H), 5.55 (brs, 2 H), 4.42 (t, J = 7.0 Hz, 2H), 2.42 (t, J = 7.1 Hz, 2H), 1.96 (m, 2 H), 1.80 (m, 2 H), 1.54 (m, 2 H), 1.46 (m, 2 H), 0.98 (t, J = 7.3 Hz, 3H), 0.92 (t, J = 7.2 Hz, 3H).

### 2-(6-amino-8-(furan-2-yl)-2-(hex-1-yn-1-yl)-9H-purin-9-yl)ethanol (14d)

After Starting Material (14a) (1 g, 3.55 mmol) is dissolved in DMF (10 mL) under nitrogen, Cs₂CO₃ and 2-bromoethan-1-ol (2.0 eq) are added thereto, and the resulting mixture is stirred at 70°C for 15 hours. After it is confirmed that the reaction is completed, the reaction mixture is extracted with EtOAc (50 mL), and then dried over anhydrous MgSO₄ and concentrated under reduced pressure. The resulting residue is purified by column chromatography under the condition of DCM/MeOH = 50/1 to obtain a target compound (14d) (710 mg, 2.18 mmol, 61%): ¹H NMR (600 MHz, CDCl₃) δ 7.76 (dd, J = 3.6, 1.1 Hz, 1H), 7.45 (dd, J = 5.1, 1.2 Hz, 1H), 7.13 (dd, J = 5.1, 3.7 Hz, 1H), 5.61 (b, 2 H), 4.53 (m, 2 H), 4.21 (m, 2 H), 3,82 (s, 1 H), 2.42 (t, J = 7.1 Hz, 2H), 1.54 (m, 2 H), 1.45 (m, 2 H), 0.92 (t, J = 7.2 Hz, 3H).

### 3-(6-amino-8-(furan-2-yl)-2-(hex-1-yn-1-yl)-9H-purin-9-yl)propane-1,2-diol (14e)

A target compound (14e) is obtained by the same synthetic method as for Compound (14a): ¹H NMR (600 MHz, CDCl₃) δ 7.75 (dd, J = 3.6, 1.2 Hz, 1H), 7.44 (dd, J = 5.1, 1.2 Hz, 1H), 6.54 (dd, J = 3.4, 1.8 Hz, 1H), 5.55 (brs, 2 H), 4.52 (m, 2 H), 4.40 (m, 1 H), 4.05 (brs, 2H), 3.60 (m, 2 H), 2.42 (t, J = 7.1 Hz, 2H), 1.64 (m, 2 H), 1.46 (m, 2 H), 0.92 (t, J = 7.2 Hz, 3H).

### 9-(2-(dimethylamino)ethyl)-8-(furan-2-yl)-2-(hex-1-yn-1-yl)-9H-purin-6-amine (14f)

A target compound (14f) is obtained by the same synthetic method as for Compound (14d): ¹H NMR (600 MHz, CDCl₃) δ 7.75 (dd, J = 3.6, 1.1 Hz, 1H), 7.46 (dd, J = 5.1, 1.2 Hz, 1H), 7.15 (dd, J = 5.1, 3.7 Hz, 1H), 5.63 (brs, 2 H), 4.50 (t, J = J = 7.1 Hz, 2H), 2.76 (t, J = 7.1 Hz, 2H), 2.42 (t, J = 7.1 Hz, 2H), 2.35 (s, 6 H), 1.53 (m, 2 H), 1.46 (m, 2 H), 0.91 (t, J = 7.2 Hz, 3H).

### 8-(furan-2-yl)-2-(hex-1-yn-1-yl)-9-(2-(piperidin-1-yl)ethyl)-9H-purin-6-amine (14g)

A target compound (14g) is obtained by the same synthetic method as for Compound (14a): ¹H NMR (600 MHz, CDCl₃) δ 7.74 (dd, J = 3.6, 1.1 Hz, 1H), 7.45 (dd, J = 5.1, 1.2 Hz, 1H), 7.11 (dd, J = 5.1, 3.7 Hz, 1H), 5.52 (brs, 2 H), 4.40 (t, J = 7.1 Hz, 2H), 2.80 (t, J = 7.1 Hz, 2H), 2.53 (m, 4 H), 2.43 (t, J = 7.1 Hz, 2H), 1.55 (m, 2 H), 1.51 (m, 6 H), 1.45 (m, 2 H), 0.92 (t, J = 7.2 Hz, 3H).

### 9-(2-ethoxyethyl)-8-(furan-2-yl)-2-(hex-1-yn-1-yl)-9H-purin-6-amine (14h)

A target compound (14h) is obtained by the same synthetic method as for Compound (14d): ¹H NMR (600 MHz, CDCl₃) δ 7.75 (dd, J = 3.6, 1.1 Hz, 1H), 7.45 (dd, J = 5.1, 1.2 Hz, 1H), 7.14 (dd, J = 5.1, 3.7 Hz, 1H), 5.54 (brs, 2 H), 4.52 (t, J = 6.1 Hz, 2H), 3.91 (m, 2H), 3.64 (m, 2 H), 2.42 (t, J = 7.1 Hz, 2H), 1.54 (m, 2 H), 1.46 (m, 2 H), 1.14 (t, J = 7.2 Hz, 3H), 0.92 (t, J = 7.2 Hz, 3H).

### 8-(furan-2-yl)-2-(hex-1-yn-1-yl)-9-isobutyl-9H-purin-6-amine (14i)

A target compound (14i) is obtained by the same synthetic method as for Compound (14a): ¹H NMR (600 MHz, CDCl₃) δ 7.76 (dd, J = 3.6, 1.1 Hz, 1H), 7.46 (dd, J = 5.1, 1.2 Hz, 1H), 6.65 (dd, J = 3.4, 1.8 Hz, 1H), 5.58 (brs, 2 H), 4.08 (d, J = 7.5 Hz, 2H), 2.52 (t, J = 7.1 Hz, 2H), 2.42 (m, 1H), 1.68 (m, 2 H), 1.55 (m, 2 H), 1.51 (m, 6H), 0.92 (t, J = 7.2 Hz, 3H).

### 8-(furan-2-yl)-2-(hex-1-yn-1-yl)-9-(2-methylbutyl)-9H-purin-6-amine (14j)

A target compound (14j) is obtained by the same synthetic method as for Compound (14d): ¹H NMR (600 MHz, CDCl₃) δ 7.76 (dd, J = 3.6, 1.1 Hz, 1H), 7.45 (dd, J = 5.1, 1.2 Hz, 1H), 7.14 (dd, J = 5.1, 3.7 Hz, 1H), 5.52 (brs, 2 H), 4.23 (dd, J = 13.5, 6.9 Hz, 1H), 4.11 (dd, J = 13.6, 8.0 Hz, 1H), 2.42 (t, J = 7.1 Hz, 2H), 2.21(m, 1 H), 1.54 (m, 2 H), 1.45 (m, 2 H), 1.43 (m, 1 H), 1.23 (m, 1 H), 0.95 (t, J = 7.3 Hz, 6H), 0.92 (t, J = 7.2 Hz, 3H).

### 9-(2-chloroethyl)-8-(furan-2-yl)-2-(hex-1-yn-1-yl)-9H-purin-6-amine (14k)

A target compound (14k) is obtained by the same synthetic method as for Compound (14d): ¹H NMR (600 MHz, CDCl₃) δ 7.75 (dd, J = 3.7, 1.1 Hz, 1H), 7.55 (dd, J = 5.1, 1.2 Hz, 1H), 7.14 (dd, J = 5.1, 3.7 Hz, 1H), 5.54 (brs, 2 H), 4.71 (t, J = 6.7 Hz, 2H), 4.12 (t, J = 6.7 Hz, 2H), 2.41 (t, J = 7.1 Hz, 2H), 1.54 (m, 2 H), 1.45 (m, 2 H), 0.92 (t, J = 7.2 Hz, 3H).

### 8-(furan-2-yl)-2-(hex-1-yn-1-yl)-9-(2-methoxyethyl)-9H-purin-6-amine (141)

A target compound (14l) is obtained by the same synthetic method as for Compound (14a): ¹H NMR (600 MHz, CDCl₃) δ 7.75 (dd, J = 3.6, 1.1 Hz, 1H), 7.45 (dd, J = 5.1, 1.2 Hz, 1H), 6.60 (dd, J = 3.4, 1.8 Hz, 1H), 5.54 (brs, 2 H), 4.53 (t, J = 6.0 Hz, 2H), 3.92 (t, J = 6.0 Hz, 2H), 3.35 (s, 3 H), 2.52 (m, 2 H), 1.68 (m, 2 H), 1.46 (m, 2 H), 0.92 (t, J = 7.2 Hz, 3H).

### 2-(hex-1-yn-1-yl)-9-propyl-8-(thiophen-2-yl)-9H-purin-6-amine (14m)

A target compound (14m) is obtained by the same synthetic method as for Compound (14a): ¹H NMR (600 MHz, CDCl₃) δ 7.87 (dd, J = 3.6, 1.2 Hz, 1H), 7.44 (dd, J = 5.1, 1.2 Hz, 1H), 7.28 (dd, J = 5.1, 3.7 Hz, 1H), 5.54 (brs, 2 H), 4.52 (q, J = 7.2 Hz, 2H), 2.42 (t, J = 7.1 Hz, 2H), 1.62 (t, J = 7.3 Hz, 3H), 1.55 (m, 2 H), 1.45 (m, 2 H), 0.92 (t, J = 7.2 Hz, 3H).

### 2-(hex-1-yn-1-yl)-9-propyl-8-(thiophen-2-yl)-9H-purin-6-amine (14n)

A target compound (14n) is obtained by the same synthetic method as for Compound (14a): ¹H NMR (600 MHz, CDCl₃) δ 7.88 (dd, J = 3.6, 1.1 Hz, 1H), 7.54 (dd, J = 5.1, 1.2 Hz, 1H), 7.29 (dd, J = 5.1, 3.7 Hz, 1H), 5.58 (brs, 2 H), 4.34 (m, 2 H), 2.42 (t, J = 7.1 Hz, 2H), 2.12 (m, 2 H), 0.95 (t, J = 7.2 Hz, 3H), 1.54 (m, 2 H), 1.45 (m, 2 H), 0.92 (t, J = 7.2 Hz, 3H).

### 2-(6-amino-2-(hex-1-yn-1-yl)-8-(thiophen-2-yl)-9H-purin-9-yl)ethanol (14o)

A target compound (14o) is obtained by the same synthetic method as for Compound (14a): ¹H NMR (600 MHz, CDCl₃) δ 7.87 (dd, 1 H, J = 3.6, 1.1 Hz), 7.55 (dd, 1 H, J = 5.1, 1.2 Hz), 7.28 (dd, 1 H, J = 5.1, 3.7 Hz), 5.54 (b, 2 H), 4.53 (m, 2 H), 4.21 (m, 2 H), 3,82 (s, 1 H), 2.42 (t, 2 H, J = 7.1 Hz), 1.54 (m, 2 H), 1.45 (m, 2 H), 0.92 (t, 3 H, J = 7.2 Hz).

### 9-(2-(dimethylamino)ethyl)-2-(hex-1-yn-1-yl)-8-(thiophen-2-yl)-9H-purin-6-amine (14p)

A target compound (14p) is obtained by the same synthetic method as for Compound (14d): ¹H NMR (600 MHz, CDCl₃) δ 7.86 (dd, 1 H, J = 3.6, 1.1 Hz), 7.55 (dd, 1 H, J = 5.1, 1.2 Hz), 7.28 (dd, 1 H, J = 5.1, 3.7 Hz), 5.54 (brs, 2 H), 4.50 (t, 2 H, J = J = 7.1 Hz), 2.76 (t, 2 H, J = 7.1 Hz), 2.42 (t, 2 H, J = 7.1 Hz), 2.35 (s, 6 H), 1.54 (m, 2 H), 1.45 (m, 2 H), 0.92 (t, 3 H, J = 7.2 Hz).

### 9-(2-ethoxyethyl)-2-(hex-1-yn-1-yl)-8-(thiophen-2-yl)-9H-purin-6-amine (14q)

A target compound (14q) is obtained by the same synthetic method as for Compound (14a): ¹H NMR (600 MHz, CDCl₃) δ 7.87 (dd, 1 H, J = 3.6, 1.1 Hz), 7.55 (dd, 1 H, J = 5.1, 1.2 Hz), 7.28 (dd, 1 H, J = 5.1, 3.7 Hz), 5.54 (brs, 2 H), 4.52 (t, 2 H, J = 6.1 Hz), 3.91 (m, 2H), 3.64 (m, 2H), 2.42 (t, 2 H, J = 7.1 Hz), 1.55 (m, 2 H), 1.45 (m, 2 H), 1.17 (t, 3 H, J = 7.2 Hz), 0.93 (t, 3 H, J = 7.2 Hz).

### 9-(2-chloroethyl)-2-(hex-1-yn-1-yl)-8-(thiophen-2-yl)-9H-purin-6-amine (14r)

A target compound (14r) is obtained by the same synthetic method as for Compound (14d): ¹H NMR (600 MHz, CDCl₃) δ 7.87 (dd, 1 H, J = 3.6, 1.1 Hz), 7.55 (dd, 1 H, J = 5.1, 1.2 Hz), 7.27 (dd, 1 H, J = 5.1, 3.7 Hz), 5.54 (brs, 2 H), 4.71 (t, 2 H, J = 6.7 Hz), 4.12 (t, 2 H, J = 6.7 Hz), 2.42 (t, 2 H, J = 7.1 Hz), 1.53 (m, 2 H), 1.45 (m, 2 H), 0.92 (t, 3 H, J = 7.2 Hz).

### Preparation Example 6: Synthesis of 18a to 18v

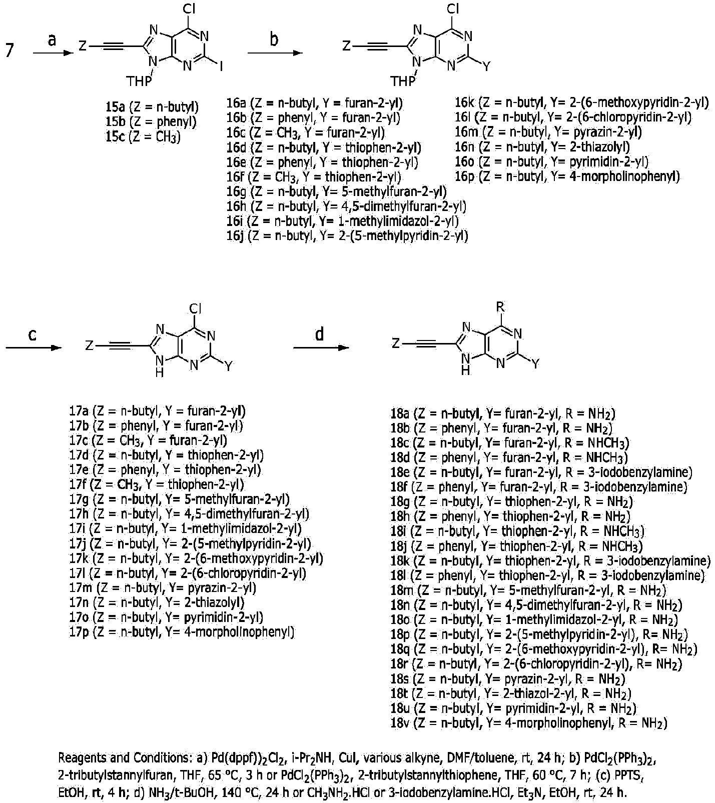

### 6-Chloro-8-(hex-1-yn-1-yl)-2-iodo-9-(tetrahydro-2H-pyran-2-yl)-9H-purine (15a)

After Starting Material (7) (500 mg, 1.01 mmol) is dissolved in anhydrous DMF (10 mL) and toluene (10 mL), bis(dibenzylideneacetone)palladium(0) (57.5 mg, 0.10 mmol), copper(I) iodide (38.4 mg, 0.20 mmol), diisopropylamine (306.60 mg, 3.03 mmol), and 1-hexyne (1.1 eq) are added thereto, and the resulting mixture is stirred for 12 hours. After it is confirmed that the reaction is completed, the reaction mixture is extracted with EtOAc (20 mL), and then dried over MgSO₄ and concentrated under reduced pressure. The resulting residue is purified by column chromatography under the condition of hexane/EtOAc = 4/1 to obtain a target compound (15a) (340 mg, 0.765 mmol, 75%): ¹H NMR (600 MHz, CD₃OD) δ 5.80 (dd, J = 2.3, 11.2 Hz, 1 H), 4.10-4.17 (m, 1 H), 3.70-3.78 (m, 1 H), 2.83-2.92 (m, 1 H), 2.64 (t, J = 6.8 Hz, 2 H), 2.03-2.18 (m, 1 H), 1.91 (d, J = 12.9 Hz, 1 H), 1.50-1.84 (m, 7 H), 0.99 (t, J = 7.3 Hz, 3 H).

### 6-Chloro-2-iodo-8-(phenylethynyl)-9-(tetrahydro-2H-pyran-2-yl)-9H-purine (15b)

A target compound (15b) is obtained using phenylacetylene by the same synthetic method as for Compound (15a): ¹H NMR (600 MHz, CDCl₃) δ 7.66-7.64 (m, 2H), 7.52-7.42 (m, 3H), 5.88 (dd, J = 11.3, 2.1 Hz, 1H), 4.23 (d, J = 11.5 Hz, 1H), 3.80-3.74 (m, 1H), 2.92 (qd, J = 12.1, 4.0 Hz, 1H), 2.17-2.14 (m, 1H), 1.96-1.55 (m, 4H);

### 6-Chloro-2-iodo-8-(prop-1-yn-1-yl)-9-(tetrahydro-2H-pyran-2-yl)-9H-purine (15c)

A target compound (15c) is obtained using 1-propyne by the same synthetic method as for Compound (15a): ¹H NMR (600 MHz, CDCl₃) δ 5.75 (dd, J = 11.0, 2.3 Hz, 1 H), 4.17 (d, J = 11.9 Hz, 1 H), 3.74-3.69 (m, 1 H), 2.89 (dd, J = 11.9, 3.7 Hz, 1 H), 2.22 (s, 3H), 2.13-2.10 (m, 1 H), 1.87-1.61 (m, 4H);

### 6-Chloro-2-(furan-2-yl)-8-(hex-1-yn-1-yl)-9-(tetrahydro-2H-pyran-2-yl)-9H-purine (16a)

Starting Material (15a) is dissolved in anhydrous THF (0.13 M), bis(triphenylphosphine)palladium(II) dichloride (0.1 eq) and 2-tributylstannylfuran (2 eq) are added thereto, and the resulting mixture is stirred at 65°C for 3 hours. After it is confirmed that the reaction is completed, the resulting residue is separated by column chromatography under the condition of hexane/EtOAc = 15/1 to 7/1 to obtain a target compound (16a); ¹H NMR (600 MHz, CDCl₃) δ 7.58 (s, 1 H), 7.30 (d, J = 3.3 Hz, 1 H), 6.51 (dd, J = 1.6, 3.3 Hz, 1 H), 5.80 (dd, J = 2.2, 11.2 Hz, 1 H), 4.15 (d, J = 11.4 Hz, 1 H), 3.69 (t, J = 11.5 Hz, 1H), 2.94-3.06 (m, 1 H), 2.52 (t, J = 6.9 Hz, 2 H), 2.09 (d, J = 9.1 Hz, 1 H), 1.87 (d, J = 12.6 Hz, 1 H), 1.58-1.83 (m, 5 H), 1.44-1.55 (m, 2 H), 0.92 (t, J = 7.3 Hz, 3 H).

### 6-Chloro-2-(furan-2-yl)-8-(phenylethynyl)-9-(tetrahydro-2H-pyran-2-yl)-9H-purine (16b)

A target compound (16b) is obtained by the same synthetic method as for Compound (16a): ¹H NMR (600 MHz, DMSO) δ (ppm): 7.96 (dd, 1 H, J = 8.0, 6.8, Hz), 7.61 (m, 2 H), 7.46 (m, 3H), 7.29 (dd, 1 H, J = 3.6, 0.8 Hz), 6.73 (dd, 1 H, J = 3.6, 1.6, Hz), 5.80 (dd, 1 H, J = 11.2, 2.4 Hz), 4.09 (d, 1 H, J = 11.2 Hz), 3.70 (m, 1 H), 3.50 (m, 1H), 2.67 (t, 2 H, J = 6.8 Hz), 2.02 (m, 2 H), 1.74 (m, 1 H).

### 6-Chloro-2-(furan-2-yl)-8-(prop-1-yn-1-yl)-9-(tetrahydro-2H-pyran-2-yl)-9H-purine (16c)

A target compound (16c) is obtained by the same synthetic method as for Compound (16a): ¹H NMR (600 MHz, CDCl₃) δ 7.62 (d, J = 0.9 Hz, 1H), 7.34 (d, J = 3.7 Hz, 1H), 6.55 (q, J = 1.7 Hz, 1H), 5.82 (dd, J = 11.5, 2.3 Hz, 1H), 4.18 (d, J = 11.5 Hz, 1H), 3.77-3.71 (m, 1H), 3.07-2.98 (m, 1H), 2.21 (s, 3H), 2.13 (d, J = 7.4 Hz, 1H), 1.91-1.63 (m, 4H);

### 6-Chloro-8-(hex-1-yn-1-yl)-9-(tetrahydro-2H-pyran-2-yl)-2-(thiophen-2-yl)-9H-purine (16d)

Starting Material (15b) is dissolved in anhydrous THF (0.13 M), bis(triphenylphosphine)palladium(II) dichloride (0.1 eq) and 2-tributylstannylthiophene (2 eq) are added thereto, and the resulting mixture is stirred at 60°C for 3 hours. After it is confirmed that the reaction is completed, the resulting residue is separated by column chromatography under the condition of hexane/EtOAc = 15/1 to 7/1 to obtain a target compound (16d); ¹H NMR (600 MHz, CDCl₃) δ 8.01 (d, J = 3.5 Hz, 1 H), 7.44 (d, J = 4.9 Hz, 1 H), 7.10 (t, J = 3.8 Hz, 1 H), 5.77 (dd, J = 2.2, 11.1 Hz, 1 H), 4.18 (d, J = 11.4 Hz, 1 H), 3.68-3.76 (m, 1 H), 3.02-3.14 (m, 1 H), 2.54 (t, J = 7.0 Hz, 2 H), 2.14 (d, J = 11.0 Hz, 1 H), 1.90 (d, J = 12.1 Hz, 1 H), 1.62-1.87 (m, 5 H), 1.45-1.57 (m, 2 H), 0.95 (t, J = 7.2 Hz, 3 H).

### 6-Chloro-8-(phenylethynyl)-9-(tetrahydro-2H-pyran-2-yl)-2-(thiophen-2-yl)-9H-purine (16e)

A target compound (16e) is obtained by the same synthetic method as for Compound (16d): ¹H NMR (600 MHz, DMSO) δ (ppm): 7.90 (dd, 1 H, J = 8.0, 6.8 Hz), 7.43 (m, 2 H), 7.46 (m, 3 H), 7.42 (dd, 1 H, J = 3.6, 0.8 Hz), 7.11 (dd, 1 H, J = 3.6, 1.6 Hz), 5.79 (dd, 1 H, J = 11.2, 2.4 Hz), 4.09 (d, 1 H, J = 11.2 Hz), 3.70 (m, 1 H), 3.50 (m, 1H), 2.69 (t, 2 H, J = 6.8 Hz), 2.03 (m, 2 H), 1.74 (m, 1 H).

### 6-Chloro-8-(prop-1-yn-1-yl)-9-(tetrahydro-2H-pyran-2-yl)-2-(thiophen-2-yl)-9H-purine (16f)

A target compound (16f) is obtained by the same synthetic method as for Compound (16d): ¹H NMR (600 MHz, CDCl₃) δ 8.03 (dd, J = 3.7, 0.9 Hz, 1H), 7.46 (dd, J = 5.0, 0.9 Hz, 1H), 7.13 (dd, J = 4.8, 3.9 Hz, 1H), 5.79 (dd, J = 11.0, 2.3 Hz, 1H), 4.22-4.19 (m, 1H), 3.78-3.72 (m, 1H), 3.10 (dd, J = 11.4, 4.1 Hz, 1H), 2.22 (s, 3H), 2.18-2.14 (m, 1H), 1.94-1.64 (m, 4H);

### 6-chloro-8-(hex-1-yn-1-yl)-2-(5-methylfuran-2-yl)-9-(tetrahydro-2H-pyran-2-yl)-9H-purine (16g)

A target compound (16g) is obtained by the same synthetic method as for Compound (16a): ¹H NMR (600 MHz, CD₃OD) δ 13.23 (bs, 1 H, D₂O exchangeable), 7.76 (s, 1 H), 7.29 (bs, 2 H, D₂O exchangeable), 6.60 (dd, J = 1.5, 3.0 Hz, 1 H), 6.55 (q, J = 1.7 Hz, 1H), 4.18 (d, J = 11.5 Hz, 1H), 3.77-3.71 (m, 1H), 3.07-2.98 (m, 1H), 2.50-2.55 (m, 2 H), 2.13 (d, J = 7.4 Hz, 1H), 1.52-1.61 (m, 2 H), 1.40-1.51 (m, 2 H), 0.98 (t, J = 7.7 Hz, 3 H), 0.92 (t, J = 7.2 Hz, 3 H).

### 6-chloro-2-(4,5-dimethylfuran-2-yl)-8-(hex-1-yn-1-yl)-9-(tetrahydro-2H-pyran-2-yl)-9H-purine (16h)

A target compound (16h) is obtained by the same synthetic method as for Compound (16a): ¹H NMR (600 MHz, MeOD) δ 7.63 (dd, J = 1.2, 2.0 Hz, 1 H), 7.17 (d, J = 2.8 Hz, 1 H), 6.55 (dd, J = 1.6, 3.2 Hz, 1 H), 5.80 (q, J = 7.2 Hz, 1 H), 4.10-4.17 (m, 1 H), 3.70-3.78 (m, 1 H), 3.14 (bs, 3 H), 2.83-2.92 (m, 1 H), 2.51 (t, J = 6.8 Hz, 2 H), 1.59-1.67 (m, 2 H), 1.47-1.56 (m, 2 H), 1.41-1.43 (m, 1 H), 1.02 (t, J = 7.2 Hz, 3 H), 0.96 (t, J = 7.6 Hz, 3 H).

### 6-chloro-8-(hex-1-yn-1-yl)-2-(1-methyl-1H-imidazol-2-yl)-9-(tetrahydro-2H-pyran-2-yl)-9H-purine (16i)

A target compound (16i) is obtained by the same synthetic method as for Compound (16a): ¹H NMR (600 MHz, CD₃OD) δ 7.47 (dd, J = 1.2, 4.8 Hz, 1 H), 7.09 (dd, J = 3.6, 5.2 Hz, 1 H), 5.80 (q, J = 7.7 Hz, 1 H), 4.10-4.17 (m, 1 H), 3.70-3.78 (m, 1 H), 2.83-2.92 (m, 1 H), 2.53 (t, J = 6.8 Hz, 2 H), 2.03-2.18 (m, 1 H), 1.61-1.69 (m, 2 H), 1.49-1.58 (m, 2 H), 0.98 (t, J = 7.6 Hz, 3 H), 0.91 (t, J = 7.6 Hz, 3 H).

### 6-chloro-8-(hex-1-yn-1-yl)-2-(5-methylpyridin-2-yl)-9-(tetrahydro-2H-pyran-2-yl)-9H-purine (16j)

A target compound (16j) is obtained by the same synthetic method as for Compound (16a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 5.80 (dd, J = 2.3, 11.2 Hz, 1 H), 4.10-4.17 (m, 1H), 3.70-3.78 (m, 1 H), 2.83-2.92 (m, 1 H), 2.53 (t, J = 6.8 Hz, 2 H), 2.03-2.18 (m, 1 H), 1.61-1.69 (m, 2 H), 1.49-1.58 (m, 2 H), 0.98 (t, J = 7.6 Hz, 3 H), 0.91 (t, J = 7.6 Hz, 3 H).

### 6-chloro-8-(hex-1-yn-1-yl)-2-(6-methoxypyridin-2-yl)-9-(tetrahydro-2H-pyran-2-yl)-9H-purine (16k)

A target compound (16k) is obtained by the same synthetic method as for Compound (16a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 5.88 (dd, J = 11.3, 2.1 Hz, 1H), 4.23 (d, J = 11.5 Hz, 1H), 3.80-3.74 (m, 1H), 2.92 (qd, J = 12.1, 4.0 Hz, 1H), 3.50 (s, 3 H), 2.53 (t, J = 6.8 Hz, 2 H), 2.17-2.14 (m, 1H), 1.61-1.69 (m, 4 H), 1.49-1.58 (m, 2 H), 0.98 (t, J = 7.6 Hz, 3 H).

### 6-chloro-2-(6-chloropyridin-2-yl)-8-(hex-1-yn-1-yl)-9-(tetrahydro-2H-pyran-2-yl)-9H-purine (161)

A target compound (16l) is obtained by the same synthetic method as for Compound (16a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 5.75 (dd, J = 11.0, 2.3 Hz, 1 H), 4.17 (d, J = 11.9 Hz, 1 H), 3.74-3.69 (m, 4 H), 2.89 (dd, J = 11.9, 3.7 Hz, 1 H), 2.53 (t, J = 6.8 Hz, 2 H), 1.61-1.69 (m, 4 H), 1.49-1.58 (m, 4 H), 0.91 (t, J = 7.6 Hz, 3 H).

### 6-chloro-8-(hex-1-yn-1-yl)-2-(pyrazin-2-yl)-9-(tetrahydro-2H-pyran-2-yl)-9H-purine (16m)

A target compound (16m) is obtained by the same synthetic method as for Compound (16a): ¹H NMR (600 MHz, CD₃OD) δ 7.45 (dd, J = 1.2, 4.8 Hz, 1 H), 7.07 (dd, J = 3.6, 5.2 Hz, 1 H), 5.80 (dd, J = 2.3, 11.2 Hz, 1 H), 4.10-4.17 (m, 2 H), 3.70-3.78 (m, 2 H), 2.83-2.92 (m, 2 H), 2.53 (t, J = 6.8 Hz, 2 H), 1.61-1.69 (m, 4 H), 1.49-1.58 (m, 2 H), 0.98 (t, J = 7.6 Hz, 3 H).

### 5-(6-chloro-8-(hex-1-yn-1-yl)-9-(tetrahydro-2H-pyran-2-yl)-9H-purin-2-yl)thiazole (16n)

A target compound (16n) is obtained by the same synthetic method as for Compound (16a): ¹H NMR (600 MHz, CD₃OD) δ 7.47 (dd, J = 1.2, 4.8 Hz, 1 H), 7.09 (dd, J = 3.6, 5.2 Hz, 1 H), 5.80 (t, J = 7.2 Hz, 1H), 3.80 (s, 2H), 3.67-3.57 (m, 2H), 2.53 (t, J = 6.8 Hz, 2 H), 2.17-1.92 (m, 2H), 1.79-1.70 (m, 2H), 1.61-1.69 (m, 2 H), 1.58-1.55 (m, 2H), 1.49-1.52 (m, 2 H), 0.98 (t, J = 7.6 Hz, 3 H).

### 6-chloro-8-(hex-1-yn-1-yl)-2-(pyrimidin-2-yl)-9-(tetrahydro-2H-pyran-2-yl)-9H-purine (16o)

A target compound (16o) is obtained by the same synthetic method as for Compound (16a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 5.80 (t, J = 7.2 Hz, 1H), 3.80 (s, 2H), 3.67-3.57 (m, 2H), 2.53 (t, J = 6.8 Hz, 2 H), 2.17-1.92 (m, 2H), 1.78-1.72 (m, 2H), 1.61-1.69 (m, 2 H), 1.58-1.55 (m, 2H), 1.49-1.55 (m, 2 H), 0.98 (t, J = 7.6 Hz, 3 H).

### 4-(4-(6-chloro-8-(hex-1-yn-1-yl)-9-(tetrahydro-2H-pyran-2-yl)-9H-purin-2-yl)phenyl)morpholine (16p)

A target compound (16p) is obtained by the same synthetic method as for Compound (16a): ¹H NMR (600 MHz, CD₃OD) δ 7.87 (dd, J = 3.6, 5.2 Hz, 1 H), 7.47 (dd, J = 1.2, 4.8 Hz, 1 H), 7.26 (dd, J = 3.6, 5.2 Hz, 1 H), 7.09 (dd, J = 3.8, 5.2 Hz, 1 H), 5.80 (t, J = 7.2 Hz, 1H), 3.80 (s, 2H), 3.67-3.57 (m, 2H), 3.52-3.55 (m, 4 H), 3.21-3.23 (m, 4 H), 2.56 (t, J = 7.2 Hz, 2 H), 2.17-1.92 (m, 2H), 1.79-1.75 (m, 2H), 1.63-1.66 (m, 2 H), 1.58-1.55 (m, 2H), 1.45-1.51 (m, 2 H), 0.99 (t, J = 7.6 Hz, 3 H).

### 6-Chloro-2-(furan-2-yl)-8-(hex-1-yn-1-yl)-9H-purine (17a)

After 6-chloro-8-(hex-1-yn-1-yl)-2-iodo-9-(tetrahydro-2H-pyran-2-yl)-9H-purine (16a), which is a starting material, is dissolved in anhydrous EtOH (0.13 M) under nitrogen, pyridinium p-toluenesulfonate (0.2 eq) is added thereto, and the resulting mixture is stirred at room temperature for 4 hours. Triethylamine (1 mL) is added to the reaction mixture, the reaction is terminated, and then the resultant is evaporated. The reaction mixture is purified by column chromatography under the condition of hexane/EtOAc = 5/1 to 2/1 to obtain a target compound (17a); ¹H NMR (600 MHz, CD₃OD) δ 7.70 (dd, J = 0.8, 1.6 Hz, 1 H), 7.27 (dd, J = 0.8, 3.6 Hz, 1 H), 6.59 (dd, J = 1.6, 3.6 Hz, 1 H), 4.83 (bs, 1 H), 2.55 (t, J = 7.2 Hz, 2 H), 1.63-1.69 (m, 2 H), 1.48-1.57 (m, 2 H), 0.97 (t, J = 7.6 Hz, 3 H).

### 6-Chloro-2-(furan-2-yl)-8-(phenylethynyl)-9H-purine(17b)

A target compound (17b) is obtained by the same synthetic method as for Compound (17a): ¹H NMR (600 MHz, CDCl₃) δ 7.64 (s, 1H), 7.46-7.33 (m, 6H), 6.52 (s, 1H);

### 6-Chloro-2-(furan-2-yl)-8-(prop-1-yn-1-yl)-9H-purine(17c)

A target compound (17c) is obtained by the same synthetic method as for Compound (17a): ¹H NMR (600 MHz, CDCl₃) δ 7.67 (s, 1H), 7.41 (d, J = 3.2 Hz, 1H), 6.61 (q, J = 1.7 Hz, 1H), 1.99 (s, 3H);

### 6-Chloro-8-(hex-1-yn-1-yl)-2-(thiophen-2-yl)-9H-purine(17d)

A target compound (17d) is obtained by the same synthetic method as for Compound (17a): ¹H NMR (800 MHz, DMSO-d₆) d 14.24 (bs, 1 H), 7.89 (d, J = 3.5 Hz, 1 H), 7.74 (d, J = 4.8 Hz, 1 H), 7.18 (dd, J = 3.6, 4.8 Hz, 1 H), 2.57 (t, J = 7.0 Hz, 2 H), 1.55-1.59 (m, 2 H), 1.43-1.47 (m, 2 H), 0.92 (t, J = 7.2 Hz, 3 H).

### 6-Chloro-8-(phenylethynyl)-2-(thiophen-2-yl)-9H-purine(17e)

A target compound (17e) is obtained by the same synthetic method as for Compound (17a): ¹H NMR (600 MHz, CDCl₃) δ 8.06 (d, J = 3.2 Hz, 1H), 7.51-7.35 (m, 6H), 7.13 (t, J = 4.3 Hz, 1H);

### 6-Chloro-8-(prop-1-yn-1-yl)-2-(thiophen-2-yl)-9H-purine(17f)

A target compound (17f) is obtained by the same synthetic method as for Compound (17a): ¹H NMR (600 MHz, CD₃OD) δ 7.93 (d, J = 2.3 Hz, 1H), 7.56-7.55 (m, 1H), 7.12 (dd, J = 4.8, 3.9 Hz, 1H), 2.19 (s, 3H).

### 6-chloro-8-(hex-1-yn-1-yl)-2-(5-methylfuran-2-yl)-9H-purine (17g)

A target compound (17g) is obtained by the same synthetic method as for Compound (17a): ¹H NMR (600 MHz, CD₃OD) δ 13.23 (bs, 1 H, D₂O exchangeable), 7.76 (s, 1 H), 7.29 (bs, 2 H, D₂O exchangeable), 6.60 (dd, J = 1.5, 3.0 Hz, 1 H), 2.50-2.55 (m, 2 H, merged with solvent peak), 1.52-1.61 (m, 2 H), 1.40-1.51 (m, 2 H), 0.98 (t, J = 7.7 Hz, 3 H), 0.92 (t, J = 7.2 Hz, 3 H).

### 6-chloro-2-(4,5-dimethylfuran-2-yl)-8-(hex-1-yn-1-yl)-9H-purine (17h)

A target compound (17h) is obtained by the same synthetic method as for Compound (17a): ¹H NMR (600 MHz, MeOD) δ 7.63 (dd, J = 1.2, 2.0 Hz, 1 H), 7.17 (d, J = 2.8 Hz, 1 H), 6.55 (dd, J = 1.6, 3.2 Hz, 1 H), 3.14 (bs, 3 H), 2.51 (t, J = 6.8 Hz, 2 H), 1.59-1.67 (m, 2 H), 1.47-1.56 (m, 2 H), 1.02 (t, J = 7.2 Hz, 3 H), 0.96 (t, J = 7.6 Hz, 3 H).

### 6-chloro-8-(hex-1-yn-1-yl)-2-(1-methyl-1H-imidazol-2-yl)-9H-purine (17i)

A target compound (17i) is obtained by the same synthetic method as for Compound (17a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H), 0.91 (t, J = 7.6 Hz, 3 H).

### 6-chloro-8-(hex-1-yn-1-yl)-2-(5-methylpyridin-2-yl)-9H-purine (17j)

A target compound (17j) is obtained by the same synthetic method as for Compound (17a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H), 0.91 (t, J = 7.6 Hz, 3 H).

### 6-chloro-8-(hex-1-yn-1-yl)-2-(6-methoxypyridin-2-yl)-9H-purine (17k)

A target compound (17k) is obtained by the same synthetic method as for Compound (17a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 3.50 (s, 3 H), 2.53 (t, J = 6.8 Hz, 2 H), 1.61-1.69 (m, 2 H), 1.49-1.58 (m, 2 H), 0.98 (t, J = 7.6 Hz, 3 H).

### 6-chloro-2-(6-chloropyridin-2-yl)-8-(hex-1-yn-1-yl)-9H-purine (17l)

A target compound (17l) is obtained by the same synthetic method as for Compound (17a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.91 (t, J = 7.6 Hz, 3 H).

### 6-chloro-8-(hex-1-yn-1-yl)-2-(pyrazin-2-yl)-9H-purine (17m)

A target compound (17m) is obtained by the same synthetic method as for Compound (17a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 5-(6-chloro-8-(hex-1-yn-1-yl)-9H-purin-2-yl)thiazole (17n)

A target compound (17n) is obtained by the same synthetic method as for Compound (17a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 6-chloro-8-(hex-1-yn-1-yl)-2-(pyrimidin-2-yl)-9H-purine (17o)

A target compound (17o) is obtained by the same synthetic method as for Compound (17a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 4-(4-(6-chloro-8-(hex-1-yn-1-yl)-9H-purin-2-yl)phenyl)morpholine (17p)

A target compound (17p) is obtained by the same synthetic method as for Compound (17a): ¹H NMR (600 MHz, CD₃OD) δ 7.87 (dd, J = 3.6, 5.2 Hz, 1 H), 7.47 (dd, J = 1.2, 4.8 Hz, 1 H), 7.26 (dd, J = 3.6, 5.2 Hz, 1 H), 7.09 (dd, J = 3.8, 5.2 Hz, 1 H), 3.52-3.55 (m, 4 H), 3.21-3.23 (m, 4 H), 2.56 (t, J = 7.2 Hz, 2 H), 1.63-1.66 (m, 2 H), 1.45-1.51 (m, 2 H), 0.99 (t, J = 7.6 Hz, 3 H).

### 2-(Furan-2-yl)-8-(hex-1-yn-1-yl)-9H-purin-6-amine (18a)

After anhydrous EtOH (0.3 M) is added to Starting Material (17a) under nitrogen and the resulting mixture is stirred, triethylamine (4 eq) and amine-saturated t-BuOH (100 eq) are added thereto and the resulting mixture is stirred at room temperature for 24 hours. The resulting residue is separated by column chromatography under the condition of DCM/MeOH to obtain a target compound (18a); ¹H NMR (600 MHz, DMSO-d₆) δ 13.23 (bs, 1 H, D₂O exchangeable), 7.76 (s, 1 H), 7.29 (bs, 2 H, D₂O exchangeable), 7.03 (d, J = 3.0 Hz, 1 H), 6.60 (dd, J = 1.5, 3.0 Hz, 1 H), 2.50-2.55 (m, 2 H, merged with solvent peak), 1.52-1.61 (m, 2 H), 1.40-1.51 (m, 2 H), 0.92 (t, J = 7.2 Hz, 3H).

### 2-(Furan-2-yl)-8-(phenylethynyl)-9H-purin-6-amine (18b)

A target compound (18b) is obtained by the same synthetic method as for Compound (18a): ¹H NMR (600 MHz, CD₃OD) δ 7.69 (d, J = 0.9 Hz, 1H), 7.65 (dd, J = 7.8, 1.8 Hz, 2H), 7.50-7.44 (m, 3H), 7.21 (d, J = 3.2 Hz, 1H), 6.60 (q, J = 1.8 Hz, 1H);

### 2-(Furan-2-yl)-8-(hex-1-yn-1-yl)-N-methyl-9H-purin-6-amine (18c)

After anhydrous EtOH (0.3 M) is added to Starting Material (17a) under nitrogen and the resulting mixture is stirred, triethylamine (4 eq) and methylamine (3 eq) are added thereto and the resulting mixture is stirred at room temperature for 24 hours. After it is confirmed that the reaction is completed, the resulting residue is separated by column chromatography under the condition of DCM/MeOH to obtain a target compound (18c); ¹H NMR (600 MHz, MeOD) δ 7.63 (dd, J = 1.2, 2.0 Hz, 1 H), 7.17 (d, J = 2.8 Hz, 1 H), 6.55 (dd, J = 1.6, 3.2 Hz, 1 H), 3.14 (bs, 3 H), 2.51 (t, J = 6.8 Hz, 2 H), 1.59-1.67 (m, 2 H), 1.47-1.56 (m, 2 H), 0.96 (t, J = 7.6 Hz, 3 H).

### 2-(Furan-2-yl)-N-methyl-8-(phenylethynyl)-9H-purin-6-amine (18d)

A target compound (18d) is obtained by the same synthetic method as for Compound (18c): ¹H NMR (600 MHz, CD₃OD) δ 7.88 (d, J = 3.2 Hz, 1H), 7.65 (dd, J = 7.5, 1.6 Hz, 2H), 7.50-7.44 (m, 4H), 7.11 (t, J = 4.3 Hz, 1H);

### 2-(Furan-2-yl)-8-(hex-1-yn-1-yl)-N-(3-iodobenzyl)-9H-purin-6-amine (18e)

After anhydrous EtOH (0.3 M) is added to Starting Material (17a) under nitrogen and the resulting mixture is stirred, triethylamine (4 eq) and 3-iodobenzyl amine (3 eq) are added thereto and the resulting mixture is stirred at room temperature for 24 hours. After it is confirmed that the reaction is completed, the resulting residue is separated by column chromatography under the condition of DCM/MeOH to obtain a target compound (18e);¹H NMR (600 MHz, CDCl₃) δ 13.19 (s, 1 H), 7.77 (s, 1 H), 7.60-7.62 (m, 1 H), 7.58 (d, J = 8.0 Hz, 1 H), 7.36 (d, J = 8.4 Hz, 1 H), 7.22 (d, J = 0.8, 3.6 Hz, 1 H), 7.04 (t, J = 8.0 Hz, 1 H), 6.53 (dd, J = 2.0, 3.6 Hz, 1 H), 6.37 (s, 1 H), 4.86 (s, 2 H), 2.29 (t, J = 7.2 Hz, 2 H), 1.45-1.53 (m, 2 H), 1.32-1.44 (m, 2 H), 0.87 (t, J = 7.2 Hz, 3 H).

### 2-(Furan-2-yl)-N-(3-iodobenzyl)-8-(phenylethynyl)-9H-purin-6-amine (18f)

A target compound (18f) is obtained by the same synthetic method as for Compound (18e): ¹H NMR (600 MHz, CD₃OD) δ 7.88 (s, 1H), 7.68 (t, J = 0.9 Hz, 1H), 7.60-7.56 (m, 3H), 7.46-7.40 (m, 4H), 7.21-7.20 (m, 1H), 7.07 (t, J = 7.8 Hz, 1H), 6.58 (q, J = 1.7 Hz, 1H), 4.81 (s, 2H);

### 8-(Hex-1-yn-1-yl)-2-(thiophen-2-yl)-9H-purin-6-amine (18g)

A target compound (18g) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 8-(Phenylethynyl)-2-(thiophen-2-yl)-9H-purin-6-amine (18h)

A target compound (18h) is obtained by the same synthetic method as for Compound (18a): ¹H NMR (600 MHz, CD₃OD) δ 7.88 (d, J = 3.2 Hz, 1H), 7.65 (dd, J = 7.5, 1.6 Hz, 2H), 7.50-7.44 (m, 4H), 7.11 (t, J = 4.3 Hz, 1H).

### 8-(Hex-1-yn-1-yl)-N-methyl-2-(thiophen-2-yl)-9H-purin-6-amine (18i)

A target compound (18i) is obtained by the same synthetic method as for Compound (18c): ¹H NMR (600 MHz, CD₃OD) d 7.86 (dd, J = 0.8, 3.6 Hz, 1 H), 7.46 (dd, J = 1.2, 5.2 Hz, 1 H), 7.09 (dd, J = 3.6, 4.8 Hz, 1 H), 3.17 (s, 3 H), 2.52 (t, J = 6.8 Hz, 2 H), 1.58-1.69 (m, 2 H), 1.48-1.58 (m, 2 H), 0.97 (t, J = 7.6 Hz, 3 H).

### N-methyl-8-(phenylethynyl)-2-(thiophen-2-yl)-9H-purin-6-amine (18j)

A target compound (18j) is obtained by the same synthetic method as for Compound (18c): ¹H NMR (600 MHz, CDCl₃) δ 8.05 (d, J = 2.7 Hz, 1H), 7.31-7.29 (m, 2H), 7.22 (t, J = 7.5 Hz, 2H), 7.15 (d, J = 7.3 Hz, 2H), 7.02 (dd, J = 4.8, 3.9 Hz, 1H), 6.39 (s, 1H), 3.28 (s, 3H);

### 8-(Hex-1-yn-1-yl)-N-(3-iodobenzyl)-2-(thiophen-2-yl)-9H-purin-6-amine (18k)

A target compound (18k) is obtained by the same synthetic method as for Compound (18e): ¹H NMR (600 MHz, CDCl₃) δ 7.92 (d, J = 2.5 Hz, 1 H), 7.76 (s, 1 H), 7.55 (d, J = 7.8 Hz, 1 H), 7.41 (d, J = 4.9 Hz, 1 H), 7.36 (d, J = 7.6 Hz, 1 H), 7.10 (t, J = 3.9 Hz, 1 H), 7.01 (t, J = 7.7 Hz, 1 H), 6.67 (bs, 1 H), 4.83 (bs, 2 H), 2.18 (t, J = 6.9 Hz, 2 H), 1.27-1.45 (m, 4 H), 0.84 (t, J = 7.2 Hz, 3 H).

### N-(3-iodobenzyl)-8-(phenylethynyl)-2-(thiophen-2-yl)-9H-purin-6-amine (181)

A target compound (18l) is obtained by the same synthetic method as for Compound (18e): ¹H NMR (600 MHz, CD₃OD) δ 7.89 (s, 1H), 7.85 (d, J = 3.2 Hz, 1H), 7.58 (t, J = 8.3 Hz, 3H), 7.43 (dt, J = 21.4, 6.4 Hz, 5H), 7.10-7.04 (m, 2H), 4.77 (s, 2H);

### 8-(hex-1-yn-1-yl)-2-(5-methylfuran-2-yl)-9H-purin-6-amine (18m)

A target compound (18m) is obtained by the same synthetic method as for Compound (18a): ¹H NMR (600 MHz, CD₃OD) δ 13.23 (bs, 1 H, D₂O exchangeable), 7.76 (s, 1 H), 7.29 (bs, 2 H, D₂O exchangeable), 6.60 (dd, J = 1.5, 3.0 Hz, 1 H), 2.50-2.55 (m, 2 H, merged with solvent peak), 1.52-1.61 (m, 2 H), 1.40-1.51 (m, 2 H), 0.98 (t, J = 7.7 Hz, 3 H), 0.92 (t, J = 7.2 Hz, 3 H).

### 2-(4,5-dimethylfuran-2-yl)-8-(hex-1-yn-1-yl)-9H-purin-6-amine (18n)

A target compound (18n) is obtained by the same synthetic method as for Compound (18a): ¹H NMR (600 MHz, MeOD) δ 7.63 (dd, J = 1.2, 2.0 Hz, 1 H), 7.17 (d, J = 2.8 Hz, 1 H), 6.55 (dd, J = 1.6, 3.2 Hz, 1 H), 3.14 (bs, 3 H), 2.51 (t, J = 6.8 Hz, 2 H), 1.59-1.67 (m, 2 H), 1.47-1.56 (m, 2 H), 1.02 (t, J = 7.2 Hz, 3 H), 0.96 (t, J = 7.6 Hz, 3 H).

### 8-(hex-1-yn-1-yl)-2-(1-methyl-1H-imidazol-2-yl)-9H-purin-6-amine (18o)

A target compound (18o) is obtained by the same synthetic method as for Compound (18a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H), 0.91 (t, J = 7.6 Hz, 3 H).

### 8-(hex-1-yn-1-yl)-2-(5-methylpyridin-2-yl)-9H-purin-6-amine (18p)

A target compound (18p) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H), 0.91 (t, J = 7.6 Hz, 3 H).

### 8-(hex-1-yn-1-yl)-2-(6-methoxypyridin-2-yl)-9H-purin-6-amine (18q)

A target compound (18q) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 3.50 (s, 3 H), 2.53 (t, J = 6.8 Hz, 2 H), 1.61-1.69 (m, 2 H), 1.49-1.58 (m, 2 H), 0.98 (t, J = 7.6 Hz, 3 H).

### 2-(6-chloropyridin-2-yl)-8-(hex-1-yn-1-yl)-9H-purin-6-amine (18r)

A target compound (18r) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.91 (t, J = 7.6 Hz, 3 H).

### 8-(hex-1-yn-1-yl)-2-(pyrazin-2-yl)-9H-purin-6-amine (18s)

A target compound (18s) is obtained by the same synthetic method as for Compound (18a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 8-(hex-1-yn-1-yl)-2-(thiazol-5-yl)-9H-purin-6-amine (18t)

A target compound (18t) is obtained by the same synthetic method as for Compound (18a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 8-(hex-1-yn-1-yl)-2-(pyrimidin-2-yl)-9H-purin-6-amine (18u)

A target compound (18u) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 8-(hex-1-yn-1-yl)-2-(4-morpholinophenyl)-9H-purin-6-amine (18v)

A target compound (18v) is obtained by the same synthetic method as for Compound (18a): ¹H NMR (600 MHz, CD₃OD) δ 7.87 (dd, J = 3.6, 5.2 Hz, 1 H), 7.47 (dd, J = 1.2, 4.8 Hz, 1 H), 7.26 (dd, J = 3.6, 5.2 Hz, 1 H), 7.09 (dd, J = 3.8, 5.2 Hz, 1 H), 3.52-3.55 (m, 4 H), 3.21-3.23 (m, 4 H), 2.56 (t, J = 7.2 Hz, 2 H), 1.63-1.66 (m, 2 H), 1.45-1.51 (m, 2 H), 0.99 (t, J = 7.6 Hz, 3 H).

### Preparation Example 7: Synthesis of 18aa to 18ar

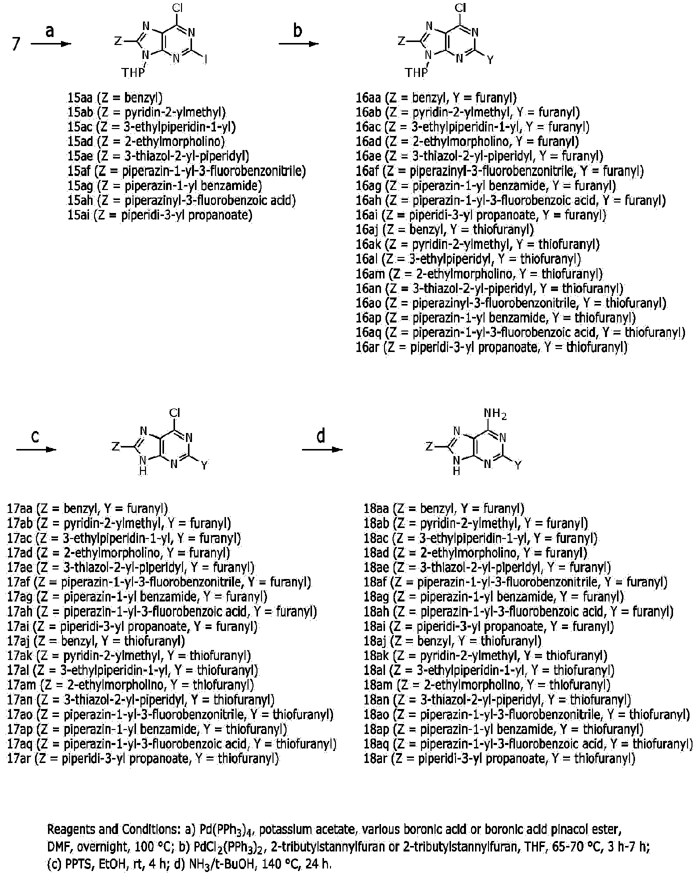

### 8-benzyl-6-chloro-2-iodo-9-(tetrahydro-2H-pyran-2-yl)-9H-purine (15aa)

After Starting Material (7) (5 g, 10.2 mmol) is dissolved in DMF (30 mL) under nitrogen, Pd(PPh₃)₄, potassium acetate (0.01 eq), and benzylboronic acid (10.7 mmol) are added thereto, and the resulting mixture is stirred at 100°C for 15 hours. After it is confirmed that the reaction is completed, the reaction mixture is extracted with diethyl ether (50 mL), and then dried over MgSO₄ and concentrated under reduced pressure. The resulting residue is purified by column chromatography under the condition of DCM/MeOH = 100/1 to obtain a target compound (15aa) (3 g, 6.63 mmol, 65%): ¹H NMR (600 MHz, CDCl₃) δ 7.30-7.23 (m, 5H), 5.80 (t, J = 7.2 Hz, 1H), 3.80 (s, 2H), 3.67-3.57 (m, 2H), 2.17-1.92 (m, 2H), 1.79-1.64 (m, 2H), 1.58-1.55 (m, 2H).

### 6-chloro-2-iodo-8-(pyridin-2-ylmethyl)-9-(tetrahydro-2H-pyran-2-yl)-9H-purine (15ab)

A target compound (15ab) is obtained by the same synthetic method as for Compound (15aa): ¹H NMR (600 MHz, CDCl₃) δ 8.51 (d, 1H), 7.67 (t, J = 7.2 Hz, 1H), 7.15 (q, J = 7.7 Hz, 2H), 6.00 (t, J = 7.7 Hz, 1H), 4.14 (s, 2H), 3.67-3.55 (m, 2H), 2.20-1.92 (m, 2H), 1.71-1.62 (m, 2H), 1.55-1.43 (m, 2H).

### 6-chloro-8-(3-ethylpiperidin-1-yl)-2-iodo-9-(tetrahydro-2H-pyran-2-yl)-9H-purine (15ac)

A target compound (15ac) is obtained by the same synthetic method as for Compound (15aa): ¹H NMR (600 MHz, CDCl₃) δ 5.91 (t, J = 7.7 Hz, 1H), 3.80-3.65 (m, 2H), 3.29-3.25 (m, 2H), 2.20-1.98 (m, 2H), 1.78-1.65 (m, 2H), 1.58 (m, 2H), 1.56-1.53 (m, 2H), 1.50-1.42 (m, 2H), 1.40 (m, 1H), 1.31 (m, 2H), 1.00 (t, J = 7.2 Hz, 1H).

### 4-(6-chloro-2-iodo-9-(tetrahydro-2H-pyran-2-yl)-9H-purin-8-yl)-2-ethylmorpholine (15ad)

A target compound (15ad) is obtained by the same synthetic method as for Compound (15aa): ¹H NMR (600 MHz, CDCl₃) δ 5.87 (t, J = 7.4 Hz, 1H), 3.78-3.62 (m, 2H), 3.29-3.25 (m, 2H), 2.20-1.98 (m, 2H), 1.77-1.61 (m, 2H), 1.58 (m, 2H), 1.57-1.52 (m, 2H), 1.43 (m, 1H), 1.33 (m, 2H), 0.98 (t, J = 7.2 Hz, 1H).

### 2-(1-(6-chloro-2-iodo-9-(tetrahydro-2H-pyran-2-yl)-9H-purin-8-yl)piperidin-3-yl)thiazole (15ae)

A target compound (15ae) is obtained by the same synthetic method as for Compound (15aa): ¹H NMR (600 MHz, CDCl₃) δ 7.69 (d, 1H), 7.24 (d, 1H), 6.00 (t, J = 7.7 Hz, 1H), 3.30-3.24 (m, 2H), 2.80 (m, 1H), 2.20-1.94 (m, 2H), 1.92-1.87 (m, 2H), 1.75-1.64 (m, 2H), 1.58-1.55 (m, 2H). 1.53-1.43( m, 2H).

### 4-(4-(6-chloro-2-iodo-9-(tetrahydro-2H-pyran-2-yl)-9H-purin-8-yl)piperazin-1-yl)-3-fluorobenzonitrile (15af)

A target compound (15af) is obtained by the same synthetic method as for Compound (15aa): ¹H NMR (600 MHz, CDCl₃) δ 7.12 (d, 1H), 7.04 (d, 1H), 6.90 (d, 1H), 5.99 (t, J = 7.2 Hz, 1H), 3.70-3.61 (m, 2H), 3.51-3.49 (m, 4H), 3.30-3.28 (m, 4H), 2.20-1.98 (m, 2H), 1.80-1.67 (m, 2H), 1.60-1.54 (m, 2H).

### 4-(4-(6-chloro-2-iodo-9-(tetrahydro-2H-pyran-2-yl)-9H-purin-8-yl)piperazin-1-yl)benzamide (15ag)

A target compound (15ag) is obtained by the same synthetic method as for Compound (15aa): ¹H NMR (600 MHz, CDCl₃) δ 7.70 (s, 1H), 7.52 (d, 1H), 7.50 (d, 1H), 7.15 (d, 1H), 7.12 (d, 1H), 5.98 (t, J = 7.4 Hz, 1H), 3.70-3.67 (m, 2H), 3.60-3.57 (m, 4H), 3.30-3.28 (m, 4H), 2.15-2.12 (m, 2H), 1.80-1.75 (m, 2H), 1.59-1.54 (m, 2H).

### 4-(4-(6-chloro-2-iodo-9-(tetrahydro-2H-pyran-2-yl)-9H-purin-8-yl)piperazin-1-yl)-3-fluorobenzoic acid (15ah)

A target compound (15ah) is obtained by the same synthetic method as for Compound (15aa): ¹H NMR (600 MHz, CDCl₃) δ 12.7 (s, 1H), 7.70 (d, 1H), 7.58 (d, 1H), 6.99 (d, 1H), 6.01 (t, J = 7.4 Hz, 1H), 3.70-3.65 (m, 2H), 3.60-3.58 (m, 24), 3.30-3.28 (m, 4H), 2.15-2.11 (m, 2H), 1.80-1.77 (m, 2H), 1.57-1.46 (m, 2H).

### methyl 3-(1-(6-chloro-2-iodo-9-(tetrahydro-2H-pyran-2-yl)-9H-purin-8-yl)piperidin-3-yl)propanoate (15ai)

A target compound (15ai) is obtained by the same synthetic method as for Compound (15aa): ¹H NMR (600 MHz, CDCl₃) δ 5.99 (t, J = 7.7 Hz, 1H), 3.70-3.65 (m, 2H), 3.62 (s, 3H), 3.30-3.25 (m, 2H), 2.35 (m, 2H), 2.20-1.97 (m, 2H), 1.88 (q, J = 7.4 Hz, 2H), 1.76-1.67 (m, 2H), 1.58-1.53 (m, 4H), 1.52-1.31 (m, 2H).

### 8-benzyl-6-chloro-2-(furan-2-yl)-9-(tetrahydro-2H-pyran-2-yl)-9H-purine (16aa)

A target compound (16aa) is obtained by the same synthetic method as for Compound (8a): ¹H NMR (600 MHz, CDCl₃) δ 8.39 (d, 1H), 7.35 (m, 1H), 7.29-7.22 (m, 6H), 5.81 (t, J = 7.2 Hz, 1H), 3.85 (s, 2H), 3.67-3.57 (m, 2H), 2.17-1.92 (m, 2H), 1.79-1.64 (m, 2H), 1.58-1.56 (m, 2H).

### 6-chloro-2-(furan-2-yl)-8-(pyridin-2-ylmethyl)-9-(tetrahydro-2H-pyran-2-yl)-9H-purine (16ab)

A target compound (16ab) is obtained by the same synthetic method as for Compound (8a): ¹H NMR (600 MHz, CDCl₃) δ 8.51 (d, 1H), 8.39 (d, 1H), 7.67 (t, J = 7.2 Hz, 1H), 7.30 (d, 1H), 7.15 (q, J = 7.7 Hz, 2H), 6.75 (d, 1), 6.00 (t, J = 7.7 Hz, 1H), 4.14 (s, 2H), 3.67-3.55 (m, 2H), 2.20-1.92 (m, 2H), 1.71-1.62 (m, 2H), 1.55-1.43 (m, 2H).

### 6-chloro-8-(3-ethylpiperidin-1-yl)-2-(furan-2-yl)-9-(tetrahydro-2H-pyran-2-yl)-9H-purine (16ac)

A target compound (16ac) is obtained by the same synthetic method as for Compound (8a): ¹H NMR (600 MHz, CDCl₃) δ 8.40 (d, 1H), 7.30 (d, 1H), 6.75 (d, 1H), 5.95 (t, J = 7.4 Hz, 1H), 3.85-3.65 (m, 2H), 3.29-3.25 (m, 2H), 2.20-1.99 (m, 2H), 1.78-1.65 (m, 2H), 1.55 (m, 2H), 1.56-1.53 (m, 2H), 1.50-1.43 (m, 2H), 1.42 (m, 1H), 1.31 (m, 2H), 0.99 (t, J = 7.2 Hz, 1H).

### 4-(6-chloro-2-(furan-2-yl)-9-(tetrahydro-2H-pyran-2-yl)-9H-purin-8-yl)-2-ethylmorpholine (16ad)

A target compound (16ad) is obtained by the same synthetic method as for Compound (8a): ¹H NMR (600 MHz, CDCl₃) δ 8.35 (d, 1H), 7.25 (d, 1H), 6.80 (d, 1H), 5.87 (t, J = 7.2 Hz, 1H), 3.78-3.62 (m, 2H), 3.29-3.25 (m, 2H), 2.20-1.98 (m, 2H), 1.77-1.61 (m, 2H), 1.58 (m, 2H), 1.57-1.52 (m, 2H), 1.43 (m, 1H), 1.33 (m, 2H), 0.98 (t, J = 7.2 Hz, 1H).

### 2-(1-(6-chloro-2-(furan-2-yl)-9-(tetrahydro-2H-pyran-2-yl)-9H-purin-8-yl)piperidin-3-yl)thiazole (16ae)

A target compound (16ae) is obtained by the same synthetic method as for Compound (8a): ¹H NMR (600 MHz, CDCl₃) δ 8.35 (d, 1H), 7.69 (d, 1H), 7.30 (d, 1H), 7.24 (d, 1H), 6.80 (d, 1H), 6.00 (t, J = 7.7 Hz, 1H), 3.30-3.24 (m, 2H), 2.80 (m, 1H), 2.20-1.94 (m, 2H), 1.92-1.87 (m, 2H), 1.75-1.64 (m, 2H), 1.58-1.55 (m, 2H). 1.53-1.43( m, 2H).

### 4-(4-(6-chloro-2-(furan-2-yl)-9-(tetrahydro-2H-pyran-2-yl)-9H-purin-8-yl)piperazin-1-yl)-3-fluorobenzonitrile (16af)

A target compound (16af) is obtained by the same synthetic method as for Compound (8a): ¹H NMR (600 MHz, CDCl₃) δ 8.40 (d, 1H), 7.30 (d, 1H), 7.12 (d, 1H), 7.04 (d, 1H), 6.90 (d, 1H), 6.85 (d, 1H), 5.99 (t, J = 7.2 Hz, 1H), 3.70-3.61 (m, 2H), 3.51-3.49 (m, 4H), 3.30-3.28 (m, 4H), 2.20-1.98 (m, 2H), 1.80-1.67 (m, 2H), 1.60-1.54 (m, 2H).

### 4-(4-(6-chloro-2-(furan-2-yl)-9-(tetrahydro-2H-pyran-2-yl)-9H-purin-8-yl)piperazin-1-yl)benzamide (16ag)

A target compound (16ag) is obtained by the same synthetic method as for Compound (8a): ¹H NMR (600 MHz, CDCl₃) δ 8.35 (d, 1H), 7.70 (s, 1H), 7.52 (d, 1H), 7.50 (d, 1H), 7.38 (d, 1H), 7.15 (d, 1H), 7.12 (d, 1H), 6.87 (d, 1H), 5.98 (t, J = 7.4 Hz, 1H), 3.70-3.67 (m, 2H), 3.60-3.57 (m, 4H), 3.30-3.28 (m, 4H), 2.15-2.12 (m, 2H), 1.80-1.75 (m, 2H), 1.59-1.54 (m, 2H).

### 4-(4-(6-chloro-2-(furan-2-yl)-9-(tetrahydro-2H-pyran-2-yl)-9H-purin-8-yl)piperazin-1-yl)-3-fluorobenzoic acid (16ah)

A target compound (16ah) is obtained by the same synthetic method as for Compound (8a): ¹H NMR (600 MHz, CDCl₃) δ 12.7 (s, 1H), 8.35 (d, 1H), 7.70 (d, 1H), 7.58 (d, 1H), 7.33 (d, 1H), 6.99 (d, 1H), 6.89 (d, 1H), 6.01 (t, J = 7.4 Hz, 1H), 3.70-3.65 (m, 2H), 3.60-3.58 (m, 24), 3.30-3.28 (m, 4H), 2.15-2.11 (m, 2H), 1.80-1.77 (m, 2H), 1.57-1.46 (m, 2H).

### methyl 3-(1-(6-chloro-2-(furan-2-yl)-9-(tetrahydro-2H-pyran-2-yl)-9H-purin-8-yl)piperidin-3-yl)propanoate (16ai)

A target compound (16ai) is obtained by the same synthetic method as for Compound (8a): ¹H NMR (600 MHz, CDCl₃) δ 8.33 (d, 1H), 7.35 (d, 1H), 6.90 (d, 1H), 5.99 (t, J = 7.2 Hz, 1H), 3.65-3.63 (m, 2H), 3.62 (s, 3H), 3.30-3.25 (m, 2H), 2.32 (m, 2H), 2.18-1.97 (m, 2H), 1.88 (q, J = 7.4 Hz, 2H), 1.78-1.67 (m, 2H), 1.59-1.53 (m, 4H), 1.52-1.31 (m, 2H).

### 8-benzyl-6-chloro-9-(tetrahydro-2H-pyran-2-yl)-2-(thiophen-2-yl)-9H-purine (16aj)

A target compound (16aj) is obtained by the same synthetic method as for Compound (8a): ¹H NMR (600 MHz, CDCl₃) δ 8. (d, 1H), 7.32 (m, 1H), 7.29-7.21 (m, 6H), 5.81 (t, J = 7.7 Hz, 1H), 3.80 (s, 2H), 3.67-3.57 (m, 2H), 2.18-1.92 (m, 2H), 1.79-1.64 (m, 2H), 1.58-1.56 (m, 2H).

### 6-chloro-8-(pyridin-2-ylmethyl)-9-(tetrahydro-2H-pyran-2-yl)-2-(thiophen-2-yl)-9H-purine (16ak)

A target compound (16ak) is obtained by the same synthetic method as for Compound (8b): ¹H NMR (600 MHz, CDCl₃) δ 8.48 (d, 1H), 8.37 (d, 1H), 7.67 (t, J = 7.4 Hz, 1H), 7.28 (d, 1H), 7.15 (q, J = 7.7 Hz, 2H), 6.75 (d, 1), 5.89 (t, J = 7.4 Hz, 1H), 4.16 (s, 2H), 3.67-3.55 (m, 2H), 2.20-1.92 (m, 2H), 1.71-1.62 (m, 2H), 1.57-1.43 (m, 2H).

### 6-chloro-8-(3-ethylpiperidin-1-yl)-9-(tetrahydro-2H-pyran-2-yl)-2-(thiophen-2-yl)-9H-purine (16al)

A target compound (16al) is obtained by the same synthetic method as for Compound (8b): ¹H NMR (600 MHz, CDCl₃) δ 8.48 (d, 1H), 7.38 (d, 1H), 6.81 (d, 1H), 5.98 (t, J = 7.7 Hz, 1H), 3.87-3.65 (m, 2H), 3.29-3.25 (m, 2H), 2.20-1.95 (m, 2H), 1.78-1.65 (m, 2H), 1.55 (m, 2H), 1.56-1.53 (m, 2H), 1.50-1.43 (m, 2H), 1.42 (m, 1H), 1.31 (m, 2H), 0.97 (t, J = 7.7 Hz, 1H).

### 4-(6-chloro-9-(tetrahydro-2H-pyran-2-yl)-2-(thiophen-2-yl)-9H-purin-8-yl)-2-ethylmorpholine (16am)

A target compound (16am) is obtained by the same synthetic method as for Compound (8b): ¹H NMR (600 MHz, CDCl₃) δ 8.40 (d, 1H), 7.35 (d, 1H), 6.90 (d, 1H), 5.90 (t, J = 7.7 Hz, 1H), 3.78-3.62 (m, 2H), 3.29-3.25 (m, 2H), 2.20-1.98 (m, 2H), 1.77-1.61 (m, 2H), 1.58 (m, 2H), 1.57-1.52 (m, 2H), 1.43 (m, 1H), 1.33 (m, 2H), 1.00 (t, J = 7.2 Hz, 1H).

### 2-(1-(6-chloro-9-(tetrahydro-2H-pyran-2-yl)-2-(thiophen-2-yl)-9H-purin-8-yl)piperidin-3-yl)thiazole (16an)

A target compound (16an) is obtained by the same synthetic method as for Compound (8b): ¹H NMR (600 MHz, CDCl₃) δ 8.38 (d, 1H), 7.70 (d, 1H), 7.45 (d, 1H), 7.31 (d, 1H), 6.70 (d, 1H), 6.00 (t, J = 7.7 Hz, 1H), 3.30-3.24 (m, 2H), 2.80 (m, 1H), 2.20-1.94 (m, 2H), 1.92-1.87 (m, 2H), 1.76-1.64 (m, 2H), 1.59-1.52 (m, 2H). 1.53-1.43( m, 2H).

### 4-(4-(6-chloro-9-(tetrahydro-2H-pyran-2-yl)-2-(thiophen-2-yl)-9H-purin-8-yl)piperazin-1-yl)-3-fluorobenzonitrile (16ao)

A target compound (16ao) is obtained by the same synthetic method as for Compound (8b): ¹H NMR (600 MHz, CDCl₃) δ 8.38 (d, 1H), 7.27 (d, 1H), 7.13 (d, 1H), 7.07 (d, 1H), 6.87 (d, 1H), 6.86 (d, 1H), 5.97 (t, J = 7.7 Hz, 1H), 3.77-3.68 (m, 2H), 3.51-3.49 (m, 4H), 3.30-3.28 (m, 4H), 2.20-1.98 (m, 2H), 1.80-1.71 (m, 2H), 1.60-1.54 (m, 2H).

### 4-(4-(6-chloro-9-(tetrahydro-2H-pyran-2-yl)-2-(thiophen-2-yl)-9H-purin-8-yl)piperazin-1-yl)benzamide (16ap)

A target compound (16ap) is obtained by the same synthetic method as for Compound (8b): ¹H NMR (600 MHz, CDCl₃) δ 8.41 (d, 1H), 7.70 (s, 1H), 7.51 (d, 1H), 7.50 (d, 1H), 7.35 (d, 1H), 7.15 (d, 1H), 7.12 (d, 1H), 6.89 (d, 1H), 5.99 (t, J = 7.4 Hz, 1H), 3.70-3.67 (m, 2H), 3.60-3.57 (m, 4H), 3.30-3.22 (m, 4H), 2.16-2.12 (m, 2H), 1.81-1.75 (m, 2H), 1.59-1.54 (m, 2H).

### 4-(4-(6-chloro-9-(tetrahydro-2H-pyran-2-yl)-2-(thiophen-2-yl)-9H-purin-8-yl)piperazin-1-yl)-3-fluorobenzoic acid (16aq)

A target compound (16aq) is obtained by the same synthetic method as for Compound (8b): ¹H NMR (600 MHz, CDCl₃) δ 12.5 (s, 1H), 8.32 (d, 1H), 7.68 (d, 1H), 7.59 (d, 1H), 7.31 (d, 1H), 6.99 (d, 1H), 6.89 (d, 1H), 6.01 (t, J = 7.7 Hz, 1H), 3.70-3.68 (m, 2H), 3.60-3.58 (m, 2H), 3.30-3.28 (m, 4H), 2.15-2.11 (m, 2H), 1.88-1.80 (m, 2H), 1.57-1.46 (m, 2H).

### methyl 3-(1-(6-chloro-9-(tetrahydro-2H-pyran-2-yl)-2-(thiophen-2-yl)-9H-purin-8-yl)piperidin-3-yl)propanoate (16ar)

A target compound (16ar) is obtained by the same synthetic method as for Compound (8b): ¹H NMR (600 MHz, CDCl₃) δ 8.38 (d, 1H), 7.37 (d, 1H), 6.92 (d, 1H), 6.01 (t, J = 7.4 Hz, 1H), 3.67-3.63 (m, 2H), 3.61 (s, 3H), 3.30-3.25 (m, 2H), 2.32 (m, 2H), 2.17-1.97 (m, 2H), 1.89 (q, J = 7.4 Hz, 2H), 1.78-1.65 (m, 2H), 1.58-1.53 (m, 4H), 1.52-1.31 (m, 2H).

### 8-benzyl-6-chloro-2-(furan-2-yl)-9H-purine (17aa)

A target compound (17aa) is obtained by the same synthetic method as for Compound (10a): ¹H NMR (600 MHz, CDCl₃) δ 8.39 (d, 1H), 7.35 (m, 1H), 7.29-7.22 (m, 6H), 3.85 (s, 2H).

### 6-chloro-2-(furan-2-yl)-8-(pyridin-2-ylmethyl)-9H-purine (17ab)

A target compound (17ab) is obtained by the same synthetic method as for Compound (10a): ¹H NMR (600 MHz, CDCl₃) δ 8.51 (d, 1H), 8.39 (d, 1H), 7.67 (t, J = 7.2 Hz, 1H), 7.30 (d, 1H), 7.15 (q, J = 7.7 Hz, 2H), 6.75 (d, 1), 4.14 (s, 2H).

### 6-chloro-8-(3-ethylpiperidin-1-yl)-2-(furan-2-yl)-9H-purine (17ac)

A target compound (17ac) is obtained by the same synthetic method as for Compound (10a): ¹H NMR (600 MHz, CDCl₃) δ 8.40 (d, 1H), 7.30 (d, 1H), 6.75 (d, 1H), 3.85-3.65 (m, 2H), 1.78-1.65 (m, 2H), 1.55 (m, 2H), 1.42 (m, 1H), 1.31 (m, 2H), 0.99 (t, J = 7.2 Hz, 1H).

### 4-(6-chloro-2-(furan-2-yl)-9H-purin-8-yl)-2-ethylmorpholine (17ad)

A target compound (17ad) is obtained by the same synthetic method as for Compound (10a): ¹H NMR (600 MHz, CDCl₃) δ 8.35 (d, 1H), 7.25 (d, 1H), 6.80 (d, 1H), 3.78-3.62 (m, 2H), 1.58 (m, 2H), 1.43 (m, 1H), 1.33 (m, 2H), 0.98 (t, J = 7.2 Hz, 1H).

### 2-(1-(6-chloro-2-(furan-2-yl)-9H-purin-8-yl)piperidin-3-yl)thiazole (17ae)

A target compound (17ae) is obtained by the same synthetic method as for Compound (10a): ¹H NMR (600 MHz, CDCl₃) δ 8.35 (d, 1H), 7.69 (d, 1H), 7.30 (d, 1H), 7.24 (d, 1H), 6.80 (d, 1H), 2.80 (m, 1H), 1.75-1.64 (m, 2H), 1.53-1.43(m, 2H).

### 4-(4-(6-chloro-2-(furan-2-yl)-9H-purin-8-yl)piperazin-1-yl)-3-fluorobenzonitrile (17af)

A target compound (17af) is obtained by the same synthetic method as for Compound (10a): ¹H NMR (600 MHz, CDCl₃) δ 8.40 (d, 1H), 7.30 (d, 1H), 7.12 (d, 1H), 7.04 (d, 1H), 6.90 (d, 1H), 6.85 (d, 1H), 3.51-3.49 (m, 4H), 3.30-3.28 (m, 4H).

### 4-(4-(6-chloro-2-(furan-2-yl)-9H-purin-8-yl)piperazin-1-yl)benzamide (17ag)

A target compound (17ag) is obtained by the same synthetic method as for Compound (10a): ¹H NMR (600 MHz, CDCl₃) δ 8.35 (d, 1H), 7.70 (s, 1H), 7.52 (d, 1H), 7.50 (d, 1H), 7.38 (d, 1H), 7.15 (d, 1H), 7.12 (d, 1H), 6.87 (d, 1H), 3.70-3.67 (m, 2H), 3.60-3.57 (m, 4H).

### 4-(4-(6-chloro-2-(furan-2-yl)-9H-purin-8-yl)piperazin-1-yl)-3-fluorobenzoic acid (17ah)

A target compound (17ah) is obtained by the same synthetic method as for Compound (10a): ¹H NMR (600 MHz, CDCl₃) δ 12.7 (s, 1H), 8.35 (d, 1H), 7.70 (d, 1H), 7.58 (d, 1H), 7.33 (d, 1H), 6.99 (d, 1H), 6.89 (d, 1H), 3.70-3.65 (m, 2H), 3.60-3.58 (m, 24).

### methyl 3-(1-(6-chloro-2-(furan-2-yl)-9H-purin-8-yl)piperidin-3-yl)propanoate (17ai)

A target compound (17ai) is obtained by the same synthetic method as for Compound (10a): ¹H NMR (600 MHz, CDCl₃) δ 8.33 (d, 1H), 7.35 (d, 1H), 6.90 (d, 1H), 3.65-3.63 (m, 2H), 3.62 (s, 3H), 2.32 (m, 2H), 1.88 (q, J = 7.4 Hz, 2H), 1.59-1.53 (m, 4H).

### 8-benzyl-6-chloro-2-(thiophen-2-yl)-9H-purine (17aj)

A target compound (17aj) is obtained by the same synthetic method as for Compound (10a): ¹H NMR (600 MHz, CDCl₃) δ 8. (d, 1H), 7.32 (m, 1H), 7.29-7.21 (m, 6H), 3.80 (s, 2H).

### 6-chloro-8-(pyridin-2-ylmethyl)-2-(thiophen-2-yl)-9H-purine (17ak)

A target compound (17ak) is obtained by the same synthetic method as for Compound (10a): ¹H NMR (600 MHz, CDCl₃) δ 8.48 (d, 1H), 8.37 (d, 1H), 7.67 (t, J = 7.4 Hz, 1H), 7.28 (d, 1H), 7.15 (q, J = 7.7 Hz, 2H), 6.75 (d, 1), 4.16 (s, 2H).

### 6-chloro-8-(3-ethylpiperidin-1-yl)-2-(thiophen-2-yl)-9H-purine (17al)

A target compound (17al) is obtained by the same synthetic method as for Compound (10a): ¹H NMR (600 MHz, CDCl₃) δ 8.48 (d, 1H), 7.38 (d, 1H), 6.81 (d, 1H), 3.87-3.65 (m, 2H), 1.55 (m, 2H), 1.56-1.53 (m, 2H), 1.42 (m, 1H), 1.31 (m, 2H), 0.97 (t, J = 7.7 Hz, 1H).

### 4-(6-chloro-2-(thiophen-2-yl)-9H-purin-8-yl)-2-ethylmorpholine (17am)

A target compound (17am) is obtained by the same synthetic method as for Compound (10a): ¹H NMR (600 MHz, CDCl₃) δ 8.40 (d, 1H), 7.35 (d, 1H), 6.90 (d, 1H), 3.78-3.62 (m, 2H), 1.58 (m, 2H), 1.43 (m, 1H), 1.33 (m, 2H), 1.00 (t, J = 7.2 Hz, 1H).

### 2-(1-(6-chloro-2-(thiophen-2-yl)-9H-purin-8-yl)piperidin-3-yl)thiazole (17an)

A target compound (17an) is obtained by the same synthetic method as for Compound (10a): ¹H NMR (600 MHz, CDCl₃) δ 8.38 (d, 1H), 7.70 (d, 1H), 7.45 (d, 1H), 7.31 (d, 1H), 6.70 (d, 1H), 2.80 (m, 1H), 1.92-1.87 (m, 2H 1.53-1.43(m, 2H).

### 4-(4-(6-chloro-2-(thiophen-2-yl)-9H-purin-8-yl)piperazin-1-yl)-3-fluorobenzonitrile (17ao)

A target compound (17ao) is obtained by the same synthetic method as for Compound (10a): ¹H NMR (600 MHz, CDCl₃) δ 8.38 (d, 1H), 7.27 (d, 1H), 7.13 (d, 1H), 7.07 (d, 1H), 6.87 (d, 1H), 6.86 (d, 1H), 3.77-3.68 (m, 2H), 3.51-3.49 (m, 4H).

### 4-(4-(6-chloro-2-(thiophen-2-yl)-9H-purin-8-yl)piperazin-1-yl)benzamide (17ap)

A target compound (17ap) is obtained by the same synthetic method as for Compound (10a): ¹H NMR (600 MHz, CDCl₃) δ 8.41 (d, 1H), 7.70 (s, 1H), 7.51 (d, 1H), 7.50 (d, 1H), 7.35 (d, 1H), 7.15 (d, 1H), 7.12 (d, 1H), 6.89 (d, 1H), 3.70-3.67 (m, 2H), 3.60-3.57 (m, 4H).

### 4-(4-(6-chloro-2-(thiophen-2-yl)-9H-purin-8-yl)piperazin-1-yl)-3-fluorobenzoic acid (17aq)

A target compound (17aq) is obtained by the same synthetic method as for Compound (10a): ¹H NMR (600 MHz, CDCl₃) δ 12.5 (s, 1H), 8.32 (d, 1H), 7.68 (d, 1H), 7.59 (d, 1H), 7.31 (d, 1H), 6.99 (d, 1H), 6.89 (d, 1H), 3.70-3.68 (m, 2H), 3.60-3.58 (m, 2H).

### methyl 3-(1-(6-chloro-2-(thiophen-2-yl)-9H-purin-8-yl)piperidin-3-yl)propanoate (17ar)

A target compound (17ar) is obtained by the same synthetic method as for Compound (10a): ¹H NMR (600 MHz, CDCl₃) δ 8.38 (d, 1H), 7.37 (d, 1H), 6.92 (d, 1H), 3.67-3.63 (m, 2H), 3.61 (s, 3H), 2.32 (m, 2H), 1.89 (q, J = 7.4 Hz, 2H), 1.58-1.53 (m, 4H).

### 8-benzyl-2-(furan-2-yl)-9H-purin-6-amine (18aa)

A target compound (18aa) is obtained by the same synthetic method as for Compound (18a): ¹H NMR (600 MHz, DMSO-d₆) δ 12.95 (s, 1 H), 8.39 (d, J = 1.7 Hz, 1H), 7.30-7.20 (m, 5H), 6.96 (s, 2H), (6.71 (dd, J = 3.4 Hz, J = 1.7 Hz, 1H), 3.86 (s, 2H).

### 2-(furan-2-yl)-8-(pyridin-2-ylmethyl)-9H-purin-6-amine (18ab)

A target compound (18ab) is obtained by the same synthetic method as for Compound (18a): ¹H NMR (600 MHz, DMSO-d₆) δ 13.65 (s, 1 H), 8.51 (d, J = 3.90 Hz, 1H), 8.39 (d, J = 1.7 Hz, 1H), 7.67 (tt, J = 3.43 Hz, 1H), 7.29-7.15 (m, 4H), 6.96 (s, 2H), 4.14(s, 2H).

### 8-(3-ethylpiperidin-1-yl)-2-(furan-2-yl)-9H-purin-6-amine (18ac)

A target compound (18ac) is obtained by the same synthetic method as for Compound (18a): ¹H NMR (600 MHz, DMSO-d₆) δ 13.55 (s, 1 H), 8.39 (d, J = 1.7 Hz, 1H), 7.29 (dd, J = 1.6, 0.7 Hz, 1H), 6.96 (s, 2H), 6.71 (dd, J = 3.4, 1.7 Hz, 1H), 3.34-3.09 (m, 4H), 1.55-1.31(m, 7H), 0.99 (t, J = 7.5 Hz, 3H).

### 8-(2-ethylmorpholino)-2-(furan-2-yl)-9H-purin-6-amine (18ad)

A target compound (18ad) is obtained by the same synthetic method as for Compound (18a): ¹H NMR (600 MHz, DMSO-d₆) δ 13.5 (s, 1 H), 8.39 (d, J = 1.7 Hz, 1H), 7.29 (dd, J = 1.6, 0.7 Hz, 1H), 6.96 (s, 2H), 6.71 (dd, J = 3.4, 1.7 Hz, 1H), 3.64-3.54 (m, 3H), 2.83-2.58 (m, 4H), 1.44 (m, 2H), 0.99 (t, J = 7.5 Hz, 3H).

### 2-(furan-2-yl)-8-(3-(thiazol-2-yl)piperidin-1-yl)-9H-purin-6-amine (18ae)

A target compound (18ae) is obtained by the same synthetic method as for Compound (18a): ¹H NMR (600 MHz, DMSO-d₆) δ 13.16 (s, 1 H), 8.39 (d, J = 1.7 Hz, 1H), 7.69 (d, J = 1=3.4 Hz, 1H), 7.29 (dd, J = 1.6, 0.7 Hz, 1H), 7.24 (d, J = 3.4 Hz, 1H), 6.96 (s, 2H), 6.71 (dd, J = 3.4, 1.7 Hz, 1H), 3.69-3.25 (m, 4H), 2.78 (d, J = 9 Hz, 1H), 1.92-1.43 (m, 4H).

### 4-(4-(6-amino-2-(furan-2-yl)-9H-purin-8-yl)piperazin-1-yl)-3-fluorobenzonitrile (18af)

A target compound (18af) is obtained by the same synthetic method as for Compound (18a): ¹H NMR (600 MHz, DMSO-d₆) δ 13.36 (s, 1 H), 8.39 (d, J = 1.7 Hz, 1H), 7.29 (dd, J = 1.6, 0.7 Hz, 1H), 7.11-7.04 (m, 2H), 6.96-6.71 (m, 4H), 3.57-3.28 (m, 8H).

### 4-(4-(6-amino-2-(furan-2-yl)-9H-purin-8-yl)piperazin-1-yl)benzamide) (18ag)

A target compound (18ag) is obtained by the same synthetic method as for Compound (18a): ¹H NMR (600 MHz, DMSO-d₆) δ 12.95 (s, 1 H), 8.39 (d, J = 1.7 Hz, 1H), 7.7 (s, 2H), 7.51-7.00 (m, 5H), 6.96 (s, 2H), 6.71 (dd, J = 3.4, 1.7 Hz, 1H), 3.57-3.28 (m, 8H).

### 4-(4-(6-amino-2-(furan-2-yl)-9H-purin-8-yl)piperazin-1-yl)-3-fluorobenzoic acid (18ah)

A target compound (18ah) is obtained by the same synthetic method as for Compound (18a): ¹H NMR (600 MHz, DMSO-d₆) δ 13.65 (s, 1 H), 12.74 (s, 1H), 8.39 (d, J = 1.7 Hz, 1H), 7.61-7.53 (m, 2H), 7.29 (dd, J = 1.6, 0.7 Hz, 1H), 6.96-6.71 (m, 4H), 3.57-3.28 (m, 8H).

### methyl 3-(1-(6-amino-2-(furan-2-yl)-9H-purin-8-yl)piperidin-3-yl)propanoate (18ai)

A target compound (18ai) is obtained by the same synthetic method as for Compound (18a): ¹H NMR (600 MHz, DMSO-d₆) δ 13.50 (s, 1 H), 8.39 (d, J = 1.7 Hz, 1H), 7.29 (dd, J = 1.6, 0.7 Hz, 1H), 6.96 (s, 2H), 6.71 (dd, J = 3.4, 1.7 Hz, 1H), 3.61 (s, 3H), 3.34-3.09 (m, 4H), 2.35-1.31 (m, 9H).

### 8-benzyl-2-(thiophen-2-yl)-9H-purin-6-amine (18aj)

A target compound (18aj) is obtained by the same synthetic method as for Compound (18a): ¹H NMR (600 MHz, DMSO-d₆) δ 13.61 (s, 1 H), 8.40 (d, J = 1.7 Hz, 1H), 7.31-7.24 (m, 5H), 6.95 (s, 2H), 6.71 (dd, J = 3.4 Hz, J = 1.7 Hz, 1H), 3.81 (s, 2H).

### 8-(pyridin-2-ylmethyl)-2-(thiophen-2-yl)-9H-purin-6-amine (18ak)

A target compound (18ak) is obtained by the same synthetic method as for Compound (18a): ¹H NMR (300 MHz, DMSO-d₆) δ 12.98 (s, 1 H), 8.57 (d, J = 3.90 Hz, 1H), 8.31 (d, J = 1.7 Hz, 1H), 7.69 (tt, J = 3.43 Hz, 1H), 7.31-7.17 (m, 4H), 6.96 (s, 2H), 4.14(s, 2H).

### 8-(3-ethylpiperidin-1-yl)-2-(thiophen-2-yl)-9H-purin-6-amine (18al)

A target compound (18al) is obtained by the same synthetic method as for Compound (18a): ¹H NMR (300 MHz, DMSO-d₆) δ 13.42 (s, 1 H), 8.45 (d, J = 1.7 Hz, 1H), 7.33 (dd, J = 1.6, 0.7 Hz, 1H), 6.98 (s, 2H), 6.72 (dd, J = 3.4, 1.7 Hz, 1H), 3.37-3.09 (m, 4H), 1.51-1.39(m, 7H), 1.00 (t, J = 7.5 Hz, 3H).

### 8-(2-ethylmorpholino)-2-(thiophen-2-yl)-9H-purin-6-amine (18am)

A target compound (18am) is obtained by the same synthetic method as for Compound (18a): ¹H NMR (300 MHz, DMSO-d₆) δ 13.15 (s, 1 H), 8.24 (d, J = 1.7 Hz, 1H), 7.15 (dd, J = 1.6, 0.7 Hz, 1H), 6.85 (s, 2H), 6.64 (dd, J = 3.4, 1.7 Hz, 1H), 3.67-3.55 (m, 3H), 2.88-2.58 (m, 4H), 1.44 (m, 2H), 0.99 (t, J = 7.5 Hz, 3H).

### 8-(3-(thiazol-2-yl)piperidin-1-yl)-2-(thiophen-2-yl)-9H-purin-6-amine (18an)

A target compound (18an) is obtained by the same synthetic method as for Compound (18a): ¹H NMR (300 MHz, DMSO-d₆) δ 12.98 (s, 1 H), 8.45 (d, J = 1.7 Hz, 1H), 7.74 (d, J = 1=3.4 Hz, 1H), 7.35 (dd, J = 1.6, 0.7 Hz, 1H), 7.26 (d, J = 3.4 Hz, 1H), 6.99 (s, 2H), 6.78 (dd, J = 3.4, 1.7 Hz, 1H), 3.70-3.25 (m, 4H), 2.81 (d, J = 9 Hz, 1H), 1.92-1.43 (m, 4H).

### 4-(4-(6-amino-2-(thiophen-2-yl)-9H-purin-8-yl)piperazin-1-yl)-3-fluorobenzonitrile (18ao)

A target compound (18ao) is obtained by the same synthetic method as for Compound (18a): ¹H NMR (300 MHz, DMSO-d₆) δ 13.66 (s, 1 H), 8.35 (d, J = 1.7 Hz, 1H), 7.28 (dd, J = 1.6, 0.7 Hz, 1H), 7.15-7.04 (m, 2H), 6.97-6.71 (m, 4H), 3.60-3.28 (m, 8H).

### 4-(4-(6-amino-2-(thiophen-2-yl)-9H-purin-8-yl)piperazin-1-yl)benzamide (18ap)

A target compound (18ap) is obtained by the same synthetic method as for Compound (18a): ¹H NMR (300 MHz, DMSO-d₆) δ 13.51 (s, 1 H), 8.45 (d, J = 1.7 Hz, 1H), 7.75 (s, 2H), 7.51-7.32 (m, 5H), 6.95 (s, 2H), 6.81 (dd, J = 3.4, 1.7 Hz, 1H), 3.67-3.58 (m, 8H).

### 4-(4-(6-amino-2-(thiophen-2-yl)-9H-purin-8-yl)piperazin-1-yl)-3-fluorobenzoic acid (18aq)

A target compound (18aq) is obtained by the same synthetic method as for Compound (18a): ¹H NMR (300 MHz, DMSO-d₆) δ 13.55 (s, 1 H), 12.74 (s, 1H), 8.39 (d, J = 1.7 Hz, 1H), 7.61-7.53 (m, 2H), 7.29 (dd, J = 1.6, 0.7 Hz, 1H), 6.96-6.71 (m, 4H), 3.57-3.28 (m, 8H).

### methyl 3-(1-(6-amino-2-(thiophen-2-yl)-9H-purin-8-yl)piperidin-3-yl)propanoate (18ar)

A target compound (18ar) is obtained by the same synthetic method as for Compound (18a): ¹H NMR (600 MHz, DMSO-d₆) δ 13.65 (s, 1 H), 7.85 (m, 2H), 7.25 (m, 1H), 6.96 (s, 2H), 3.61 (s, 3H), 3.34-3.09 (m, 4H), 2.35-1.88 (m, 4H), 1.56-1.31 (m, 5H).

### Preparation Example 8: Synthesis of 18ba to 18bn

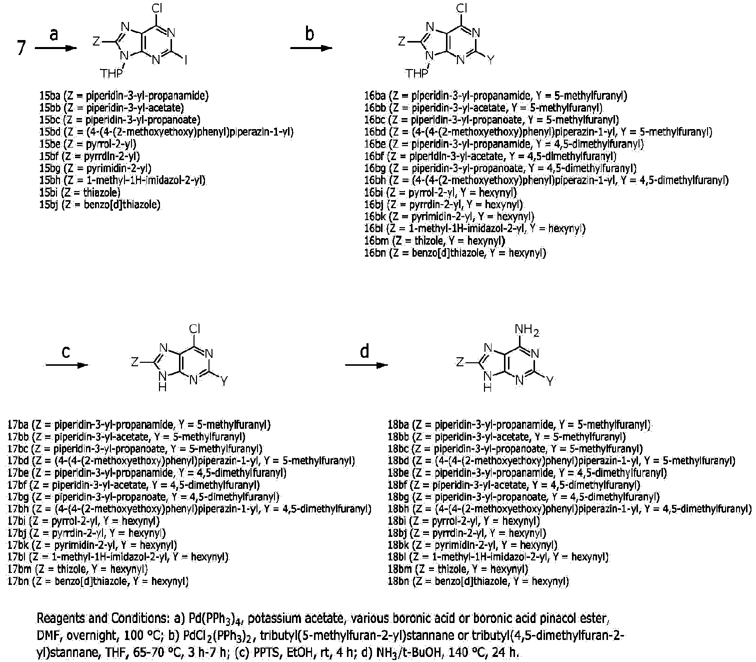

### 3-(1-(6-chloro-2-iodo-9-(tetrahydro-2H-pyran-2-yl)-9H-purin-8-yl)piperidin-3-yl)propanamide (15ba)

A target compound (15ba) is obtained by the same synthetic method as for Compound (15aa): ¹H NMR (600 MHz, CDCl₃) δ 7.03 (s, 2H), 5.95 (t, J = 7.7 Hz, 1H), 3.65-3.61 (m, 2H), 3.30-3.22 (m, 2H), 2.20-1.98 (m, 2H), 2.05 (m, 2H), 1.77 (m, 2H), 1.75-1.68 (m, 2H), 1.58-1.31 (m, 4H).

### methyl 2-(1-(6-chloro-2-iodo-9-(tetrahydro-2H-pyran-2-yl)-9H-purin-8-yl)piperidin-3-yl)acetate (15bb)

A target compound (15bb) is obtained by the same synthetic method as for Compound (15aa): ¹H NMR (600 MHz, CDCl₃) δ 5.99 (t, J = 7.4 Hz, 1H), 3.71 (s, 3H), 3.62-3.59 (m, 2H), 3.32-3.28 (m, 2H), 2.21-1.98 (m, 2H), 2.05 (m, 2H), 1.75 (m, 2H), 1.75-1.68 (m, 2H), 1.58-1.33 (m, 4H).

### methyl 3-(1-(6-chloro-2-iodo-9-(tetrahydro-2H-pyran-2-yl)-9H-purin-8-yl)piperidin-3-yl)propanoate (15bc)

A target compound (15bc) is obtained by the same synthetic method as for Compound (15aa): ¹H NMR (600 MHz, CDCl₃) δ 6.01 (t, J = 7.2 Hz, 1H), 3.75 (s, 3H), 3.64-3.58 (m, 2H), 3.33-3.28 (m, 2H), 2.40 (m, 2H), 2.24-1.98 (m, 2H), 2.07 (m, 2H), 1.75 (m, 2H), 1.75-1.68 (m, 2H), 1.58-1.33 (m, 4H).

### 6-chloro-2-iodo-8-(4-(4-(2-methoxyethoxy)phenyl)piperazin-1-yl)-9-(tetrahydro-2H-pyran-2-yl)-9H-purine (15bd)

A target compound (15bd) is obtained by the same synthetic method as for Compound (15aa): ¹H NMR (600 MHz, CDCl₃) δ 6.74 (d, 2H), 6.61 (d, 2H), 5.95 (t, J = 7.2 Hz, 1H), 4.41 (t, J = 7.7 Hz, 2H), 3.78 (t, J = 7.2 Hz, 2H), 3.66-3.59 (m, 2H), 3.57 (m, 4H), 3.28 (m, 4H), 2.17-1.92 (m, 2H), 1.74-1.64 (m, 2H), 1.58-1.55 (m, 2H).

### 6-chloro-2-iodo-8-(1H-pyrrol-2-yl)-9-(tetrahydro-2H-pyran-2-yl)-9H-purine(15be)

A target compound (15be) is obtained by the same synthetic method as for Compound (15aa): ¹H NMR (600 MHz, CDCl₃) δ 11.95 (s, 1H), 6.95 (d, 1H), 6.48 (d, 1H), 6.15 (d, 1H), 5.97 (t, J = 7.7 Hz, 1H), 3.70-3.67 (m, 2H), 2.20-1.92 (m, 2H), 1.75-1.65 (m, 2H), 1.58-1.55 (m, 2H).

### 6-chloro-2-iodo-8-(pyridin-2-yl)-9-(tetrahydro-2H-pyran-2-yl)-9H-purine (15bf)

A target compound (15bf) is obtained by the same synthetic method as for Compound (15aa): ¹H NMR (600 MHz, CDCl₃) δ 8.59 (d, 1H), 8.24 (d, 1H), 7.85 (t, J = 7.2 Hz, 1H), 7.50 (t, J = 7.7 Hz, 1H), 5.99 (t, J = 7.7 Hz, 1H), 3.72-3.67 (m, 2H), 2.20-1.92 (m, 2H), 1.77-1.65 (m, 2H), 1.59-1.55 (m, 2H).

### 6-chloro-2-iodo-8-(pyrimidin-2-yl)-9-(tetrahydro-2H-pyran-2-yl)-9H-purine (15bg)

A target compound (15bg) is obtained by the same synthetic method as for Compound (15aa): ¹H NMR (600 MHz, CDCl₃) δ 9.08 (d, 1H), 9.05 (d, 1H), 7.85 (t, J = 7.2 Hz, 1H), 6.05 (t, J = 7.2 Hz, 1H), 3.85-3.77 (m, 2H), 2.35-2.11 (m, 2H), 1.87-1.65 (m, 2H), 1.59-1.55 (m, 2H).

### 6-chloro-2-iodo-8-(1-methyl-1H-imidazol-2-yl)-9-(tetrahydro-2H-pyran-2-yl)-9H-purine (15bh)

A target compound (15bh) is obtained by the same synthetic method as for Compound (15aa): ¹H NMR (600 MHz, CDCl₃) δ 7.13 (d, 1H), 6.87 (d, 1H), 5.97 (t, J = 7.7 Hz, 1H), 3.72 (s, 3H), 3.70-3.67 (m, 2H), 2.20-1.92 (m, 2H), 1.75-1.65 (m, 2H), 1.58-1.55 (m, 2H).

### 2-(6-chloro-2-iodo-9-(tetrahydro-2H-pyran-2-yl)-9H-purin-8-yl)thiazole (15bi)

A target compound (15bi) is obtained by the same synthetic method as for Compound (15aa): ¹H NMR (600 MHz, CDCl₃) δ 8.07 (d, 1H), 7.71 (d, 1H), 6.01 (t, J = 7.4 Hz, 1H), 3.67-3.61 (m, 2H), 2.22-1.92 (m, 2H), 1.78-1.65 (m, 2H), 1.58-1.55 (m, 2H).

### 2-(6-chloro-2-iodo-9-(tetrahydro-2H-pyran-2-yl)-9H-purin-8-yl)benzo[d]thiazole (15bj)

A target compound (15bj) is obtained by the same synthetic method as for Compound (15aa): ¹H NMR (600 MHz, CDCl₃) δ 8.18 (d, 1H), 8.02 (d, 1H), 7.53-7.51 (m, 2H), 5.98 (t, J = 7.4 Hz, 1H), 3.67-3.57 (m, 2H), 2.20-1.99 (m, 2H), 1.80-1.67 (m, 2H), 1.58-1.55 (m, 2H).

### 3-(1-(6-chloro-2-(5-methylfuran-2-yl)-9-(tetrahydro-2H-pyran-2-yl)-9H-purin-8-yl)piperidin-3-yl)propanamide (16ba)

A target compound (16ba) is obtained using tributyl(5-methylfuran-2-yl)stannane by the same synthetic method as for Compound (8a): ¹H NMR (600 MHz, CDCl₃) δ 7.03 (s, 2H), 6.97 (d, 1H), 6.15 (d, 1H), 5.95 (t, J = 7.7 Hz, 1H), 3.65-3.61 (m, 2H), 3.30-3.22 (m, 2H), 2.35 (s, 3H), 2.20-1.98 (m, 2H), 2.05 (m, 2H), 1.77 (m, 2H), 1.75-1.68 (m, 2H), 1.58-1.31 (m, 4H).

### methyl 2-(1-(6-chloro-2-(5-methylfuran-2-yl)-9-(tetrahydro-2H-pyran-2-yl)-9H-purin-8-yl)piperidin-3-yl)acetate (16bb)

A target compound (16bb) is obtained by the same synthetic method as for Compound (16ba): ¹H NMR (600 MHz, CDCl₃) δ 6.99 (d, 1H), 6.11 (d, 1H), 5.99 (t, J = 7.4 Hz, 1H), 3.71 (s, 3H), 3.62-3.59 (m, 2H), 3.32-3.28 (m, 2H), 2.35 (s, 3H), 2.21-1.98 (m, 2H), 2.05 (m, 2H), 1.75 (m, 2H), 1.75-1.68 (m, 2H), 1.58-1.33 (m, 4H).

### methyl 3-(1-(6-chloro-2-(5-methylfuran-2-yl)-9-(tetrahydro-2H-pyran-2-yl)-9H-purin-8-yl)piperidin-3-yl)propanoate (16bc)

A target compound (16bc) is obtained by the same synthetic method as for Compound (16ba): ¹H NMR (600 MHz, CDCl₃) δ 7.01 (d, 1H), 6.13 (d, 1H), 6.01 (t, J = 7.2 Hz, 1H), 3.75 (s, 3H), 3.64-3.58 (m, 2H), 3.33-3.28 (m, 2H), 2.40 (m, 2H), 2.37 (s, 3H), 2.24-1.98 (m, 2H), 2.07 (m, 2H), 1.75 (m, 2H), 1.75-1.68 (m, 2H), 1.58-1.33 (m, 4H).

### 6-chloro-8-(4-(4-(2-methoxyethoxy)phenyl)piperazin-1-yl)-2-(5-methylfuran-2-yl)-9-(tetrahydro-2H-pyran-2-yl)-9H-purine (16bd)

A target compound (16bd) is obtained by the same synthetic method as for Compound (16ba): ¹H NMR (600 MHz, CDCl₃) δ 6.87 (d, 1H), 6.74 (d, 2H), 6.61 (d, 2H), 6.17 (d, 1H), 5.95 (t, J = 7.2 Hz, 1H), 4.41 (t, J = 7.7 Hz, 2H), 3.78 (t, J = 7.2 Hz, 2H), 3.66-3.59 (m, 2H), 3.57 (m, 4H), 3.28 (m, 4H), 2.45 (s, 3H), 2.17-1.92 (m, 2H), 1.74-1.64 (m, 2H), 1.58-1.55 (m, 2H).

### 3-(1-(6-chloro-2-(4,5-dimethylfuran-2-yl)-9-(tetrahydro-2H-pyran-2-yl)-9H-purin-8-yl)piperidin-3-yl)propanamide (16be)

A target compound (16be) is obtained using tributyl(4,5-dimethylfuran-2-yl)stannane by the same synthetic method as for Compound (8a): ¹H NMR (600 MHz, CDCl₃) δ 7.03 (s, 2H), 5.95 (t, J = 7.7 Hz, 1H), 3.65-3.61 (m, 2H), 3.30-3.22 (m, 2H), 2.35 (s, 3H), 2.20-1.98 (m, 2H), 2.01 (s, 3H), 2.05 (m, 2H), 1.77 (m, 2H), 1.75-1.68 (m, 2H), 1.58-1.31 (m, 4H).

### methyl 2-(1-(6-chloro-2-(4,5-dimethylfuran-2-yl)-9-(tetrahydro-2H-pyran-2-yl)-9H-purin-8-yl)piperidin-3-yl)acetate (16bf)

A target compound (16bf) is obtained by the same synthetic method as for Compound (16be): ¹H NMR (600 MHz, CDCl₃) δ 5.99 (t, J = 7.4 Hz, 1H), 3.71 (s, 3H), 3.62-3.59 (m, 2H), 3.32-3.28 (m, 2H), 2.35 (s, 3H), 2.21-1.98 (m, 2H), 2.07 (s, 3H), 2.05 (m, 2H), 1.75 (m, 2H), 1.75-1.68 (m, 2H), 1.58-1.33 (m, 4H).

### methyl 3-(1-(6-chloro-2-(4,5-dimethylfuran-2-yl)-9-(tetrahydro-2H-pyran-2-yl)-9H-purin-8-yl)piperidin-3-yl)propanoate (16bg)

A target compound (16bg) is obtained by the same synthetic method as for Compound (16be): ¹H NMR (600 MHz, CDCl₃) δ 6.01 (t, J = 7.2 Hz, 1H), 3.75 (s, 3H), 3.64-3.58 (m, 2H), 3.33-3.28 (m, 2H), 2.40 (m, 2H), 2.38 (s, 3H), 2.24-1.98 (m, 2H), 2.07 (m, 2H), 2.01 (s, 3H), 1.75 (m, 2H), 1.75-1.68 (m, 2H), 1.58-1.33 (m, 4H).

### 6-chloro-2-(4,5-dimethylfuran-2-yl)-8-(4-(4-(2-methoxyethoxy)phenyl)piperazin-1-yl)-9-(tetrahydro-2H-pyran-2-yl)-9H-purine (16bh)

A target compound (16bh) is obtained by the same synthetic method as for Compound (16be): ¹H NMR (600 MHz, CDCl₃) δ 6.74 (d, 2H), 6.61 (d, 2H), 5.95 (t, J = 7.2 Hz, 1H), 4.41 (t, J = 7.7 Hz, 2H), 3.78 (t, J = 7.2 Hz, 2H), 3.66-3.59 (m, 2H), 3.57 (m, 4H), 3.28 (m, 4H), 2.39 (s, 3H), 2.17-1.92 (m, 2H), 2.11 (s, 3H), 1.74-1.64 (m, 2H), 1.58-1.55 (m, 2H).

### 6-chloro-2-(hex-1-yn-1-yl)-8-(1H-pyrrol-2-yl)-9-(tetrahydro-2H-pyran-2-yl)-9H-purine (16bi)

A target compound (16bi) is obtained by the same synthetic method as for Compound (9a): ¹H NMR (600 MHz, CDCl₃) δ 11.95 (s, 1H), 6.95 (d, 1H), 6.48 (d, 1H), 6.15 (d, 1H), 5.97 (t, J = 7.7 Hz, 1H), 3.70-3.67 (m, 2H), 2.46-2.45 (m, 2H), 2.20-1.92 (m, 2H), 1.75-1.65 (m, 2H), 1.58-1.55 (m, 2H). 1.44 (m, 2H), 1.30-1.28 (m, 2H), 0.89 (t, J = 7.7 Hz, 3H).

### 6-chloro-2-(hex-1-yn-1-yl)-8-(pyridin-2-yl)-9-(tetrahydro-2H-pyran-2-yl)-9H-purine (16bj)

A target compound (16bj) is obtained by the same synthetic method as for Compound (9a): ¹H NMR (600 MHz, CDCl₃) δ 8.59 (d, 1H), 8.24 (d, 1H), 7.85 (t, J = 7.2 Hz, 1H), 7.50 (t, J = 7.7 Hz, 1H), 5.99 (t, J = 7.7 Hz, 1H), 3.72-3.67 (m, 2H), 2.45 -2.44 (m, 2H), 2.20-1.92 (m, 2H), 1.77-1.65 (m, 2H), 1.59-1.55 (m, 2H), 1.47-1.46 (m, 2H), 1.31-1.30 (m, 2H), 1.01 (t, J = 7.2 Hz, 3H).

### 6-chloro-2-(hex-1-yn-1-yl)-8-(pyrimidin-2-yl)-9-(tetrahydro-2H-pyran-2-yl)-9H-purine (16bk)

A target compound (16bk) is obtained by the same synthetic method as for Compound (9a): ¹H NMR (600 MHz, CDCl₃) δ 9.08 (d, 1H), 9.05 (d, 1H), 7.85 (t, J = 7.2 Hz, 1H), 6.05 (t, J = 7.2 Hz, 1H), 3.85-3.77 (m, 2H), 2.47-2.46 (m, 2H), 2.35-2.11 (m, 2H), 1.87-1.65 (m, 2H), 1.59-1.55 (m, 2H), 1.50-1.46 (m, 2H), 1.35-1.33 (m, 2H), 1.05 (t, J = 7.2 Hz, 3H).

### 6-chloro-2-(hex-1-yn-1-yl)-8-(1-methyl-1H-imidazol-2-yl)-9-(tetrahydro-2H-pyran-2-yl)-9H-purine (16bl)

A target compound (16bl) is obtained by the same synthetic method as for Compound (9a): ¹H NMR (600 MHz, CDCl₃) δ 7.13 (d, 1H), 6.87 (d, 1H), 5.97 (t, J = 7.7 Hz, 1H), 3.72 (s, 3H), 3.70-3.67 (m, 2H), 2.47-2.44 (m, 2H), 2.20-1.92 (m, 2H), 1.75-1.65 (m, 2H), 1.58-1.55 (m, 2H), 1.47-1.45 (m, 2H), 1.31-1.29 (m, 2H), 1.02 (t, J = 7.4 Hz, 3H).

### 2-(6-chloro-2-(hex-1-yn-1-yl)-9-(tetrahydro-2H-pyran-2-yl)-9H-purin-8-yl)thiazole (16bm)

A target compound (16bm) is obtained by the same synthetic method as for Compound (9a): ¹H NMR (600 MHz, CDCl₃) δ 8.07 (d, 1H), 7.71 (d, 1H), 6.01 (t, J = 7.4 Hz, 1H), 3.67-3.61 (m, 2H), 2.55-2.52 (m, 2H), 2.22-1.92 (m, 2H), 1.78-1.65 (m, 2H), 1.61-1.60 (m, 2H), 1.58-1.55 (m, 2H), 1.48-1.47 (m, 2H), 1.33-1.29 (m, 2H), 0.98 (t, J = 7.2 Hz, 3H).

### 2-(6-chloro-2-(hex-1-yn-1-yl)-9-(tetrahydro-2H-pyran-2-yl)-9H-purin-8-yl)benzo[d]thiazole (16bn)

A target compound (16bn) is obtained by the same synthetic method as for Compound (9a): ¹H NMR (600 MHz, CDCl₃) δ 8.18 (d, 1H), 8.02 (d, 1H), 7.53-7.51 (m, 2H), 5.98 (t, J = 7.4 Hz, 1H), 3.67-3.57 (m, 2H), 2.44-2.42 (m, 2H), 2.20-1.99 (m, 2H), 1.80-1.67 (m, 2H), 1.58-1.55 (m, 2H), 1.44-1.42 (m, 2H), 1.30-1.28 (m, 2H), 0.89 (t, J = 7.7 Hz, 3H).

### 3-(1-(6-chloro-2-(5-methylfuran-2-yl)-9H-purin-8-yl)piperidin-3-yl)propanamide (17ba)

A target compound (17ba) is obtained by the same synthetic method as for Compound (17a): ¹H NMR (600 MHz, CDCl₃) δ 13.65 (s, 1H), 7.03 (s, 2H), 6.97 (d, 1H), 6.15 (d, 1H), 3.30-3.22 (m, 2H), 2.35 (s, 3H), 2.05 (m, 2H), 1.77 (m, 2H).

### methyl 2-(1-(6-chloro-2-(5-methylfuran-2-yl)-9H-purin-8-yl)piperidin-3-yl)acetate (17bb)

A target compound (17bb) is obtained by the same synthetic method as for Compound (17a): ¹H NMR (600 MHz, CDCl₃) δ 6.99 (d, 1H), 6.11 (d, 1H), 3.71 (s, 3H), 3.62-3.59 (m, 2H2.35 (s, 3H), 2.05 (m, 2H), 1.75 (m, 2H).

### methyl 3-(1-(6-chloro-2-(5-methylfuran-2-yl)-9H-purin-8-yl)piperidin-3-yl)propanoate (17bc)

A target compound (17bc) is obtained by the same synthetic method as for Compound (17a): ¹H NMR (600 MHz, CDCl₃) δ 7.01 (d, 1H), 6.13 (d, 1H), 3.75 (s, 3H), 3.64-3.58 (m, 2H), 2.40 (m, 2H), 2.37 (s, 3H), 2.07 (m, 2H), 1.75 (m, 2H).

### 6-chloro-8-(4-(4-(2-methoxyethoxy)phenyl)piperazin-1-yl)-2-(5-methylfuran-2-yl)-9H-purine (17bd)

A target compound (17bd) is obtained by the same synthetic method as for Compound (17a): ¹H NMR (600 MHz, CDCl₃) δ 6.87 (d, 1H), 6.74 (d, 2H), 6.61 (d, 2H), 6.17 (d, 1H), 4.41 (t, J = 7.7 Hz, 2H), 3.78 (t, J = 7.2 Hz, 2H), 3.57 (m, 4H), 3.28 (m, 4H), 2.45 (s, 3H).

### 3-(1-(6-chloro-2-(4,5-dimethylfuran-2-yl)-9H-purin-8-yl)piperidin-3-yl)propanamide (17be)

A target compound (17be) is obtained by the same synthetic method as for Compound (17a): ¹H NMR (600 MHz, CDCl₃) δ 7.03 (s, 2H), 3.65-3.61 (m, 2H), 2.35 (s, 3H), 2.01 (s, 3H), 2.05 (m, 2H), 1.77 (m, 2H).

### methyl 2-(1-(6-chloro-2-(4,5-dimethylfuran-2-yl)-9H-purin-8-yl)piperidin-3-yl)acetate (17bf)

A target compound (17bf) is obtained by the same synthetic method as for Compound (17a): ¹H NMR (600 MHz, CDCl₃) δ 3.71 (s, 3H), 3.62-3.59 (m, 2H), 2.35 (s, 3H), 2.07 (s, 3H), 2.05 (m, 2H), 1.75 (m, 2H).

### methyl 3-(1-(6-chloro-2-(4,5-dimethylfuran-2-yl)-9H-purin-8-yl)piperidin-3-yl)propanoate (17bg)

A target compound (17bg) is obtained by the same synthetic method as for Compound (17a): ¹H NMR (600 MHz, CDCl₃) δ 3.75 (s, 3H), 3.64-3.58 (m, 2H), 2.40 (m, 2H), 2.38 (s, 3H), 2.07 (m, 2H), 2.01 (s, 3H), 1.75 (m, 2H).

### 6-chloro-2-(4,5-dimethylfuran-2-yl)-8-(4-(4-(2-methoxyethoxy)phenyl)piperazin-1-yl)-9H-purine (17bh)

A target compound (17bh) is obtained by the same synthetic method as for Compound (17a): ¹H NMR (600 MHz, CDCl₃) δ 6.74 (d, 2H), 6.61 (d, 2H), 4.41 (t, J = 7.7 Hz, 2H), 3.78 (t, J = 7.2 Hz, 2H), 3.57 (m, 4H), 3.28 (m, 4H), 2.39 (s, 3H), 2.11 (s, 3H).

### 6-chloro-2-(hex-1-yn-1-yl)-8-(1H-pyrrol-2-yl)-9H-purine (17bi)

A target compound (17bi) is obtained by the same synthetic method as for Compound (17a): ¹H NMR (600 MHz, CDCl₃) δ 11.95 (s, 1H), 6.95 (d, 1H), 6.48 (d, 1H), 6.15 (d, 1H), 2.46-2.45 (m, 2H), 1.58-1.55 (m, 2H). 1.44 (m, 2H), 0.89 (t, J = 7.7 Hz, 3H).

### 6-chloro-2-(hex-1-yn-1-yl)-8-(pyridin-2-yl)-9H-purine (17bj)

A target compound (17bj) is obtained by the same synthetic method as for Compound (17a): ¹H NMR (600 MHz, CDCl₃) δ 8.59 (d, 1H), 8.24 (d, 1H), 7.85 (t, J = 7.2 Hz, 1H), 7.50 (t, J = 7.7 Hz, 1H), 2.45 -2.44 (m, 2H), 1.77-1.65 (m, 2H), 1.59-1.55 (m, 2H), 1.01 (t, J = 7.2 Hz, 3H).

### 6-chloro-2-(hex-1-yn-1-yl)-8-(pyrimidin-2-yl)-9H-purine (17bk)

A target compound (17bk) is obtained by the same synthetic method as for Compound (17a): ¹H NMR (600 MHz, CDCl₃) δ 9.08 (d, 1H), 9.05 (d, 1H), 7.85 (t, J = 7.2 Hz, 1H 2.47-2.46 (m, 2H), 1.87-1.65 (m, 2H),.50-1.46 (m, 2H), 1.05 (t, J = 7.2 Hz, 3H).

### 6-chloro-2-(hex-1-yn-1-yl)-8-(1-methyl-1H-imidazol-2-yl)-9H-purine (17bl)

A target compound (17bl) is obtained by the same synthetic method as for Compound (17a): ¹H NMR (600 MHz, CDCl₃) δ 7.13 (d, 1H), 6.87 (d, 1H), 3.72 (s, 3H), 2.47-2.44 (m, 2H), 1.75-1.65 (m, 2H), 1.47-1.45 (m, 2H), 1.02 (t, J = 7.4 Hz, 3H).

### 2-(6-chloro-2-(hex-1-yn-1-yl)-9H-purin-8-yl)thiazole (17bm)

A target compound (17bm) is obtained by the same synthetic method as for Compound (17a): ¹H NMR (600 MHz, CDCl₃) δ 8.07 (d, 1H), 7.71 (d, 1H), 2.55-2.52 (m, 2H), 1.78-1.65 (m, 2H), 1.58-1.55 (m, 2H), 1.48-1.47 (m, 2H), 0.98 (t, J = 7.2 Hz, 3H).

### 2-(6-chloro-2-(hex-1-yn-1-yl)-9H-purin-8-yl)benzo[d]thiazole (17bn)

A target compound (17bn) is obtained by the same synthetic method as for Compound (17a): ¹H NMR (600 MHz, CDCl₃) δ 8.18 (d, 1H), 8.02 (d, 1H), 7.53-7.51 (m, 2H), 2.44-2.42 (m, 2H), 1.80-1.67 (m, 2H), 1.44-1.42 (m, 2H), 0.89 (t, J = 7.7 Hz, 3H).

### 3-(1-(6-amino-2-(5-methylfuran-2-yl)-9H-purin-8-yl)piperidin-3-yl)propanamide (18ba)

A target compound (18ba) is obtained by the same synthetic method as for Compound (18a): ¹H NMR (600 MHz, DMSO-d₆) δ 13.65 (s, 1H), 7.03 (s, 2H), 7.01 (s, 2H), 6.97 (d, 1H), 6.15 (d, 1H), 3.30-3.22 (m, 2H), 2.35 (s, 3H), 2.05 (m, 2H), 1.77 (m, 2H).

### methyl 2-(1-(6-amino-2-(5-methylfuran-2-yl)-9H-purin-8-yl)piperidin-3-yl)acetate (18bb)

A target compound (18bb) is obtained by the same synthetic method as for Compound (18a): ¹H NMR (600 MHz, DMSO-d₆) δ 7.01 (s, 2H), 6.99 (d, 1H), 6.11 (d, 1H), 3.71 (s, 3H), 3.62-3.59 (m, 2H2.35 (s, 3H), 2.05 (m, 2H), 1.75 (m, 2H).

### methyl 3-(1-(6-amino-2-(5-methylfuran-2-yl)-9H-purin-8-yl)piperidin-3-yl)propanoate (18bc)

A target compound (18bc) is obtained by the same synthetic method as for Compound (18a): ¹H NMR (600 MHz, DMSO-d₆) δ 7.01 (d, 1H), 6.95 (s, 2H), 6.13 (d, 1H), 3.75 (s, 3H), 3.64-3.58 (m, 2H), 2.40 (m, 2H), 2.37 (s, 3H), 2.07 (m, 2H), 1.75 (m, 2H).

### 8-(4-(4-(2-methoxyethoxy)phenyl)piperazin-1-yl)-2-(5-methylfuran-2-yl)-9H-purin-6-amine (18bd)

A target compound (18bd) is obtained by the same synthetic method as for Compound (18a): ¹H NMR (600 MHz, DMSO-d₆) δ 7.00 (s, 2H), 6.87 (d, 1H), 6.74 (d, 2H), 6.61 (d, 2H), 6.17 (d, 1H), 4.41 (t, J = 7.7 Hz, 2H), 3.78 (t, J = 7.2 Hz, 2H), 3.57 (m, 4H), 3.28 (m, 4H), 2.45 (s, 3H).

### 3-(1-(6-amino-2-(4,5-dimethylfuran-2-yl)-9H-purin-8-yl)piperidin-3-yl)propanamide (18be)

A target compound (18be) is obtained by the same synthetic method as for Compound (18a): ¹H NMR (600 MHz, DMSO-d₆) δ 7.03 (s, 2H), 6.98 (s, 2H), 3.65-3.61 (m, 2H), 2.35 (s, 3H), 2.01 (s, 3H), 2.05 (m, 2H), 1.77 (m, 2H).

### methyl 2-(1-(6-amino-2-(4,5-dimethylfuran-2-yl)-9H-purin-8-yl)piperidin-3-yl)acetate (18bf)

A target compound (18bf) is obtained by the same synthetic method as for Compound (18a): ¹H NMR (600 MHz, DMSO-d₆) δ 6.89 (s, 2H), 3.71 (s, 3H), 3.62-3.59 (m, 2H), 2.35 (s, 3H), 2.07 (s, 3H), 2.05 (m, 2H), 1.75 (m, 2H).

### methyl 3-(1-(6-amino-2-(4,5-dimethylfuran-2-yl)-9H-purin-8-yl)piperidin-3-yl)propanoate (18bg)

A target compound (18bg) is obtained by the same synthetic method as for Compound (18a): ¹H NMR (600 MHz, DMSO-d₆) δ 6.97 (s, 2H), 3.75 (s, 3H), 3.64-3.58 (m, 2H), 2.40 (m, 2H), 2.38 (s, 3H), 2.07 (m, 2H), 2.01 (s, 3H), 1.75 (m, 2H).

### 2-(4,5-dimethylfuran-2-yl)-8-(4-(4-(2-methoxyethoxy)phenyl)piperazin-1-yl)-9H-purin-6-amine (18bh)

A target compound (18bh) is obtained by the same synthetic method as for Compound (18a): ¹H NMR (600 MHz, DMSO-d₆) δ 7.02 (s, 2H), 6.74 (d, 2H), 6.61 (d, 2H), 4.41 (t, J = 7.7 Hz, 2H), 3.78 (t, J = 7.2 Hz, 2H), 3.57 (m, 4H), 3.28 (m, 4H), 2.39 (s, 3H), 2.11 (s, 3H).

### 2-(Hex-1-yn-1-yl)-8-(1H-pyrrol-2-yl)-9H-purin-6-amine (18bi)

A target compound (18bi) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 2-(Hex-1-yn-1-yl)-8-(pyridin-2-yl)-9H-purin-6-amine (18bj)

A target compound (18bj) is obtained by the same synthetic method as for Compound (18a): ¹H NMR (600 MHz, CD₃OD) δ 7.85 (dd, J = 1.2, 3.6 Hz, 1 H), 7.65 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 2-(Hex-1-yn-1-yl)-8-(pyrimidin-2-yl)-9H-purin-6-amine (18bk)

A target compound (18bk) is obtained by the same synthetic method as for Compound (18a): ¹H NMR (600 MHz, CD₃OD) δ 7.85 (dd, J = 1.2, 3.6 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 2-(Hex-1-yn-1-yl)-8-(1-methyl-1H-imidazol-2-yl)-9H-purin-6-amine (18b1)

A target compound (18bl) is obtained by the same synthetic method as for Compound (18a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H), 0.91 (t, J = 7.6 Hz, 3 H).

### 2-(Hex-1-yn-1-yl)-8-(thiazol-2-yl)-9H-purin-6-amine (18bm)

A target compound (18bm) is obtained by the same synthetic method as for Compound (18a): ¹H NMR (600 MHz, CD₃OD) δ 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### 8-(Benzo[d]thiazol-2-yl)-2-(hex-1-yn-1-yl)-9H-purin-6-amine (18bn)

A target compound (18bn) is obtained by the same synthetic method as for Compound (18a): ¹H NMR (600 MHz, CD₃OD) δ 9.47 (dd, J = 1.2, 4.8 Hz, 1 H), 8.09 (dd, J = 3.6, 5.2 Hz, 1 H), 7.47 (dd, J = 1.2, 4.8 Hz, 1 H), 7.09 (dd, J = 3.6, 5.2 Hz, 1 H), 2.53 (t, J = 6.8 Hz, 2 H), 1.61-1.69 (m, 2 H), 1.49-1.58 (m, 2 H), 0.98 (t, J = 7.6 Hz, 3 H).

### Preparation Example 9: Synthesis of 20a to 20x

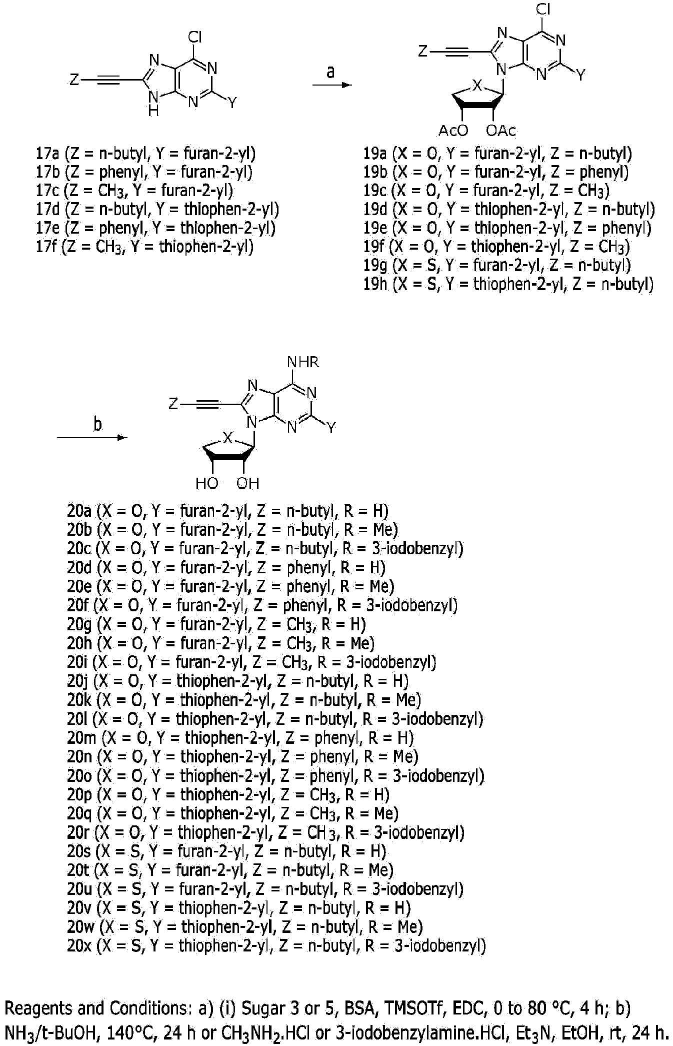

### (2R,3R,4R)-2-(6-Chloro-2-(furan-2-yl)-8-(hex-1-yn-1-yl)-9H-purin-9-yl)tetrahydrofuran-3,4-diyl diacetate (19a)

After Starting Material (17a) (0.5 mmol) is dissolved in EDC (5 mL), N,O-bis(trimethylsilyl)acetamide (BSA) (1.44 eq) is slowly added thereto, the resulting mixture is stirred at 40°C for 1 hour, and Sugar 3 (1.2 eq) is dissolved in EDC (2 mL) and slowly added thereto. After the reaction mixture is cooled to 0°C, TMSOTf (0.68 eq) is added thereto, and the resulting mixture is warmed to 80°C and stirred for 3 hours. After it is confirmed that the reaction is completed, an aqueous saturated NaHCOs solution is added thereto, and then the resultant is extracted with DCM and concentrated under reduced pressure. The resulting residue is purified by column chromatography under the condition of hexane/EtOAc = 4/1 to obtain a target compound (19a) (58%): ¹H NMR (600 MHz, CDCl₃) δ 7.64 (d, J = 0.9 Hz, 1H), 7.36 (d, J = 3.2 Hz, 1H), 6.58 (q, J = 1.7 Hz, 1H), 6.33 (t, J = 4.6 Hz, 2H), 5.94 (t, J = 2.1 Hz, 1H), 4.89 (dd, J = 10.3, 3.9 Hz, 1H), 4.21 (dd, J = 10.3, 2.1 Hz, 1H), 2.58 (t, J = 7.1 Hz, 2H), 2.20 (s, 3H), 2.08 (s, 3H), 1.68 (q, J = 7.5 Hz, 2H), 1.59-1.50 (m, 2H), 0.98 (t, J = 7.3 Hz, 3H).

### (2R,3R,4R)-2-(6-Chloro-2-(furan-2-yl)-8-(phenylethynyl)-9H-purin-9-yl)tetrahydrofuran-3,4-diyl diacetate (19b)

A target compound (19b) is obtained using Sugar 3 by the same synthetic method as for Compound (19a): ¹H NMR (600 MHz, CDCl₃) δ 7.65 (dd, J = 8.2, 1.4 Hz, 3H), 7.51-7.41 (m, 3H), 7.39-7.38 (m, 1H), 6.59 (q, J = 1.7 Hz, 1H), 6.43 (d, J = 5.5 Hz, 1H), 6.37 (t, J = 5.0 Hz, 1H), 5.98-5.95 (m, 1H), 4.92 (dd, J = 10.5, 4.1 Hz, 1H), 4.24 (dd, J = 10.4, 2.2 Hz, 1H), 2.21 (s, 3H), 2.08 (s, 3H).

### (2R,3R,4R)-2-(6-Chloro-2-(furan-2-yl)-8-(prop-1-yn-1-yl)-9H-purin-9-yl)tetrahydrofuran-3,4-diyl diacetate (19c)

A target compound (19c) is obtained using Sugar 3 by the same synthetic method as for Compound (19a): ¹H NMR (600 MHz, CDCl₃) δ 7.64 (s, 1H), 7.37 (s, 1H), 6.59 (s, 1H), 6.37-6.32 (m, 2H), 5.96 (s, 1H), 4.87 (d, J = 10.1 Hz, 1H), 4.21 (d, J = 10.6 Hz, 1H), 2.24 (t, J = 0.9 Hz, 3H), 2.21 (t, J = 1.1 Hz, 3H), 2.14-2.09 (m, 3H).

### (2R,3R,4R)-2-(6-Chloro-8-(hex-1-yn-1-yl)-2-(thiophen-2-yl)-9H-purin-9-yl)tetrahydrofuran-3,4-diyl diacetate (19d)

A target compound (19d) is obtained using Sugar 3 by the same synthetic method as for Compound (19a): ¹H NMR (600 MHz, CDCl₃) δ 8.03 (q, J = 1.7 Hz, 1H), 7.49 (dd, J = 5.1, 0.9 Hz, 1H), 7.15 (dd, J = 5.1, 3.7 Hz, 1H), 6.37-6.31 (m, 2H), 5.93 (t, J = 3.4 Hz, 1H), 4.90 (dd, J = 10.6, 3.7 Hz, 1H), 4.24 (dd, J = 10.6, 1.8 Hz, 1H), 2.58 (t, J = 6.9 Hz, 2H), 2.20 (d, J = 4.6 Hz, 3H), 2.09-2.07 (m, 3H), 1.69 (t, J = 7.4 Hz, 2H), 1.53 (q, J = 7.5 Hz, 2H), 0.98 (t, J = 7.4 Hz, 3H).

### (2R,3R,4R)-2-(6-Chloro-8-(phenylethynyl)-2-(thiophen-2-yl)-9H-purin-9-yl)tetrahydrofuran-3,4-diyl diacetate (19e)

A target compound (19e) is obtained using Sugar 3 by the same synthetic method as for Compound (19a): ¹H NMR (600 MHz, CDCl₃) δ 8.04 (d, J = 3.2 Hz, 1H), 7.66 (d, J = 6.9 Hz, 2H), 7.51-7.42 (m, 4H), 7.15 (t, J = 4.3 Hz, 1H), 6.41 (s, 2H), 5.95 (s, 1H), 4.93 (dd, J = 10.5, 4.1 Hz, 1H), 4.27 (d, J = 10.5 Hz, 1H), 2.22 (s, 3H), 2.08 (s, 3H).

### (2R,3R,4R)-2-(6-Chloro-8-(prop-1-yn-1-yl)-2-(thiophen-2-yl)-9H-purin-9-yl)tetrahydrofuran-3,4-diyl diacetate (19f)

A target compound (19f) is obtained using Sugar 3 by the same synthetic method as for Compound (19a): ¹H NMR (600 MHz, CDCl₃) δ 8.01 (q, J = 1.7 Hz, 1H), 7.48 (dd, J = 5.0, 0.9 Hz, 1H), 7.13 (dd, J = 5.0, 4.1 Hz, 1H), 6.40 (t, J = 5.3 Hz, 1H), 6.29 (d, J = 5.5 Hz, 1H), 5.94-5.92 (m, 1H), 4.86 (dd, J = 10.5, 3.7 Hz, 1H), 4.22 (dd, J = 10.1, 1.8 Hz, 1H), 2.23 (s, 3H), 2.20 (s, 3H), 2.08 (s, 3H).

### (2R,3R,4S)-2-(6-Chloro-2-(furan-2-yl)-8-(hex-1-yn-1-yl)-9H-purin-9-yl)tetrahydrothiophene-3,4-diyl diacetate (19g)

A target compound (19g) is obtained using Sugar 5 by the same synthetic method as for Compound (19a): ¹H NMR (600 MHz, DMSO) δ (ppm): 7.96 (dd, 1 H, J = 8.0, 6.8, Hz), 7.29 (dd, 1 H, J = 3.6, 0.8 Hz), 6.73 (dd, 1 H, J = 3.6, 1.6 Hz), 5.32 (m, 1 H), 4.88 (m, 2 H), 2.88 (m, 2 H), 2.67 (t, 2 H, J = 6.8 Hz), 2.24 (s, 6 H), 1.67 (m, 2 H), 1.52 (m, 2 H), 0.92 (t, 3H, J = 7.2 Hz).

### (2R,3R,4S)-2-(6-Chloro-2-(thiophene-2-yl)-8-(hex-1-yn-1-yl)-9H-purin-9-yl)tetrahydrothiophene-3,4-diyl diacetate (19h)

A target compound (19h) is obtained using Sugar 5 by the same synthetic method as for Compound (19a): ¹H NMR (600 MHz, DMSO) δ (ppm): 7.91 (dd, 1 H, J = 8.0, 6.8, Hz), 7.42 (dd, 1 H, J = 3.6, 0.8 Hz), 7.13 (dd, 1 H, J = 3.6, 0.9 Hz), 5.32 (m, 1 H), 4.87 (m, 2 H), 2.90 (m, 2 H), 2.67 (t, 2 H, J = 6.8 Hz), 2.25 (s, 6 H), 1.67 (m, 2 H), 1.52 (m, 2 H), 0.92 (t, 3 H, J = 7.2 Hz).

### (2R,3R,4R)-2-(6-Amino-2-(furan-2-yl)-8-(hex-1-yn-1-yl)-9H-purin-9-yl)tetrahydrofuran-3,4-diol (20a)

A target compound (20a) is obtained by the same synthetic method as for Compound (18a): ¹H NMR (600 MHz, MeOD) δ 7.69 (s, 1 H), 7.20 (d, J = 3.2 Hz, 1 H), 6.61 (q, J = 1.6, 2.8 Hz, 1 H), 6.19 (d, J = 6.4 Hz, 1 H), 5.4 (t, J = 5.2 Hz, 1 H), 4.70 (dd, J = 3.6, 9.6 Hz, 1 H), 4.59 (t, J = 6.8 Hz, 1 H), 4.06 (d, J = 9.6 Hz, 1 H), 2.63 (t, J = 7.2, 2 H), 1.67-1.73 (m, 2 H), 1.55-1.61 (m, 2 H), 1.03 (t, J = 14.8 Hz, 3 H).

### (2R,3R,4R)-2-(2-(Furan-2-yl)-8-(hex-1-yn-1-yl)-6-(methylamino)-9H-purin-9-yl)tetrahydrofuran-3,4-diol (20b)

A target compound (20b) is obtained by the same synthetic method as for Compound (18b): ¹H NMR (600 MHz, MeOD) δ 7.62 (dd, J = 0.8, 1.7 Hz, 1 H), 7.16 (dd, J = 0.6, 3.3 Hz, 1 H), 6.54 (dd, J = 1.7, 3.3 Hz, 1 H), 6.13 (d, J = 6.1 Hz, 1 H), 5.34 (dd, J = 4.7, 6.0 Hz, 1 H), 4.65 (dd, J = 3.4, 9.5 Hz, 1 H), 4.52-4.54 (m, 1 H), 3.99 (d, J = 1.4, 9.5 Hz, 1 H), 3.14 (bs, 3 H), 2.56 (t, J = 6.9, 2 H), 1.61-1.66 (m, 2 H), 1.48-1.56 (m, 2 H), 0.96 (t, J = 7.3 Hz, 3 H).

### (2R,3R,4R)-2-(2-(Furan-2-yl)-8-(hex-1-yn-1-yl)-6-((3-iodobenzyl)amino)-9H-purin-9-yl)tetrahydrofuran-3,4-diol (20c)

A target compound (20c) is obtained by the same synthetic method as for Compound (18e): ¹H NMR (600 MHz, CDCl₃) δ 7.76 (s, 1 H), 7.58 (d, J = 7.8 Hz, 1 H), 7.51 (d, J = 0.7 Hz, 1 H), 7.34 (d, J = 7.7 Hz, 1 H), 7.06 (d, J = 3.2 Hz, 1 H), 7.03 (t, J = 7.7 Hz, 1 H), 6.47 (dd, J = 1.7, 3.3 Hz, 1 H), 6.13 (d, J = 5.4 Hz, 1 H) 4.78 (bs, 2 H), 4.67 (bs, 1 H), 4.58 (dd, J = 3.7, 9.8 Hz, 1H), 4.10 (dd, J = 1.3, 9.8 Hz, 1 H), 3.12 (bs, 1 H), 2.42 (t, J = 7.0 Hz, 2 H), 1.54-1.62 (m, 2 H), 1.39-1.48 (m, 2 H), 0.91 (t, J = 7.2 Hz, 3 H).

### (2R,3R,4R)-2-(6-Amino-2-(furan-2-yl)-8-(phenylethynyl)-9H-purin-9-yl)tetrahydrofuran-3,4-diol (20d)

A target compound (20d) is obtained by the same synthetic method as for Compound (18a): ¹H NMR (600 MHz, MeOD) δ 7.64-7.67 (m, 3 H), 7.42-7.48 (m, 3 H), 7.18 (dd, J = 0.7, 3.4 Hz, 1H), 6.56 (dd, J = 1.7, 3.4 Hz, 1 H), 6.24 (d, J = 6.1 Hz, 1 H), 5.39 (dd, J = 4.6, 6.1 Hz, 1 H), 4.69 (dd, J = 3.3, 9.5 Hz, 1 H), 4.53-4.57 (m, 1 H), 4.04 (dd, J = 1.3, 9.6 Hz, 1 H).

### (2R,3R,4R)-2-(2-(Furan-2-yl)-6-(methylamino)-8-(phenylethynyl)-9H-purin-9-yl)tetrahydrofuran-3,4-diol (20e)

A target compound (20e) is obtained by the same synthetic method as for Compound (18b): ¹H NMR (600 MHz, MeOD) δ 7.63-7.67 (m, 3 H), 7.42-7.50 (m, 3 H), 7.19 (d, J = 2.9 Hz, 1 H), 6.57 (dd, J = 1.3, 3.4 Hz, 1 H), 6.24 (d, J = 6.2 Hz, 1 H), 5.40 (dd, J = 4.7, 6.1 Hz, 1 H), 4.70 (dd, J = 3.4, 9.5 Hz, 1 H), 4.57 (bs, 1 H), 4.53-4.56 (m, 1 H), 4.03 (dd, J = 1.3, 9.5 Hz, 1 H), 3.16 (s, 3 H).

### (2R,3R,4R)-2-(2-(Furan-2-yl)-6-((3-iodobenzyl)amino)-8-(phenylethynyl)-9H-purin-9-yl)tetrahydrofuran-3,4-diol (20f)

A target compound (20f) is obtained by the same synthetic method as for Compound (18e): ¹H NMR (600 MHz, DMSO-d₆) δ 8.81 (s, 1H), 7.86 (d, J = 5.9 Hz, 2H), 7.69-7.67 (m, 2H), 7.59 (d, J = 8.2 Hz, 1H), 7.56-7.50 (m, 3H), 7.45 (d, J = 7.3 Hz, 1H), 7.19 (d, J = 2.7 Hz, 1H), 7.13 (t, J = 7.5 Hz, 1H), 6.65 (q, J = 1.7 Hz, 1H), 6.11 (d, J = 6.4 Hz, 1H), 5.52 (d, J = 6.4 Hz, 1H), 5.30 (d, J = 3.7 Hz, 1H), 5.17 (t, J = 5.7 Hz, 2H), 4.71 (d, J = 5.0 Hz, 1H), 4.52 (dd, J = 9.1, 3.2 Hz, 1H), 4.41 (d, J = 3.2 Hz, 1H), 3.92 (d, J = 9.1 Hz, 1H);

### (2R,3R,4R)-2-(6-Amino-2-(furan-2-yl)-8-(prop-1-yn-1-yl)-9H-purin-9-yl)tetrahydrofuran-3,4-diol (20g)

A target compound (20g) is obtained by the same synthetic method as for Compound (18a): ¹H NMR (600 MHz, CD₃OD δ 7.67 (s, 1H), 7.18 (d, J = 3.7 Hz, 1H), 6.59 (q, J = 1.7 Hz, 1H), 6.16 (d, J = 5.9 Hz, 1H), 5.37 (t, J = 5.3 Hz, 1H), 4.67 (dd, J = 9.6, 3.2 Hz, 1H), 4.55 (t, J = 3.9 Hz, 1H), 4.03 (d, J = 9.6 Hz, 1H), 2.22 (s, 3H);

### (2R,3R,4R)-2-(2-(Furan-2-yl)-6-(methylamino)-8-(prop-1-yn-1-yl)-9H-purin-9-yl)tetrahydrofuran-3,4-diol (20h)

A target compound (20h) is obtained by the same synthetic method as for Compound (18b): ¹H NMR (600 MHz, CD₃OD) δ 7.61 (s, 1H), 7.15 (d, J = 2.7 Hz, 1H), 6.53 (q, J = 1.7 Hz, 1H), 6.12 (d, J = 6.4 Hz, 1H), 5.34 (t, J = 5.5 Hz, 1H), 4.64 (dd, J = 9.4, 3.4 Hz, 1H), 4.52 (d, J = 3.7 Hz, 1H), 3.98 (d, J = 9.6 Hz, 1H), 3.13 (s, 3H), 2.17 (s, 3H);

### (2R,3R,4R)-2-(2-(Furan-2-yl)-6-((3-iodobenzyl)amino)-8-(prop-1-yn-1-yl)-9H-purin-9-yl)tetrahydrofuran-3,4-diol (20i)

A target compound (20i) is obtained by the same synthetic method as for Compound (18e): ¹H NMR (600 MHz, CDCl₃) δ 7.79 (s, 1H), 7.60 (d, J = 7.8 Hz, 1H), 7.51 (s, 1H), 7.36 (d, J = 7.8 Hz, 1H), 7.07 (d, J = 7.8 Hz, 2H), 6.47 (s, 1H), 6.14 (d, J = 5.5 Hz, 1H), 5.41 (s, 1H), 4.77 (s, 2H), 4.64 (s, 1H), 4.58 (dd, J = 9.6, 3.7 Hz, 1H), 4.11 (d, J = 9.6 Hz, 1H), 2.07 (s, 3H);

### (2R,3R,4R)-2-(6-Amino-8-(hex-1-yn-1-yl)-2-(thiophen-2-yl)-9H-purin-9-yl)tetrahydrofuran-3,4-diol (20j)

A target compound (20j) is obtained by the same synthetic method as for Compound (18a): ¹H NMR (600 MHz, CDCl₃) δ 7.82 (dd, J = 1.2, 3.6 Hz, 1 H), 7.45 (dd, J = 1.2, 5.0 Hz, 1 H), 7.07 (dd, J = 3.6, 5.0 Hz, 1 H), 6.11 (d, J = 6.0 Hz, 1 H), 5.39 (dd, J = 4.7, 5.9 Hz, 1 H), 4.67 (dd, J = 3.3, 9.4 Hz, 1 H), 4.53-4.56 (m, 1 H), 4.01 (dd, J = 1.4, 9.5 Hz, 1 H), 2.56 (t, J = 6.9 Hz, 2 H), 1.61-1.67 (m, 2 H), 1.49-1.55 (m, 2 H), 0.97 (t, J = 7.3 Hz, 3 H).

### (2R,3R,4R)-2-(8-(Hex-1-yn-1-yl)-6-(methylamino)-2-(thiophen-2-yl)-9H-purin-9-yl)tetrahydrofuran-3,4-diol (20k)

A target compound (20k) is obtained by the same synthetic method as for Compound (18b): ¹H NMR (600 MHz, MeOD) δ 7.84 (dd, J = 0.8, 3.6 Hz, 1 H), 7.42 (dd, J = 1.2, 5.2 Hz, 1 H), 7.06 (dd, J = 3.6, 4.8 Hz, 1 H), 6.10 (d, J = 6.0 Hz, 1 H), 5.40 (t, J = 4.4 Hz, 1 H), 4.68 (dd, J = 3.6, 9.6 Hz, 1 H), 4.55-4.57 (m, 1 H), 4.02 (dd, J = 1.6, 10.0 Hz, 1 H), 3.14 (bs, 3 H), 2.53 (t, J = 6.8 Hz, 2 H), 1.59-1.67 (m, 2 H), 1.47-1.54 (m, 2 H), 0.95 (t, J = 7.6 Hz, 3 H).

### (2R,3R,4R)-2-(8-(Hex-1-yn-1-yl)-6-((3-iodobenzyl)amino)-2-(thiophen-2-yl)-9H-purin-9-yl)tetrahydrofuran-3,4-diol (201)

A target compound (201) is obtained by the same synthetic method as for Compound (18e): ¹H NMR (600 MHz, CDCl₃) δ 7.76-7.80 (m, 2 H), 7.56 (d, J = 7.8 Hz, 1 H), 7.31-7.37 (m, 2 H), 7.00-7.07 (m, 2 H), 6.22 (bs, 1 H), 6.06 (d, J = Hz, 1 H), 5.44 (t, J = 0.8 Hz, 1 H), 4.66-4.91 (m, 3 H), 4.60 (dd, J = 3.6, 9.8 Hz, 1 H), 4.10 (dd, J = 1.3, 9.8 Hz, 1 H), 3.13-3.23 (m, 1 H), 2.36 (t, J = 7.0 Hz, 2 H), 1.50-1.58 (m, 2 H), 1.35-1.44 (m, 2 H), 0.88 (t, J = 7.2 Hz, 3 H).

### (2R,3R,4R)-2-(6-Amino-8-(phenylethynyl)-2-(thiophen-2-yl)-9H-purin-9-yl)tetrahydrofuran-3,4-diol (20m)

A target compound (20m) is obtained by the same synthetic method as for Compound (18a): ¹H NMR (600 MHz, MeOD) δ 7.85 (dd, J = 1.2, 3.6 Hz, 1 H), 7.64-7.68 (m, 2 H), 7.42-7.52 (m, 4 H), 7.08 (dd, J = 3.6, 5.0 Hz, 1 H), 6.22 (d, J = 6.0 Hz, 1 H), 5.45 (dd, J = 4.7, 6.0 Hz, 1 H), 4.72 (dd, J = 3.3, 9.5 Hz, 1 H), 4.55-4.57 (m, 1 H), 4.05 (dd, J = 1.3, 9.5 Hz, 1H).

### (2R,3R,4R)-2-(6-(Methylamino)-8-(phenylethynyl)-2-(thiophen-2-yl)-9H-purin-9-yl)tetrahydrofuran-3,4-diol (20n)

A target compound (20n) is obtained by the same synthetic method as for Compound (18b): ¹H NMR (600 MHz, MeOD) δ 7.87 (dd, J = 1.0, 3.6 Hz, 1 H), 7.63-7.67 (m, 2 H), 7.41-7.50 (m, 4 H), 7.08 (dd, J = 3.6, 5.0 Hz, 1 H), 6.22 (d, J = 6.0 Hz, 1 H), 5.46 (dd, J = 4.7, 5.8 Hz, 1 H), 4.72 (dd, J = 3.3, 9.5 Hz, 1 H), 4.05 (dd, J = 1.3, 9.5 Hz, 1 H), 3.17 (s, 3 H).

### (2R,3R,4R)-2-(6-((3-iodobenzyl)amino)-8-(phenylethynyl)-2-(thiophen-2-yl)-9H-purin-9-yl)tetrahydrofuran-3,4-diol (20o)

A target compound (20o) is obtained by the same synthetic method as for Compound (18e): ¹H NMR (600 MHz, DMSO-d₆) δ 8.82 (bs, 1 H), 7.79-7.83 (m, 2 H), 7.61-7.65 (m, 3 H), 7.42-7.55 (m, 5 H), 7.07-7.13 (m, 2 H), 6.04 (d, J = 6.3 Hz, 1 H), 5.50 (bs, 1 H), 5.27 (bs, 1 H), 5.14 (bs, 1 H), 4.65 (d, J = 4.9 Hz, 2 H), 4.50 (dd, J = 2.8, 9.2 Hz, 1 H), 4.38 (bs, 1 H), 3.90 (d, J = 9.2 Hz, 1 H).

### (2R,3R,4R)-2-(6-Amino-8-(prop-1-yn-1-yl)-2-(thiophen-2-yl)-9H-purin-9-yl)tetrahydrofuran-3,4-diol (20p)

A target compound (20p) is obtained by the same synthetic method as for Compound (18e): ¹H NMR (600 MHz, CD₃OD) δ 7.85 (q, J = 1.7 Hz, 1H), 7.48 (dd, J = 5.0, 1.4 Hz, 1H), 7.10 (dd, J = 5.0, 3.7 Hz, 1H), 6.14 (d, J = 6.4 Hz, 1H), 5.43 (t, J = 5.5 Hz, 1H), 4.71 (dd, J = 9.6, 3.2 Hz, 1H), 4.57 (t, J = 3.4 Hz, 1H), 4.05 (d, J = 9.6 Hz, 1H), 2.22 (s, 3H);

### (2R,3R,4R)-2-(6-(Methylamino)-8-(prop-1-yn-1-yl)-2-(thiophen-2-yl)-9H-purin-9-yl)tetrahydrofuran-3,4-diol (20q)

A target compound (20q) is obtained by the same synthetic method as for Compound (18b): ¹H NMR (600 MHz, CD₃OD) δ 7.88 (d, J = 3.7 Hz, 1H), 7.47 (d, J = 5.0 Hz, 1H), 7.10 (t, J = 4.3 Hz, 1H), 6.13 (d, J = 5.9 Hz, 1H), 5.43 (t, J = 5.5 Hz, 1H), 4.71 (dd, J = 9.6, 3.2 Hz, 1H), 4.57 (t, J = 3.4 Hz, 1H), 4.04 (d, J = 9.4 Hz, 1H), 3.17 (s, 3H), 2.21 (s, 3H);

### (2R,3R,4R)-2-(6-((3-iodobenzyl)amino)-8-(prop-1-yn-1-yl)-2-(thiophen-2-yl)-9H-purin-9-yl)tetrahydrofuran-3,4-diol (20r)

A target compound (20r) is obtained by the same synthetic method as for Compound (18e): ¹H NMR (600 MHz, CDCl₃) δ 7.78 (s, 2H), 7.58 (d, J = 7.8 Hz, 1H), 7.35 (t, J = 7.8 Hz, 2H), 7.04 (t, J = 7.8 Hz, 2H), 6.32 (s, 1H), 6.08 (d, J = 5.1 Hz, 1H), 5.50 (s, 1H), 4.70 (d, J = 28.5 Hz, 2H), 4.61 (d, J = 9.7 Hz, 1H), 4.11 (d, J = 10.1 Hz, 1H), 2.01 (s, 3H);

### (2R,3R,4S)-2-(6-Amino-2-(furan-2-yl)-8-(hex-1-yn-1-yl)-9H-purin-9-yl)tetrahydrothiophene-3,4-diol (20s).

A target compound (20s) is obtained by the same synthetic method as for Compound (18a): ¹H NMR (600 MHz, DMSO-d₆) δ 7.80 (s, 1 H), 7.46 (bs, 2 H, D₂O exchangeable), 7.10 (d, J = 3.3 Hz, 1 H), 6.62 (dd, J = 1.6, 3.0 Hz, 1 H), 6.05 (d, J = 7.5 Hz, 1 H), 5.45 (bs, 1 H, D₂O exchangeable), 5.34 (s, 1 H), 5.26 (bs, 1 H, D₂O exchangeable), 4.45-4.50 (m, 1 H), 3.46 (dd, J = 3.3, 11.2 Hz, 1 H), 2.85 (d, J = 9.7 Hz, 1 H), 2.60 (t, J = 6.8 Hz, 2 H), 1.55-1.63 (m, 2 H), 1.42-1.53 (m, 2 H), 0.93 (t, J = 7.3 Hz, 3 H); ¹H NMR (600 MHz, MeOD) δ 7.80 (d, J = 1.6 Hz, 1 H), 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4S)-2-(2-(Furan-2-yl)-8-(hex-1-yn-1-yl)-6-(methylamino)-9H-purin-9-yl)tetrahydrothiophene-3,4-diol (20t)

A target compound (20t) is obtained by the same synthetic method as for Compound (18b): ¹H NMR (800 MHz, DMSO-d₆) δ 7.95 (bs, 1 H), 7.82 (s, 1 H), 7.17 (bs, 1 H), 6.63 (dd, J = 1.7, 3.2 Hz, 1 H), 6.06 (d, J = 7.5 Hz, 1 H), 5.48 (d, J = 6.4 Hz, 1 H), 5.36 (d, J = 3.6 Hz, 1 H), 5.25-5.27 (m, 1 H), 4.46-4.48 (m, 1 H), 3.48 (dd, J = 3.5, 11.2 Hz, 1 H), 3.00 (bs, 3 H), 2.85 (dd, J = 2.1, 11.2 Hz, 1 H), 2.60 (t, J = 6.9 Hz, 2 H), 1.57-1.60 (m, 2 H), 1.44-1.49 (m, 2 H), 0.93 (t, J = 7.3 Hz, 3 H).

### (2R,3R,4S)-2-(2-(Furan-2-yl)-8-(hex-1-yn-1-yl)-6-((3-iodobenzyl)amino)-9H-purin-9-yl)tetrahydrothiophene-3,4-diol (20u)

A target compound (20u) is obtained by the same synthetic method as for Compound (18e): ¹H NMR (800 MHz, MeOD) δ 7.87 (s, 1 H), 7.65 (d, J = 0.7 Hz, 1 H), 7.57 (d, J = 7.8 Hz, 1 H), 7.44 (d, J = 7.7 Hz, 1 H), 7.16 (d, J = 3.2 Hz, 1 H), 7.07 (t, J = 7.7 Hz, 1 H), 6.56 (dd, J = 1.6, 3.2 Hz, 1 H), 6.23 (d, J = 7.0 Hz, 1 H), 5.44 (dd, J = 3.5, 7.0 Hz, 1 H), 4.78 (bs, 2 H), 4.66 (dd, J = 3.4, 6.0 Hz, 1 H), 3.68 (dd, J = 3.6, 11.4 Hz, 1 H), 2.96 (dd, J = 2.4, 11.4 Hz, 1 H), 2.58 (t, J = 7.0 Hz, 2 H), 1.65-1.68 (m, 2 H), 1.52-1.56 (m, 2 H), 0.98 (t, J = 7.3 Hz, 3 H).

### (2R,3R,4S)-2-(6-Amino-8-(hex-1-yn-1-yl)-2-(thiophen-2-yl)-9H-purin-9-yl)tetrahydrothiophene-3,4-diol (20v)

A target compound (20v) is obtained by the same synthetic method as for Compound (18a): ¹H NMR (600 MHz, DMSO-d₆) δ 7.76 (d, J = 4.0 Hz, 1 H), 7.58 (d, J = 5.2 Hz, 1 H), 7.45 (bs, 2 H), 7.10 (t, J = 4.4 Hz, 1 H), 5.96 (d, J = 7.6 Hz, 1 H), 5.47 (d, J = 6.0 Hz, 1 H), 5.32 (d, J = 3.6 Hz, 1 H), 5.23-5.29 (m, 1 H), 4.47 (bs, 1 H), 3.48 (dd, J = 3.2, 11.2 Hz, 1 H), 2.81-2.87 (m, 1 H), 2.56 (t, J = 6.8 Hz, 2 H), 1.51-1.60 (m, 2 H), 1.37-1.49 (m, 2 H), 0.89 (t, J = 6.8 Hz, 3 H).

### (2R,3R,4S)-2-(8-(Hex-1-yn-1-yl)-6-(methylamino)-2-(thiophen-2-yl)-9H-purin-9-yl)tetrahydrothiophene-3,4-diol (20w)

A target compound (20w) is obtained by the same synthetic method as for Compound (18b): ¹H NMR (600 MHz, MeOD) δ 7.89 (d, J = 2.4 Hz, 1 H), 7.46 (dd, J = 0.9, 5.0 Hz, 1 H), 7.09 (dd, J = 3.6, 5.0 Hz, 1 H), 6.19 (d, J = 6.8 Hz, 1 H), 5.51 (dd, J = 3.6, 6.8 Hz, 1 H), 4.70 (dd, J = 3.1, 5.8 Hz, 1 H), 3.71 (dd, J = 3.6, 11.4 Hz, 1 H), 3.16 (bs, 3 H), 2.98 (dd, J = 2.7, 11.4 Hz, 1 H), 2.59 (t, J = 7.3 Hz, 2 H), 1.65-1.70 (m, 2 H), 1.52-1.58 (m, 2 H), 0.99 (t, J = 7.3 Hz, 3 H).

### (2R,3R,4S)-2-(8-(Hex-1-yn-1-yl)-6-((3-iodobenzyl)amino)-2-(thiophen-2-yl)-9H-purin-9-yl)tetrahydrothiophene-3,4-diol (20x)

A target compound (20x) is obtained by the same synthetic method as for Compound (18e): ¹H NMR (600 MHz, CDCl₃) δ 7.81 (bs, 1 H), 7.76 (s, 1 H), 7.56 (d, J = 7.8 Hz, 1 H), 7.28-7.45 (m, 2 H), 6.97-7.14 (m, 2 H), 6.31 (bs, 1 H), 6.04 (d, J = 6.3 Hz, 1 H), 5.47 (dd, J = 3.7, 6.3 Hz, 1 H), 4.73 (bs, 2 H), 3.70 (dd, J = 3.7, 11.6 Hz, 1 H), 3.04 (dd, J = 2.1, 11.6 Hz, 1 H), 3.00 (bs, 1 H), 2.37 (t, J = 7.0 Hz, 2 H), 1.52-1.59 (m, 2 H), 1.36-1.45 (m, 2 H), 0.89 (t, J = 9.2 Hz, 3 H).

### Preparation Example 10: Synthesis of 21a to 21r

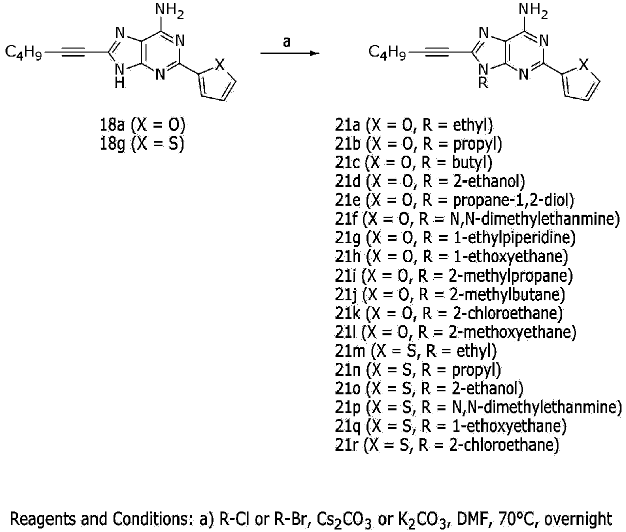

### 9-ethyl-2-(furan-2-yl)-8-(hex-1-yn-1-yl)-9H-purin-6-amine (21a)

A target compound (21a) is obtained by the same synthetic method as for Compound (14a): ¹H NMR (600 MHz, CDCl₃) δ 7.59 (dd, 1 H, J = 1.7, 0.9 Hz), 7.22 (dd, 1 H, J = 3.4, 0.9 Hz), 6.53 (dd, 1 H, J = 3.4, 1.8 Hz), 5.63 (brs, 2 H), 4.43 (q, 2 H, J = 7.3 Hz), 2.54 (t, 2 H, J = 7.1 Hz), 1.67 (m, 2H), 1.53 (m, 2 H), 1.49 (t, 3 H, J = 7.3 Hz), 0.97 (t, 3 H, J = 7.3 Hz).

### 2-(furan-2-yl)-8-(hex-1-yn-1-yl)-9-propyl-9H-purin-6-amine (21b)

A target compound (21b) is obtained by the same synthetic method as for Compound (14a): ¹H NMR (600 MHz, CDCl₃) δ 7.58 (dd, 1 H, J = 1.7, 0.9Hz), 7.22 (dd, 1 H, J = 3.4, 0.9 Hz), 6.53 (dd, 1 H, J = 3.4, 1.8 Hz), 5.54 (brs, 2 H), 4.25 (m, 2 H), 2.54 (t, 2 H, J = 7.0 Hz), 1.95 (m, 2 H), 1.67 (m, 2 H), 1.51 (m, 2 H), 0.97 (t, 3 H, J = 7.3 Hz), 0.97 (t, 3 H, J = 7.3 Hz).

### 9-butyl-2-(furan-2-yl)-8-(hex-1-yn-1-yl)-9H-purin-6-amine (21c)

A target compound (21c) is obtained by the same synthetic method as for Compound (14a): ¹H NMR (600 MHz, CDCl₃) δ 7.58 (dd, 1 H, J = 1.7, 0.9 Hz), 7.22 (dd, 1 H, J = 3.4, 0.9 Hz), 6.53 (dd, 1 H, J = 3.4, 1.8 Hz), 5.59 (brs, 2 H), 4.28 (t, 1 H, J = 7.2 Hz), 2.54 (t, 1 H, J = 7.0 Hz), 1.89 (m, 2 H), 1.66 (m, 2 H), 1.52 (m, 2 H), 1.37 (m, 2 H), 0.97 (t, 3 H, J = 7.3 Hz), 0.97 (t, 3 H, J = 7.3 Hz).

### 2-(6-amino-2-(furan-2-yl)-8-(hex-1-yn-1-yl)-9H-purin-9-yl)ethanol (21d)

A target compound (21d) is obtained by the same synthetic method as for Compound (14d): ¹H NMR (600 MHz, CDCl₃) δ 7.58 (dd, 1 H, J = 1.7, 0.8 Hz), 7.19 (dd, 1 H, J = 3.4, 0.9 Hz), 6.53 (dd, 1 H, J = 3.4, 1.8 Hz), 5.65 (m, 3 H), 4.43 (m, 2 H), 4.10 (m, 2 H), 2.54 (t, 2 H, J = 7.1 Hz), 1.67 (m, 2 H), 1.50 (m, 2 H), 0.97 (t, 3 H, J = 7.3 Hz).

### 3-(6-amino-2-(furan-2-yl)-8-(hex-1-yn-1-yl)-9H-purin-9-yl)propane-1,2-diol (21e)

A target compound (21e) is obtained by the same synthetic method as for Compound (14a): ¹H NMR (600 MHz, CDCl₃) δ 7.58 (dd, 1 H, J = 1.7, 0.8 Hz), 7.19 (dd, 1 H, J = 3.4, 0.9 Hz), 6.54 (dd, 1 H, J = 3.4, 1.8 Hz), 5.75 (brs, 2 H), 4.43 (m, 2 H), 4.39 (m, 1 H), 4.04 (brs, 2 H), 3.56 (m, 2 H), 2.53 (t, 2 H, J = 7.1 Hz), 1.65 (m, 2 H), 1.50 (m, 2 H), 0.96 (t, 3 H, J = 7.3 Hz).

### 9-(2-(dimethylamino)ethyl)-2-(furan-2-yl)-8-(hex-1-yn-1-yl)-9H-purin-6-amine (21f)

A target compound (21f) is obtained by the same synthetic method as for Compound (14a): ¹H NMR (600 MHz, CDCl₃) δ 7.58 (dd, 1 H, J = 1.7, 0.9 Hz), 7.21 (dd, 1 H, J = 3.4, 0.9 Hz), 6.53 (dd, 1 H, J = 3.4, 1.8 Hz), 5.63 (brs, 2 H), 4.37 (t, 2 H, J = 7.0 Hz), 2.80 (t, 2 H, J = 7.0 Hz), 2.54 (t, 2 H, J = 7.0 Hz), 2.33 (s, 6 H), 1.66 (m, 2 H), 1.51 (m, 2 H), 0.96 (t, 3 H, J = 7.3 Hz).

### 2-(furan-2-yl)-8-(hex-1-yn-1-yl)-9-(2-(piperidin-1-yl)ethyl)-9H-purin-6-amine (21g)

A target compound (21g) is obtained by the same synthetic method as for Compound (14a): ¹H NMR (600 MHz, CDCl₃) δ 7.57 (dd, 1 H, J = 1.7, 0.9 Hz), 7.21 (dd, 1 H, J = 3.4, 0.9 Hz), 6.52 (dd, 1 H, J = 3.4, 1.8 Hz), 5.61(brs, 2 H), 4.38 (t, 2 H, J = 7.0 Hz), 2.78 (t, 2 H, J = 7.0 Hz), 2.53 (t, 2 H, J = 7.0 Hz), 2.52 (m, 4 H), 1.66 (m, 2 H), 1.51 (m, 6 H), 1.41 (m, 2 H), 0.96 (t, 3H, J = 7.3 Hz).

### 9-(2-ethoxyethyl)-2-(furan-2-yl)-8-(hex-1-yn-1-yl)-9H-purin-6-amine (21h)

A target compound (21h) is obtained by the same synthetic method as for Compound (14a): ¹H NMR (600 MHz, CDCl₃) δ 7.58 (dd, 1 H, J = 1.8, 0.9 Hz), 7.22 (dd, 1 H, J = 3.4, 0.9 Hz), 6.52 (dd, 1 H, J = 3.4, 1.8 Hz), 5.74 (brs, 2 H), 4.46 (t, 2 H, J = 6.1 Hz), 3.85 (m, 2 H), 3.52 (m, 2H), 2.53 (m, 2 H), 1.66 (m, 2 H), 1.50 (m, 2 H), 1.14 (t, 3 H, J = 7.0 Hz), 0.96 (t, 3 H, J = 7.3 Hz).

### 2-(furan-2-yl)-8-(hex-1-yn-1-yl)-9-isobutyl-9H-purin-6-amine (21i)

A target compound (21i) is obtained by the same synthetic method as for Compound (14a): ¹H NMR (600 MHz, CDCl₃) δ 7.58 (dd, 1 H, J = 1.8, 0.9 Hz), 7.22 (dd, 1 H, J = 3.4, 0.9 Hz), 6.52 (dd, 1 H, J = 3.4, 1.8 Hz), 5.66 (brs, 2 H), 4.08 (d, 2 H, J = 7.5 Hz), 2.54 (t, 2 H, J = 7.0 Hz), 2.42 (m, 1H), 1.66 (m, 2 H), 1.52 (m, 2 H), 1.51 (m, 6 H), 0.95 (t, 3 H, J = 7.3 Hz).

### 2-(furan-2-yl)-8-(hex-1-yn-1-yl)-9-(2-methylbutyl)-9H-purin-6-amine (21j)

A target compound (21j) is obtained by the same synthetic method as for Compound (14d): ¹H NMR (600 MHz, CDCl₃) δ 7.57 (dd, 1 H, J = 1.7, 0.9 Hz), 7.21 (dd, 1 H, J = 3.4, 0.9 Hz), 6.52 (dd, 1 H, J = 3.4, 1.8 Hz), 5.64 (brs, 2 H), 4.18 (dd, 1 H, J = 13.6, 6.8 Hz), 4.05 (dd, 1 H, J = 13.6, 8.1 Hz), 2.53 (t, 2H, J = 7.0 Hz), 2.19 (m, 1 H), 1.65 (m, 2 H), 1.52 (m, 2 H), 1.42 (m, 1 H), 1.24 (m, 1 H), 0.96 (t, 6 H, J = 7.3 Hz), 0.91 (t, 3 H, J = 6.8 Hz).

### 9-(2-chloroethyl)-2-(furan-2-yl)-8-(hex-1-yn-1-yl)-9H-purin-6-amine (21k)

A target compound (21k) is obtained by the same synthetic method as for Compound (14d): ¹H NMR (600 MHz, CDCl₃) δ 7.58 (dd, 1 H, J = 1.7, 0.9 Hz), 7.23 (dd, 1 H, J = 3.4, 0.9 Hz), 6.53 (dd, 1 H, J = 3.4, 1.8 Hz), 5.77 (brs, 2 H), 4.61 (t, 2 H, J = 6.6 Hz), 3.98 (t, 2 H, J = 6.6 Hz), 2.55 (t, 2 H, J = 7.1 Hz), 1.67 (m, 2 H), 1.52 (m, 2 H), 0.96 (t, 3 H, J = 7.3 Hz).

### 2-(furan-2-yl)-8-(hex-1-yn-1-yl)-9-(2-methoxyethyl)-9H-purin-6-amine (211)

A target compound (211) is obtained by the same synthetic method as for Compound (14a): ¹H NMR (600 MHz, CDCl₃) δ 7.58 (dd, 1 H, J = 1.8, 0.9 Hz), 7.22 (dd, 1 H, J = 3.4, 0.9 Hz), 6.52 (dd, 1 H, J = 3.4, 1.8 Hz), 5.74 (brs, 2 H), 4.46 (t, 2 H, J = 6.0 Hz), 3.83 (t, 2 H, J = 6.0 Hz), 3.36 (s, 3 H), 2.53 (m, 2 H), 1.66 (m, 2 H), 1.50 (m, 2 H), 0.96 (t, 3 H, J = 7.3 Hz).

### 9-ethyl-8-(hex-1-yn-1-yl)-2-(thiophen-2-yl)-9H-purin-6-amine (21m)

A target compound (21m) is obtained by the same synthetic method as for Compound (14a): ¹H NMR (600 MHz, CDCl₃) δ 7.92 (dd, 1 H, J = 3.7, 1.1 Hz), 7.42 (dd, 1 H, J = 5.0, 1.2 Hz), 7.12 (dd, 1 H, J = 5.0, 3.7 Hz), 5.66 (brs, 2 H), 4.34 (q, 2 H, J = 7.3 Hz), 2.31 (t, 2 H, J = 7.1 Hz), 1.50 (m, 2 H), 1.41 (m, 2 H), 1.38 (t, 3 H, J = 7.2 Hz), 0.90 (t, 3 H, J = 7.2 Hz).

### 8-(hex-1-yn-1-yl)-9-propyl-2-(thiophen-2-yl)-9H-purin-6-amine (21n)

A target compound (21n) is obtained by the same synthetic method as for Compound (14a): ¹H NMR (600 MHz, CDCl₃) δ 7.91 (dd, 1 H, J = 3.7, 1.1 Hz), 7.42 (dd, 1 H, J = 5.0, 1.2 Hz), 7.12 (dd, 1 H, J = 5.0, 3.7 Hz), 5.66 (brs, 2 H), 4.20 (m, 2 H), 2.31 (t, 2 H, J = 7.1 Hz), 2.05 (m, 2 H), 1.50 (m, 2 H), 1.41 (m, 2 H), 0.92 (t, 3 H, J = 7.3 Hz), 0.90 (t, 3 H, J = 7.2 Hz).

### 2-(6-amino-8-(hex-1-yn-1-yl)-2-(thiophen-2-yl)-9H-purin-9-yl)ethanol (21o)

A target compound (21o) is obtained by the same synthetic method as for Compound (14a): ¹H NMR (600 MHz, CDCl₃) δ 7.92 (dd, 1 H, J = 3.7, 1.1 Hz), 7.41 (dd, 1 H, J = 5.0, 1.2 Hz), 7.13 (dd, 1 H, J = 5.0, 3.7 Hz), 5.56 (m, 3 H), 4.41 (dd, 2H, J = 5.4, 3.5 Hz), 4.07 (m, 2 H), 2.31 (t, 2 H, J = 7.1 Hz), 1.50 (m, 2 H), 1.41 (m, 2 H), 0.90 (t, 3 H, J = 7.2 Hz).

### 9-(2-(dimethylamino)ethyl)-8-(hex-1-yn-1-yl)-2-(thiophen-2-yl)-9H-purin-6-amine (21p)

A target compound (21p) is obtained by the same synthetic method as for Compound (14d): ¹H NMR (600 MHz, CDCl₃) δ 7.92 (dd, 1 H, J = 3.7, 1.1 Hz), 7.42 (dd, 1 H, J = 5.0, 1.2 Hz), 7.12 (dd, 1 H, J = 5.0, 3.7 Hz), 5.68 (brs, 2 H), 4.45 (t, 2 H, J = 7.0 Hz), 2.91 (t, 2H, J = 7.0 Hz), 2.35 (s, 6 H), 2.31 (t, 2 H, J = 7.1 Hz), 1.50 (m, 2 H), 1.41 (m, 2 H), 0.90 (t, 3 H, J = 7.2 Hz).

### 9-(2-ethoxyethyl)-8-(hex-1-yn-1-yl)-2-(thiophen-2-yl)-9H-purin-6-amine (21q)

A target compound (21q) is obtained by the same synthetic method as for Compound (14a): ¹H NMR (600 MHz, CDCl₃) δ 7.92 (dd, 1 H, J = 3.7, 1.1 Hz), 7.42 (dd, 1 H, J = 5.0, 1.2 Hz), 7.12 (dd, 1 H, J = 5.0, 3.7 Hz), 5.52 (brs, 2 H), 4.42 (t, 2H, 6.0 Hz), 3.83 (m, 2 H), 3.51 (m, 2H), 2.31 (t, 2 H, J = 7.1 Hz), 1.50 (m, 2 H), 1.41 (m, 2 H), 1.16 (t, 3 H, J = 7.0 Hz), 0.91 (t, 3 H, J = 7.2 Hz).

### 9-(2-chloroethyl)-8-(hex-1-yn-1-yl)-2-(thiophen-2-yl)-9H-purin-6-amine (21r)

A target compound (21r) is obtained by the same synthetic method as for Compound (14d): ¹H NMR (600 MHz, CDCl₃) δ 7.91 (dd, 1 H, J = 3.7, 1.1 Hz), 7.43 (dd, 1 H, J = 5.0, 1.3 Hz), 7.12 (dd, 1 H, J = 5.0, 3.7 Hz), 5.48 (brs, 2 H), 4.62 (t, 2H, J = 6.6 Hz), 4.01 (t, 2 H, J = 6.6 Hz), 2.31 (t, 2 H, J = 7.1 Hz), 1.50 (m, 2 H), 1.40 (m, 2 H), 0.90 (t, 3 H, J = 7.2 Hz).

### Preparation Example 11: Synthesis of 24a to 24c

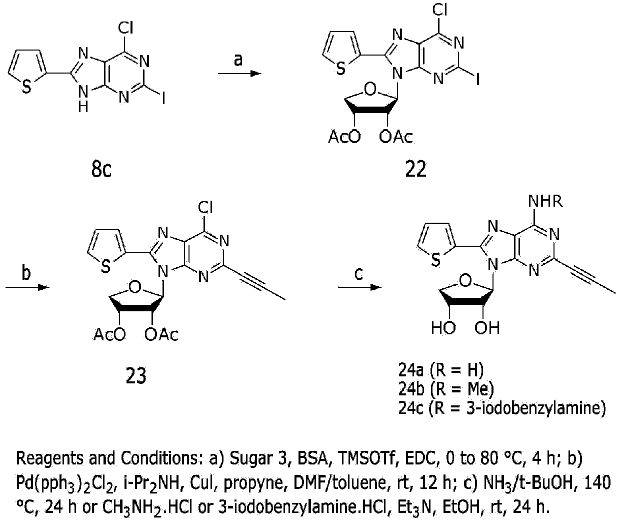

### (2R,3R,4R)-2-(6-Chloro-2-iodo-8-(thiophen-2-yl)-9H-purin-9-yl)tetrahydrofuran-3,4-diyl diacetate (22)

A target compound (22) is obtained using Starting Material (8c) and Sugar 3 by the same synthetic method as for Compound (12a): ¹H NMR (600 MHz, CDCl₃) δ 7.70 (dd, J = 5.3, 1.1 Hz, 1H), 7.67 (q, J = 1.7 Hz, 1H), 7.26 (s, 1H, merged with solvent), 6.42 (t, J = 5.5 Hz, 1H), 6.31 (d, J = 5.5 Hz, 1H), 5.87-5.85 (m, 1H), 4.78 (dd, J = 10.5, 3.7 Hz, 1H), 4.21 (dd, J = 10.9, 1.5 Hz, 1H), 2.17 (s, 3H), 2.07 (s, 3H);

### (2R,3R,4R)-2-(6-Chloro-2-(prop-1-yn-1-yl)-8-(thiophen-2-yl)-9H-purin-9-yl)tetrahydrofuran-3,4-diyl diacetate (23)

A target compound (23) is obtained by the same synthetic method as for Compound (9a): ¹H NMR (600 MHz, CDCl₃) δ 7.68-7.65 (m, 2H), 7.25-7.24 (m, 1H), 6.53 (t, J = 5.3 Hz, 1H), 6.34 (d, J = 5.5 Hz, 1H), 5.86 (t, J = 3.9 Hz, 1H), 4.83 (dd, J = 10.5, 3.7 Hz, 1H), 4.19 (dd, J = 10.7, 1.1 Hz, 1H), 2.16 (s, 3H), 2.14 (s, 3H), 2.04 (s, 3H);

### (2R,3R,4R)-2-(6-Amino-2-(prop-1-yn-1-yl)-8-(thiophen-2-yl)-9H-purin-9-yl)tetrahydrofuran-3,4-diol (24a)

A target compound (24a) is obtained by the same synthetic method as for Compound (13a): ¹H NMR (600 MHz, CD₃OD) δ 7.74 (dd, J = 5.1, 1.4 Hz, 1H), 7.69 (dd, J = 3.7, 0.9 Hz, 1H), 7.25 (dd, J = 5.1, 3.7 Hz, 1H), 6.13 (d, J = 6.4 Hz, 1H), 5.53 (dd, J = 6.4, 4.6 Hz, 1H), 4.64 (dd, J = 9.7, 3.2 Hz, 1H), 4.49 (d, J = 3.7 Hz, 1H), 3.99 (dd, J = 9.7, 0.9 Hz, 1H), 2.06 (s, 3H);

### (2R,3R,4R)-2-(6-(Methylamino)-2-(prop-1-yn-1-yl)-8-(thiophen-2-yl)-9H-purin-9-yl)tetrahydrofuran-3,4-diol (24b)

A target compound (24b) is obtained by the same synthetic method as for Compound (13a): ¹H NMR (600 MHz, DMSO-d₆) δ 7.94 (d, J = 3.7 Hz, 1H), 7.88-7.86 (m, 1H), 7.58 (dd, J = 3.7, 0.9 Hz, 1H), 7.29 (dd, J = 5.0, 3.7 Hz, 1H), 5.96 (d, J = 6.9 Hz, 1H), 5.53 (d, J = 6.4 Hz, 1H), 5.29 (dd, J = 11.2, 6.6 Hz, 1H), 5.24 (d, J = 3.7 Hz, 1H), 4.43 (dd, J = 9.1, 3.2 Hz, 1H), 4.31 (d, J = 2.7 Hz, 1H), 3.84 (d, J = 9.6 Hz, 1H), 2.93 (d, J = 2.7 Hz, 3H), 2.06 (s, 3H).

### (2R,3R,4R)-2-(6-((3-iodobenzyl)amino)-2-(prop-1-yn-1-yl)-8-(thiophen-2-yl)-9H-purin-9-yl)tetrahydrofuran-3,4-diol (24c)

A target compound (24c) is obtained by the same synthetic method as for Compound (13b): ¹H NMR (600 MHz, CD₃OD) δ 7.78 (s, 1H), 7.70 (dd, J = 5.3, 1.1 Hz, 1H), 7.66 (d, J = 3.7 Hz, 1H), 7.58 (d, J = 7.8 Hz, 1H), 7.38 (d, J = 7.4 Hz, 1H), 7.22 (dd, J = 5.1, 3.7 Hz, 1H), 7.07 (t, J = 7.8 Hz, 1H), 6.11 (d, J = 6.4 Hz, 1H), 5.51 (dd, J = 6.2, 4.8 Hz, 1H), 4.62 (dd, J = 9.4, 3.4 Hz, 1H), 4.46 (t, J = 3.4 Hz, 1H), 3.96 (dd, J = 9.7, 1.4 Hz, 1H), 2.04 (s, 3H).

### Preparation Example 12: Synthesis of 27a to 27c

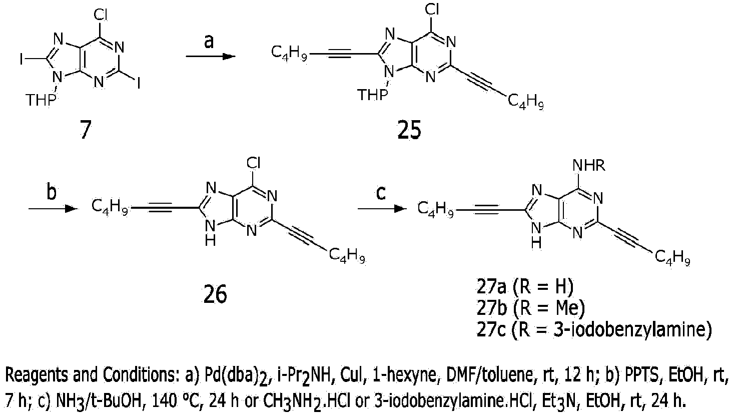

### 6-chloro-2,8-di(hex-1-yn-1-yl)-9-(tetrahydro-2H-pyran-2-yl)-9H-purine (25)

After Starting Material (7) (1 mmol) is dissolved in anhydrous DMF/toluene (1:1, 0.02 M), bis(triphenylphosphine)palladium(II) dichloride (0.2 eq), copper(I) iodide (0.2 eq), diisopropylamine (3 eq), and 1-hexyne (2 eq) are added thereto at room temperature, and then the resulting mixture is stirred at room temperature for 12 hours. After it is confirmed that the reaction is completed, the reaction mixture is extracted with EtOAc, and then dried over anhydrous MgSO₄ and concentrated under reduced pressure to obtain a target compound (26) (67%): ¹H NMR (600 MHz, CDCl₃) δ 5.97 (t, J = 7.7 Hz, 1H), 3.67-3.55 (m, 2H), 2.50-2.48 (m, 4H), 2.20-1.92 (m, 2H), 1.75-1.65 (m, 2H), 1.57-1.53 (m, 2H), 1.46-1.44 (m, 4H), 1.37-1.35 (m, 4H), 1.07 (t, J = 7.2 Hz, 3H), 1.02 (t, J = 7.2 Hz, 3H).

### 6-Chloro-2,8-di(hex-1-yn-1-yl)-9H-purine (26)

After Starting Material (25) is dissolved in THF/EtOH (0.15 M), a pyridinium p-toluenesulfonate (PPTS) (0.2 eq) solution (1 mL of water) is slowly added thereto at room temperature, and the resulting mixture is stirred for 4 hours. After it is confirmed that the reaction is completed, the reaction mixture is extracted with EtOAc. The resulting residue is dried over anhydrous MgSO₄ and concentrated under reduced pressure to obtain a target compound (26): ¹H NMR (600 MHz, CDCl₃) δ 2.55-2.51 (m, 4H), 1.54-1.52 (m, 4H), 1.34-1.32 (m, 4H), 1.05 (t, J = 7.2 Hz, 3H), 0.99 (t, J = 7.4 Hz, 3H).

### 2,8-Di(hex-1-yn-1-yl)-9H-purin-6-amine (27a)

A target compound (27a) is obtained using Starting Material (7) by the same synthetic method as for Compound (13a): ¹H NMR (600 MHz, CD₃OD) δ 2.53 (t, J = 6.9 Hz, 2H), 2.44 (t, J = 6.9 Hz, 2H), 1.66-1.59 (m, 4H), 1.55-1.48 (m, 4H), 0.97 (td, J = 7.2, 5.6 Hz, 6H).

### 2,8-Di(hex-1-yn-1-yl)-N-methyl-9H-purin-6-amine (27b)

A target compound (27b) is obtained using Starting Material (7) by the same synthetic method as for Compound (13b): ¹H NMR (600 MHz, CD₃OD) δ 3.21 (s, 3H), 2.41 (td, J = 7.2, 3.5 Hz, 4H), 1.62-1.55 (m, 4H), 1.47-1.37 (m, 4H), 0.88 (t, J = 7.3 Hz, 6H).

### 2,8-Di(hex-1-yn-1-yl)-N-(3-iodobenzyl)-9H-purin-6-amine (27c)

A target compound (27c) is obtained using Starting Material (7) by the same synthetic method as for Compound (13b): ¹H NMR (600 MHz, CDCl₃) δ 7.69 (s, 1H), 7.56 (d, J = 7.8 Hz, 1H), 7.30 (d, J = 7.8 Hz, 1H), 7.01 (t, J = 7.8 Hz, 1H), 2.43-2.36 (m, 4H), 1.57 (q, J = 7.5 Hz, 4H), 1.41 (q, J = 7.5 Hz, 4H), 0.88 (t, J = 7.3 Hz, 6H).

### Preparation Example 13: Synthesis of 29a to 29c

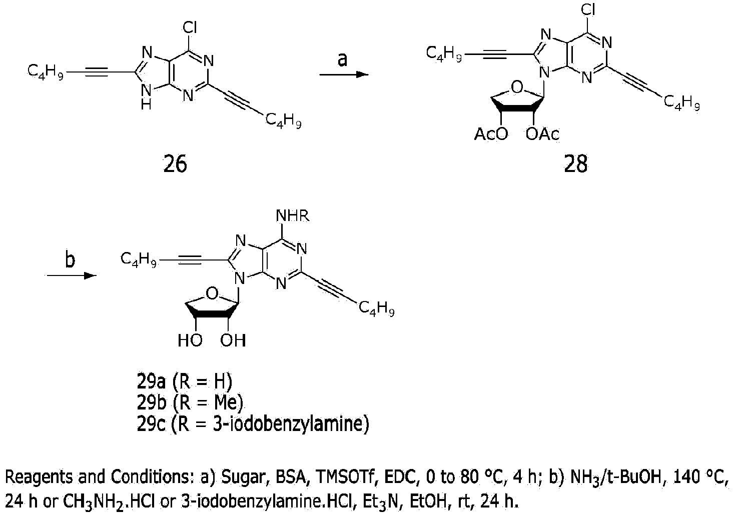

### (2R,3R,4R)-2-(6-Chloro-2,8-di(hex-1-yn-1-yl)-9H-purin-9-yl)tetrahydrofuran-3,4-diyl diacetate (28)

A target compound (28) is obtained using Starting Material (26) and Sugar 3 by the same synthetic method as for Compound (12a): ¹H NMR (600 MHz, CDCl₃) δ 6.32 (d, J = 6.0 Hz, 1H), 6.23 (t, J = 5.5 Hz, 1H), 5.78-5.75 (m, 1H), 4.78 (dd, J = 10.6, 3.7 Hz, 1H), 4.16 (dd, J = 10.6, 0.9 Hz, 1H), 2.56 (t, J = 6.9 Hz, 2H), 2.46 (t, J = 7.4 Hz, 2H), 2.16 (s, 3H), 2.03 (s, 3H), 1.69-1.60 (m, 4H), 1.44-1.54 (m, 4H), 0.94 (q, J = 7.4 Hz, 6H).

### (2R,3R,4R)-2-(6-Amino-2,8-di(hex-1-yn-1-yl)-9H-purin-9-yl)tetrahydrofuran-3,4-diol (29a)

A target compound (29a) is obtained by the same synthetic method as for Compound (13a): ¹H NMR (600 MHz, CD₃OD) δ 6.11 (d, J = 6.4 Hz, 1H), 5.28 (dd, J = 6.7, 4.8 Hz, 1H), 4.56 (dd, J = 9.4, 3.4 Hz, 1H), 4.44 (t, J = 3.4 Hz, 1H), 3.99 (dd, J = 9.7, 0.9 Hz, 1H), 2.59 (t, J = 6.9 Hz, 2H), 2.44 (t, J = 6.9 Hz, 2H), 1.69-1.49 (m, 8H), 0.98 (q, J = 7.2 Hz, 6H).

### (2R,3R,4R)-2-(2,8-Di(hex-1-yn-1-yl)-6-(methylamino)-9H-purin-9-yl)tetrahydrofuran-3,4-diol (29b)

A target compound (29b) is obtained by the same synthetic method as for Compound (13b): ¹H NMR (600 MHz, CDCl₃) δ 6.12 (d, J = 6.0 Hz, 1H), 5.42 (t, J = 5.3 Hz, 1H), 4.60 (s, 1H), 4.54 (dd, J = 10.1, 3.7 Hz, 1H), 4.11 (d, J = 9.7 Hz, 1H), 3.12 (s, 3H), 2.44 (q, J = 7.4 Hz, 4H), 1.67-1.56 (m, 4H), 1.53-1.42 (m, 4H), 0.94 (q, J = 7.7 Hz, 6H).

### (2R,3R,4R)-2-(2,8-Di(hex-1-yn-1-yl)-6-((3-iodobenzyl)amino)-9H-purin-9-yl)tetrahydrofuran-3,4-diol (29c)

A target compound (29c) is obtained by the same synthetic method as for Compound (13b): ¹H NMR (600 MHz, CDCl₃) δ 7.72 (s, 1H), 7.61 (d, J = 7.8 Hz, 1H), 7.32 (d, J = 7.8 Hz, 1H), 7.06 (t, J = 7.8 Hz, 1H), 6.12 (d, J = 5.5 Hz, 1H), 5.34 (t, J = 5.3 Hz, 1H), 4.78 (s, 2H), 4.60 (s, 1H), 4.55 (dd, J = 9.7, 3.7 Hz, 1H), 4.08 (d, J = 9.7 Hz, 1H), 2.49-2.41 (m, 4H), 1.62 (td, J = 13.9, 7.2 Hz, 4H), 1.48 (td, J = 14.1, 7.2 Hz, 4H), 0.94 (td, J = 7.1, 3.7 Hz, 6H).

### Preparation Example 14: Synthesis of 32 and 34

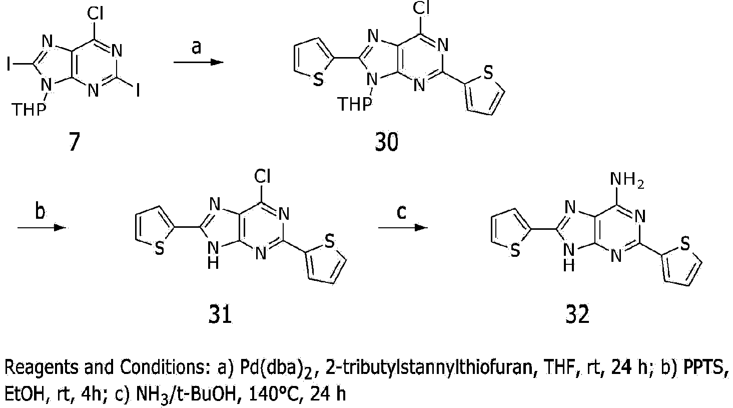

### 6-chloro-9-(tetrahydro-2H-pyran-2-yl)-2,8-di(thiophen-2-yl)-9H-purine (30)

After Starting Material (7) (1 mmol) is dissolved in THF (0.03 M), Pd(dba)₂ (0.01 eq) and 2-tributylstannylthiophene (2 eq) are added thereto, and the resulting mixture is stirred at room temperature for 24 hours. After it is confirmed that the reaction is completed, the reaction mixture is extracted with EtOAc, and then dried over anhydrous MgSO₄ and concentrated under reduced pressure. The resulting residue is purified by column chromatography under the condition of hexane/EtOAc = 4/1 to obtain a target compound (30): ¹H NMR (600 MHz, CDCl₃) δ 7.89 (d, 1H), 7.87 (d, 1H) 7.85 (d, 1H), 7.78 (d, 1H), 7.25 (d, 1H) 7.22 (d, 2H), 6.01 (t, J = 7.7 Hz, 1H), 3.70-3.67 (m, 2H), 2.20-1.98 (m, 2H), 1.78-1.71 (m, 2H), 1.57-1.51 (m, 2H).

### 6-chloro-2,8-di(thiophen-2-yl)-9H-purine (31)

After Starting Material (30) (1 mmol) is dissolved in EtOH (0.03 M), a pyridinium p-toluenesulfonate (PPTS) (0.2 eq) solution (1 mL of water) is slowly added thereto at room temperature, and the resulting mixture is stirred for 4 hours. After it is confirmed that the reaction is completed, the reaction mixture is extracted with EtOAc. The resulting residue is dried over anhydrous MgSO₄ and concentrated under reduced pressure to obtain a target compound (31): ¹H NMR (600 MHz, CD₃OD) δ 13.65 (s, 1H), 7.91 (d, 1H), 7.88 (d, 1H), 7.86 (d, 1H), 7.81 (d, 1H), 7.32 (d, 1H), 7.26 (d, 1H).

### 2,8-Di(thiophen-2-yl)-9H-purin-6-amine (32)

A target compound (32) is obtained by the same synthetic method as for Compound (11a): ¹H NMR (600 MHz, CD₃OD) δ 7.87 (q, J = 1.7 Hz, 1H), 7.75 (q, J = 1.7 Hz, 1H), 7.65 (dd, J = 5.0, 1.4 Hz, 1H), 7.48 (dd, J = 5.0, 0.9 Hz, 1H), 7.21 (dd, J = 5.0, 3.7 Hz, 1H), 7.11 (dd, J = 5.0, 3.7 Hz, 1H).

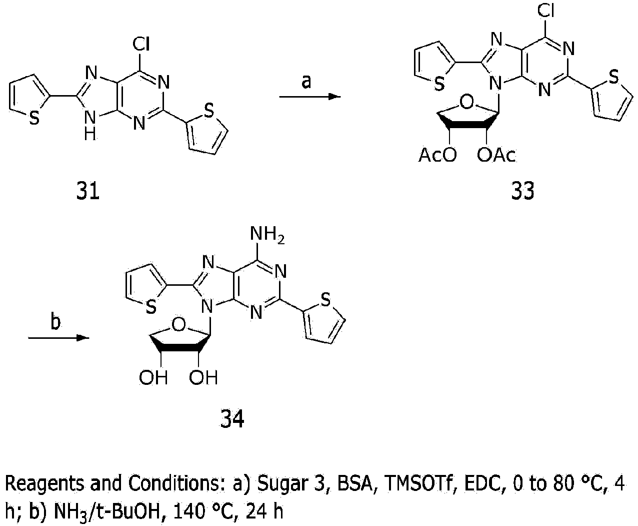

### (2R,3R,4R)-2-(6-Cloro-2,8-di(thiophen-2-yl)-9H-purin-9-yl)tetrahydrofuran-3,4-diyl diacetate (33)

A target compound (33) is obtained using Starting Material (31) and Sugar 3 by the same synthetic method as for Compound (12a): ¹H NMR (600 MHz, CDCl₃) δ 8.03 (q, J = 1.6 Hz, 1H), 7.67 (qd, J = 3.9, 1.2 Hz, 2H), 7.49-7.47 (m, 1H), 7.24 (dd, J = 4.9, 3.7 Hz, 1H), 7.14 (dd, J = 4.9, 3.7 Hz, 1H), 6.70 (t, J = 5.2 Hz, 1H), 6.33 (d, J = 4.9 Hz, 1H), 6.04-6.01 (m, 1H), 4.87 (dd, J = 10.7, 4.0 Hz, 1H), 4.24 (dd, J = 10.4, 1.8 Hz, 1H), 2.18 (s, 3H), 2.08 (d, J = 13.5 Hz, 3H).

### (2R,3R,4R)-2-(6-Aino-2,8-di(thiophen-2-yl)-9H-purin-9-yl)tetrahydrofuran-3,4-diol (34)

A target compound (34) is obtained by the same synthetic method as for Compound (13a): ¹H-NMR (600 MHz, CD₃OD) δ 7.85-7.82 (m, 2H), 7.71-7.67 (m, 2H), 7.46-7.45 (m, 1H), 7.24 (q, J = 3.1 Hz, 1H), 7.09 (q, J = 3.1 Hz, 1H), 6.17 (d, J = 5.5 Hz, 1H), 5.62 (t, J = 5.2 Hz, 1H), 4.79 (dd, J = 9.5, 3.4 Hz, 1H), 4.63 (t, J = 3.4 Hz, 1H), 4.06-4.04 (m, 1H);

### Preparation Example 15: Synthesis of 36a to 361

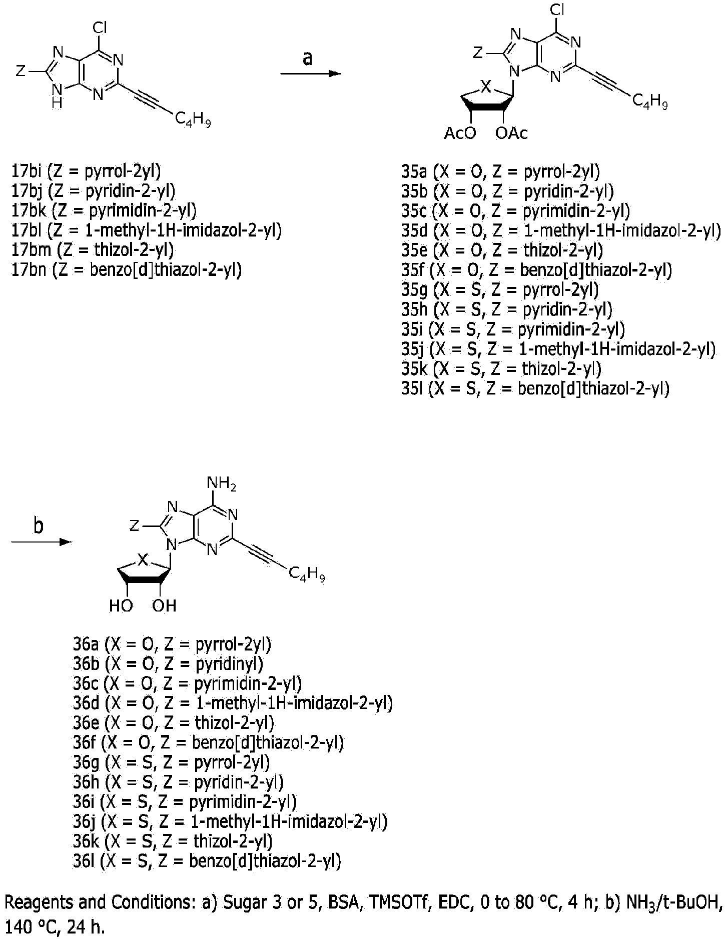

### (2R,3R,4R)-2-(6-chloro-2-(hex-1-yn-1-yl)-8-(1H-pyrrol-2-yl)-9H-purin-9-yl)tetrahydrofuran-3,4-diyl diacetate (35a)

A target compound (35a) is obtained using Sugar 3 by the same synthetic method as for Compound (19a): ¹H-NMR (600 MHz, CD₃OD) δ 11.94 (s, 1H), 7.01 (d, 1H), 6.50 (d, 1H), 6.75 (t, J = 5.2 Hz, 1H), 6.13 (d, 1H), 5.61-5.59 (m, 4H), 2.51 (t, J = 7.7 Hz, 2H), 2.05 (s, 6H), 1.42-1.40 (m, 2H), 1.31-1.30 (m, 2H), 0.91 (t, J = 7.2 Hz, 3H).

### (2R,3R,4R)-2-(6-chloro-2-(hex-1-yn-1-yl)-8-(pyridin-2-yl)-9H-purin-9-yl)tetrahydrofuran-3,4-diyl diacetate (35b)

A target compound (35b) is obtained using Sugar 3 by the same synthetic method as for Compound (19a): ¹H-NMR (600 MHz, CD₃OD) δ 8.57 (d, 1H), 8.31 (d, 1H), 7.95 (d, 1H), 7.42 (d, 1H), 6.72 (t, J = 5.4 Hz, 1H), 5.61-5.57 (m, 4H), 2.49 (t, J = 7.7 Hz, 2H), 2.07 (s, 6H), 1.42-1.40 (m, 2H), 1.32-1.30 (m, 2H), 0.89 (t, J= 7.2 Hz, 3H).

### (2R,3R,4R)-2-(6-chloro-2-(hex-1-yn-1-yl)-8-(pyrimidin-2-yl)-9H-purin-9-yl)tetrahydrofuran-3,4-diyl diacetate (35c)

A target compound (35c) is obtained using Sugar 3 by the same synthetic method as for Compound (19a): ¹H-NMR (600 MHz, CD₃OD) δ 9.02 (d, 1H), 9.00 (d, 1H), 7.71 (d, 1H), 6.75 (t, J = 5.2 Hz, 1H), 5.63-5.60 (m, 4H), 2.49 (t, J = 7.7 Hz, 2H), 2.05 (s, 6H), 1.42-1.40 (m, 2H), 1.31-1.30 (m, 2H), 0.87 (t, J = 7.2 Hz, 3H).

### (2R,3R,4R)-2-(6-chloro-2-(hex-1-yn-1-yl)-8-(1-methyl-1H-imidazol-2-yl)-9H-purin-9-yl)tetrahydrofuran-3,4-diyl diacetate (35d)

A target compound (35d) is obtained using Sugar 3 by the same synthetic method as for Compound (19a): ¹H-NMR (600 MHz, CD₃OD) δ 7.03 (d, 1H), 6.50 (d, 1H), 6.75 (t, J = 5.2 Hz, 1H), 6.13 (d, 1H), 5.61-5.59 (m, 4H), 3.72 (s, 3H), 2.51 (t, J = 7.7 Hz, 2H), 2.05 (s, 6H), 1.42-1.40 (m, 2H), 1.31-1.30 (m, 2H), 0.91 (t, J = 7.2 Hz, 3H).

### (2R,3R,4R)-2-(2-(hex-1-yn-1-yl)-8-(thiazol-2-yl)-9H-purin-9-yl)tetrahydrofuran-3,4-diyl diacetate (35e)

A target compound (35e) is obtained using Sugar 3 by the same synthetic method as for Compound (19a): ¹H-NMR (600 MHz, CD₃OD) δ 8.07 (d, 1H), 7.72 (d, 1H), 6.81 (t, J = 5.4 Hz, 1H), 6.11 (d, 1H), 5.61-5.59 (m, 4H), 2.51 (t, J = 7.7 Hz, 2H), 2.05 (s, 6H), 1.42-1.40 (m, 2H), 1.31-1.30 (m, 2H), 0.90 (t, J = 7.2 Hz, 3H).

### (2R,3R,4R)-2-(8-(benzo[d]thiazol-2-yl)-6-chloro-2-(hex-1-yn-1-yl)-9H-purin-9-yl)tetrahydrofuran-3,4-diyl diacetate (35f)

A target compound (35f) is obtained using Sugar 3 by the same synthetic method as for Compound (19a): ¹H-NMR (600 MHz, CD₃OD) δ 8.21 (d, 1H), 8.09 (d, 1H), 7.57 (d, 1H), 7.53 (d, 1H), 6.81 (t, J = 5.4 Hz, 1H), 6.13 (d, 1H), 5.63-5.59 (m, 4H), 2.51 (t, J = 7.7 Hz, 2H), 2.05 (s, 6H), 1.42-1.40 (m, 2H), 1.31-1.30 (m, 2H), 0.87 (t, J = 7.4 Hz, 3H).

### (2R,3R,4S)-2-(6-chloro-2-(hex-1-yn-1-yl)-8-(1H-pyrrol-2-yl)-9H-purin-9-yl)tetrahydrothiophene-3,4-diyl diacetate (35g)

A target compound (35g) is obtained using Sugar 5 by the same synthetic method as for Compound (19a): ¹H-NMR (600 MHz, CD₃OD) δ 11.87 (s, 1H), 7.01 (d, 1H), 6.52 (d, 1H), 6.79 (t, J = 5.2 Hz, 1H), 6.13 (d, 1H), 5.61-5.59 (m, 4H), 2.51 (t, J = 7.7 Hz, 2H), 2.05 (s, 6H), 1.42-1.40 (m, 2H), 1.31-1.30 (m, 2H), 0.87 (t, J = 7.2 Hz, 3H).

### (2R,3R,4S)-2-(6-chloro-2-(hex-1-yn-1-yl)-8-(pyridin-2-yl)-9H-purin-9-yl)tetrahydrothiophene-3,4-diyl diacetate (35h)

A target compound (35h) is obtained using Sugar 5 by the same synthetic method as for Compound (19a): ¹H-NMR (600 MHz, CD₃OD) δ 8.51 (d, 1H), 8.34 (d, 1H), 7.95 (d, 1H), 7.44 (d, 1H), 6.75 (t, J = 5.4 Hz, 1H), 5.63-5.57 (m, 4H), 2.49 (t, J = 7.7 Hz, 2H), 2.07 (s, 6H), 1.42-1.40 (m, 2H), 1.32-1.30 (m, 2H), 0.90 (t, J = 7.2 Hz, 3H).

### (2R,3R,4S)-2-(6-chloro-2-(hex-1-yn-1-yl)-8-(pyrimidin-2-yl)-9H-purin-9-yl)tetrahydrothiophene-3,4-diyl diacetate (35i)

A target compound (35i) is obtained using Sugar 5 by the same synthetic method as for Compound (19a): ¹H-NMR (600 MHz, CD₃OD) δ 9.07 (d, 1H), 9.02 (d, 1H), 7.73 (d, 1H), 6.77 (t, J = 5.2 Hz, 1H), 5.63-5.60 (m, 4H), 2.51 (t, J = 7.7 Hz, 2H), 2.05 (s, 6H), 1.42-1.40 (m, 2H), 1.31-1.30 (m, 2H), 0.89 (t, J = 7.2 Hz, 3H).

### (2R,3R,4S)-2-(6-chloro-2-(hex-1-yn-1-yl)-8-(1-methyl-1H-imidazol-2-yl)-9H-purin-9-yl)tetrahydrothiophene-3,4-diyl diacetate (35j)

A target compound (35j) is obtained using Sugar 5 by the same synthetic method as for Compound (19a): ¹H-NMR (600 MHz, CD₃OD) δ 7.05 (d, 1H), 6.55 (d, 1H), 6.80 (t, J = 5.2 Hz, 1H), 6.14 (d, 1H), 5.61-5.59 (m, 4H), 3.72 (s, 3H), 2.52 (t, J = 7.7 Hz, 2H), 2.05 (s, 6H), 1.42-1.40 (m, 2H), 1.31-1.30 (m, 2H), 0.89 (t, J = 7.2 Hz, 3H).

### (2R,3R,4S)-2-(2-(hex-1-yn-1-yl)-8-(thiazol-2-yl)-9H-purin-9-yl)tetrahydrothiophene-3,4-diyl diacetate (35k)

A target compound (35k) is obtained using Sugar 5 by the same synthetic method as for Compound (19a): ¹H-NMR (600 MHz, CD₃OD) δ 8.01 (d, 1H), 7.71 (d, 1H), 6.79 (t, J = 5.4 Hz, 1H), 6.11 (d, 1H), 5.63-5.59 (m, 4H), 2.51 (t, J = 7.7 Hz, 2H), 2.01 (s, 6H), 1.42-1.40 (m, 2H), 1.30-1.29 (m, 2H), 0.87 (t, J = 7.2 Hz, 3H).

### (2R,3R,4S)-2-(8-(benzo[d]thiazol-2-yl)-6-chloro-2-(hex-1-yn-1-yl)-9H-purin-9-yl)tetrahydrothiophene-3,4-diyl diacetate (351)

A target compound (351) is obtained using Sugar 5 by the same synthetic method as for Compound (19a): ¹H-NMR (600 MHz, CD₃OD) δ 8.32 (d, 1H), 8.01 (d, 1H), 7.67 (d, 1H), 7.58 (d, 1H), 6.83 (t, J = 5.4 Hz, 1H), 6.13 (d, 1H), 5.64-5.59 (m, 4H), 2.51 (t, J = 7.7 Hz, 2H), 2.05 (s, 6H), 1.42-1.40 (m, 2H), 1.32-1.30 (m, 2H), 0.89 (t, J = 7.4 Hz, 3H).

### (2R,3R,4R)-2-(6-amino-2-(hex-1-yn-1-yl)-8-(1H-pyrrol-2-yl)-9H-purin-9-yl)tetrahydrofuran-3,4-diol (36a)

A target compound (36a) is obtained by the same synthetic method as for Compound (18a): ¹H NMR (600 MHz, MeOD) δ 7.80 (d, J = 1.6 Hz, 1 H), 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4R)-2-(6-Amino-2-(hex-1-yn-1-yl)-8-(pyridin-2-yl)-9H-purin-9-yl)tetrahydrofuran-3,4-diol (36b)

A target compound (36b) is obtained by the same synthetic method as for Compound (18a): ¹H NMR (600 MHz, MeOD) δ 7.80 (d, J = 1.6 Hz, 1 H), 7.50 (d, J = 1.6 Hz, 1 H), 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4R)-2-(6-Amino-2-(hex-1-yn-1-yl)-8-(pyrimidin-2-yl)-9H-purin-9-yl)tetrahydrofuran-3,4-diol (36c)

A target compound (36c) is obtained by the same synthetic method as for Compound (18a): ¹H NMR (600 MHz, MeOD) δ 7.50 (d, J = 1.6 Hz, 1 H), 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4R)-2-(6-Amino-2-(hex-1-yn-1-yl)-8-(1-methyl-1H-imidazol-2-yl)-9H-purin-9-yl)tetrahydrofuran-3,4-diol (36d)

A target compound (36d) is obtained by the same synthetic method as for Compound (18a): ¹H NMR (600 MHz, MeOD) δ 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2H), 1.35-1.43 (m, 2 H), 1.21 (t, J = 7.2 Hz, 3 H), 1.11 (t, J = 7.2 Hz, 3 H).

### (2R,3R,4R)-2-(6-Amino-2-(hex-1-yn-1-yl)-8-(thiazol-2-yl)-9H-purin-9-yl)tetrahydrofuran-3,4-diol (36e)

A target compound (36e) is obtained by the same synthetic method as for Compound (18a): ¹H NMR (600 MHz, MeOD) δ 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2H), 1.35-1.43 (m, 2 H), 1.21 (t, J = 7.2 Hz, 3 H).

### (2R,3R,4R)-2-(6-Amino-8-(benzo[d]thiazol-2-yl)-2-(hex-1-yn-1-yl)-9H-purin-9-yl)tetrahydrofuran-3,4-diol (36f)

A target compound (36f) is obtained by the same synthetic method as for Compound (18a): ¹H NMR (600 MHz, MeOD) δ 7.77 (d, J = 1.6 Hz, 1 H), 7.50 (d, J = 1.6 Hz, 1 H), 7.17 (d, J = 2.8 Hz, 1 H), 6.97-7.04 (m, 1 H), 6.68 (dd, J = 1.6, 3.6 Hz, 1 H), 6.35-6.40 (m, 2 H), 5.69 (dd, J = 3.6, 7.6 Hz, 1 H), 4.60 (dd, J = 3.6, 6.0 Hz, 1 H), 3.68 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4S)-2-(6-amino-2-(hex-1-yn-1-yl)-8-(1H-pyrrol-2-yl)-9H-purin-9-yl)tetrahydrothiophene-3,4-diol (36g)

A target compound (36g) is obtained by the same synthetic method as for Compound (18a): ¹H NMR (600 MHz, MeOD) δ 7.85 (d, J = 1.6 Hz, 1 H), 7.11 (d, J = 2.8 Hz, 1 H), 6.97-7.04 (m, 1 H), 6.38 (dd, J = 1.6, 3.6 Hz, 1 H), 6.25-6.30 (m, 2 H), 5.49 (dd, J = 3.6, 7.6 Hz, 1 H), 4.30 (dd, J = 3.6, 6.0 Hz, 1 H), 3.48 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4S)-2-(6-amino-2-(hex-1-yn-1-yl)-8-(pyridin-2-yl)-9H-purin-9-yl)tetrahydrothiophene-3,4-diol (36h)

A target compound (36h) is obtained by the same synthetic method as for Compound (18a): ¹H NMR (600 MHz, MeOD) δ 7.85 (d, J = 1.6 Hz, 1 H), 7.50 (d, J = 1.6 Hz, 1 H), 7.11 (d, J = 2.8 Hz, 1 H), 6.97-7.04 (m, 1 H), 6.38 (dd, J = 1.6, 3.6 Hz, 1 H), 6.25-6.30 (m, 2 H), 5.49 (dd, J = 3.6, 7.6 Hz, 1 H), 4.30 (dd, J = 3.6, 6.0 Hz, 1 H), 3.48 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4S)-2-(6-amino-2-(hex-1-yn-1-yl)-8-(pyrimidin-2-yl)-9H-purin-9-yl)tetrahydrothiophene-3,4-diol (36i)

A target compound (36i) is obtained by the same synthetic method as for Compound (18a): ¹H NMR (600 MHz, MeOD) δ 7.50 (d, J = 1.6 Hz, 1 H), 7.11 (d, J = 2.8 Hz, 1 H), 6.97-7.04 (m, 1 H), 6.38 (dd, J = 1.6, 3.6 Hz, 1 H), 6.25-6.30 (m, 2 H), 5.49 (dd, J = 3.6, 7.6 Hz, 1 H), 4.30 (dd, J = 3.6, 6.0 Hz, 1 H), 3.48 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### (2R,3R,4S)-2-(6-amino-2-(hex-1-yn-1-yl)-8-(1-methyl-1H-imidazol-2-yl)-9H-purin-9-yl)tetrahydrothiophene-3,4-diol (36j)

A target compound (36j) is obtained by the same synthetic method as for Compound (18a): ¹H NMR (600 MHz, MeOD) δ 7.11 (d, J = 2.8 Hz, 1 H), 6.97-7.04 (m, 1 H), 6.38 (dd, J = 1.6, 3.6 Hz, 1 H), 6.25-6.30 (m, 2 H), 5.49 (dd, J = 3.6, 7.6 Hz, 1 H), 4.30 (dd, J = 3.6, 6.0 Hz, 1 H), 3.48 (dd, J = 3.2, 10.8 Hz, 1 H), 2.93 (dd, J = 2.4, 11.6 Hz, 1 H), 2.22-2.30 (m, 2 H), 1.45-1.54 (m, 2H), 1.35-1.43 (m, 2 H), 1.21 (t, J = 7.2 Hz, 3 H), 1.11 (t, J = 7.2 Hz, 3 H).

### (2R,3R,4S)-2-(6-amino-2-(hex-1-yn-1-yl)-8-(thiazol-2-yl)-9H-purin-9-yl)tetrahydrothiophene-3,4-diol (36k)

A target compound (36k) is obtained by the same synthetic method as for Compound (18a): ¹H NMR (600 MHz, MeOD) δ 7.11 (d, J = 2.8 Hz, 1 H), 6.97-7.04 (m, 1 H), 6.38 (dd, J = 1.6, 3.6 Hz, 1 H), 6.25-6.30 (m, 2 H), 5.49 (dd, J = 3.6, 7.6 Hz, 1 H), 4.30 (dd, J = 3.6, 6.0 Hz, 1 H), 3.48 (dd, J = 3.2, 10.8 Hz, 1 H), 2.93 (dd, J = 2.4, 11.6 Hz, 1 H), 2.22-2.30 (m, 2 H), 1.45-1.54 (m, 2H), 1.35-1.43 (m, 2 H), 1.21 (t, J = 7.2 Hz, 3 H).

### (2R,3R,4S)-2-(6-amino-8-(benzo[d]thiazol-2-yl)-2-(hex-1-yn-1-yl)-9H-purin-9-yl)tetrahydrothiophene-3,4-diol (361)

A target compound (361) is obtained by the same synthetic method as for Compound (18a): ¹H NMR (600 MHz, MeOD) δ 7.87 (d, J = 1.6 Hz, 1 H), 7.50 (d, J = 1.6 Hz, 1 H), 7.11 (d, J = 2.8 Hz, 1 H), 6.97-7.04 (m, 1 H), 6.38 (dd, J = 1.6, 3.6 Hz, 1 H), 6.25-6.30 (m, 2 H), 5.49 (dd, J = 3.6, 7.6 Hz, 1 H), 4.30 (dd, J = 3.6, 6.0 Hz, 1 H), 3.48 (dd, J = 3.2, 10.8 Hz, 1 H), 2.93 (dd, J = 2.4, 11.6 Hz, 1 H), 2.22-2.30 (m, 2 H), 1.45-1.54 (m, 2 H), 1.35-1.43 (m, 2 H), 1.21 (t, J = 7.2 Hz, 3 H).

### Experimental Examples: Evaluation of binding affinity for adenosine receptor (1)

In order to evaluate the affinity of the adenosine derivatives of the present invention prepared in the Preparation Examples for A₁, A_{2A}, A_{2B} and A₃ receptors in a human adenosine receptor (hAR), the following experiments were performed.

### Experimental Example 1: Competition Binding in Human A1 Receptors

An adenosine A1 receptor competition binding experiment was performed in a multiscreen GF/C 96-well plate (Millipore, Madrid, Spain) pretreated with a binding buffer (20 mM Hepes, 100 mM NaCl, 10 mM MgCh, 2 U/mL adenosine deaminase, and pH = 7.4). In each well, 5 µg of a membrane obtained from Euroscreen Ha1 cell line was incubated and prepared by the testing laboratory (Lot: A002/13-04-2011, protein concentration = 5864 µg/ml), and 1 nM [3H]-DPCPX (137 Ci/mmol, 1 mCi/mL, PerkinElmer NET974001MC) and compounds were studied and standardized. Non-specific binding was determined in the presence of 10 µM R-PIA (Sigma P4532). Before being measured by a microplate beta scintillation counter (Microbeta Trilux PerkinElmer, Madrid, Spain), the reaction mixture (Vt: 200 µL/well) was incubated at 25°C for 60 minutes, then filtered by a filter and then washed four times with 250 µl of a wash buffer (20 mM Hepes, 100 mM NaCl, 10 mM MgCh, and pH = 7.4).

### Experimental Example 2: Competition Binding in Human A2A Receptors

An adenosine A2A receptor competition binding experiment was performed in a multiscreen GF/C 96-well plate (Millipore, Madrid, Spain) pretreated with a binding buffer (50 mM Tris-HCl, 1 mM EDTA, 10 mM MgCh, 2 U/mL adenosine deaminase, and pH = 7.4). In each well, 5 µg of a membrane obtained from HeLa-A2A cell line was incubated and prepared by the testing laboratory (Lot: A001/18-10-2010, protein concentration = 4288 µg/ml), and 3 nM [3H]-ZM241385 (50 Ci/mmol, 1 mCi/mL, ARC-ITISA 0884) and compounds were studied and standardized. Non-specific binding was determined in the presence of 50 µM NECA (Sigma E2387). Before being measured by a microplate beta scintillation counter (Microbeta Trilux PerkinElmer, Madrid, Spain), the reaction mixture (Vt: 200 µL/well) was incubated at 25°C for 30 minutes, then filtered by a filter and then washed four times with 250 µl of a wash buffer (50 mM Tris-HCl, 1 mM EDTA, 10 mM MgCh, and pH = 7.4).

### Experimental Example 3: Competition Binding in Human A2B Receptors

An adenosine A2B receptor competition binding experiment was performed in a 96-well plate. In each well, 20 µg of a membrane obtained from Euroscreen Ha2B cell line was incubated and prepared by the testing laboratory (Lot: A007/22-02-2016, protein concentration = 3211 µg/ml), and 25 nM [3H]-DPCPX (137 Ci/mmol, 1 mCi/mL, PerkinElmer NET974001MC) and compounds were studied and standardized. Non-specific binding was determined in the presence of 1000 µM NECA (Sigma E2397). Before being measured by a microplate beta scintillation counter (Microbeta Trilux PerkinElmer, Madrid, Spain), the reaction mixture (Vt: 200 µL/well) was incubated at 25°C for 30 minutes, 200 µL of the incubated reaction mixture was incubated in a GF/C 96-well plate (Millipore, Madrid, Spain) pretreated with a binding buffer (50 mA Tris-HCl, 1 mM EDTA, 5 mM MgCh, 100 µg/µL Bacitracin, 2 U/mL adenosine deaminase, and pH = 6.5), then filtered by a filter, and then washed four times with 250 µL of a wash buffer (50 mM Tris-HCl, 1 mM EDTA, 5 mM MgCh, and pH = 6.5).

### Experimental Example 4: Competition Binding in Human A3 Receptors

An adenosine A3 receptor competition binding experiment was performed in a multiscreen GF/B 96-well plate (Millipore, Madrid, Spain) pretreated with a binding buffer (50 mM Tris-HCl, 1 mM EDTA, 5 mM MgCh, 2 U/mL adenosine deaminase, and pH = 7.4). In each well, 60 µg of a membrane obtained from HeLa-A3 cell line was incubated and prepared by the testing laboratory (Lot: A004/03-05-2019, protein concentration = 1829 µg/ml), and 10 nM [3H]-NECA (26.3 Ci/mmol, 1 mCi/mL, PerkinElmer NET811250UC) and compounds were studied and standardized. Non-specific binding was determined in the presence of 100 µM R-PIA (Sigma P4532). Before being measured by a microplate beta scintillation counter (Microbeta Trilux PerkinElmer, Madrid, Spain), the reaction mixture (Vt: 200 µL/well) was incubated at 25°C for 180 minutes, then filtered by a filter and then washed six times with 250 µl of a wash buffer (50 mM Tris-HCl and pH = 7.4).

**[Table 1]**

| Compound | Binding affinity (% inhibition) | | | |
|---|---|---|---|---|
| | hA₁ | hA_{2A} | hA_{2B} | hA₃ |
| 11a | - | +++ | - | ++ |
| 11b | - | +++ | - | +++ |
| 11c | - | ++ | - | ++ |
| 11d | - | ++ | - | ++ |
| 11e | - | + | - | + |
| 11f | - | + | - | + |
| 11g | - | - | - | - |
| 11h | - | - | - | - |
| 11i | - | - | - | - |
| 11j | - | ++ | - | ++ |
| 11k | - | ++ | - | ++ |
| 11l | - | + | - | + |
| 11m | - | + | - | + |
| 11n | - | - | - | - |
| 11o | - | - | - | - |
| 11p | - | - | - | - |
| 11q | - | ++ | - | +++ |
| 11r | + | - | - | +++ |
| 11s | + | + | - | +++ |
| 11t | - | - | - | +++ |
| 13a | - | +++ | - | + |
| 13b | - | - | - | +++ |
| 13c | - | - | - | - |
| 13d | - | +++ | - | + |
| 13e | - | ++ | - | ++ |
| 13f | - | - | - | - |
| 13g | - | + | - | - |
| 13h | - | - | - | - |
| 13i | - | - | - | - |
| 13j | + | ++ | + | - |
| 13k | - | - | - | + |
| 131 | - | - | - | - |
| 13m | + | +++ | - | +++ |
| 13n | - | - | - | - |
| 13o | - | - | - | + |
| 13p | - | - | - | ++ |
| 14a | + | ++ | + | - |
| 14b | + | +++ | + | - |
| 14c | + | ++ | + | - |
| 14d | - | + | - | - |
| 14e | - | + | + | - |
| 14f | - | - | - | - |
| 14g | + | - | + | - |
| 14h | + | + | + | - |
| 14i | - | - | + | ++ |
| 14j | + | + | - | - |
| 14k | + | +++ | + | - |
| 141 | + | ++ | +++ | - |
| 14m | + | ++ | + | - |
| 14n | + | +++ | + | - |
| 14o | - | + | - | - |
| 14p | - | - | - | - |
| 14q | + | + | + | - |
| 14r | + | +++ | + | - |
| 18a | - | +++ | - | - |
| 18b | - | - | - | - |
| 18c | - | ++ | - | + |
| 18d | - | +++ | + | +++ |
| 18e | - | ++ | - | + |
| 18f | - | - | - | +++ |
| 18g | +++ | +++ | + | ++ |
| 18h | - | + | - | ++ |
| 18i | + | ++ | - | +++ |
| 18j | +++ | +++ | - | +++ |
| 18k | - | ++ | - | + |
| 181 | + | +++ | - | +++ |
| 18m | + | +++ | - | + |
| 18n | ++ | ++ | - | - |
| 18o | - | - | - | - |
| 18p | - | - | - | - |
| 18q | - | - | - | +++ |
| 18r | - | - | - | - |
| 18s | - | + | - | ++ |
| 18t | - | ++ | - | - |
| 18u | - | +++ | ++ | ++ |
| 18v | - | +++ | - | - |
| 18aa | - | +++ | ++ | ++ |
| 18ab | - | - | - | - |
| 18ac | + | +++ | + | - |
| 18ad | - | +++ | ++ | ++ |
| 18ae | - | +++ | - | - |
| 18af | + | +++ | + | - |
| 18ag | + | ++ | +++ | - |
| 18ah | + | ++ | + | - |
| 18ai | + | ++ | +++ | - |
| 18aj | - | +++ | ++ | ++ |
| 18ak | - | +++ | - | - |
| 18al | - | - | - | - |
| 18am | + | +++ | + | - |
| 18an | - | +++ | ++ | ++ |
| 18ao | - | +++ | - | - |
| 18ap | + | +++ | + | - |
| 18aq | + | ++ | +++ | - |
| 18ar | + | ++ | + | - |
| 18ba | - | +++ | ++ | ++ |
| 18bb | - | - | - | - |
| 18bc | + | +++ | + | - |
| 18bd | - | +++ | ++ | ++ |
| 18be | - | +++ | - | - |
| 18bf | + | +++ | + | - |
| 18bg | + | ++ | +++ | - |
| 18bh | + | ++ | + | - |
| 18bi | + | ++ | +++ | - |
| 18bj | - | +++ | ++ | ++ |
| 18bk | - | +++ | - | - |
| 18bl | - | - | - | - |
| 18bm | + | +++ | + | - |
| 18bn | + | ++ | + | - |
| 20a | - | ++ | - | ++ |
| 20b | - | - | - | ++ |
| 20c | - | - | - | - |
| 20d | +++ | +++ | - | +++ |
| 20e | - | - | - | +++ |
| 20f | - | - | - | +++ |
| 20g | ++ | ++ | - | +++ |
| 20h | - | - | - | +++ |
| 20i | - | - | - | +++ |
| 20j | + | ++ | - | ++ |
| 20k | - | - | - | ++ |
| 201 | - | - | - | - |
| 20m | - | - | - | - |
| 20n | - | - | - | +++ |
| 20ο | - | - | - | - |
| 20p | + | + | + | - |
| 20q | - | - | - | ++ |
| 20r | - | - | - | - |
| 20s | - | ++ | + | +++ |
| 20t | + | +++ | + | - |
| 20u | + | ++ | + | - |
| 20v | - | - | - | +++ |
| 20w | + | +++ | + | - |
| 20x | + | ++ | + | - |
| 21a | - | +++ | + | - |
| 21b | + | +++ | + | - |
| 21c | + | ++ | + | - |
| 21d | - | + | - | - |
| 21e | - | + | - | - |
| 21f | - | - | - | - |
| 21g | + | - | + | - |
| 21h | + | + | + | - |
| 21i | - | - | + | ++ |
| 21j | + | + | - | - |
| 21k | + | +++ | + | - |
| 211 | + | ++ | +++ | - |
| 21m | - | ++ | + | - |
| 21n | + | ++ | + | - |
| 21o | - | + | - | - |
| 21p | - | - | - | - |
| 21q | + | + | + | - |
| 21r | + | ++ | + | - |
| 24a | + | ++ | - | + |
| 24b | + | ++ | - | + |
| 24c | + | + | - | + |
| 27a | - | - | - | ++ |
| 27b | - | - | - | ++ |
| 27c | - | - | - | - |
| 29a | - | ++ | - | +++ |
| 29b | - | - | - | +++ |
| 29c | - | - | - | +++ |
| 32 | +++ | +++ | +++ | +++ |
| 34 | - | - | - | +++ |
| 36a | - | +++ | ++ | ++ |
| 36b | - | - | - | - |
| 36c | + | +++ | + | - |
| 36d | - | +++ | ++ | ++ |
| 36e | - | +++ | - | - |
| 36f | + | +++ | + | - |
| 36g | + | ++ | +++ | - |
| 36h | + | ++ | + | - |
| 36i | + | ++ | +++ | - |
| 36j | - | +++ | ++ | ++ |
| 36k | - | +++ | - | - |
| 361 | - | - | - | - |

As shown in Table 1 above, the adenosine derivative compounds of the present invention exhibited high binding affinity for human adenosine A_{2A} and A₃ receptors and exhibited generally low affinity, that is, high selectivity for adenosine A₁ and A_{2B} receptors.

### Preparation Examples: Preparation of adenosine derivative of present invention (2)

### Preparation Example 1: Synthesis of tetrahydrofuran-2,3-diyl diacetate (107)

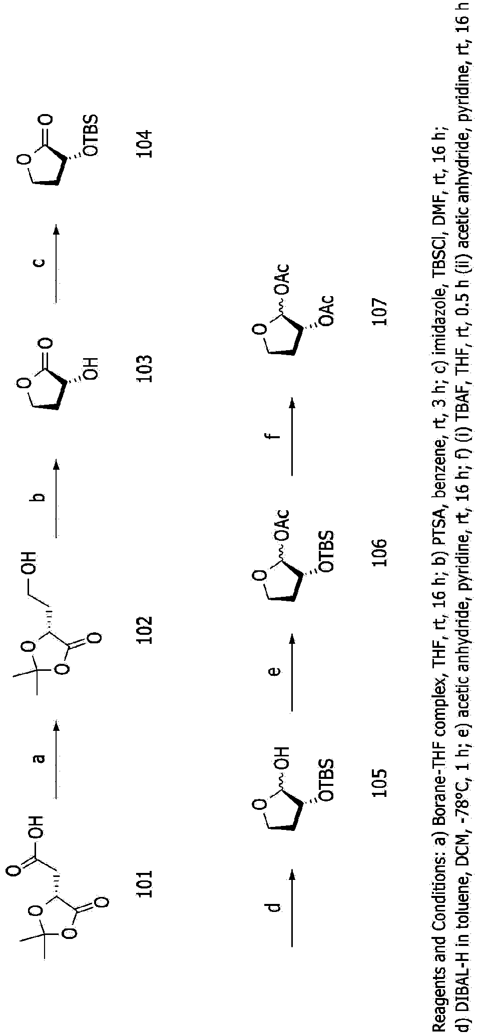

### (R)-5-(2-hydroxyethyl)-2,2-dimethyl-1,3-dioxolan-4-one (102)

Starting Material (101) (50 g, 287 mmol) is dissolved in THF (300 mL) under nitrogen, and 316 mL of a borane-THF complex (1.0 M in THF) is added dropwise at 0°C for 1 hour. The reaction mixture is stirred at 0°C for 2.5 hours and then at room temperature for 19 hours. After MeOH (300 mL) is slowly added to the reaction mixture, the resulting mixture is concentrated under reduced pressure at 25°C. The resulting residue is purified by column chromatography under the condition of hexane/EtOAc = 3:1 to obtain a target compound (102) (18.9 g, 41%) in the form of a colorless oil: ¹H NMR (400 MHz, CDCl₃) δ 4.55 - 4.60 (m, 1H), 3.91 - 3.79 (m, 2H), 2.19 - 2.11 (m, 2H), 2.06 - 1.96 (1H, m), 1.63 (s, 3H), 1.56 (s, 3H).

### (R)-3-hydroxydihydrofuran-2(3H)-one (103)

Starting Material (102) (32 g, 200 mmol) is dissolved in benzene (350 mL), PTSA monohydrate (380 mg) is added thereto, and then the resultant is stirred at room temperature for 3 hours. After it is confirmed that the reaction is completed, the reaction mixture is concentrated under reduced pressure. The resulting residue is purified by column chromatography under the condition of hexane/EtOAc = 1/1 to obtain a target compound (103) (17.7 g, 86%) in the form of a colorless oil: ¹H NMR (400 MHz, CDCl₃) δ 4.52 - 4.57 (m, 1H), 4.44 (td, J = 9.0 Hz, 1H), 2.58 - 2.66 (m, 1H), 2.23 - 2.35 (m, 1H).

### (R)-3-((tert-butyldimethylsilyl)oxy)dihydrofuran-2(3H)-one (104)

(R)-3-hydroxydihydrofuran-2(3H)-one (103) (20 g, 169 mmol) is dissolved in DMF (400 mL), and 58 g (845 mmol) of imidazole and 89 g (592 mmol) of TBSCl are added thereto at 0°C. The reaction mixture is brought to room temperature, and then stirred for 18 hours. After it is confirmed that the reaction is completed, the reaction mixture is extracted with EtOAc. The resulting residue is dried over anhydrous MgSO₄ and concentrated under reduced pressure to obtain a target compound (104) (45 g, 77%) in the form of a white solid. The target compound was used in the next reaction without further purification: ¹H NMR (400 MHz, CDCl₃) δ 4.35 - 4.45 (m, 2H), 4.19 (td, J = 9.2, 6.5 Hz, 1H), 2.37 - 2.53 (m, 1H), 2.17 - 2.30 (m, 1H), 2.91 (s, 9H), 0.17 (s, 3H), 0.15 (s, 3H).

### (3R)-3-((tert-butyldimethylsilyl)oxy)tetrahydrofuran-2-ol (105)

Starting Material (104) (32.5 g, 150 mmol) is dissolved in DCM (325 mL) under nitrogen, DIBAL-H in toluene (180 mL) is slowly added there to at -78°C, and the resultant is stirred at the same temperature for 1 hour. The reaction is terminated by slowly adding MeOH (15 mL) to the reaction mixture, and a 2 M aqueous sulfuric acid solution is added thereto until the pH becomes 3. The reaction mixture is extracted with EtOAc/H₂O, then dried over MgSO₄, and concentrated under reduced pressure. The resulting residue is purified by column chromatography under the condition of hexane/EtOAc = 4/1 to obtain a target compound (105) (25.4 g, 77%) in the form of a colorless oil: ¹H NMR

### (3R)-3-((tert-butyldimethylsilyl)oxy)tetrahydrofuran-2-yl acetate (106)

After Starting Material (105) (25 g, 114 mmol) is dissolved in pyridine under nitrogen, anhydrous acetic acid (20 mL) is slowly added thereto, and then the resultant is stirred at room temperature for 18 hours. After it is confirmed that the reaction is terminated, the reaction mixture is extracted with EtOAc/H₂O and concentrated under reduced pressure to obtain a target compound (106) (25.3 g, 85.3%) in the form of a brown oil: ¹H NMR (400 MHz, CDCl₃) δ 5.96 (s, 1H), 4.26 (d, J = 3.9 Hz, 1H), 4.05 - 4.19 (m, 2H), 2.06 - 2.18 (m, 1H), 2.04 (s, 3H), 1.76-1.87 (m, 1H), 0.90 (s, 9H), 0.10 (s, 3H), 0.09 (s, 3H).

### (3R)-tetrahydrofuran-2,3-diyl diacetate (107)

After Starting Material (106) (29.7 g, 114 mmol) is dissolved in THF (200 mL), TBAF is slowly added thereto, and the resultant is stirred for 1 hour. After it is confirmed that the reaction is terminated, the reaction mixture is extracted with EtOAc/H₂O and concentrated under reduced pressure. After the resulting residue is dissolved in pyridine, anhydrous acetic acid is slowly added thereto, and the resultant is stirred at room temperature for 18 hours. After it is confirmed that the reaction is completed, the resulting residue obtained by concentration under reduced pressure is purified by column chromatography under the condition of hexane/EtOAc = 4/1 to obtain a target compound (107) (11.8 g, 55%) in the form of a colorless oil: ¹H NMR (400 MHz, DMSO- d6) δ 6.00 (s, 1H), 5.06 (d, J = 4.8 Hz, 1H), 3.97 - 4.04 (m, 2H), 2.22 - 2.33 (m, 1H), 2.02 (s, 3H), 2.00 (s, 3H), 1.92 - 1.99 (m, 1H).

### Preparation Example 2: Synthesis of 116a to 116f

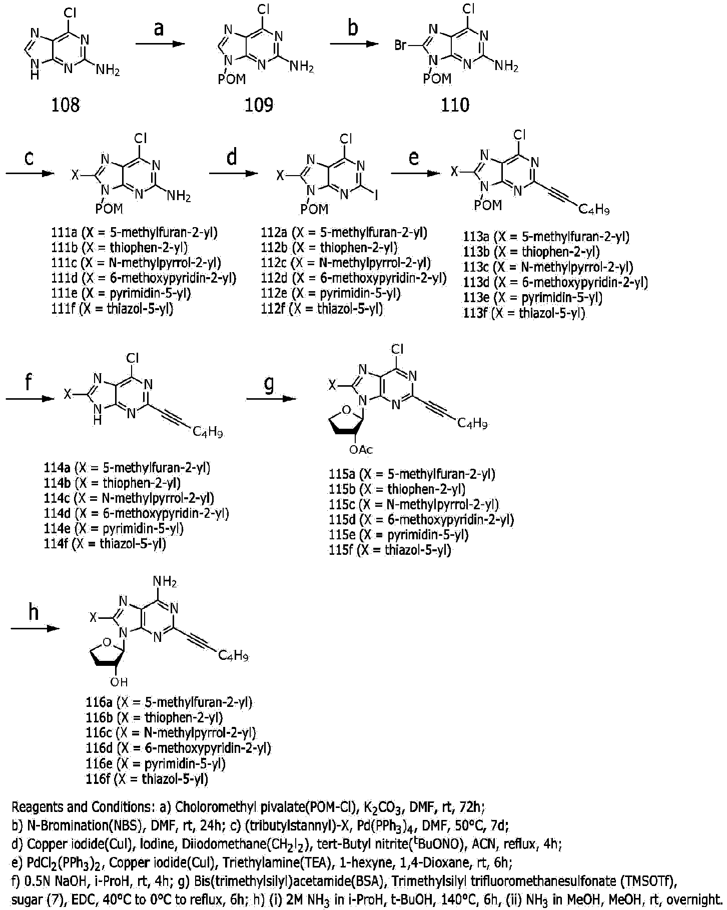

### (2-amino-6-chloro-9H-purin-9-yl)methyl pivalate (109)

Starting Material (108) (50 g, 169.6 mmol) is suspended in an anhydrous DMF (1,200 mL) solvent, potassium carbonate (48.9 g) and choloromethyl pivalate (51 mL) are slowly added thereto at room temperature, and then the resultant is stirred for 72 hours. After it is confirmed that the reaction is completed, the reaction mixture is filtered with THF (300 mL). The filtrate is diluted with toluene and concentrated under reduced pressure. The resulting residue is purified by column chromatography under the condition of hexane/THF = 2/1 to 1/1 to obtain a target compound (109) (51.9 g, 62%): ¹H NMR (400 MHz, DMSO- d6) δ 8.20 (s, 1H), 7.12 (s, 2H), 6.00 (s, 2H), 1.10 (s, 9H).

### (2-amino-8-bromo-6-chloro-9H-purin-9-yl)methyl pivalate (110)

Starting Material (109) (26 g, 91.6 mmol) and N-bromosuccinimide (32.6 g) are suspended in anhydrous DMF (300 mL) under nitrogen, and the resultant is stirred at room temperature for 24 hours. After it is confirmed that the reaction is completed, the resultant is neutralized to pH 7 with a 20% sodium thiosulfate solution. The reaction mixture is extracted with EtOAc and brine, then dried over anhydrous MgSO₄, and concentrated under reduced pressure. The resulting residue is purified by column chromatography under the condition of DCM/EtOAc = 35/1 to 20/1 to obtain a target compound (110) (16.2 g, 49%) as a yellow solid: ¹H NMR (400 MHz, DMSO- d6) δ 7.25 (s, 2H), 5.96 (s, 2H), 1.13 (s, 9H).

### (2-amino-6-chloro-8-(5-methylfuran-2-yl)-9H-purin-9-yl)methyl pivalate (111a)

Starting Material (110) (1 g, 2.8 mmol), tetrakis(triphenylphosphine)palladium(0) (160 mg), and 5-methyl-2-(tributylstannyl)furan (1.54 g) are suspended in anhydrous DMF (10 mL), and the resultant is stirred at 50°C for 4 days. After it is confirmed that the reaction is completed, the resultant is cooled to room temperature, and the reaction mixture is diluted with toluene and concentrated under reduced pressure. The resulting residue is purified by column chromatography under the condition of hexane/THF = 5/1 to obtain a target compound (111a) (402 mg, 40%) as a yellow solid: ¹H NMR (400 MHz, CDCl3) δ 7.39 (d, 1H, J = 4.0 Hz), 6.29 (d, 1H, J = 4.0 Hz), 6.10 (s, 2H), 5.28 (s, 2H), 2.44 (s, 3H), 1.23 (s, 9H).

### (2-amino-6-chloro-8-(thiophen-2-yl)-9H-purin-9-yl)methyl pivalate (111b)

The resultant is stirred at 30°C for 3 days by the same synthetic method as for Compound (111a) to obtain a target compound (111b) (60%) as a yellow solid: ¹H NMR (400 MHz, CDCl3) δ 7.76 (d, 1H, J = 4.0 Hz), 7.68 (d, 1H, J = 4.0 Hz), 7.24 (t, 1H, J = 4.0 Hz), 6.20 (s, 2H), 6.03 (s, 2H), 1.21 (s, 9H).

### (2-amino-6-chloro-8-(1-methyl-1H-pyrrol-2-yl)-9H-purin-9-yl)methyl pivalate (111c)

The resultant is stirred at 80°C for 5 days by the same synthetic method as for Compound (111a) to obtain a target compound (111c) (42%) as a yellow solid: ¹H NMR (400 MHz, CDCl₃) δ 7.38 (d, 1H, J = 4.0 Hz), 5.92 (s, 2H), 5.46 (s, 2H), 5.29 (d, 1H, J=2.4), 4.92 (d, 1H, J=3.2), 3.14 (s, 2H), 1.19 (s, 9H).

### (2-amino-6-chloro-8-(6-methoxypyridin-2-yl)-9H-purin-9-yl)methyl pivalate (111d)

The resultant is stirred at 80°C for 3 days by the same synthetic method as for Compound (111a) to obtain a target compound (111d) (43%) as a yellow solid: ¹H NMR (400 MHz, CDCl₃) δ 7.09 (d, 1H, J = 2.0 Hz), 6.92 (d, 1H, J = 2.8), 6.17 (d, 1H, J=3.7), 6.06 (s, 2H), 5.81 (s, 2H), 1.29 (s, 9H), 0.34 (s, 3H).

### (2-amino-6-chloro-8-(pyrimidin-5-yl)-9H-purin-9-yl)methyl pivalate (111e)

The resultant is stirred at 80°C for 7 days by the same synthetic method as for Compound (111a) to obtain a target compound (111e) (52%) as a yellow solid: ¹H NMR (400 MHz, CDCl₃) δ 9.03 (s, 1H), 8.93 (m, 2H), 5.93 (s, 2H), 5.52 (s, 2H), 1.23 (s, 9H).

### (2-amino-6-chloro-8-(thiazol-5-yl)-9H-purin-9-yl)methyl pivalate (111f)

The resultant is stirred at 80°C for 7 days by the same synthetic method as for Compound (111a) to obtain a target compound (111f) (38%) as a yellow solid: ¹H NMR (400 MHz, CDCl₃) δ 8.97 (s, 1H), 7.52 (s, 1H), 5.89 (s, 2H), 5.38 (s, 2H), 1.19 (s, 9H).

### (6-chloro-2-iodo-8-(5-methylfuran-2-yl)-9H-purin-9-yl)methyl pivalate (112a)

Starting Material (111a) (100 mg, 0.275 mmol), copper iodide (CuI) (55 mg), iodine (I₂) (70 mg), CH₂I₂ (0.22 mL), and tert-butyl nitrite (0.1 mL) were suspended in ACN under nitrogen, and the resultant is stirred at 80°C for 2 hours while blocking the light with silver foil paper. After it is confirmed that the reaction is completed, the resultant is cooled to room temperature. The reaction mixture is neutralized with a 20% sodium thiosulfate solution and concentrated under reduced pressure. The resulting residue is purified by column chromatography under the condition of hexane/THF = 20/1 to obtain a target compound (112a) (67.4 mg, 52%) as a white solid: ¹H NMR (400 MHz, CDCl3) δ 7.78 (d, 1H, J = 4.0 Hz), 7.70 (d, 1H, J = 4.0 Hz), 7.25 (t, 1H, J = 0.0, 4.0 Hz), 6.33 (s, 2H), 1.21 (s, 9H).

### (6-chloro-2-iodo-8-(thiophen-2-yl)-9H-purin-9-yl)methyl pivalate (112b)

A target compound (112b) (87%) is obtained by the same synthetic method as for Compound (112a): ¹H NMR (400 MHz, CDCl3) δ 7.78 (d, 1H, J = 4.0 Hz), 7.70 (d, 1H, J = 4.0 Hz), 7.25 (t, 1H, J = 0.0, 4.0 Hz), 6.33 (s, 2H), 1.21 (s, 9H).

### (6-chloro-2-iodo-8-(1-methyl-1H-pyrrol-2-yl)-9H-purin-9-yl)methyl pivalate (112c)

A target compound (112c) (55%) is obtained by the same synthetic method as for Compound (112a): ¹H NMR (400 MHz, CDCl₃) δ 7.36 (d, 1H, J = 4.0 Hz), 5.91 (s, 2H), 5.27 (d, 1H, J=2.4), 4.89 (d, 1H, J=3.2), 3.20 (s, 2H), 1.20 (s, 9H).

### (6-chloro-2-iodo-8-(6-methoxypyridin-2-yl)-9H-purin-9-yl)methyl pivalate (112d)

A target compound (112d) (53%) is obtained by the same synthetic method as for Compound (112a): ¹H NMR (400 MHz, CDCl₃) δ 7.09 (d, 1H, J = 2.0 Hz), 6.97 (d, 1H, J = 2.8), 6.19 (d, 1H, J=3.7), 6.05 (s, 2H), 1.32 (s, 9H), 0.35 (s, 3H).

### (6-chloro-2-iodo-8-(pyrimidin-5-yl)-9H-purin-9-yl)methyl pivalate (112e)

A target compound (112e) (59%) is obtained by the same synthetic method as for Compound (112a): ¹H NMR (400 MHz, CDCl₃) δ 9.03 (s, 1H), 8.79 (m, 2H), 5.96 (s, 2H), 1.25 (s, 9H).

### (6-chloro-2-iodo-8-(thiazol-5-yl)-9H-purin-9-yl)methyl pivalate (1121)

A target compound (112f) (58%) is obtained by the same synthetic method as for Compound (112a): ¹H NMR (400 MHz, CDCl₃) δ 8.92 (s, 1H), 7.57 (s, 1H), 5.87 (s, 2H), 1.20 (s, 9H).

### (6-chloro-2-(hex-1-yn-1-yl)-8-(5-methylfuran-2-yl)-9H-purin-9-yl)methyl pivalate (113a)

After Starting Material (112a) (340 mg, 0.72 mmol), CuI (20 mg) and PdCl₂(PPh₃)₂ (76 mg) are dissolved in 1,4-dioxane (3 mL) under nitrogen, triethylamine (0.6 mL) and 1-hexyne (0.1 mL) are added thereto, and the resulting mixture is stirred at room temperature for 3 hours. After it is confirmed that the reaction is completed, the resultant is extracted with ethyl acetate and brine, and the resulting organic layer is dried over MgSO₄ and concentrated under reduced pressure. The resulting residue is purified by column chromatography under the condition of hexane/THF = 9/1 to obtain a target compound (113a) (214.5 mg, 69.4%): ¹H NMR (400 MHz, CDCl3) δ 7.39 (d, 1H, J = 4.0 Hz), 6.45 (s, 2H), 6.29 (d, 1H, J = 4.0 Hz), 2.51 (t, 2H, J = 8.0, 8.0 Hz), 2.44 (s, 3H), 1.70-1.64 (m, 2H), 1.53-1.48 (m, 2H), 1.15 (s, 9H), 0.96 (t, 3H, J = 8.0, 8.0 Hz).

### (6-chloro-2-(hex-1-yn-1-yl)-8-(thiophen-2-yl)-9H-purin-9-yl)methyl pivalate (113b)

A target compound (113b) (79%) is obtained by the same synthetic method as for Compound (113a): ¹H NMR (400 MHz, CDCl3) δ 7.76 (d, 1H, J = 4.0 Hz), 7.68 (d, 1H, J = 4.0 Hz), 7.24 (t, 1H, J = 4.0, 4.0 Hz), 6.37 (s, 2H), 2.51 (t, 2H, J = 8.0, 8.0 Hz), 1.70-1.64 (m, 2H), 1.53-1.48 (m, 2H), 1.20 (s, 9H), 0.96 (t, 3H, J = 8.0, 8.0 Hz).

### (6-chloro-2-(hex-1-yn-1-yl)-8-(1-methyl-1H-pyrrol-2-yl)-9H-purin-9-yl)methyl pivalate (113c)

A target compound (113c) (59%) is obtained by the same synthetic method as for Compound (113a): ¹H NMR (400 MHz, CDCl₃) δ 7.36 (d, 1H, J = 4.0 Hz), 5.91 (s, 2H), 5.27 (d, 1H, J=2.4), 4.89 (d, 1H, J=3.2), 3.20 (s, 2H), 2.38 (t, 2H, J = 2.5), 1.63 (m, 2H, J = 2.4), 1.51 (m, 2H, J = 3.1), 1.20 (s, 9H), 0.98 (t, 3H, J = 3.2).

### (6-chloro-2-(hex-1-yn-1-yl)-8-(6-methoxypyridin-2-yl)-9H-purin-9-yl)methyl pivalate (113d)

A target compound (113d) (60%) is obtained by the same synthetic method as for Compound (113a): ¹H NMR (400 MHz, CDCl₃) δ 7.09 (d, 1H, J = 2.0 Hz), 6.97 (d, 1H, J = 2.8), 6.19 (d, 1H, J = 3.7), 6.05 (s, 2H), 2.51 (t, 2H, J = 2.5), 1.72 (m, 2H, J = 2.4), 1.60 (m, 2H, J = 3.1), 1.32 (s, 9H), 1.02 (t, 3H, J = 3.2) 0.35 (s, 3H).

### (6-chloro-2-(hex-1-yn-1-yl)-8-(pyrimidin-5-yl)-9H-purin-9-yl)methyl pivalate (113e)

A target compound (113e) (61%) is obtained by the same synthetic method as for Compound (113a): ¹H NMR (400 MHz, CDCl₃) δ 9.03 (s, 1H), 8.79 (m, 2H), 5.96 (s, 2H), 2.48 (t, 2H, J = 2.5), 1.69 (m, 2H, J = 2.4), 1.62 (m, 2H, J = 3.1), 1.25 (s, 9H), 0.99 (t, 3H, J = 3.3).

### (6-chloro-2-(hex-1-yn-1-yl)-8-(thiazol-5-yl)-9H-purin-9-yl)methyl pivalate (113f)

A target compound (113f) (58%) is obtained by the same synthetic method as for Compound (113a): ¹H NMR (400 MHz, CDCl₃) δ 8.92 (s, 1H), 7.57 (s, 1H), 5.87 (s, 2H), 2.41 (t, 2H, J = 2.5), 1.62 (m, 2H, J = 2.4), 1.56 (m, 2H, J = 3.1), 1.20 (s, 9H), 0.96 (t, 3H, J = 3.5).

### 6-chloro-2-(hex-1-yn-1-yl)-8-(5-methylfuran-2-yl)-9H-purine (114a)

Starting Material (113a) (200 mg, 0.47 mmol) is suspended in 0.5 N NaOH (3 mL) and i-PrOH (5 mL), and the resultant is stirred at room temperature for 24 hours. After it is confirmed that the reaction is completed, the resultant is neutralized to pH 7 with AcOH and concentrated under reduced pressure. The resulting residue is purified by column chromatography under the condition of DCM/MeOH = 50/1 to obtain a target compound (114a) (117 mg, 79%) as a yellow solid: ¹H NMR (400 MHz, DMSO- d6) δ 7.39 (d, 1H, J = 4.0 Hz), 6.29 (d, 1H, J = 4.0 Hz), 2.51 (t, 2H, J = 4.0, 8.0), 2.44 (s, 3H), 1.60-1.54 (m, 2H), 1.49-1.44 (m, 2H), 0.94 (t, 3H, J = 8.0, 8.0 Hz).

### 6-chloro-2-(hex-1-yn-1-yl)-8-(thiophen-2-yl)-9H-purine (114b)

A target compound 114b (85%) is obtained by the same synthetic method as for Compound (114a): ¹H NMR (400 MHz, DMSO- d6) δ 8.04 (d, 1H, J = 0.0), 7.94 (d, 1H, J = 8.0), 7.32 (t, 1H J = 4.0, 4.0), 2.51 (t, 2H, J = 4.0, 8.0), 1.60-1.54 (m, 2H), 1.49-1.44 (m, 2H), 0.94 (t, 3H, J = 8.0, 8.0 Hz).

### 6-chloro-2-(hex-1-yn-1-yl)-8-(1-methyl-1H-pyrrol-2-yl)-9H-purine (114c)

A target compound (114c) (68%) is obtained by the same synthetic method as for Compound (114a): ¹H NMR (400 MHz, CDCl₃) δ 12.82 (s, 1H), 7.36 (d, 1H, J = 4.0 Hz), 5.91 (s, 2H), 5.27 (d, 1H, J=2.4), 4.89 (d, 1H, J=3.2), 3.20 (s, 2H), 2.38 (t, 2H, J = 2.5), 1.63 (m, 2H, J = 2.4), 1.51 (m, 2H, J = 3.1), 0.98 (t, 3H, J = 3.2).

### 6-chloro-2-(hex-1-yn-1-yl)-8-(6-methoxypyridin-2-yl)-9H-purine (114d)

A target compound (114d) (46%) is obtained by the same synthetic method as for Compound (114a): ¹H NMR (400 MHz, CDCl₃) δ 12.76 (s, 1H)7.09 (d, 1H, J = 2.0 Hz), 6.97 (d, 1H, J = 2.8), 6.19 (d, 1H, J = 3.7), 6.05 (s, 2H), 2.51 (t, 2H, J = 2.5), 1.72 (m, 2H, J = 2.4), 1.60 (m, 2H, J = 3.1), 1.02 (t, 3H, J = 3.2) 0.35 (s, 3H).

### 6-chloro-2-(hex-1-yn-1-yl)-8-(pyrimidin-5-yl)-9H-purine (114e)

A target compound (114e) (45%) is obtained by the same synthetic method as for Compound (114a): ¹H NMR (400 MHz, CDCl₃) δ 12.87 (s, 1H) 9.03 (s, 1H), 8.79 (m, 2H), 5.96 (s, 2H), 2.48 (t, 2H, J = 2.5), 1.69 (m, 2H, J = 2.4), 1.62 (m, 2H, J = 3.1), 0.99 (t, 3H, J = 3.3).

### 5-(6-chloro-2-(hex-1-yn-1-yl)-9H-purin-8-yl)thiazole (114f)

A target compound (114c) (38%) is obtained by the same synthetic method as for Compound (114a): ¹H NMR (400 MHz, CDCl₃) δ 12.71 (s, 1H), 8.92 (s, 1H), 7.57 (s, 1H), 5.87 (s, 2H), 2.41 (t, 2H, J = 2.5), 1.62 (m, 2H, J = 2.4), 1.56 (m, 2H, J = 3.1), 0.96 (t, 3H, J = 3.5).

### (2R,3R)-2-(6-chloro-2-(hex-1-yn-1-yl)-8-(5-methylfuran-2-yl)-9H-purin-9-yl)tetrahydrofuran-3-yl acetate (115a)

Starting Material (114a) (187 mg, 0.60 mmol) and bis(trimethylsilyl)acetamide (0.13 ml) are added to EDC (16 ml) and the resultant is dissolved under stirring at 40°C. Tetrahydrofuran-2,3-diyl diacetate (83.5 mg) is added to the mixture at room temperature, trimethyl silyltrifluoromethane sulfonate (50 µl) is added thereto at 0°C, and then the resulting mixture is stirred at 80°C for 2 hours. After it is confirmed that the reaction is completed, the resultant is neutralized to pH 7 with NaHCO₃ and extracted with DCM. The resulting organic layer is dried over MgSO₄ and then concentrated under reduced pressure. The resulting residue is purified by column chromatography under the condition of DCM/EtOAc = 10/1 to obtain a target compound (115a) (40 mg, 15%): ¹H NMR (400 MHz, CDCl3) δ 7.39 (d, 1H, J = 4.0 Hz), 6.52 (t, 1H, J = 4.0, 4.0 Hz), 6.36 (d, 1H, J = 4.0), 6.29 (d, 1H, J = 4.0 Hz), 5.88 (t, 2H, J = 4.0, 4.0 Hz), 4.85 (dd, 1H, J = 4.0, 8.0 Hz), 4.20 (d, 1H, J = 8.0 Hz), 2.50 (t, 2H, J = 8.0, 8.0 Hz), 2.44 (s, 3H), 2.17 (s, 3H), 1.70-1.64 (m, 2H), 1.54-1.49 (m, 2H), 0.97 (t, 3H, J = 8.0, 8.0 Hz).

### (2R,3R)-2-(6-chloro-2-(hex-1-yn-1-yl)-8-(thiophen-2-yl)-9H-purin-9-yl)tetrahydrofuran-3-yl acetate (115b)

A target compound (115b) (66%) is obtained using sugar (107) by the same synthetic method as for Compound (115a): ¹H NMR (400 MHz, CDCl3) δ 7.68 (t, 2H, J = 4.0, 4.0 Hz), 7.26 (d, 1H, J = 4.0 Hz), 6.52 (t, 1H, J = 4.0, 4.0 Hz), 6.36 (d, 1H, J = 4.0), 5.88 (t, 2H, J = 4.0, 4.0 Hz), 4.85 (dd, 1H, J = 4.0, 8.0 Hz), 4.20 (d, 1H, J = 8.0 Hz), 2.50 (t, 2H, J = 8.0, 8.0 Hz), 2.17 (s, 3H), 1.70-1.64 (m, 2H), 1.54-1.49 (m, 2H), 0.97 (t, 3H, J = 8.0, 8.0 Hz).

### (2R,3R)-2-(6-chloro-2-(hex-1-yn-1-yl)-8-(1-methyl-1H-pyrrol-2-yl)-9H-purin-9-yl)tetrahydrofuran-3-yl acetate (115c)

A target compound (115c) (40%) is obtained using sugar (7) by the same synthetic method as for Compound (115a): ¹H NMR (400 MHz, CDCl₃) δ 7.36 (d, 1H, J = 4.0 Hz), 6.38 (d, 1H, J = 2.0), 5.91 (s, 2H), 5.27 (d, 1H, J=2.4), 4.89 (d, 1H, J=3.2), 3.20 (s, 2H), 2.92 (m, 1H, J = 2.2), 2.83 (m, 2H, J = 2.3), 2.52 (t, 3H, J = 2.5), 2.35 (t, 2H, J = 2.5), 2.08 (s, 3H), 1.63 (m, 2H, J = 2.4), 1.51 (m, 2H, J = 3.1), 0.98 (t, 3H, J = 3.2).

### (2R,3R)-2-(6-chloro-2-(hex-1-yn-1-yl)-8-(6-methoxypyridin-2-yl)-9H-purin-9-yl)tetrahydrofuran-3-yl acetate (115d)

A target compound (115d) (29%) is obtained using sugar (107) by the same synthetic method as for Compound (115a): ¹H NMR (400 MHz, CDCl₃) δ 7.09 (d, 1H, J = 2.0 Hz), 6.97 (d, 1H, J = 2.8), 6.31 (d, 1H, J = 2.0), 6.19 (d, 1H, J = 3.7), 6.05 (s, 2H), 2.98 (m, 1H, J = 2.2), 2.80 (m, 2H, J = 2.3), 2.57 (t, 3H, J = 2.5), 2.51 (t, 2H, J = 2.5), 2.08 (s, 3H), 1.72 (m, 2H, J = 2.4), 1.60 (m, 2H, J = 3.1), 1.02 (t, 3H, J = 3.2) 0.35 (s, 3H).

### (2R,3R)-2-(6-chloro-2-(hex-1-yn-1-yl)-8-(pyrimidin-5-yl)-9H-purin-9-yl)tetrahydrofuran-3-yl acetate (115e)

A target compound (115e) (32%) is obtained using sugar (107) by the same synthetic method as for Compound (115a): ¹H NMR (400 MHz, CDCl₃) δ 9.03 (s, 1H), 8.79 (m, 2H), 6.31 (d, 1H, J = 2.0), 5.96 (s, 2H), 2.98 (m, 1H, J = 2.2), 2.80 (m, 2H, J = 2.3), 2.57 (t, 3H, J = 2.5) 2.48 (t, 2H, J = 2.5), 2.07 (s, 3H), 1.69 (m, 2H, J = 2.4), 1.62 (m, 2H, J = 3.1), 0.99 (t, 3H, J = 3.3).

### (2R,3R)-2-(6-chloro-2-(hex-1-yn-1-yl)-8-(thiazol-5-yl)-9H-purin-9-yl)tetrahydrofuran-3-yl acetate (115f)

A target compound (115f) (31%) is obtained using sugar (107) by the same synthetic method as for Compound (115a): ¹H NMR (400 MHz, CDCl₃) δ 9.03 (s, 1H), 8.79 (m, 2H), 6.37 (d, 1H, J = 2.1), 5.96 (s, 2H), 2.98 (m, 1H, J = 2.3), 2.81 (m, 2H, J = 3.0), 2.56 (t, 2H, J = 2.5), 2.48 (t, 2H, J = 2.5), 2.03 (s, 3H), 1.69 (m, 2H, J = 2.4), 1.62 (m, 2H, J = 3.1), 0.99 (t, 3H, J = 3.3).

### (2R,3R)-2-(6-chloro-2-(hex-1-yn-1-yl)-8-(5-methylfuran-2-yl)-9H-purin-9-yl)tetrahydrofuran-3-ol (116a)

Starting Material 115a (70 mg, 0.16 mmol) is dissolved in NH₃ in isopropyl alcohol (10 mL), and the resulting solution is stirred at 140°C for 18 hours. After it is confirmed that the reaction is completed, a pale yellow solid obtained by concentrating the reaction mixture under reduced pressure is dissolved in NH₃ in MeOH, and then the resulting solution is stirred at 140°C for 1 hour. After it is confirmed that the reaction is completed, the reaction mixture is concentrated under reduced pressure. The resulting residue is purified by the column chromatography under the condition of DCM/MeOH = 97/3 to obtain a target compound (116a) (3 mg, 50%) as a pale yellow solid: ¹H NMR (400 MHz, MeOD) δ 7.09 (d, 1H, J = 4.0 Hz), 6.32 (d, 1H, J = 4.0 Hz), 6.17 (d, 1H, J = 0.0 Hz), 5.32 (bs, 2H), 4.40 (t, 1H, J = 8.0, 4.0 Hz), 4.21 (t, 1H, J = 8.0, 8.0 Hz), 2.85-2.80 (m, 1H), 2.47-2.44 (m, 6H), 2.11-2.07 (m, 1H), 1.88-1.59 (m, 2H), 1.59-1.50 (m, 2H), 0.98 (t, 3H, J = 8.0, 8.0 Hz).

### (2R,3R)-2-(6-chloro-2-(hex-1-yn-1-yl)-8-(thiophen-2-yl)-9H-purin-9-yl)tetrahydrofuran-3-ol (116b)

A target compound (116b) (49%) is obtained by the same synthetic method as for Compound (116a): ¹H NMR (400 MHz, MeOD) δ 7.74 (d, 1H, J = 4.0 Hz), 7.71 (d, 1H, J = 4.0 Hz), 7.25 (t, 1H, J = 4.0, 4.0 Hz), 6.00 (d, 1H, J = 4.0 Hz), 5.38 (bs, 2H), 4.41-4.39 (m, 1H), 4.21-4.19 (m, 1H), 2.86-2.82 (m, 1H), 2.45 (t, 3H, J = 8.0, 8.0 Hz), 2.10-2.07 (m, 1H), 1.65-1.59 (m, 2H), 1.57-1.50 (m, 2H), 0.98 (t, 3H, J = 8.0, 8.0 Hz).

### (2R,3R)-2-(6-chloro-2-(hex-1-yn-1-yl)-8-(1-methyl-1H-pyrrol-2-yl)-9H-purin-9-yl)tetrahydrofuran-3-ol (116c)

A target compound (116c) (42%) is obtained by the same synthetic method as for Compound (116a): ¹H NMR (400 MHz, MeOD) δ 7.36 (d, 1H, J = 4.0 Hz), 6.38 (d, 1H, J = 2.0), 5.91 (s, 2H), 5.27 (d, 1H, J=2.4), 4.89 (d, 1H, J=3.2), 3.20 (s, 2H), 2.92 (m, 1H, J = 2.2), 2.83 (m, 2H, J = 2.3), 2.52 (t, 3H, J = 2.5), 2.35 (t, 2H, J = 2.5), 1.63 (m, 2H, J = 2.4), 1.51 (m, 2H, J = 3.1), 0.98 (t, 3H, J = 3.2).

### (2R,3R)-2-(6-chloro-2-(hex-1-yn-1-yl)-8-(6-methoxypyridin-2-yl)-9H-purin-9-yl)tetrahydrofuran-3-ol (116d)

A target compound (116d) (39%) is obtained by the same synthetic method as for Compound (116a): ¹H NMR (400 MHz, MeOD) δ 7.09 (d, 1H, J = 2.0 Hz), 6.97 (d, 1H, J = 2.8), 6.31 (d, 1H, J = 2.0), 6.19 (d, 1H, J = 3.7), 6.05 (s, 2H), 2.98 (m, 1H, J = 2.2), 2.80 (m, 2H, J = 2.3), 2.57 (t, 3H, J = 2.5), 2.51 (t, 2H, J = 2.5), 1.72 (m, 2H, J = 2.4), 1.60 (m, 2H, J = 3.1), 1.02 (t, 3H, J = 3.2) 0.35 (s, 3H).

### (2R,3R)-2-(6-chloro-2-(hex-1-yn-1-yl)-8-(pyrimidin-5-yl)-9H-purin-9-yl)tetrahydrofuran-3-ol (116e)

A target compound (116e) (32%) is obtained by the same synthetic method as for Compound (116a): ¹H NMR (400 MHz, MeOD) δ 9.03 (s, 1H), 8.79 (m, 2H), 6.31 (d, 1H, J = 2.0), 5.96 (s, 2H), 2.98 (m, 1H, J = 2.2), 2.80 (m, 2H, J = 2.3), 2.57 (t, 3H, J = 2.5) 2.48 (t, 2H, J = 2.5), 1.69 (m, 2H, J = 2.4), 1.62 (m, 2H, J = 3.1), 0.99 (t, 3H, J = 3.3).

### (2R,3R)-2-(6-chloro-2-(hex-1-yn-1-yl)-8-(thiazol-5-yl)-9H-purin-9-yl)tetrahydrofuran-3-ol (116f)

A target compound (116f) (30%) is obtained by the same synthetic method as for Compound (116a): ¹H NMR (400 MHz, MeOD) δ 9.03 (s, 1H), 8.79 (m, 2H), 6.37 (d, 1H, J = 2.1), 5.96 (s, 2H), 2.98 (m, 1H, J = 2.3), 2.81 (m, 2H, J = 3.0), 2.56 (t, 2H, J = 2.5), 2.48 (t, 2H, J = 2.5), 1.69 (m, 2H, J = 2.4), 1.62 (m, 2H, J = 3.1), 0.99 (t, 3H, J = 3.3).

### Preparation Example 3: Synthesis of 121a to 121x

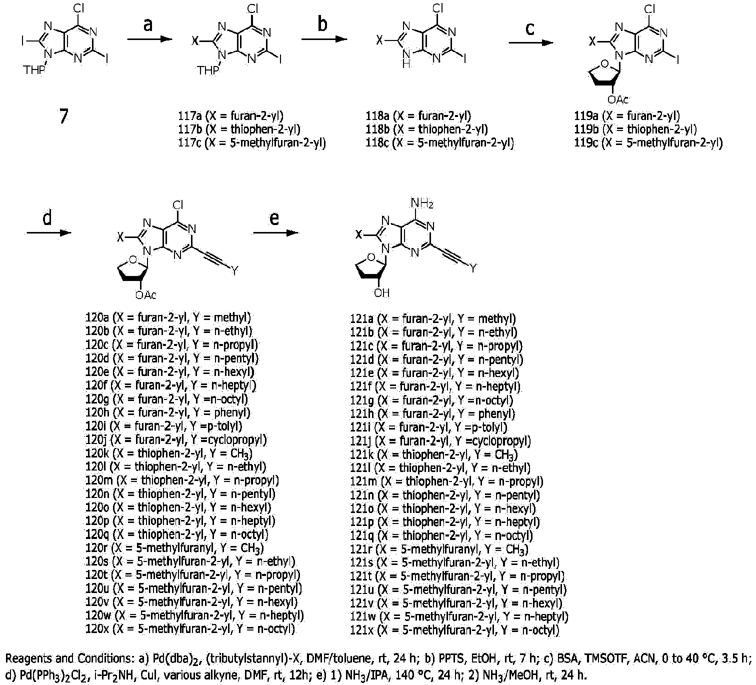

### 6-chloro-8-(furan-2-yl)-2-iodo-9-(tetrahydro-2H-pyran-2-yl)-9H-purine (117a)

After Starting Material (7) (35 g, 71 mmol) is dissolved in DMF/toluene (250 mL) under nitrogen, Pd(dba)₂ (0.01 mmol) and 2-tributylstannylfuran (74.55 mmol) are added thereto, and the resulting mixture is stirred at room temperature for 24 hours. After it is confirmed that the reaction is completed, the reaction mixture is extracted with EtOAc (350 mL), dried over anhydrous MgSO₄ and concentrated under reduced pressure. The resulting residue is purified by column chromatography under the condition of DCM/MeOH = 100/1 to obtain a target compound (117a) (16.8 g, 55%) in the form of an ivory solid: ¹H NMR (600 MHz, CDCl₃) δ 7.91 (d, 1H), 7.13 (d, 1H), 6.70 (t, J = 7.2 Hz, 1H), 5.80 (t, J = 7.7 Hz, 1H), 3.67-3.57 (m, 2H), 2.17-1.92 (m, 2H), 1.74-1.64 (m, 2H), 1.58-1.55 (m, 2H).

### 6-chloro-2-iodo-9-(tetrahydro-2H-pyran-2-yl)-8-(thiophen-2-yl)-9H-purine (117b)

A target compound (117b) is obtained using 2-tributylstannylthiofuran by the same synthetic method as for Compound (117a): ¹H NMR (600 MHz, CDCl₃) δ 7.87 (d, 1H), 7.85 (d, 1H), 7.24 (t, 1H, J = 7.4 Hz), 5.75 (t, 1H, J = 7.2 Hz), 3.55-3.48 (m, 2H), 2.07-1.84 (m, 2H), 1.65-1.54 (m, 2H), 1.48-1.45 (m, 2H).

### 6-chloro-2-iodo-8-(5-methylfuran-2-yl)-9-(tetrahydro-2H-pyran-2-yl)-9H-purine (117c)

A target compound (117c) is obtained using 5-methyl-2-(tributylstannyl)furan by the same synthetic method as for Compound (117a): ¹H NMR (400 MHz, DMSO) δ 6.87 (d, 1H, J = 2.0), 6.02 (d, 2H, J = 2.1), 5.66 (t, 1H, J = 1.3), 3.52 (m, 2H), 2.21 (s, 3H), 1.91 (m, 2H, J = 1.6), 1.52 (m, 4H).

### 6-chloro-8-(furan-2-yl)-2-iodo-9H-purine (118a)

After Starting Material (117a) (500 mg, 1.2 mmol) is dissolved in THF/Ethanol, PPTS in H₂O (0.5 mL) is slowly added thereto at room temperature. The reaction mixture is stirred at room temperature for 16 hours, and then concentrated under reduced pressure. The resulting residue is purified by column chromatography under the condition of hexane/EA = 3/2 to obtain a target compound (117c) (180 mg, 43%): ¹H NMR (400 MHz, DMSO) δ 12.00 (s, 1H), 8.26 (d, 1H, J = 2.6), 7.63 (d, 1H, J = 2.0), 6.89 (t, 1H, J = 2.3).

### 6-chloro-2-iodo-8-(thiophen-2-yl)-9H-purine (118b)

A target compound (118b) is obtained by the same synthetic method as for Compound (118a): ¹H NMR (400 MHz, DMSO) δ 12.00 (s, 1H), 8.07 (d, 1H, J = 2.6), 7.63 (d, 1H, J = 2.0), 7.27 (t, 1H, J = 2.3).

### 6-chloro-2-iodo-8-(5-methylfuran-2-yl)-9H-purine (118c)

A target compound (118c) is obtained by the same synthetic method as for Compound (118a): ¹H NMR (400 MHz, CDCl₃) δ 12.00 (s, 1H), 6.52 (d, 1H, J = 2.3), 6.01 (d, 1H, J = 2.1), 2.03 (s, 3H).

### (2R,3R)-2-(6-chloro-8-(furan-2-yl)-2-iodo-9H-purin-9-yl)tetrahydrofuran-3-yl acetate (119a)

After Starting Material (118a) (1.5 g, 4.1 mmol) is dissolved in EDC (45 mL), N,O-bis(trimethylsilyl)acetamide (1.3 mL) is slowly added thereto, the resultant is stirred at 40°C for 1 hour, and then cooled to 0°C, sugar (107) (922 mg) dissolved in EDC (5 mL) is added thereto, TMSOTf (0.52 mL) is added thereto, and then the resultant is stirred at 80°C for 3 hours. The reaction mixture is cooled to 0°C to terminate the reaction using a saturated NaHCO₃ solution, extracted with EtOAc/H₂O, and then dried over anhydrous MgSO₄ and concentrated under reduced pressure. The resulting residue is purified by column chromatography under the condition of hexane/EtOAc = 4/1 to obtain a target compound (119a) (980 mg, 49%): ¹H NMR (400 MHz, DMSO) δ 6.52 (d, 1H), 6.39 (s, 1H), 6.07 (t, 1H, J = 2.0), 5.32 (d, 1H, J = 2.8), 3.25 (t, 2H, J = 3.2), 2.28 (t, 2H, J = 2.8), 1.89 (s, 3H).

### (2R,3R)-2-(6-chloro-2-iodo-8-(thiophen-2-yl)-9H-purin-9-yl)tetrahydrofuran-3-yl acetate (119b)

A target compound (119b) is obtained by the same synthetic method as for Compound (119a): ¹H NMR (400 MHz, DMSO) δ 7.14 (d, 1H, J = 2.4), 7.01 (d, 1H, J = 2.6), 6.84 (t, 1H, J = 2.1), 6.39 (s, 1H), 5.32 (d, 1H, J = 2.8), 3.25 (t, 2H, J = 3.2), 2.28 (t, 2H, J = 2.8), 1.89 (s, 3H).

### (2R,3R)-2-(6-chloro-2-iodo-8-(5-methylfuran-2-yl)-9H-purin-9-yl)tetrahydrofurn-3-yl acetate (119c)

A target compound (119c) is obtained by the same synthetic method as for Compound (119a): ¹H NMR (400 MHz, DMSO) δ 6.52 (d, 1H), 6.39 (s, 1H), 6.07 (t, 1H, J = 2.0), 3.25 (t, 2H, J = 3.2), 2.51 (s, 3H), 2.28 (t, 2H, J = 2.8), 1.89 (s, 3H).

### (2R,3R)-2-(6-chloro-8-(furan-2-yl)-2-(prop-1-yn-1-yl)-9H-purin-9-yl)tetrahydrofuran-3-yl acetate (120a)

After Starting Material (119a) (100 mg, 0.21 mmol) is dissolved in DMF, CuI (8 mg), Pd(PPh₃)₂Cl₂ (15 mg), DIPA (0.09 mL), and 1-propyne (0.03 mL) is slowly added thereto. The reaction mixture is sufficiently degassed with nitrogen and then stirred at room temperature for 1 hour. After it is confirmed that the reaction is completed, the reaction mixture is extracted with EtOAc/H₂O, dried over anhydrous MgSO₄, and concentrated under reduced pressure. The resulting residue is purified by column chromatography under the condition of hexane/EtOAc = 5/1 to obtain a target compound (120a) (84 mg, 96%) as a yellow solid: ¹H NMR (400 MHz, CDCl₃) δ 7.71 (s, 1H), 7.42 (d, J = 2.9 Hz, 1H), 6.63 - 6.68 (m, 1H), 6.46 (d, J = 2.0 Hz, 1H), 6.19 - 6.24 (m, 1H), 4.39 - 4.48 (m, 1H), 4.20 - 4.30 (m, 1H), 3.10 - 3.12 (m, 1H), 2.20 - 2.31 (m, 1H), 2.13 (s, 3H), 2.09 (s, 3H).

### (2R,3R)-2-(2-(but-1-yn-1-yl)-6-chloro-8-(furan-2-yl)-9H-purin-9-yl)tetrahydrofuran-3-yl acetate (120b)

A target compound (120b) (68 mg, 35%) is obtained using 1-butyne by the same synthetic method as for Compound (120a): ¹H NMR (400 MHz, CDCl₃) δ 7.71 (s, 1H), 7.42 (d, J = 3.4 Hz, 1H), 6.63 - 6.67 (m, 1H), 6.46 (d, J = 1.8 Hz, 1H), 6.16 - 6.25 (m, 1H), 4.36 - 4.49 (m, 1H), 4.22 - 4.32 (m, 1H), 3.09 - 3.23 (m, 1H), 2.49 (q, J = 7.5 Hz, 2H), 2.17 - 2.32 (m, 1H), 2.09 (s, 3H), 1.29 (t, J = 7.5 Hz, 3H).

### (2R,3R)-2-(6-chloro-8-(furan-2-yl)-2-(pent-1-yn-1-yl)-9H-purin-9-yl)tetrahydrofuran-3-yl acetate (120c)

A target compound (120c) (102 mg, 63%) is obtained using 1-pentyne by the same synthetic method as for Compound (120a): ¹H NMR (400 MHz, CDCl₃) δ 7.71 (s, 1H), 7.42 (d, J = 3.1 Hz, 1H), 6.63 - 6.67 (m, 1H), 6.46 (d, J = 1.9 Hz, 1H), 6.16 - 6.26 (m, 1H), 4.37 - 4.50 (m, 1H), 4.21 - 4.32 (m, 1H), 3.07 - 3.24 (m, 1H), 2.46 (t, J = 7.1 Hz, 2H), 2.21 - 2.32 (m, 1H), 2.09 (s, 3H), 1.63 - 1.77 (m, 2H), 1.08 (t, J = 7.4 Hz, 3H).

### (2R,3R)-2-(6-chloro-8-(furan-2-yl)-2-(hept-1-yn-1-yl)-9H-purin-9-yl)tetrahydrofuran-3-yl acetate (120d)

A target compound (120d) (98 mg, 61%) is obtained using 1-heptyne by the same synthetic method as for Compound (120a): ¹H NMR (400 MHz, CDCl₃) δ 7.70 (s, 1H), 7.42 (d, J = 3.6 Hz, 1H), 6.63 - 6.68 (m, 1H), 6.46 (d, J = 1.9 Hz, 1H), 6.17 - 6.25 (m, 1H), 4.37 - 4.49 (m, 1H), 4.20 - 4.34 (m, 1H), 3.11 - 3.23 (m, 1H), 2.47 (t, J = 7.2 Hz, 2H), 2.16 - 2.34 (m, 1H), 2.09 (s, 3H), 1.62 - 1.74 (m, 2H), 1.30 - 1.51 (m, 4H), 0.93 (t, J = 7.2 Hz, 3H).

### (2R,3R)-2-(6-chloro-8-(furan-2-yl)-2-(oct-1-yn-1-yl)-9H-purin-9-yl)tetrahydrofuran-3-yl acetate (120e)

A target compound (120e) (79 mg, 61%) is obtained using 1-octyne by the same synthetic method as for Compound (120a): ¹H NMR (400 MHz, CDCl₃) δ 7.70 (s, 1H), 7.42 (d, J = 3.5 Hz, 1H), 6.63 - 6.68 (m, 1H), 6.46 (d, J = 1.9 Hz, 1H), 6.17 - 6.23 (m, 1H), 4.40 - 4.48 (m, 1H), 4.21 - 4.31 (m, 1H), 3.10 - 3.22 (m, 1H), 2.47 (t, J = 7.2 Hz, 2H), 2.19 - 2.32 (m, 1H), 2.09 (s, 3H), 1.61 - 1.72 (m, 2H), 1.43 - 1.52 (m, 2H), 1.28 - 1.39 (m, 4H), 0.90 (t, J = 6.7 Hz, 3H).

### (2R,3R)-2-(6-chloro-8-(furan-2-yl)-2-(non-1-yn-1-yl)-9H-purin-9-yl)tetrahydrofuran-3-yl acetate (120f)

A target compound (120f) (91 mg, 61%) is obtained using 1-nonyne by the same synthetic method as for Compound (120a): ¹H NMR (400 MHz, CDCl₃) δ 7.70 (s, 1H), 7.41 (d, J = 3.5 Hz, 1H), 6.63 - 6.68 (m, 1H), 6.45 (d, J = 1.9 Hz, 1H), 6.15 - 6.23 (m, 1H), 4.38 - 4.49 (m, 1H), 4.20 - 4.31 (m, 1H), 3.08 - 3.21 (m, 1H), 2.46 (t, J = 7.2 Hz, 2H), 2.19 - 2.31, 2.08 (s, 3H), (m, 1H), 1.56 - 1.73 (m, 2H), 1.41 - 1.54 (m, 2H), 1.19 - 1.39 (m, 8H), 0.88 (t, J = 6.6 Hz, 3H).

### (2R,3R)-2-(6-chloro-2-(dec-1-yn-1-yl)-8-(furan-2-yl)-9H-purin-9-yl)tetrahydrofuran-3-yl acetate (120g)

A target compound (120g) (91 mg, 53%) is obtained using 1-decyne by the same synthetic method as for Compound (120a): ¹H NMR (400 MHz, CDCl₃) δ 7.70 (s, 1H), 7.42 (d, J = 3.5 Hz, 1H), 6.63 - 6.68 (m, 1H), 6.45 (d, J = 1.9 Hz, 1H), 6.17 - 6.23 (m, 1H), 4.38 - 4.47 (m, 1H), 4.20 - 4.31 (m, 1H), 3.08 - 3.22 (m, 1H), 2.46 (t, J = 7.2 Hz, 2H), 2.20 - 2.29 (m, 1H), 2.08 (s, 3H), 1.59 - 1.73 (m, 2H), 1.40 - 1.53 (m, 2H), 1.21 - 1.36 (m, 8H), 0.89 (t, J = 6.4 Hz, 3H).

### (2R,3R)-2-(6-chloro-8-(furan-2-yl)-2-(phenylethynyl)-9H-purin-9-yl)tetrahydrofuran-3-yl acetate (120h)

A target compound (120h) (87 mg, 52%) is obtained using phenylacetylene by the same synthetic method as for Compound (120a): ¹H NMR (400 MHz, CDCl₃) δ 7.72 (s, 1H), 7.65 - 7.70 (m, 2H), 7.45 (d, J = 3.6 Hz, 1H), 7.37 - 7.43 (m, 3H), 6.65 - 6.70 (m, 1H), 6.50 (d, J = 1.9 Hz, 1H), 6.22 - 6.28 (m, 1H), 4.42 - 4.55 (m, 1H), 4.22 - 4.35 (m, 1H), 3.13 - 3.28 (m, 1H), 2.21 - 2.36 (m, 1H), 2.10 (s, 3H).

### (2R,3R)-2-(6-chloro-8-(furan-2-yl)-2-(p-tolylethynyl)-9H-purin-9-yl)tetrahydrofuran-3-yl acetate (120i)

A target compound (120i) (99 mg, 51%) is obtained using 4-ethynyltoluene by the same synthetic method as for Compound (120a): ¹H NMR (400 MHz, CDCl₃) δ 7.72 (s, 1H), 7.57 (d, J = 7.9 Hz, 2H), 7.44 (d, J = 3.3 Hz, 1H), 7.20 (d, J = 8.0 Hz, 2H), 6.65 - 6.68 (m, 1H), 6.49 (d, J = 1.6 Hz, 1H), 6.20 - 6.28 (m, 1H), 4.42 - 4.55 (m, 1H), 4.22 - 4.33 (m, 1H), 3.13 - 3.28 (m, 1H), 2.40 (s, 3H), 2.23 - 2.33 (m, 1H), 2.11 (s, 3H).

### (2R,3R)-2-(6-chloro-2-(cyclopropylethynyl)-8-(furan-2-yl)-9H-purin-9-yl)tetrahydrofuran-3-yl acetate (120j)

A target compound (120j) (102 mg, 45%) is obtained using cyclopropylene by the same synthetic method as for Compound (120a): ¹H NMR (400 MHz, CDCl₃) δ 7.70 (s, 1H), 7.41 (d, J = 3.5 Hz, 1H), 6.62 - 6.67 (m, 1H), 6.45 (d, J = 1.9 Hz, 1H), 6.12 - 6.23 (m, 1H), 4.36 - 4.48 (m, 1H), 4.20 - 4.31 (m, 1H), 3.07 - 3.24 (m, 1H), 2.20 - 2.29 (m, 1H), 2.09 (s, 3H), 1.49 - 1.55 (m, 1H), 0.92 - 1.01 (m, 4H).

### (2R,3R)-2-(6-chloro-2-(prop-1-yn-1-yl)-8-(thiophen-2-yl)-9H-purin-9-yl)tetrahydrofuran-3-yl acetate (120k)

After Starting Material (119b) (100 mg, 0.20 mmol) is dissolved in DMF under nitrogen, CuI (8 mg), Pd(PPh₃)₂Cl₂ (15 mg), DIPA (0.09 mL), and 1-propyne (0.02 mL) are slowly added thereto, and the resulting mixture is stirred at room temperature for 1 hour. The reaction mixture is extracted with EtOAc/H₂O, then dried over MgSO₄, and concentrated under reduced pressure. The resulting residue is purified by column chromatography under the condition of hexane/EtOAc = 5/1 to obtain a target compound (120k) (21 mg, 25%): ¹H NMR (400 MHz, DMSO-d₆) δ 7.89 (s, 1H), 7.03 (d, J = 3.5 Hz, 1H), 6.63 - 6.91 (m, 1H), 6.25 (d, J = 3.6 Hz, 1H), 5.55 (d, J = 4.8 Hz, 1H), 4.22 - 4.38 (m, 1H), 4.11 - 4.20 (m, 1H), 2.40-2.48 (m, 1H), 2.28 (s, 3H), 2.05 (s, 3H), 1.88 - 1.93 (m, 1H).

### (2R,3R)-2-(2-(but-1-yn-1-yl)-6-chloro-8-(thiophen-2-yl)-9H-purin-9-yl)tetrahydrofuran-3-yl acetate (1201)

A target compound (120l) (91 mg, 75%) is obtained by the same synthetic method as for Compound (120k): ¹H NMR (400 MHz, CDCl₃) δ 7.64(s, 1H), 7.42 (d, J = 3.4 Hz, 1H), 6.55 (dd, J = 3.4 Hz, 1H), 5.40 - 5.60 (m, 2H), 4.35 - 4.44 (m, 1H), 2.89-3.01 (m, 2H), 2.80 (t, J = 7.2 Hz, 2H), 2.30 (s, 3H), 1.59 - 1.68 (m, 2H), 0.99 (t, J = 7.8 Hz, 3H).

### (2R,3R)-2-(6-chloro-2-(pent-1-yn-1-yl)-8-(thiophen-2-yl)-9H-purin-9-yl)tetrahydrofuran-3-yl acetate (120m)

A target compound (120m) (101 mg, 77%) is obtained by the same synthetic method as for Compound (120k): ¹H NMR (400 MHz, DMSO-d₆) δ 7.87 (s, 1H), 7.49 (s, 2H), 7.10 (d, J = 3.5 Hz, 1H), 6.77 - 6.89 (m, 1H), 6.11 (d, J = 3.5 Hz, 1H), 5.48 (d, J = 4.8 Hz, 1H), 5.10 - 5.16 (m, 1H), 4.20 - 4.28 (m, 1H), 2.40 - 2.50 (m, 1H), 2.34 - 2.39 (m, 2H), 2.21 (s, 3H), 1.78 - 1.85 (m, 1H), 0.98 (t, J = 8.0 Hz, 3H), 0.79 - 0.90 (m, 2H).

### (2R,3R)-2-(6-chloro-2-(hept-1-yn-1-yl)-8-(thiophen-2-yl)-9H-purin-9-yl)tetrahydrofuran-3-yl acetate (120n)

A target compound (120n) (87 mg, 71%) is obtained by the same synthetic method as for Compound (120k): ¹H NMR (400 MHz, CDCl₃) δ 7.61 (s, 1H), 7.08 (d, J = 3.1 Hz, 1H), 6.55 (dd, J = 6.2 Hz, 1H), 6.10 (d, J = 3.5 Hz, 1H), 5.49 - 5.55 (m, 2H), 4.28-4.35 (m, 1H), 4.11 - 4.25 (m, 1H), 2.77 - 2.90 (m, 1H), 2.40 (t, J = 7.5 Hz, 2H), 2.25 (s, 3H), 2.11 - 2.21 (m, 1H), 1.50 - 1.77 (m, 2H), 1.44 - 1.50 (m, 2H), 1.28 - 1.35 (m, 2H), 0.88 (t, J = 7.5 H), 3H).

### (2R,3R)-2-(6-chloro-2-(oct-1-yn-1-yl)-8-(thiophen-2-yl)-9H-purin-9-yl)tetrahydrofuran-3-yl acetate (120o)

A target compound (120o) (79 mg, 77%) is obtained by the same synthetic method as for Compound (120k): ¹H NMR (400 MHz, CDCl₃) δ 7.58 (s, 1H), 7.08 (d, J = 3.8 Hz, 1H), 6.68 (dd, J = 3.5, 1H), 6.17 (d, J = 3.5 Hz, 1H), 5.43 - 5.58 (m, 3H), 4.37-4.46 (m, 1H), 4.20 - 4.33 (m, 1H), 2.87 - 2.92 (m, 1H), 2.52 (t, J = 7.2 Hz, 2H), 2.28 (s, 3H), 2.07 - 2.25 (m, 1H), 1.51 - 1.61 (m, 2H), 1.35 - 1.46 (m, 2H), 1.22 - 1.30 (m, 4H), 0.87 (t, J = 7.5 Hz, 3H).

### (2R,3R)-2-(6-chloro-2-(non-1-yn-1-yl)-8-(thiophen-2-yl)-9H-purin-9-yl)tetrahydrofuran-3-yl acetate (120p)

A target compound (120p) (97 mg, 72%) is obtained by the same synthetic method as for Compound (120k): ¹H NMR (400 MHz, CDCl₃) δ 7.55 (s, 1H), 7.25 (d, J = 3.5 Hz, 1H), 6.61 (dd, J = 3.1 Hz, 1H), 6.33 (d, J = 3.5 Hz, 1H), 5.50 - 5.54 (m, 3H), 4.41-4.52 (m, 1H), 4.22 - 4.38 (m, 2H), 2.82 - 2.91 (m, 1H), 2.25 (s, 3H), 2.10 - 2.21 (m, 1H), 1.61 - 1.68 (m, 2H), 1.42 - 1.59 (m, 2H), 1.22 - 1.35 (m, 6H), 0.85 (t, J = 4.8 Hz, 3H).

### (2R,3R)-2-(6-chloro-2-(dec-1-yn-1-yl)-8-(thiophen-2-yl)-9H-purin-9-yl)tetrahydrofuran-3-yl acetate (120q)

A target compound (120q) (125 mg, 79%) is obtained by the same synthetic method as for Compound (120k): ¹H NMR (400 MHz, CDCl₃) δ 7.50 (s, 1H), 6.97 (d, J = 2.2 Hz, 1H), 6.68 -6.85 (m, 1H), 6.23 (d, J = 4.4 Hz, 1H), 5.68 (br s, 2H), 4.51 - 4.53 (m, 1H), 4.17 - 4.27 (m, 2H), 2.45 (t, J = 7.5 Hz, 2H), 2.22 (s, 3H), 2.10 - 2.21 (m, 1H), 1.62 - 1.68 (m, 2H), 1.38 - 1.49 (m, 2H), 1.20 - 1.35 (m, 8H), 0.86 (t, J= 5.8 Hz, 3H).

### (2R,3R)-2-(6-chloro-8-(5-methylfuran-2-yl)-2-(prop-1-yn-1-yl)-9H-purin-9-yl)tetrahydrofuran-3-yl acetate (120r)

After Starting Material (119c) (150 mg, 0.31 mmol) is dissolved in DMF under nitrogen, CuI (12 mg), Pd(PPh₃)₂Cl₂ (22 mg), DIPA (0.14 mL), and 1-propyne (0.03 mL) are slowly added thereto, and the resulting mixture is stirred at room temperature for 1 hour. The reaction mixture is extracted with EtOAc/H₂O, then dried over MgSO₄, and concentrated under reduced pressure. The resulting residue is purified by column chromatography under the condition of hexane/EtOAc = 5/1 to obtain a target compound (120r) (63 mg, 47%) as a yellow solid: ¹H NMR (400 MHz, DMSO-d₆) δ 7.35 (d, J = 3.8 Hz, 1H), 6.48 (d, J = 3.0 Hz, 1H), 6.40 (d, J = 1.7 Hz, 1H), 5.97 - 6.03 (m, 1H), 4.27 - 4.39 (m, 1H), 4.11 - 4.23 (m, 1H), 2.77 - 2.89 (m, 1H), 2.42 (s, 3H), 2.16 - 2.23 (m, 1H), 2.13 (s, 3H), 2.04 (s, 3H).

### (2R,3R)-2-(2-(but-1-yn-1-yl)-6-chloro-8-(5-methylfuran-2-yl)-9H-purin-9-yl)tetrahydrofuran-3-yl acetate (120s)

A target compound (120s) (69 mg, 71%) is obtained by the same synthetic method as for Compound (120r): ¹H NMR (400 MHz, CDCl₃) δ ¹H NMR (400 MHz, CDCl₃) δ 7.29 (d, J = 3.5 Hz, 1H), 6.41 (d, J = 1.6 Hz, 1H), 6.25 - 6.29 (m, 1H), 6.15 - 6.21 (m, 1H), 4.40 - 4.51 (m, 1H), 4.20 - 4.31 (m, 1H), 3.08 - 3.20 (m, 1H), 2.50 (q, J = 7.5 Hz, 2H), 2.45 (s, 3H), 2.18 - 2.30 (m, 1H), 2.10 (s, 3H), 0.92 (t, J = 7.2 Hz, 3H).

### (2R,3R)-2-(6-chloro-8-(5-methylfuran-2-yl)-2-(pent-1-yn-1-yl)-9H-purin-9-yl)tetrahydrofuran-3-yl acetate (120t)

A target compound (120t) (67 mg, 66%) is obtained by the same synthetic method as for Compound (120r): ¹H NMR (400 MHz, CDCl₃) δ ¹H NMR (400 MHz, CDCl₃) δ 7.29 (d, J = 3.5 Hz, 1H), 6.41 (d, J = 1.6 Hz, 1H), 6.25 - 6.29 (m, 1H), 6.15 - 6.21 (m, 1H), 4.40 - 4.51 (m, 1H), 4.20 - 4.31 (m, 1H), 3.08 - 3.20 (m, 1H), 2.45 (t, J = 7.2 Hz, 2H), 2.45 (s, 3H), 2.18 - 2.30 (m, 1H), 2.10 (s, 3H), 1.62 - 1.76 (m, 2H), 0.92 (t, J = 7.2 Hz, 3H).

### (2R,3R)-2-(6-chloro-2-(hept-1-yn-1-yl)-8-(5-methylfuran-2-yl)-9H-purin-9-yl)tetrahydrofuran-3-yl acetate (120u)

A target compound (120u) (68 mg, 59%) is obtained by the same synthetic method as for Compound (120r): ¹H NMR (400 MHz, CDCl₃) δ 7.29 (d, J = 3.4 Hz, 1H), 6.41 (d, J = 1.5 Hz, 1H), 6.25 - 6.29 (m, 1H), 6.15 - 6.21 (m, 1H), 4.40 - 4.51 (m, 1H), 4.20 - 4.31 (m, 1H), 3.08 - 3.20 (m, 1H), 2.42 - 2.49 (m, 5H), 2.18 - 2.30 (m, 1H), 2.10 (s, 3H), 1.62 - 1.72 (m, 2H), 1.32 - 1.51 (m, 4H), 0.92 (t, J = 7.2 Hz, 3H).

### (2R,3R)-2-(6-chloro-8-(5-methylfuran-2-yl)-2-(oct-1-yn-1-yl)-9H-purin-9-yl)tetrahydrofuran-3-yl acetate (120v)

A target compound (120v) (82 mg, 66%) is obtained by the same synthetic method as for Compound (120r): ¹H NMR (400 MHz, CDCl₃) δ 7.29 (d, J = 3.4 Hz, 1H), 6.41 (d, J = 1.5 Hz, 1H), 6.25 - 6.29 (m, 1H), 6.15 - 6.21 (m, 1H), 4.40 - 4.48 (m, 1H), 4.21 - 4.31 (m, 1H), 3.10 - 3.22 (m, 1H), 2.47 (s, 3H), 2.19 - 2.31 (m, 1H), 2.09 (s, 3H), 1.61 - 1.70 (m, 2H), 1.43 - 1.50 (m, 2H), 1.27 - 1.37 (m, 4H), 0.90 (t, J = 6.7 Hz, 3H).

### (2R,3R)-2-2-(6-chloro-8-(5-methylfuran-2-yl)-2-(non-1-yn-1-yl)-9H-purin-9-yl)tetrahydrofuran-3-yl acetate (120w)

A target compound (120w) (81 mg, 67%) is obtained by the same synthetic method as for Compound (120r): ¹H NMR (400 MHz, CDCl₃) δ 7.29 (d, J = 3.2 Hz, 1H), 6.40 (d, J = 1.5 Hz, 1H), 6.25 - 6.29 (m, 1H), 6.15 - 6.19 (m, 1H), 4.37 - 4.52 (m, 1H), 4.20 - 4.31 (m, 1H), 3.02 - 3.14 (m, 1H), 2.45 (s, 3H), 2.17 - 2.28 (m, 1H), 2.08 (s, 3H), 1.59 - 1.67 (m, 2H), 1.40 - 1.49 (m, 2H), 1.17 - 1.32 (m, 8H), 0.88 (t, J = 6.1 Hz, 3H).

### (2R,3R)-2-(6-chloro-2-(dec-1-yn-1-yl)-8-(5-methylfuran-2-yl)-9H-purin-9-yl)tetrahydrofuran-3-yl acetate (120x)

A target compound (120x) (75 mg, 70%) is obtained by the same synthetic method as for Compound (120r): ¹H NMR (400 MHz, CDCl₃) δ 7.29 (d, J = 3.2 Hz, 1H), 6.40 (d, J = 1.5 Hz, 1H), 6.25 - 6.29 (m, 1H), 6.15 - 6.19 (m, 1H), 4.37 - 4.52 (m, 1H), 4.20 - 4.31 (m, 1H), 3.02 - 3.14 (m, 1H), 2.40-2.51 (m, 5H), 2.17 - 2.28 (m, 1H), 2.08 (s, 3H), 1.59 - 1.67 (m, 2H), 1.40 - 1.49 (m, 2H), 1.17 - 1.32 (m, 8H), 0.88 (t, J = 6.3 Hz, 3H).

### (2R,3R)-2-(6-chloro-8-(furan-2-yl)-2-(prop-1-yn-1-yl)-9H-purin-9-yl)tetrahydrofuran-3-ol (121a)

NH₃ in isopropyl alcohol (excess) is added to Starting Material (120a) (85 mg, 0.2 mmol), and the resulting mixture is stirred at 140°C for 12 hours. After it is confirmed that the reaction is completed, the reaction mixture is concentrated under reduced pressure. The resulting residue is purified by column chromatography under the condition of DCM/MeOH = 20/1 to obtain a target compound (121a) (10.5 mg, 41%) as a pale yellow solid: ¹H NMR (400 MHz, DMSO-d₆) δ 7.99 (s, 1H), 7.46 (s, 2H), 7.08 (d, J = 3.1 Hz, 1H), 6.72 - 6.78 (m, 1H), 5.98 (d, J = 3.4 Hz, 1H), 5.51 (d, J = 4.6 Hz, 1H), 4.38 - 4.50 (m, 1H), 4.19 - 4.29 (m, 1H), 3.96 - 4.09 (m, 1H), 2.57-2.67 (m, 1H), 2.02 (s, 3H), 1.87 - 1.99 (m, 1H).

### (2R, 3R)-2-(2-(but-1-yn-1-yl)-6-chloro-8-(furan-2-yl)-9H-purin-9-yl)tetrahydrofuran-3-ol (121b)

A target compound (121b) (37 mg, 55%) is obtained by the same synthetic method as for Compound (121a): ¹H NMR (400 MHz, DMSO-d₆) δ 7.99 (s, 1H), 7.47 (s, 2H), 7.09 (d, J = 3.2 Hz, 1H), 6.73 - 6.78 (m, 1H), 5.98 (d, J = 3.3 Hz, 1H), 5.52 (d, J = 4.8 Hz, 1H), 5.07 - 5.12 (m, 1H), 4.15 - 4.29 (m, 1H), 3.98 - 4.15 (m, 1H), 2.58 - 2.68 (m, 1H), 2.33 - 2.44 (m, 2H), 1.88 - 1.99 (m, 1H), 0.84 - 0.94 (m, 2H), 1.15 (t, J = 7.5 Hz, 3H).

### (2R, 3R)-2-(6-chloro-8-(furan-2-yl)-2-(pent-1-yn-1-yl)-9H-purin-9-yl)tetrahydrofuran-3-ol (121c)

A target compound (121c) (15 mg, 21%) as a white solid is obtained by the same synthetic method as for Compound (121a): ¹H NMR (400 MHz, CDCl₃) δ 7.64(s, 1H) 7.14, (d, J = 3.5 Hz, 1H), 6.59 (dd, J = 3.4, 1.8 Hz, 1H), 5.56 - 5.63 (m, 3H), 4.43 - 4.36 (m, 1H), 4.20-4.28 (m, 1H), 2.82-2.97 (m, 2H), 2.43 (t, J = 7.2 Hz, 2H), 1.63 - 1.74 (m, 2H), 1.07 (t, J = 7.4 Hz, 3H).

### (2R, 3R)-2-(6-chloro-8-(furan-2-yl)-2-(hept-1-yn-1-yl)-9H-purin-9-yl)tetrahydrofuran-3-ol (121d)

A target compound (121d) (31 mg, 50%) as a pale yellow solid is obtained by the same synthetic method as for Compound (121a): ¹H NMR (400 MHz, CDCl₃) δ 7.63 (s, 1H), 7.13 (d, J = 3.5 Hz, 1H), 6.58 (dd, J = 3.4, 1.7 Hz, 1H), 6.15 (d, J = 3.3 Hz, 1H), 5.55 - 5.64 (m, 3H), 4.36-4.44 (m, 1H), 4.20 - 4.30 (m, 1H), 2.99 (br s, 1H), 2.85 - 2.95 (m, 1H), 2.44 (t, J = 7.3 Hz, 2H), 2.12 - 2.21 (m, 1H), 1.63 - 1.70 (m, 2H), 1.41 - 1.50 (m, 2H), 1.31 - 1.40 (m, 2H), 0.92 (t, J = 7.2 Hz, 3H).

### (2R, 3R)-2-(6-chloro-8-(furan-2-yl)-2-(oct-1-yn-1-yl)-9H-purin-9-yl)tetrahydrofuran-3-ol (121e)

A target compound (121e) (37 mg, 63%) as a pale yellow solid is obtained by the same synthetic method as for Compound (121a): ¹H NMR (400 MHz, CDCl₃) δ 7.63 (s, 1H), 7.13 (d, J = 3.5 Hz, 1H), 6.58 (dd, J = 3.4, 1.7 Hz, 1H), 6.15 (d, J = 3.3 Hz, 1H), 5.55 - 5.65 (m, 3H), 4.36-4.44 (m, 1H), 4.20 - 4.30 (m, 1H), 3.02 (br s, 1H), 2.85 - 2.96 (m, 1H), 2.44 (t, J = 7.2 Hz, 2H), 2.10 - 2.21 (m, 1H), 1.63 - 1.70 (m, 2H), 1.41 - 1.52 (m, 2H), 1.26 - 1.38 (m, 4H), 0.90 (t, J = 6.7 Hz, 3H).

### (2R, 3R)-2-(6-chloro-8-(furan-2-yl)-2-(non-1-yn-1-yl)-9H-purin-9-yl)tetrahydrofuran-3-ol (121f)

A target compound (121f) (10 mg, 16%) as a pale yellow solid is obtained by the same synthetic method as for Compound (121a): ¹H NMR (400 MHz, CDCl₃) δ 7.63 (s, 1H), 7.13 (d, J = 3.4 Hz, 1H), 6.58 (dd, J = 3.4, 1.8 Hz, 1H), 6.15 (d, J = 3.3 Hz, 1H), 5.55 - 5.65 (m, 3H), 4.36-4.44 (m, 1H), 4.20 - 4.30 (m, 1H), 3.04 (br s, 1H), 2.85 - 2.96 (m, 1H), 2.44 (t, J = 7.2 Hz, 2H), 2.10 - 2.22 (m, 1H), 1.61 - 1.71 (m, 2H), 1.39 - 1.51 (m, 2H), 1.24 - 1.38 (m, 6H), 0.89 (t, J = 6.3 Hz, 3H).

### (2R, 3R)-2-(6-chloro-2-(dec-1-yn-1-yl)-8-(furan-2-yl)-9H-purin-9-yl)tetrahydrofuran-3-ol (121g)

A target compound (121g) (68 mg, 55%) as a pale yellow solid is obtained by the same synthetic method as for Compound (121a): ¹H NMR (400 MHz, CDCl₃) δ 7.58 (s, 1H), 7.07 (d, J = 3.3 Hz, 1H), 7.02 -7.11 (m, 1H), 6.13 (d, J = 3.6 Hz, 1H), 5.66 (br s, 2H), 5.63 (br s, 1H), 4.33 - 4.56 (m, 1H), 4.17 - 4.27 (m, 1H), 3.75 (s, 1H), 4.12 - 4.27 (m, 1H), 2.43 (t, J = 7.3 Hz, 2H), 2.11 - 2.24 (m, 1H), 1.59 - 1.70 (m, 2H), 1.39 - 1.50 (m, 2H), 1.21 - 1.39 (m, 8H), 0.88 (t, J = 6.7 Hz, 3H).

### (2R, 3R)-2-(6-chloro-8-(furan-2-yl)-2-(phenylethynyl)-9H-purin-9-yl)tetrahydrofuran-3-ol (121h)

A target compound (121h) (12 mg, 40%) as a pale yellow solid is obtained by the same synthetic method as for Compound (121a): ¹H NMR (400 MHz, CD₆OD) δ 7.86 (s, 1H), 7.59 - 7.64 (m, 2H), 7.40 - 7.45 (m, 2H), 7.24 (d, J = 3.2 Hz, 1H), 6.71 - 6.76 (m, 1H), 6.23 (d, J = 3.2 Hz, 1H), 5.35 (br s, 1H), 4.40 - 4.52 (m, 1H), 4.16 - 4.27 (m, 1H), 2.79 - 2.92 (m, 2H), 2.06 - 2.17 (m, 1H).

### (2R, 3R)-2-(6-chloro-8-(furan-2-yl)-2-(p-tolylethynyl)-9H-purin-9-yl)tetrahydrofuran-3-ol (121i)

A target compound (121i) (29 mg, 74%) as a pale yellow solid is obtained by the same synthetic method as for Compound (121a): ¹H NMR (400 MHz, CDCl₃) δ 7.65 (s, 1H) 7.54 (d, J = 8.0 Hz, 2H), 7.14 - 7.20 (m, 3H), 6.57 - 6.62 (m, 1H), 6.18 (d, J = 3.2 Hz, 1H), 5.59 - 5.69 (m, 3H), 4.38 - 4.50 (m, 1H), 4.21 - 4.31 (m, 1H), 2.92 - 3.02 (m, 1H), 2.85 (d, J = 4.7 Hz, 1H), 2.37 (s, 3H), 2.14 - 2.24 (m, 1H).

### (2R, 3R)-2-(6-chloro-2-(cyclopropylethynyl)-8-(furan-2-yl)-9H-purin-9-yl)tetrahydrofuran-3-ol (121j)

A target compound (121j) (34 mg, 61%) as a white solid is obtained by the same synthetic method as for Compound (121a): ¹H NMR (400 MHz, DMSO-d₆) δ 7.99 (s, 1H), 7.47 (s, 2H), 7.08 (d, J = 2.9 Hz, 1H), 6.70 - 6.77 (m, 1H), 5.98 (d, J = 3.4 Hz, 1H), 5.52 (d, J = 4.6 Hz, 1H), 5.01 - 5.16 (m. 1H), 4.17 - 4.30 (m, 1H), 3.97 - 4.10 (m, 1H), 2.55 - 2.65 (m, 1H), 1.89 - 2.00 (m, 1H), 1.48 - 1.58 (m, 1H), 0.84 - 0.94 (m, 2H), 0.71 - 0.79 (m, 2H).

### (2R, 3R)-2-(6-chloro-2-(prop-1-yn-1-yl)-8-(thiophen-2-yl)-9H-purin-9-yl)tetrahydrofuran-3-ol (121k)

NH₃ in isopropyl alcohol (excess) is added to Starting Material (120b) (20 mg, 0.05 mmol) under nitrogen, and the resulting mixture is stirred at 140°C for 12 hours. After it is confirmed that the reaction is completed, the reaction mixture is concentrated under reduced pressure and purified by column chromatography under the condition of DCM/MeOH = 20/1 to obtain a target compound (121k) (3.6 mg, 21%) as a white solid: ¹H NMR (400 MHz, DMSO-d₆) δ 7.91 (s, 1H), 7.57 (s, 2H), 7.05 (d, J = 3.3 Hz, 1H), 6.73 - 6.90 (m, 1H), 6.27 (d, J = 3.8 Hz, 1H), 5.75 (d, J = 4.4 Hz, 1H), 4.30 - 4.38 (m, 1H), 4.17 - 4.25 (m, 1H), 4.04 - 4.11 (m, 1H), 2.45-2.58 (m, 1H), 2.05 (s, 3H), 1.88 - 1.98 (m, 1H).

### (2R, 3R)-2-(2-(but-1-yn-1-yl)-6-chloro-8-(thiophen-2-yl)-9H-purin-9-yl)tetrahydrofuran-3-ol (1211)

A target compound (121l) (31 mg, 47%) as a pale yellow solid is obtained by the same synthetic method as for Compound (121k): ¹H NMR (400 MHz, CDCl₃) δ 7.84(s, 1H), 7.42 (d, J = 3.5 Hz, 1H), 6.75 (dd, J = 3.5 Hz, 1H), 5.50 - 5.61 (m, 2H), 4.15 - 4.22 (m, 1H), 2.99-3.12 (m, 2H), 2.81 (t, J = 7.4 Hz, 2H), 1.45 - 1.64 (m, 2H), 0.85 (t, J = 7.4 Hz, 3H).

### (2R, 3R)-2-(6-chloro-2-(pent-1-yn-1-yl)-8-(thiophen-2-yl)-9H-purin-9-yl)tetrahydrofuran-3-ol (121m)

A target compound (121m) (35 mg, 49%) as a pale yellow solid is obtained by the same synthetic method as for Compound (121k): ¹H NMR (400 MHz, DMSO-d₆) δ 7.72 (s, 1H), 7.44 (s, 2H), 7.18 (d, J = 4.4 Hz, 1H), 6.40 - 6.81 (m, 1H), 6.02 (d, J = 3.4 Hz, 1H), 5.58 (d, J = 4.4 Hz, 1H), 5.08 - 5.20 (m, 1H), 4.18 - 4.25 (m, 1H), 3.97 - 4.11 (m, 1H), 2.44 - 2.51 (m, 1H), 2.31 - 2.38 (m, 2H), 1.75 - 1.92 (m, 1H), 0.99 (t, J = 7.5 Hz, 3H), 0.79 - 0.92 (m, 2H).

### (2R, 3R)-2-(6-chloro-2-(hept-1-yn-1-yl)-8-(thiophen-2-yl)-9H-purin-9-yl)tetrahydrofuran-3-ol (121n)

A target compound (121n) (26 mg, 43%) as a pale yellow solid is obtained by the same synthetic method as for Compound (121k): ¹H NMR (400 MHz, CDCl₃) δ 7.63 (s, 1H), 7.11 (d, J = 3.5 Hz, 1H), 6.75 (dd, J = 6.4 Hz, 1H), 6.05 (d, J = 3.7 Hz, 1H), 5.41 - 5.55 (m, 3H), 4.25-4.35 (m, 1H), 4.10 - 4.22 (m, 2H), 2.75 - 2.85 (m, 1H), 2.41 (t, J = 7.7 Hz, 2H), 2.11 - 2.20 (m, 1H), 1.52 - 1.75 (m, 2H), 1.42 - 1.48 (m, 2H), 1.28 - 1.35 (m, 2H), 0.85 (t, J = 7.7 Hz, 3H).

### (2R, 3R)-2-(6-chloro-2-(oct-1-yn-1-yl)-8-(thiophen-2-yl)-9H-purin-9-yl)tetrahydrofuran-3-ol (121o)

A target compound (121o) (23 mg, 45%) as a pale yellow solid is obtained by the same synthetic method as for Compound (121k): ¹H NMR (400 MHz, CDCl₃) δ 7.54 (s, 1H), 7.18 (d, J = 4.4 Hz, 1H), 6.72 (dd, J = 3.4 1H), 6.21 (d, J = 3.4 Hz, 1H), 5.41 - 5.50 (m, 3H), 4.37-4.51 (m, 1H), 4.20 - 4.31 (m, 1H), 2.87 - 2.93 (m, 1H), 2.52 (t, J = 7.4 Hz, 2H), 2.25 (s, 3H), 2.07 - 2.25 (m, 1H), 1.50 - 1.62 (m, 2H), 1.34 - 1.45 (m, 2H), 1.21 - 1.29 (m, 4H), 0.75 (t, J = 7.7 Hz, 3H).

### (2R, 3R)-2-(6-chloro-2-(non-1-yn-1-yl)-8-(thiophen-2-yl)-9H-purin-9-yl)tetrahydrofuran-3-ol (121p)

A target compound (121p) (12 mg, 41%) as a pale yellow solid is obtained by the same synthetic method as for Compound (121k): ¹H NMR (400 MHz, CDCl₃) δ 7.51 (s, 2H), 7.20 (d, J = 3.3 Hz, 1H), 6.55 (dd, J = 3.4 Hz, 1H), 6.31 (d, J = 3.5 Hz, 1H), 5.48 - 5.54 (m, 1H), 4.38-4.49 (m, 1H), 4.22 - 4.31 (m, 1H), 2.84 - 2.95 (m, 1H), 2.55 (t, J = 7.5 Hz, 2H), 2.10 - 2.21 (m, 1H), 1.59 - 1.68 (m, 2H), 1.42 - 1.55 (m, 2H), 1.22 - 1.35 (m, 6H), 0.92 (t, J = 3.5 Hz, 3H).

### (2R, 3R)-2-(6-chloro-2-(dec-1-yn-1-yl)-8-(thiophen-2-yl)-9H-purin-9-yl)tetrahydrofuran-3-ol (121q)

A target compound (121q) (31 mg, 51%) as a pale yellow solid is obtained by the same synthetic method as for Compound (121k): ¹H NMR (400 MHz, CDCl₃) δ 7.49 (s, 1H), 6.95 (d, J = 3.2 Hz, 1H), 6.65 -6.75 (m, 1H), 6.25 (d, J = 4.4 Hz, 1H), 5.68 (br s, 2H), 4.50 - 4.53 (m, 1H), 4.19 - 4.27 (m, 1H), 4.11 - 4.17 (m, 1H), 2.45 (t, J = 7.4 Hz, 2H), 2.10 - 2.21 (m, 1H), 1.63 - 1.71 (m, 2H), 1.38 - 1.49 (m, 2H), 1.20 - 1.31 (m, 8H), 0.84 (t, J = 4.4 Hz, 3H).

### (2R, 3R)-2-(6-chloro-8-(5-methylfuran-2-yl)-2-(prop-1-yn-1-yl)-9H-purin-9-yl)tetrahydrofuran-3-ol (121r)

NH₃ in isopropyl alcohol (excess) is added to Starting Material (120c) (50 mg, 0.12 mmol) under nitrogen, and the resulting mixture is stirred at 140°C for 12 hours. After it is confirmed that the reaction is completed, the reaction mixture is concentrated under reduced pressure and purified by column chromatography under the condition of DCM/MeOH = 20/1 to obtain a target compound (121r) (27 mg, 32%) as a pale yellow solid: ¹H NMR (400 MHz, DMSO-d₆) δ 7.44 (br s, 2H), 6.96 (d, J = 3.2 Hz, 1H), 6.37 (d, J = 2.4 Hz, 1H) 5.94 (d, J = 3.3 Hz, 1H), 5.51 (d, J = 4.7 Hz, 1H), 5.09 - 5.16 (m, 1H), 4.19 - 4.28 (m, 1H), 4.00 - 4.09 (m, 1H), 2.55 - 2.67 (m, 1H), 2.38 (s, 3H), 2.01 (s, 3H), 1.88 - 1.99 (m, 1H).

### (2R, 3R)-2-(2-(but-1-yn-1-yl)-6-chloro-8-(5-methylfuran-2-yl)-9H-purin-9-yl)tetrahydrofuran-3-ol (121s)

A target compound (121s) (9 mg, 47%) as a white solid is obtained by the same synthetic method as for Compound (121r): ¹H NMR (400 MHz, DMSO-d₆) δ 7.43 (br s, 2H), 6.96 (d, J = 3.1 Hz, 1H), 6.38 (d, J = 2.4 Hz, 1H) 5.94 (d, J = 3.3 Hz, 1H), 5.51 (d, J = 4.6 Hz, 1H), 5.09 - 5.16 (m, 1H), 4.19 - 4.28 (m, 1H), 4.00 - 4.09 (m, 1H), 2.55 - 2.67 (m, 1H), 2.36 - 2.48 (m, 5H), 1.88 - 1.99 (m, 1H), 0.92 (t, J = 7.2 Hz, 3H).

### (2R, 3R)-2-(6-chloro-8-(5-methylfuran-2-yl)-2-(pent-1-yn-1-yl)-9H-purin-9-yl)tetrahydrofuran-3-ol (121t)

A target compound (121t) (23 mg, 65%) as a white solid is obtained by the same synthetic method as for Compound (121r): ¹H NMR (400 MHz, DMSO-d₆) δ 7.43 (br s, 2H), 6.98 (d, J = 3.1 Hz, 1H), 6.35 (d, J = 2.4 Hz, 1H) 5.94 (d, J = 3.3 Hz, 1H), 5.51 (d, J = 4.6 Hz, 1H), 5.09 - 5.16 (m, 1H), 4.19 - 4.28 (m, 1H), 4.00 - 4.09 (m, 1H), 2.55 - 2.67 (m, 1H), 2.36 - 2.48 (m, 5H), 1.88 - 1.99 (m, 1H), 1.61 - 1.72 (m, 2H), 0.92 (t, J = 7.1 Hz, 3H).

### (2R, 3R)-2-(6-chloro-2-(hept-1-yn-1-yl)-8-(5-methylfuran-2-yl)-9H-purin-9-yl)tetrahydrofuran-3-ol (121u)

A target compound (121u) (6.9 mg, 19%) as a white solid is obtained by the same synthetic method as for Compound (121r): ¹H NMR (400 MHz, CDCl₃) δ 7.01 (d, J = 3.1 Hz, 1H), 6.17 (d, J = 2.4 Hz, 1H), 6.10 - 6.13 (m, 1H), 5.51 - 5.65 (m, 3H), 4.35 - 4.44 (m, 1H), 4.20 - 4.29 (m, 1H), 3.13 (s, 1H), 2.84 - 2.95 (m, 1H), 2.40 - 2.46 (m, 5H), 2.11 - 2.20 (m, 1H), 1.62 - 1.71 (m, 2H), 1.32 - 1.49 (m, 4H), 0.92 (t, J = 7.2 Hz, 3H).

### (2R, 3R)-2-(6-chloro-8-(5-methylfuran-2-yl)-2-(oct-1-yn-1-yl)-9H-purin-9-yl)tetrahydrofuran-3-ol (121v)

A target compound (121v) (7.8 mg, 45%) as a white solid is obtained by the same synthetic method as for Compound (121r): ¹H NMR (400 MHz, CDCl₃) δ 6.95 (d, J = 3.3 Hz, 1H), 6.09 - 6.15 (m, 2H), 5.66 (s, 2H), 5.60 (s, 1H), 4.35 - 4.45 (m, 1H), 4.19 - 4.29 (m, 1H), 3.84 (s, 1H), 2.79 - 2.92 (m, 1H), 2.37 - 2.47 (m, 5H), 2.11 - 2.23 (m, 1H), 1.59 - 1.70 (m, 2H), 1.40 - 1.51 (m, 2H), 1.26 - 1.37 (m, 4H), 0.90 (t, J = 6.9 Hz, 3H).

### (2R, 3R)-2-(6-chloro-8-(5-methylfuran-2-yl)-2-(non-1-yn-1-yl)-9H-purin-9-yl)tetrahydrofuran-3-ol (121w)

A target compound (121w) (7.7 mg, 19%) as a white solid is obtained by the same synthetic method as for Compound (121r): ¹H NMR (400 MHz, CDCl₃) δ 6.94 (d, J = 3.3 Hz, 1H), 6.08 - 6.13 (m, 2H), 6.10 - 6.13 (m, 1H), 4.01 (s, 1H), 2.77 - 2.91 (m, 1H), 2.36 - 2.46 (m, 5H), 2.11 - 2.23 (m, 1H), 1.59 - 1.69 (m, 2H), 1.38 - 1.50 (m, 2H), 1.24 - 1.36 (m, 6H), 0.89 (t, J = 7.2 Hz, 3H).

### (2R, 3R)-2-(6-chloro-2-(dec-1-yn-1-yl)-8-(5-methylfuran-2-yl)-9H-purin-9-yl)tetrahydrofuran-3-ol (121x)

A target compound (121x) (5 mg, 35%) as a white solid is obtained by the same synthetic method as for Compound (121r): ¹H NMR (400 MHz, CDCl₃) δ 6.93 (d, J = 3.3 Hz, 1H), 6.08 - 6.13 (m, 2H), 6.10 - 6.13 (m, 1H), 4.01 (s, 1H), 2.77 - 2.91 (m, 1H), 2.36 - 2.45 (m, 5H), 2.10 - 2.23 (m, 1H), 1.59 - 1.69 (m, 2H), 1.38 - 1.50 (m, 2H), 1.22 - 1.38 (m, 8H), 0.89 (t, J = 7.1 Hz, 3H).

### Experimental Examples: Evaluation of binding affinity for adenosine receptor (2)

In order to evaluate the affinity of the adenosine derivatives of the present invention prepared in the Preparation Examples for A₁, A_{2A}, A_{2B} and A₃ receptors in a human adenosine receptor (hAR), experiments were performed in the same manner as in Experimental Examples 1 to 4, and the experimental results are shown as in the following Table 2.

**[Table 2]**

| Compound | Binding affinity (% inhibition) | | | |
|---|---|---|---|---|
| | hA₁ | hA_{2A} | hA_{2B} | hA₃ |
| 116a | + | +++ | - | ++ |
| 116b | - | ++ | + | + |
| 116c | + | +++ | - | + |
| 116d | + | +++ | - | ++ |
| 116e | + | +++ | - | + |
| 116f | - | ++ | + | - |
| 121a | + | +++ | - | + |
| 121b | + | +++ | + | + |
| 121c | + | +++ | + | - |
| 121d | + | +++ | + | - |
| 121e | - | +++ | - | + |
| 121f | + | +++ | ++ | + |
| 121g | - | +++ | - | - |
| 121h | ++ | +++ | ++ | ++ |
| 121i | + | +++ | + | ++ |
| 121j | - | +++ | - | + |
| 121k | - | +++ | + | - |
| 121l | + | +++ | + | - |
| 121m | - | +++ | + | ++ |
| 121n | + | +++ | + | + |
| 121o | + | +++ | - | + |
| 121p | - | +++ | + | ++ |
| 121q | + | +++ | - | + |
| 121r | + | +++ | ++ | + |
| 121s | - | +++ | + | + |
| 121t | + | +++ | + | ++ |
| 121u | + | +++ | + | + |
| 121v | - | +++ | ++ | + |
| 121w | + | +++ | + | ++ |
| 121x | + | +++ | ++ | ++ |

As shown in Table 2 above, the adenosine derivative compounds of the present invention exhibited high binding affinity for human adenosine A_{2A} and A₃ receptors and exhibited generally low affinity, that is, high selectivity for adenosine A₁ and A_{2B} receptors.

Therefore, it can be expected that the adenosine derivative compounds of the present invention regulate the activity of an A_{2A} or A₃ adenosine receptor, and thus can effectively prevent or treat brain diseases, cardiovascular diseases, sleep disorders, inflammation, immune system diseases and the like in which the receptors are involved.

As described above, although the present invention is mainly described with reference to the embodiments of the present invention, this is merely an example and does not limit the present invention, and it will be appreciated that a person with ordinary skill in the art to which the present invention pertains can make various modifications and applications which are not exemplified above within a range not departing from the essential characteristics of the embodiments of the present invention. For example, each constituent element specifically shown in the embodiments of the present invention can be modified and implemented. And differences related to these modifications and applications should be construed as being included in the scope of the present invention defined in the appended claims.

## Claims

1. An adenosine derivative represented by the following Chemical Formula 3 or a pharmaceutically acceptable salt thereof. 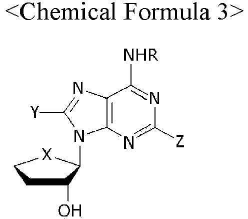 (in Chemical Formula 3,
X is oxygen (O) or sulfur (S),
Y and Z are each independently a substituted or unsubstituted C₂ to C₁₀ heteroaryl; a substituted or unsubstituted C₂ to C₁₀ heterocycloalkyl; a substituted or unsubstituted C₆ to C₂₀ aryl or alkylaryl; or a substituted or unsubstituted C₂ to C₁₀ alkynyl group,
R is hydrogen (H); a substituted or unsubstituted C₁ to C₁₀ alkyl; or a substituted or unsubstituted C₆ to C₂₀ aryl or alkylaryl, and
when Y or Z is substituted, Y or Z is optionally substituted with one or more selected from the group consisting of an alkyl, an aryl, carboxylic acid, an alkoxy, an alkylamide, a heteroaryl, a nitrile, an amide, a halogen, a cycloalkyl and a carboxylate)

2. The adenosine derivative or the pharmaceutically acceptable salt thereof of claim 1, wherein the heteroaryl is furanyl, thiophenyl, pyrrolyl, pyranyl, pyrazolyl, pyridinyl, triazolyl, imidazolyl, thiazolyl, pyrimidinyl, pyrazinyl, pyridazinyl, indolyl, quinolinyl or purinyl,
the heterocycloalkyl is tetrahydrofuranyl, thiolanyl, pyrrolidinyl, tetrahydropyranyl, piperidinyl, dioxanyl, morpholino or tetrahydropyrimidinyl,
the aryl is phenyl, naphthalenyl, anthracenyl or phenanthrenyl, and the alkylaryl is benzyl,
the alkynyl is ethynyl, propynyl, butynyl, pentynyl, hexynyl, heptynyl, octynyl, nonynyl or decynyl, and
the alkyl is methyl, ethyl, propyl, butyl or pentyl.

3. The adenosine derivative or the pharmaceutically acceptable salt thereof of claim 1, wherein the adenosine derivative or the pharmaceutically acceptable salt thereof is an adenosine derivative represented by the following Chemical Formula 3-1 or a pharmaceutically acceptable salt thereof. 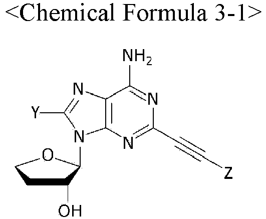 (in Chemical Formula 3-1,
Y is benzyl, furanyl, methylfuranyl, dimethylfuranyl, thiophenyl, thiazolyl, pyridinyl, methoxypyridinyl, pyrrolyl, methylpyrrolyl, pyrimidinyl, piperidinyl, ethylpiperidinyl, piperidinyl propanoate, piperidinylpropanamide, piperidinyl acetate, thiazolylpiperidinyl, piperazinyl, (methoxyethoxy)phenylpiperazinyl, piperazinylbenzonitrile, piperazinylfluorobenzonitrile, piperazinylbenzamide, piperazinylbenzoic acid, piperazinylfluorobenzoic acid, morpholino, ethylmorpholino, imidazolyl, methylimidazolyl, thiazolyl or benzo[d]thiazolyl, and
Z is methyl, ethyl, propyl, butyl, pentyl, hexyl, heptyl, octyl, phenyl, tolyl, cyclopropyl, propylamide, butyric acid or phenyl)

4. The adenosine derivative or the pharmaceutically acceptable salt thereof of claim 3, wherein Y is furanyl, methylfuranyl, thiophenyl, methylpyrrolyl, methoxypyridinyl, pyrimidinyl or thiazolyl, and
Z is methyl, ethyl, propyl, pentyl, hexyl, heptyl, octyl, phenyl, tolyl or cyclopropyl.

5. A pharmaceutical composition for preventing or treating A_{2A} or A₃ adenosine receptor-related diseases, comprising the adenosine derivative, or the pharmaceutically acceptable salt thereof according to any one of claims 1 to 4.

6. A method for preparing an adenosine derivative, the method comprising the following Steps (a) to (h) according to the following Reaction Scheme 1, or the following Steps (i) to (m) according to the following Reaction Scheme 2. 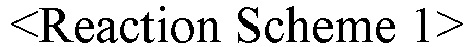 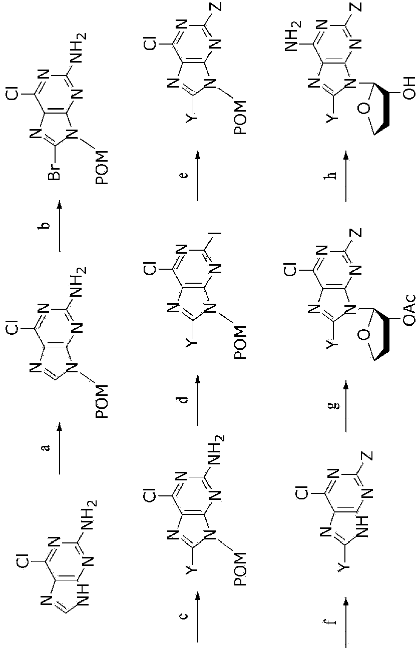
(a) forming a pivaloyloxymethyl (POM) group by being allowed to react with chloromethyl pivalate;
(b) performing bromination by being allowed to react with N-bromosuccinimide (NBS);
(c) substituting a -Br group with a -Y group by being allowed to react with stannyl-Y;
(d) substituting a -NH₂ group with a -I group by being allowed to react with one or more of CuI, I₂ and CH₂I₂;
(e) substituting the -I group with a -Z group by being allowed to react with Z;
(f) removing the POM group by being allowed to react with NaOH;
(g) being allowed to react with tetrahydrofuran-2,3-diyl diacetate; and
(h) being allowed to react with NH₃ 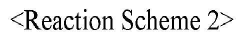 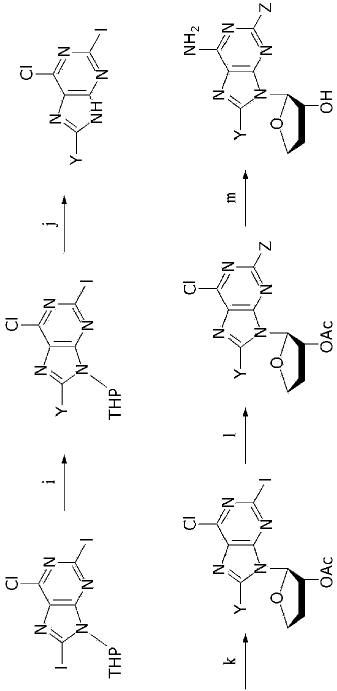
(i) substituting an -I group with a -Y group by being allowed to react with stannyl-Y;
(j) removing a tetrahydropyran (THP) group by being allowed to react with pyridinium p-toluenesulfonate (PPTS);
(k) being allowed to react with tetrahydrofuran-2,3-diyl diacetate;
(l) substituting an -I group with a -Z group by being allowed to react with Z; and
(m) being allowed to react with NH₃
(in Reaction Scheme 1 or 2, Y and Z are each independently a substituted or unsubstituted C₂ to C₁₀ heteroaryl; a substituted or unsubstituted C₂ to C₁₀ heterocycloalkyl; a substituted or unsubstituted C₆ to C₂₀ aryl or alkylaryl; or a substituted or unsubstituted C₂ to C₁₀ alkynyl group)
